(19) Europäisches Patentamt
European Patent Office
Office européen des brevets

(11) EP 4 450 504 A1

(12) EUROPEAN PATENT APPLICATION
published in accordance with Art. 153(4) EPC

(43) Date of publication:
**23.10.2024 Bulletin 2024/43**

(21) Application number: **22906674.1**

(22) Date of filing: **16.12.2022**

(51) International Patent Classification (IPC):
*C07D 471/14* (2006.01) *C07D 519/00* (2006.01)
*A61K 31/519* (2006.01) *A61P 35/00* (2006.01)

(52) Cooperative Patent Classification (CPC):
**A61K 31/519; A61K 31/5377; A61P 35/00; C07D 471/14; C07D 519/00**

(86) International application number:
**PCT/CN2022/139447**

(87) International publication number:
**WO 2023/109929 (22.06.2023 Gazette 2023/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.12.2021 CN 202111561282**
**07.06.2022 CN 202210634934**

(71) Applicant: **CSPC Zhongqi Pharmaceutical Technology (Shijiazhuang) Co., Ltd.**
**Shijiazhuang, Hebei 050035 (CN)**

(72) Inventors:
- **WANG, Zhenyu**
  **Shijiazhuang, Hebei 050035 (CN)**
- **WANG, Kuanglei**
  **Shijiazhuang, Hebei 050035 (CN)**
- **AN, Hui**
  **Shijiazhuang, Hebei 050035 (CN)**
- **HUA, Xinxing**
  **Shijiazhuang, Hebei 050035 (CN)**
- **HU, Jiming**
  **Shijiazhuang, Hebei 050035 (CN)**
- **GAO, Jun**
  **Shijiazhuang, Hebei 050035 (CN)**
- **LI, Zizhen**
  **Shijiazhuang, Hebei 050035 (CN)**
- **FAN, Lixue**
  **Shijiazhuang, Hebei 050035 (CN)**
- **YANG, Yinping**
  **Shijiazhuang, Hebei 050035 (CN)**
- **ZHU, Xingbo**
  **Shijiazhuang, Hebei 050035 (CN)**

(74) Representative: **Grünecker Patent- und Rechtsanwälte PartG mbB**
**Leopoldstraße 4**
**80802 München (DE)**

# (54) HETEROCYCLIC COMPOUND HAVING ANTI-TUMOR ACTIVITY AND USE THEREOF

(57) Provided are a class of compounds, a stereoisomer, an optical isomer, a pharmaceutically acceptable salt, a prodrug, a solvate (for example, a hydrate) or an isotope derivative thereof. The compounds have a tri-heterocyclic structure (for example, the structure represented by formula (A)), which is a novel structure, thereby providing a new direction for the development of SOS1 inhibitor drugs. In-vitro enzyme activity inhibition activity studies show that the compounds have a relatively strong inhibition effect on SOS1 and can be used as a prospective compound for preventing and/or treating SOS1-mediated diseases. Moreover, said compounds also exhibit significant inhibitory activity on NCI-H358 cell proliferation. Furthermore, a specific synthesis method is provided. The synthesis method is simple in process, convenient to operate and beneficial to large-scale industrial production and use.

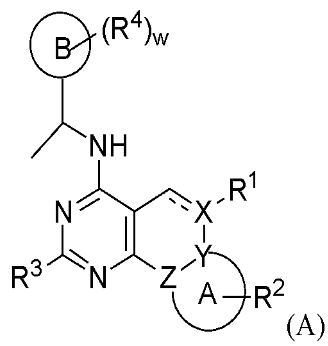

(A)

EP 4 450 504 A1

## Description

### CROSS-REFERENCE TO RELATED APPLICATION

The present disclosure claims priority to and the benefits of Chinese patent application No. 202111561282.9 filed on December 17, 2021 and entitled "HETEROCYCLIC COMPOUND HAVING ANTI-TUMOR ACTIVITY AND USE THEREOF", and priority to and the benefits of Chinese patent application No. 202210634934.5 filed on June 7, 2022 and entitled "HETEROCYCLIC COMPOUND HAVING ANTI-TUMOR ACTIVITY AND USE THEREOF", the disclosures of which are incorporated herein by reference in their entirety.

### TECHNICAL FIELD

The present disclosure pertains to the technical field of medicine, and specifically relates to a compound as SOS1 inhibitor, a composition comprising the same, a preparation method therefor, and use thereof.

### BACKGROUND

There are currently three known genes in the RAS family: KRAS (Kirsten Rat Sarcoma Viral Oncogene Homolog), NRAS (neuroblastoma RAS Viral Oncogene Homolog), and HRAS (Harvey Murine Sarcoma Virus Oncogene). RAS-family proteins are a class of small GTPases and RAS is also the first oncogene identified in human tumors. RAS-family proteins have a weak intrinsic GTPase activity and slow nucleotide exchange rates. Binding of GTPase-activating proteins (GAPs) such as NF1 increases the GTPase activity of the RAS-family proteins.

Mutations in RAS enzymes are bound up with tumorigenesis. The types of RAS mutation vary in different types of tumor. In human tumors, KRAS mutations (e.g., amino acids G12, G13, Q61, A146) are the most common, accounting for about 85%, while NRAS mutations (*e.g.,* amino acids G12, G13, Q61, A146) and HRAS mutations (*e.g.,* amino acids G12, G13, Q61) account for 12% and 3%, respectively. Alterations (*e.g.*, mutation, over-expression, gene amplification) in the RAS-family proteins have also been described as a resistant mechanism against cancer drugs such as the EGFR antibodies cetuximab and panitumumab and the EGFR tyrosine kinase inhibitor osimertinib. For oncogenic RAS mutants, the activity of GAP is attenuated or greatly reduced, leading to permanent activation, which underlies oncogenic RAS signaling. Direct inhibition of RAS has been proven to be extremely challenging and lack of drugability as a result of the picomolar affinity of RAS for GTP at the binding site, the lack of other well-defined pockets, and the extensive and flat protein-protein interactions of RAS with GEF, GAP, and effectors. Therefore, inhibition of RAS activation by targeting the upstream guanine nucleotide exchange factors such as SOS proteins may hold new promise.

There are two human isoforms of SOS, *i.e.,* SOS1 and SOS2, but most of the studies focus on SOS1. Human SOS1 includes 1333 amino acids (15 kDa), having a structure comprising an N-terminal histone-like domain, a Dbl homology (DH) domain, a pleckstrin homology (PH) domain, a Helical linker (HL), a Ras exchanger motif (Rem) domain, and a Cdc25 domain, and also a C-terminal domain. Of these, the PH, Rem, and Cdc25 are components of the catalytic core domain of $SOS^{cat}$.

In the last decades, the RAS-family protein - SOS1 protein interaction has gained increasing recognition. Besides, studies have recently been conducted to combine rational design and screening platform to screen out and identify small-molecule inhibitors of SOS1, *i.e.,* the compounds which bind to SOS1 and inhibit the protein-protein interactions with the RAS-family proteins. For example, a plurality of fused cyclic SOS1 inhibitors is described in WO2021105960A1.

Despite the fact that some small-molecule SOS 1 inhibitors have been disclosed, no SOS1 inhibitors have yet been developed and marketed at present. Therefore, there still remains an urgent need to develop new compounds with market potential and better pharmacodynamic and pharmacokinetic properties.

### SUMMARY

The present disclosure aims to provide a novel compound as SOS1 inhibitor, a composition comprising the same, a preparation method therefor, and use thereof for treating a disease mediated by SOS1.

According to the first aspect of the present disclosure, provided is a compound represented by the following formula (A), or a stereoisomer, optical isomer, pharmaceutical salt, prodrug, solvate or isotope derivative thereof,

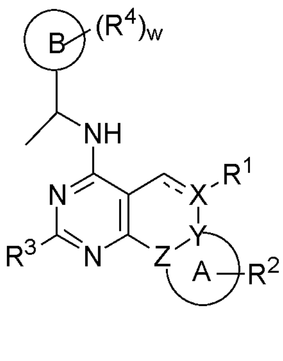

(A)

wherein ------ represents a single bond or a double bond;

Y and Z are both C or N, where Z is C when Y is N and Z is N when Y is C;

Y and Z together with the atoms to which they are linked form a ring A, where the ring A is 5- to 12-membered heterocyclyl or 5- to 12-membered heteroaryl;

there is one, two or three $R^2$, each of which at each occurrence is independently hydrogen, halogen, hydroxyl, cyano, amino, nitro, formyl, oxo, $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more cyano, hydroxyl or halogen;

$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;

ring B is $C_{4-12}$ cycloalkyl, $C_{4-12}$ cycloalkenyl, 4- to 12-membered heterocyclyl, $C_{6-12}$ aryl, 5- to 12-membered heteroaryl, $C_{6-12}$ aryl-fused $C_{4-12}$ cycloalkyl, $C_{6-12}$ aryl-fused 4- to 12-membered heterocyclyl or $C_{6-12}$ aryl-fused $C_{4-12}$ cycloalkenyl;

each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2, 3 or 4;

when ----- is a double bond, X is C, and $R^1$ linked thereto is -O-$R^A$, -N($R^D$)$R^B$ or $R^C$;

when $R^1$ is -O-$R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -O$C_{1-6}$ alkyl, -S$C_{1-6}$ alkyl, -CO$C_{1-6}$ alkyl, -$CH_2$CO$C_{1-6}$ alkyl, -$CH_2$CON($C_{1-6}$ alkyl)$_2$, -$CH_2$CONH$C_{1-6}$ alkyl, -NH$C_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

when $R^1$ is -N($R^D$)$R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, -O$C_{1-6}$ alkyl, -S$C_{1-6}$ alkyl, -CO$C_{1-6}$ alkyl, -$CH_2$CO$C_{1-6}$ alkyl, -$CH_2$CON($C_{1-6}$ alkyl)$_2$, -$CH_2$CONH$C_{1-6}$ alkyl, -NH$C_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, -O$C_{1-6}$ alkyl, -S$C_{1-6}$ alkyl, -CO$C_{1-6}$ alkyl, -$CH_2$CO$C_{1-6}$ alkyl, -$CH_2$CON($C_{1-6}$ alkyl)$_2$, -$CH_2$CONH$C_{1-6}$ alkyl, -NH$C_{1-6}$ alkyl or -N($C_{1-6}$ alkyl)$_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from deuterium, hydroxyl, cyano, $C_{1-3}$ alkyl, $C_{1-3}$ alkoxy or halogen;

when ----- is a single bond, X is N, and $R^1$ linked thereto is $C_{3-10}$ cycloalkyl, $C_{3-10}$ cycloalkenyl, $C_{6-10}$ aryl, 3- to 10-membered heterocyclyl or 5- to 10-membered heteroaryl, wherein the $C_{3-10}$ cycloalkyl, $C_{3-10}$ cycloalkenyl, $C_{6-10}$ aryl, 3- to 10-membered heterocyclyl, and 5- to 10-membered heteroaryl are all optionally substituted with one or more identical or different $R^{a4}$ and/or $R^{b4}$;

each of $R^{a4}$, if present, is independently $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{b4}$ and/or $R^{c4}$;

each of $R^{b4}$, if present, is independently $-OR^{c4}$, $-NR^{c4}R^{c4}$, halogen, -CN, $-C(O)R^{c4}$, $-C(O)OR^{c4}$, $-C(O)NR^{c4}R^{c4}$, $-OC(O)R^{c4}$, $-S(O)_2R^{c4}$, $-S(O)_2NR^{c4}R^{c4}$, $-NHC(O)R^{c4}$, $-N(C_{1-4}$ alkyl$)C(O)R^{c4}$, $-NHC(O)OR^{c4}$ or a divalent substituent =O or =NH, where the =O and =NH may only be substituents in a non-aromatic ring system;

each of $R^{c4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{d4}$ and/or $R^{e4}$;

each of $R^{d4}$, if present, is independently $-OR^{e4}$, $-NR^{e4}R^{e4}$, halogen, -CN, $-C(O)R^{e4}$, $-C(O)OR^{e4}$, $-C(O)NR^{e4}R^{e4}$, $-S(O)_2R^{e4}$, $-S(O)_2NR^{e4}R^{e4}$, $-NHC(O)R^{e4}$, $-N(C_{1-4}$ alkyl$)C(O)R^{e4}$ or a divalent substituent =O, where the =O may only be a substituent in a non-aromatic ring system;

each of $R^{e4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more hydrogen, cyano, hydroxyl or halogen;

wherein Z and Y are deemed as the atoms of ring A and counted in the number of the atoms of ring A.

Unless otherwise stated, heteroatoms in the heteroaryl and heterocyclyl are each independently O, N or S, and the number of heteroatoms is 1, 2, 3 or 4.

Preferably, the present disclosure provides a compound, or a stereoisomer, optical isomer, pharmaceutical salt, prodrug, solvate or isotope derivative thereof, wherein the compound has a structure represented by formula (A'):

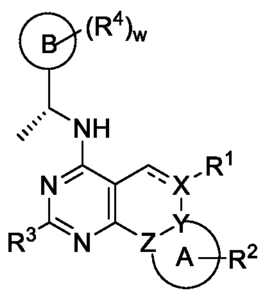

(A')

the substituents in formula (A') are as defined in formula (A).

The present disclosure further provides a compound represented by the following formula (I), or a stereoisomer, optical isomer, pharmaceutical salt, prodrug or solvate:

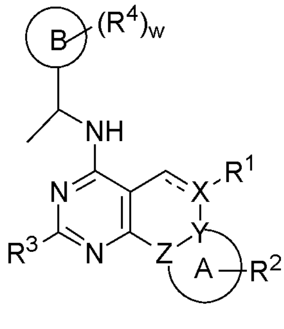

(I)

wherein ----- represents a single bond or a double bond;

Y and Z are both C or N, where Z is C when Y is N and Z is N when Y is C;

Y and Z together with the atoms to which they are linked form a ring A, where the ring A is 5- to 12-membered heterocyclyl or 5- to 12-membered heteroaryl;

there is one, two or three $R^2$, each of which at each occurrence is independently hydrogen, halogen, hydroxyl, cyano, amino, nitro, formyl, oxo, $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more cyano, hydroxyl or halogen;

$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;

ring B is $C_{4-12}$ cycloalkyl, $C_{4-12}$ cycloalkenyl, $C_{4-12}$ heterocyclyl, $C_{6-12}$ aryl, $C_{5-12}$ heteroaryl, $C_{6-12}$ aryl-fused $C_{4-12}$ cycloalkyl, $C_{6-12}$ aryl-fused $C_{4-12}$ heterocyclyl or $C_{6-12}$ aryl-fused $C_{4-12}$ cycloalkenyl;

each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2, 3 or 4;

when ----- is a double bond, X is C, and $R^1$ linked thereto is -O-$R^A$, -N($R^D$)$R^B$ or $R^C$;

when $R^1$ is -O-$R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

when $R^1$ is -N($R^D$)$R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl or -N($C_{1-6}$ alkyl)$_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{c1}$;

each of $R^{c1}$, if present, is independently halogen, hydroxyl, cyano, amino, nitro, oxo, formyl, $C_{1-6}$ alkyl, $C_{2-6}$ alkenyl,

$C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from hydroxyl, $C_{1-3}$ alkyl, $C_{1-3}$ alkoxy or halogen;

when ----- is a single bond, X is N, and $R^1$ linked thereto is $C_{3-10}$ cycloalkyl, $C_{3-10}$ cycloalkenyl, $C_{6-10}$ aryl, 3- to 10-membered heterocyclyl or 5- to 10-membered heteroaryl, wherein the $C_{3-10}$ cycloalkyl, $C_{3-10}$ cycloalkenyl, $C_{6-10}$ aryl, 3- to 10-membered heterocyclyl, and 5- to 10-membered heteroaryl are all optionally substituted with one or more identical or different $R^{a4}$ and/or $R^{b4}$;

each of $R^{a4}$, if present, is independently $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{b4}$ and/or $R^{c4}$;

each of $R^{b4}$, if present, is independently $-OR^{c4}$, $-NR^{c4}R^{c4}$, halogen, -CN, $-C(O)R^{c4}$, $-C(O)OR^{c4}$, $-C(O)NR^{c4}R^{c4}$, $-OC(O)R^{c4}$, $-S(O)_2R^{c4}$, $-S(O)_2NR^{c4}R^{c4}$, $-NHC(O)R^{c4}$, $-N(C_{1-4}$ alkyl$)C(O)R^{c4}$, $-NHC(O)OR^{c4}$ or a divalent substituent =O or =NH, where the =O and =NH may only be substituents in a non-aromatic ring system;

each of $R^{c4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{d4}$ and/or $R^{e4}$;

each of $R^{d4}$, if present, is independently $-OR^{e4}$, $-NR^{e4}R^{e4}$, halogen, -CN, $-C(O)R^{e4}$, $-C(O)OR^{e4}$, $-C(O)NR^{e4}R^{e4}$, $-S(O)_2R^{e4}$, $-S(O)_2NR^{e4}R^{e4}$, $-NHC(O)R^{e4}$, $-N(C_{1-4}$ alkyl$)C(O)R^{e4}$ or a divalent substituent =O, where the =O may only be a substituent in a non-aromatic ring system;

each of $R^{e4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more hydrogen, cyano, hydroxyl or halogen;

wherein Z and Y are deemed as the atoms of ring A and counted in the number of the atoms of ring A.

Unless otherwise stated, heteroatoms in the heteroaryl and heterocyclyl are each independently O, N or S, and the number of heteroatoms is 1, 2, 3 or 4.

Preferably, the present disclosure provides a compound, or a stereoisomer, optical isomer, pharmaceutical salt, prodrug or solvate thereof, wherein the compound has a structure represented by formula (II):

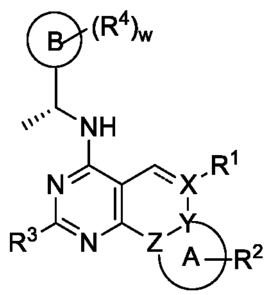

(II)

the substituents in formula (II) are as defined in formula (I).

Preferably, the present disclosure provides a compound, or a stereoisomer, optical isomer, pharmaceutical salt, prodrug or solvate thereof, wherein the compounds has a structure represented by formula (III):

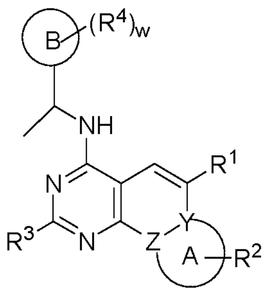
(III)

the substituents in formula (III) are as defined in formula (I).

Preferably, the present disclosure provides a compound, or a stereoisomer, optical isomer, pharmaceutical salt, prodrug or solvate thereof, wherein the compound has a structure represented by formula (IV):

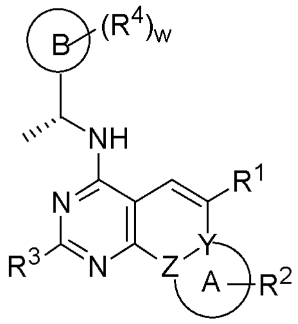
(IV)

the substituents in formula (IV) are as defined in formula (I).

In one preferred embodiment of the present disclosure, ----- is a double bond, X is C, and $R^1$ linked thereto is $-O-R^A$.

Preferably, $R^A$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl or $-N(C_{1-6}$ alkyl$)_2$.

Further preferably, $R^A$ is $C_{3-6}$ cycloalkyl, $C_{6-10}$ aryl, 3- to 6-membered heterocyclyl or 5- to 6-membered heteroaryl, wherein the $C_{3-6}$ cycloalkyl, $C_{6-10}$ aryl, 3-to 6-membered heterocyclyl, and 5- to 6-membered heteroaryl are all optionally substituted with 1 to 3 identical or different $R^{a1}$;

each of $R^{a1}$, if present, is independently halogen, hydroxyl, cyano, amino, oxo, formyl, $C_{1-6}$ alkyl, $-OC_{1-6}$ alkyl, $-SC_{1-6}$ alkyl or $-COC_{1-6}$ alkyl.

Further preferably, $R^A$ is 5- to 6-membered heterocyclyl or 5- to 6-membered heteroaryl, wherein the 5- to 6-membered heterocyclyl and 5- to 6-membered heteroaryl are both optionally substituted with 1 to 3 identical or different $R^{a1}$;

each of $R^{a1}$, if present, is independently halogen, hydroxyl, cyano, amino, oxo, formyl, $C_{1-4}$ alkyl, $-OC_{1-4}$ alkyl, $-SC_{1-4}$ alkyl or $-COC_{1-4}$ alkyl.

Further preferably, $R^A$ is 5- to 6-membered heterocyclyl, wherein the 5- to 6-membered heterocyclyl is optionally substituted with one or two identical or different $R^{a1}$;

each of $R^{a1}$, if present, is independently halogen, oxo, formyl, acetyl, methyl, ethyl, n-propyl, isopropyl or methoxy.

Further preferably, $R^A$ is 5- to 6-membered monocyclic heterocyclyl, wherein the 5- to 6-membered monocyclic heterocyclyl is optionally substituted with one or two identical or different $R^{a1}$; heteroatoms in the 5- to 6-membered monocyclic heterocyclyl are each independently O, N or S, and the number of heteroatoms is 1;

each of $R^{a1}$, if present, is independently halogen, oxo, formyl, acetyl, methyl, ethyl, n-propyl, isopropyl or methoxy.

Further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;

each of $R^{a1}$, if present, is independently halogen, oxo, formyl, acetyl, methyl, ethyl, n-propyl, isopropyl or methoxy.

Even further preferably, $R^A$ is the following group:

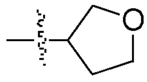, 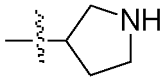,

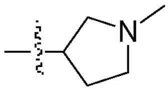, 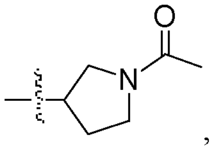, 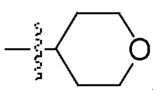, 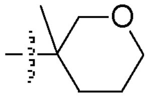 or 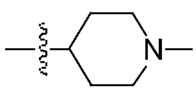.

In one preferred embodiment of the present disclosure, $\overline{\text{-----}}$ is a double bond, X is C, and $R^1$ linked thereto is $-N(R^D)R^B$.

Preferably, $R^B$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl.

Further preferably, $R^B$ is $C_{1-6}$ alkyl, 3- to 10-membered heterocyclyl or 5- to 10-membered heteroaryl, wherein the $C_{1-6}$ alkyl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl.

Further preferably, $R^B$ is $C_{1-6}$ alkyl or 5- to 6-membered monocyclic heterocyclyl, wherein the $C_{1-6}$ alkyl and 5- to 6-membered monocyclic heterocyclyl are both optionally substituted with one or two identical or different $R^{b1}$; heteroatoms in the 5-to 6-membered monocyclic heterocyclyl are each independently O, N or S, and the number of heteroatoms is 1;
each of $R^{b1}$, if present, is independently halogen, oxo, formyl, $C_{1-4}$ alkyl, $-OC_{1-4}$ alkyl or $-COC_{1-4}$ alkyl.

Further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;
each of $R^{b1}$, if present, is independently oxo, formyl, acetyl, methyl, ethyl, methoxy or ethoxy.

Even further preferably, $R^B$ is the following group:

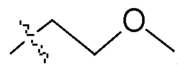, 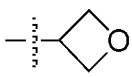 or

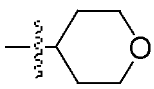.

Preferably, $R^D$ is hydrogen, $C_{1-6}$ alkyl or $-OC_{1-6}$ alkyl; further preferably, $R^D$ is hydrogen or $C_{1-3}$ alkyl; even further preferably, $R^D$ is hydrogen or methyl; most preferably, $R^D$ is hydrogen.

In one preferred embodiment of the present disclosure, ------ is a double bond, X is C, and $R^1$ linked thereto is $R^C$.

Preferably, $R^C$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{c1}$;
each of $R^{c1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl or 3- to 10-membered heterocyclyl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, and 3- to 10-membered heterocyclyl are all optionally substituted with one or more substituents selected from hydroxyl, $C_{1-3}$ alkyl, $C_{1-3}$ alkoxy or halogen.

Further preferably, $R^C$ is $C_{1-6}$ alkyl, $C_{3-10}$ cycloalkyl, 3- to 10-membered heterocyclyl or 5- to 10-membered

heteroaryl, wherein the $C_{1-6}$ alkyl, $C_{3-10}$ cycloalkyl, 3- to 10-membered heterocyclyl, and 5- to 10-membered heteroaryl are all optionally substituted with 1 to 3 identical or different $R^{c1}$;

each of $R^{c1}$, if present, is independently halogen, hydroxyl, cyano, amino, nitro, oxo, formyl, $C_{1-4}$ alkyl, $-OC_{1-4}$ alkyl, $-SC_{1-4}$ alkyl, $-COC_{1-4}$ alkyl, $-CH_2CON(C_{1-4}\ alkyl)_2$, $-CH_2CONHC_{1-4}$ alkyl or 3- to 6-membered heterocyclyl, wherein the $C_{1-4}$ alkyl, $-OC_{1-4}$ alkyl, $-SC_{1-4}$ alkyl, $-COC_{1-4}$ alkyl, $-CH_2CON(C_{1-4}\ alkyl)_2$, $-CH_2CONHC_{1-4}$ alkyl, and 3- to 6-membered heterocyclyl are all optionally substituted with one or more substituents selected from hydroxyl, methyl, methoxy or halogen.

Further preferably, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 3 identical or different $R^{c1}$,

each of $R^{c1}$, if present, is independently halogen, hydroxyl, cyano, amino, oxo, formyl, $C_{1-4}$ alkyl, $-OC_{1-4}$ alkyl, $-COC_{1-4}$ alkyl, $-CH_2CON(C_{1-4}\ alkyl)_2$ or 3- to 6-membered heterocyclyl, wherein the $C_{1-4}$ alkyl, $-OC_{1-4}$ alkyl, $-COC_{1-4}$ alkyl, $-CH_2CON(C_{1-4}\ alkyl)_2$, and 3- to 6-membered heterocyclyl are all optionally substituted with one or more substituents selected from hydroxyl, methyl, methoxy or halogen.

Further preferably, $R^C$ is 5- to 6-membered monocyclic heterocyclyl, 6- to 10-membered spiro heterocyclyl, 6- to 8-membered bridged heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, and 5- to 6-membered monocyclic heteroaryl are all optionally substituted with 1 to 3 identical or different $R^{c1}$;

each of $R^{c1}$, if present, is independently halogen, hydroxyl, cyano, amino, oxo, formyl, acetyl, propionyl, methoxy, ethoxy, methyl, ethyl, n-propyl, isopropyl, $-CH_2CON(CH_3)_2$ or 6-membered heterocyclyl, wherein the acetyl, propionyl, methoxy, ethoxy, methyl, ethyl, n-propyl, isopropyl, $-CH_2CON(CH_3)_2$, and 6-membered heterocyclyl are all optionally substituted with one or more substituents selected from hydroxyl, methyl, methoxy or halogen.

Even further preferably, $R^C$ is 6-membered monocyclic heterocyclyl, 4-membered/6-membered spiro heterocyclyl, 4-membered/4-membered spiro heterocyclyl, 7-membered bridged heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 7-membered bridged heterocyclyl, and 6-membered monocyclic heteroaryl are all optionally substituted with 1 to 3 identical or different $R^{c1}$;

each of $R^{c1}$, if present, is independently halogen, hydroxyl, cyano, amino, oxo, formyl, acetyl, $-COCH_2CH_3$, $-COCH_2OH$, hydroxymethyl, hydroxyethyl, $-CH_2OCH_3$, methoxy, ethoxy, methyl, ethyl, n-propyl, isopropyl, $-CH_2CON(CH_3)_2$ or 6-membered heterocyclyl.

Further preferably, $R^C$ is

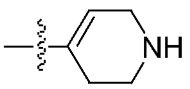, 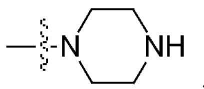, 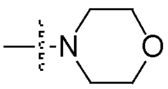,

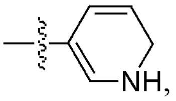 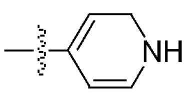, 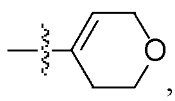, 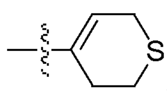, 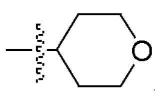, 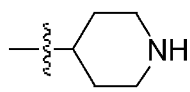,

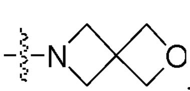, 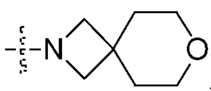, 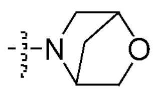 or 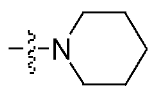,

wherein the $R^C$ are all optionally substituted with one or two identical or different $R^{c1}$;

each of $R^{c1}$, if present, is independently F, Cl, Br, hydroxyl, cyano, amino, oxo, acetyl, $-COCH_2CH_3$, $-COCH_2OH$, hydroxymethyl, hydroxyethyl, $-CH_2OCH_3$, methoxy, methyl, ethyl, isopropyl, $-CH_2CON(CH_3)_2$ or morpholinyl.

Even further preferably, $R^C$ optionally substituted with $R^{c1}$ is the following group:

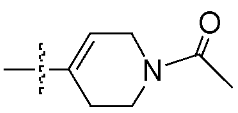, 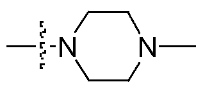, 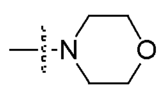, 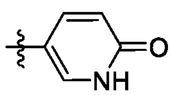, 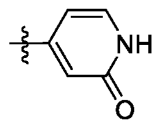,

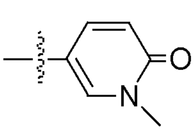, 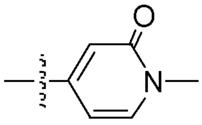, 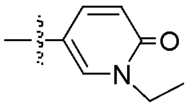, 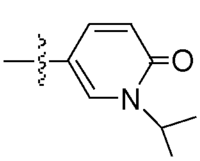, 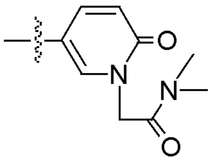,

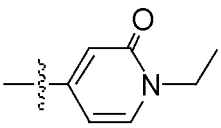, 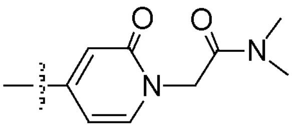, 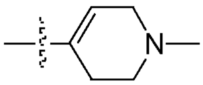, 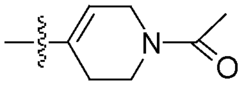,

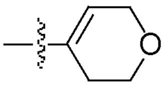, 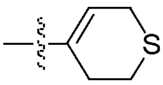, 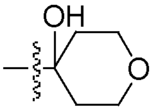, 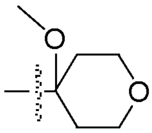, 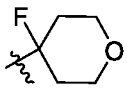, 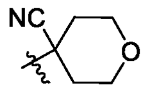,

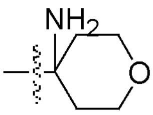, 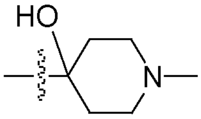, 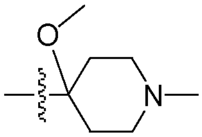, 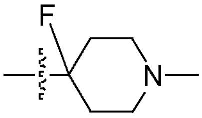, 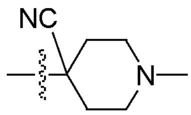,

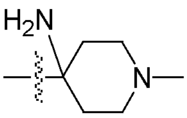, 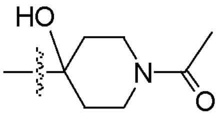, 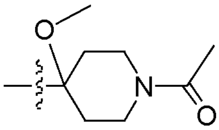, 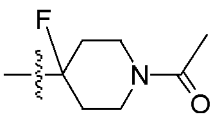,

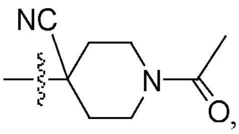, 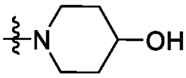, 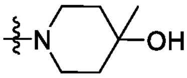, 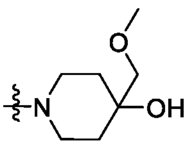, 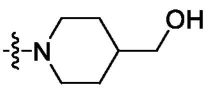,

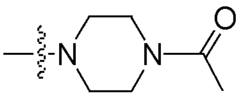, 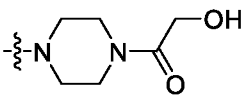, 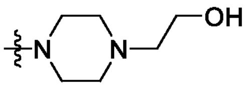, 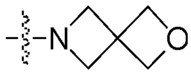,

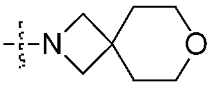, 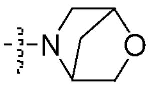, 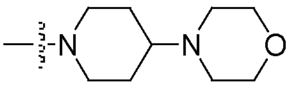, 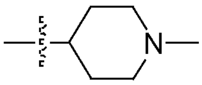,

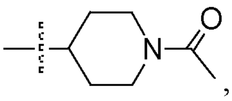, 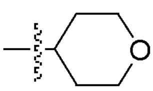 or 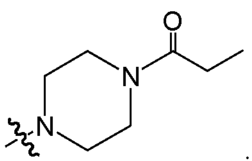.

In one preferred embodiment of the present disclosure, ring A is 5- to 10-membered heterocyclyl or 5- to 10-membered heteroaryl, wherein heteroatoms in the 5- to 10-membered heterocyclyl and 5- to 10-membered heteroaryl are each independently O or N, and the number of heteroatoms is 1 to 4.

Further preferably, ring A is 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/4-membered fused heterocyclyl, 5-membered/6-membered fused heterocyclyl, 6-membered/5-membered fused heterocyclyl, 6-membered/4-membered fused heterocyclyl, 6-membered/6-membered fused heterocyclyl, 5-membered/3-membered spiro heterocyclyl, 5-membered/5-membered spiro heterocyclyl, 5-membered/4-membered spiro heterocyclyl, 5-membered/6-membered spiro heterocyclyl, 6-membered/3-membered spiro heterocyclyl, 6-membered/5-membered spiro heterocyclyl, 6-membered/4-membered spiro heterocyclyl or 6-membered/6-membered spiro heterocyclyl, wherein heteroatoms in the 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/4-membered fused heterocyclyl, 5-membered/6-membered fused heterocyclyl, 6-membered/5-membered fused heterocyclyl, 6-membered/4-membered fused heterocyclyl, 6-membered/6-membered fused heterocyclyl, 5-membered/3-membered spiro heterocyclyl, 5-membered/5-membered spiro heterocyclyl, 5-membered/4-membered spiro heterocyclyl, 5-membered/6-membered spiro heterocyclyl, 6-membered/3-membered spiro heterocyclyl, 6-membered/5-membered spiro heterocyclyl, 6-membered/4-membered spiro heterocyclyl, and 6-membered/6-membered spiro heterocyclyl are each independently N, and the number of heteroatoms is 1 to 4.

Further preferably, ring A is 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/6-membered fused heterocyclyl or 5-membered/3-membered spiro heterocyclyl, wherein heteroatoms in the 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/6-membered fused heterocyclyl, and 5-membered/3-membered spiro heterocyclyl are each independently N, and the number of heteroatoms is 1 to 3.

Further preferably, ring A is 5-membered monocyclic heterocyclyl or 5-membered monocyclic heteroaryl, wherein heteroatoms in the 5-membered monocyclic heterocyclyl and 5-membered monocyclic heteroaryl are N, and the number of heteroatoms is 1 to 3.

Even further preferably, ring A is the following group:

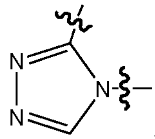 , 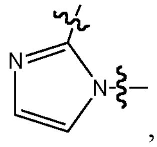 ,

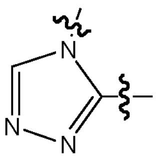 , 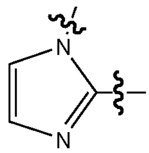 , 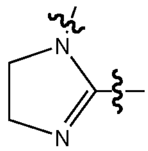 , 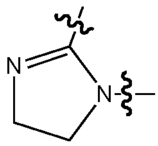 , 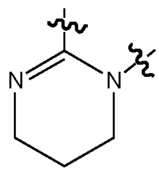 , 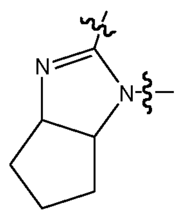 ,

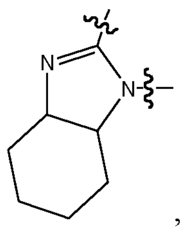 , 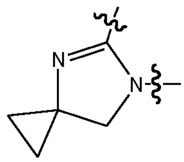 or 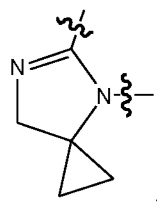 .

Even further preferably, ring A is the following group:

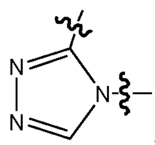 , 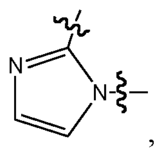 ,

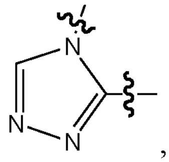, 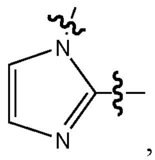, 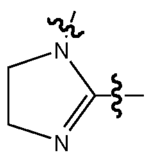 or 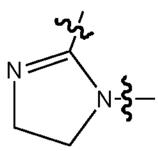.

In one preferred embodiment of the present disclosure, there is one, two or three $R^2$, each of which at each occurrence is independently hydrogen, halogen, hydroxyl, cyano, amino, nitro, formyl, oxo, $C_{1-3}$ alkyl, $C_{1-3}$ alkoxy, -$C_{1-3}$ alkyl-NH($C_{1-3}$ alkyl) or -$C_{1-3}$ alkyl-N($C_{1-3}$ alkyl)$_2$, wherein the $C_{1-3}$ alkyl, $C_{1-3}$ alkoxy, -$C_{1-3}$ alkyl-NH($C_{1-3}$ alkyl), and -$C_{1-3}$ alkyl-N($C_{1-3}$ alkyl)$_2$ are all optionally substituted with one or more hydroxyl or halogen;

further preferably, there is one, two or three $R^2$, each of which at each occurrence is independently hydrogen, halogen, hydroxyl, cyano, amino, nitro, formyl, oxo, methoxy, methyl, ethyl, n-propyl or isopropyl;
further preferably, there is one or two $R^2$, each of which at each occurrence is independently hydrogen, halogen, hydroxyl, cyano, amino, nitro, methoxy or methyl;
even further preferably, there is one or two $R^2$, each of which at each occurrence is independently hydrogen or methyl.

In one preferred embodiment of the present disclosure, $R^3$ is hydrogen, halogen, hydroxyl, amino, cyano, $C_{1-4}$ alkyl, $C_{1-4}$ alkoxy or $C_{3-6}$ cycloalkyl, wherein the $C_{1-4}$ alkyl, $C_{1-4}$ alkoxy, and $C_{3-6}$ cycloalkyl are all optionally substituted with one or more hydroxyl or halogen;

further preferably, $R^3$ is hydrogen, halogen, hydroxyl, amino, cyano, methyl, ethyl, n-propyl, isopropyl, cyclopropyl, cyclobutyl, cyclopentyl, cyclohexyl or methoxy;
further preferably, $R^3$ is hydrogen, halogen, hydroxyl, amino, cyano, methyl, ethyl, n-propyl, isopropyl or cyclopropyl;
even further preferably, $R^3$ is hydrogen, F, Cl, Br, amino, methyl, ethyl or cyclopropyl.

In one preferred embodiment of the present disclosure, ring B is $C_{4-12}$ cycloalkenyl, $C_{4-12}$ heterocyclyl, $C_{6-12}$ aryl, $C_{6-8}$ aryl-fused $C_{4-6}$ cycloalkyl, $C_{6-8}$ aryl-fused $C_{4-6}$ heterocyclyl or $C_{5-12}$ heteroaryl.

Further preferably, ring B is $C_{6-10}$ aryl or $C_{5-10}$ heteroaryl.

Further preferably, ring B is phenyl or pyridinyl.

In one preferred embodiment of the present disclosure, each of $R^4$, if present, is independently hydrogen, cyano, halogen, amino, nitro, $C_{1-4}$ alkyl, $C_{1-4}$ haloalkyl, $C_{1-4}$ hydroxyalkyl, $C_{3-6}$ cycloalkyl, $C_{3-6}$ halocycloalkyl or 3- to 6-membered heterocyclyl, wherein the $C_{1-4}$ alkyl, $C_{1-4}$ haloalkyl, $C_{1-4}$ hydroxyalkyl, $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2 or 3.

Further preferably, each of $R^4$ is independently hydrogen, cyano, halogen, amino, nitro, $C_{1-4}$ alkyl, $C_{1-4}$ haloalkyl or $C_{1-4}$ hydroxyalkyl, wherein the $C_{1-4}$ alkyl, $C_{1-4}$ haloalkyl, and $C_{1-4}$ hydroxyalkyl are all optionally substituted with one or more of the following substituents: halogen, hydroxyl or amino; w is 1, 2 or 3.

Further preferably, each of $R^4$ is independently hydrogen, cyano, halogen, amino, nitro, methyl, ethyl, n-propyl or isopropyl, wherein the methyl, ethyl, n-propyl, and isopropyl are all optionally substituted with one or more of the following substituents: halogen or hydroxyl; w is 1, 2 or 3.

Further preferably, each of $R^4$ is independently hydrogen, halogen, amino, methyl, ethyl or isopropyl, wherein the methyl, ethyl, and isopropyl are all optionally substituted with one or more of the following substituents: halogen or hydroxyl; w is 1, 2 or 3.

Further preferably, each of $R^4$ is independently hydrogen, halogen, amino, methyl, trifluoromethyl, difluoromethyl, monofluoromethyl, -$CF_2CH_2OH$, -$C(CH_3)_2OH$ or -$CF_2CH_3$; w is 1, 2 or 3.

The present disclosure further provides a compound, or a stereoisomer, optical isomer, pharmaceutical salt, prodrug, solvate or isotope derivative thereof, wherein the compound has a structure represented by formula (B):

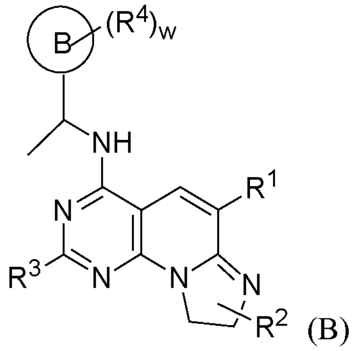

(B)

wherein $R^1$ is -O-$R^A$, -N($R^D$)$R^B$ or $R^C$;

when $R^1$ is -O-$R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl, -N($C_{1-6}$ alkyl$)_2$, $C_{3-10}$ cycloalkyl, $C_{6-10}$ aryl, 3- to 10-membered heterocyclyl or 5- to 10-membered heteroaryl;

when $R^1$ is -N($R^D$)$R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl, -N($C_{1-6}$ alkyl$)_2$, $C_{3-10}$ cycloalkyl, $C_{6-10}$ aryl, 3- to 10-membered heterocyclyl or 5- to 10-membered heteroaryl;

$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl or -N($C_{1-6}$ alkyl$)_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$COC_{3-6}$ cycloalkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl, -N($C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$COC_{3-6}$ cycloalkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl, -N($C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from deuterium, hydroxyl, cyano, $C_{1-3}$ alkyl, $C_{1-3}$ alkoxy or halogen;

there is one or two $R^2$, each of which at each occurrence is independently hydrogen, halogen, hydroxyl, cyano, amino, nitro, formyl, oxo, $C_{1-6}$ alkyl, $C_{1-6}$ alkoxy, halogenated $C_{1-6}$ alkyl or halogenated $C_{1-6}$ alkoxy;

$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;

ring B is $C_{4-12}$ cycloalkyl, $C_{4-12}$ cycloalkenyl, $C_{4-12}$ heterocyclyl, $C_{6-12}$ aryl, $C_{5-12}$ heteroaryl, $C_{6-12}$ aryl-fused $C_{4-12}$ cycloalkyl, $C_{6-12}$ aryl-fused $C_{4-12}$ heterocyclyl or $C_{6-12}$ aryl-fused $C_{4-12}$ cycloalkenyl;

each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2, 3 or 4;

unless otherwise stated, heteroatoms in the heteroaryl and heterocyclyl are each independently O, N or S, and the number of heteroatoms is 1, 2, 3 or 4.

In one preferred embodiment of the present disclosure, $R^1$ is $-O-R^A$, $R^A$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl or $-N(C_{1-6}$ alkyl$)_2$.

Further preferably, $R^A$ is $C_{3-6}$ cycloalkyl, $C_{6-10}$ aryl, 3- to 6-membered heterocyclyl or 5- to 6-membered heteroaryl, wherein the $C_{3-6}$ cycloalkyl, $C_{6-10}$ aryl, 3-to 6-membered heterocyclyl, and 5- to 6-membered heteroaryl are all optionally substituted with 1 to 3 identical or different $R^{a1}$;

each of $R^{a1}$, if present, is independently halogen, hydroxyl, cyano, amino, oxo, formyl, $C_{1-6}$ alkyl, $-OC_{1-6}$ alkyl, $-SC_{1-6}$ alkyl or $-COC_{1-6}$ alkyl.

Further preferably, $R^A$ is 5- to 6-membered heterocyclyl or 5- to 6-membered heteroaryl, wherein the 5- to 6-membered heterocyclyl and 5- to 6-membered heteroaryl are both optionally substituted with 1 to 3 identical or different $R^{a1}$;

each of $R^{a1}$, if present, is independently halogen, hydroxyl, cyano, amino, oxo, formyl, $C_{1-4}$ alkyl, $-OC_{1-4}$ alkyl, $-SC_{1-4}$ alkyl or $-COC_{1-4}$ alkyl.

Further preferably, $R^A$ is 5- to 6-membered heterocyclyl, wherein the 5- to 6-membered heterocyclyl is optionally substituted with one or two identical or different $R^{a1}$;

each of $R^{a1}$, if present, is independently halogen, oxo, formyl, acetyl, methyl, ethyl, n-propyl, isopropyl or methoxy.

Further preferably, $R^A$ is 5- to 6-membered monocyclic heterocyclyl, wherein the 5- to 6-membered monocyclic heterocyclyl is optionally substituted with one or two identical or different $R^{a1}$; heteroatoms in the 5- to 6-membered monocyclic heterocyclyl are each independently O, N or S, and the number of heteroatoms is 1;

each of $R^{a1}$, if present, is independently halogen, oxo, formyl, acetyl, methyl, ethyl, n-propyl, isopropyl or methoxy.

Further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;

each of $R^{a1}$, if present, is independently halogen, oxo, formyl, acetyl, methyl, ethyl, n-propyl, isopropyl or methoxy.

Even further preferably, $R^A$ is the following group:

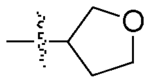 , 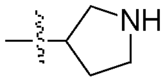 ,

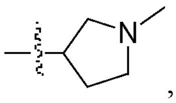 , 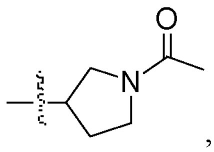 , 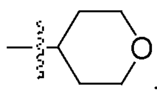 , 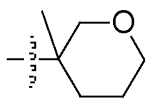 or 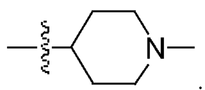 .

In one preferred embodiment of the present disclosure, $R^1$ is $-N(R^D)R^B$, where $R^B$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl.

Further preferably, $R^B$ is $C_{1-6}$ alkyl, 3- to 10-membered heterocyclyl or 5- to 10-membered heteroaryl, wherein the $C_{1-6}$ alkyl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl.

Further preferably, $R^B$ is $C_{1-6}$ alkyl or 5- to 6-membered monocyclic heterocyclyl, wherein the $C_{1-6}$ alkyl and 5- to 6-membered monocyclic heterocyclyl are both optionally substituted with one or two identical or different $R^{b1}$; heteroatoms in the 5-to 6-membered monocyclic heterocyclyl are each independently O, N or S, and the number of heteroatoms is 1;

each of $R^{b1}$, if present, is independently halogen, oxo, formyl, $C_{1-4}$ alkyl, $-OC_{1-4}$ alkyl or $-COC_{1-4}$ alkyl.

Further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;

each of $R^{b1}$, if present, is independently oxo, formyl, acetyl, methyl, ethyl, methoxy or ethoxy.

Even further preferably, $R^B$ is the following group:

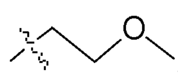, 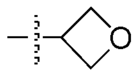 or

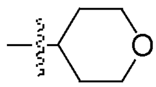.

$R^D$ is hydrogen, $C_{1-6}$ alkyl or $-OC_{1-6}$ alkyl; further preferably, $R^D$ is hydrogen or $C_{1-3}$ alkyl; even further preferably, $R^D$ is hydrogen or methyl; most preferably, $R^D$ is hydrogen.

In one preferred embodiment of the present disclosure, $R^1$ is $R^C$, and $R^C$ is $C_{3-10}$ cycloalkyl, $C_{6-10}$ aryl, 3- to 10-membered heterocyclyl or 5- to 10-membered heteroaryl, wherein the $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl or 3- to 10-membered heterocyclyl, wherein the $C_{1-6}$ alkyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, and 3- to 10-membered heterocyclyl are all optionally substituted with one or more substituents selected from deuterium, hydroxyl, cyano, $C_{1-3}$ alkyl, $C_{1-3}$ alkoxy or halogen.

Further preferably, $R^C$ is $C_{3-10}$ cycloalkyl, 3- to 10-membered heterocyclyl or 5- to 10-membered heteroaryl, wherein the $C_{3-10}$ cycloalkyl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-4}$ alkyl, $-OC_{1-4}$ alkyl, $-SC_{1-4}$ alkyl, $-COC_{1-4}$ alkyl, $-COC_{3-6}$ cycloalkyl, $-CH_2COC_{1-4}$ alkyl, $-CH_2CON(C_{1-4}$ alkyl$)_2$, $-CH_2CONHC_{1-4}$ alkyl, $C_{3-6}$ cycloalkyl or 3- to 6-membered heterocyclyl, wherein the $C_{1-4}$ alkyl, $-OC_{1-4}$ alkyl, $-SC_{1-4}$ alkyl, $-COC_{1-4}$ alkyl, $-COC_{3-6}$ cycloalkyl, $-CH_2COC_{1-4}$ alkyl, $-CH_2CON(C_{1-4}$ alkyl$)_2$, $-CH_2CONHC_{1-4}$ alkyl, $C_{3-6}$ cycloalkyl, and 3- to 6-membered heterocyclyl are all optionally substituted with one or more substituents selected from deuterium, hydroxyl, cyano, $C_{1-3}$ alkyl, $C_{1-3}$ alkoxy or halogen.

Further preferably, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$,

each of $R^{c1}$, if present, is independently halogen, hydroxyl, cyano, amino, oxo, formyl, methylsulfonyl, $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$ or 3- to 6-membered heterocyclyl, wherein the $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$, and 3- to 6-membered heterocyclyl are all optionally substituted with one or more substituents selected from deuterium, hydroxyl, cyano, methyl, methoxy or halogen.

Further preferably, $R^C$ is 5- to 6-membered monocyclic heterocyclyl, 6- to 10-membered spiro heterocyclyl, 6- to 8-membered bridged heterocyclyl, 8- to 10-membered fused heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, 8- to 10-membered fused heterocyclyl, and 5-to 6-membered monocyclic heteroaryl are all optionally substituted with 1 to 4 identical or different $R^{c1}$;

each of $R^{c1}$, if present, is independently halogen, hydroxyl, cyano, amino, oxo, formyl, acetyl, propionyl, methylsulfonyl, methoxy, ethoxy, methyl, ethyl, n-propyl, isopropyl, $-CH_2CON(CH_3)_2$, -CO-cyclopropyl, -CO-cyclobutyl, 4-membered heterocyclyl, 5-membered heterocyclyl or 6-membered heterocyclyl, wherein the acetyl, propionyl, methylsulfonyl, methoxy, ethoxy, methyl, ethyl, n-propyl, isopropyl, $-CH_2CON(CH_3)_2$, -CO-cyclopropyl, -CO-cyclobutyl, 4-membered heterocyclyl, 5-membered heterocyclyl, and 6-membered heterocyclyl are all optionally substituted with one or more substituents selected from deuterium, hydroxyl, cyano, methyl, methoxy or halogen.

Even further preferably, $R^C$ is 6-membered monocyclic heterocyclyl, 4-membered/6-membered spiro heterocyclyl, 4-membered/4-membered spiro heterocyclyl, 6-membered/5-membered fused heterocyclyl, 7-membered bridged heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 6-membered/4-membered fused heterocyclyl, 7-membered bridged heterocyclyl, and 6-membered monocyclic heteroaryl are all optionally substituted with 1 to 4 identical or different $R^{c1}$;
each of $R^{c1}$, if present, is independently halogen, hydroxyl, cyano, amino, oxo, formyl, acetyl, propionyl, methylsulfonyl, methoxy, ethoxy, methyl, ethyl, n-propyl, isopropyl, $-CH_2CON(CH_3)_2$, -CO-cyclopropyl, 4-membered heterocyclyl, 5-membered heterocyclyl or 6-membered heterocyclyl, wherein the acetyl, propionyl, methylsulfonyl, methoxy, ethoxy, methyl, ethyl, n-propyl, isopropyl, $-CH_2CON(CH_3)_2$, -CO-cyclopropyl, 4-membered heterocyclyl, 5-membered heterocyclyl, and 6-membered heterocyclyl are all optionally substituted with one or more substituents selected from deuterium, hydroxyl, cyano, methyl, methoxy or halogen.

Further preferably, $R^C$ is

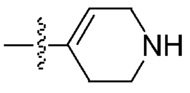, 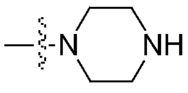, 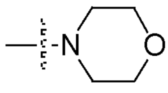,

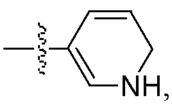, 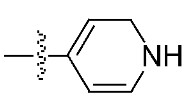, 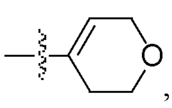, 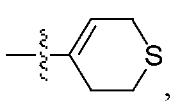, 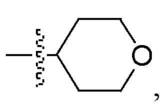, 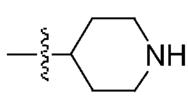,

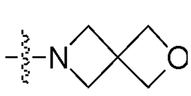, 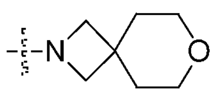, 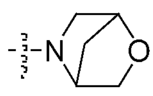, 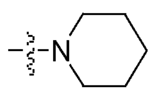, 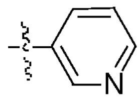, 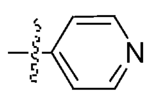,

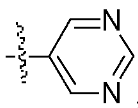, 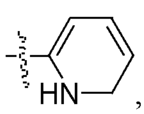, 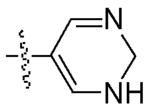, 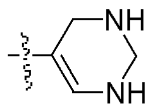 or 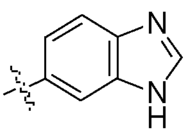,

and the $R^C$ are all optionally substituted with 1 to 4 identical or different $R^{c1}$;
each of $R^{c1}$, if present, is independently F, Cl, Br, hydroxyl, cyano, amino, oxo, methylsulfonyl, acetyl, $-COCH_2CH_3$, $-COCH_2OH$, $-COCH_2CN$, $-CH(OH)(CH_3)_2$, hydroxymethyl, hydroxyethyl, $-CH_2OCH_3$, $-CH_2CH_2OCH_3$, methoxy, methyl, $CD_3$, ethyl, isopropyl, monofluoromethyl, difluoromethyl, trifluoromethyl, monofluoroethyl, difluoroethyl, trifluoroethyl,

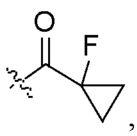, 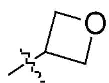,

$-CH_2CON(CH_3)_2$ or morpholinyl.

Even further preferably, $R^C$ optionally substituted with $R^{c1}$ is the following group:

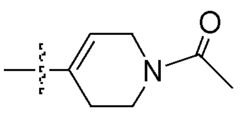, 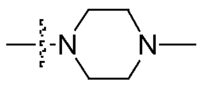, 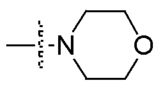, 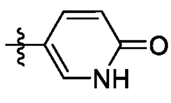, 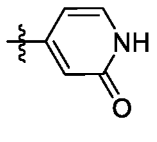,

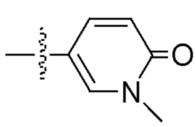, 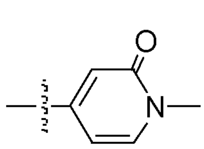, 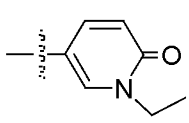, 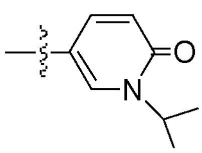, 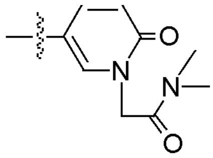,

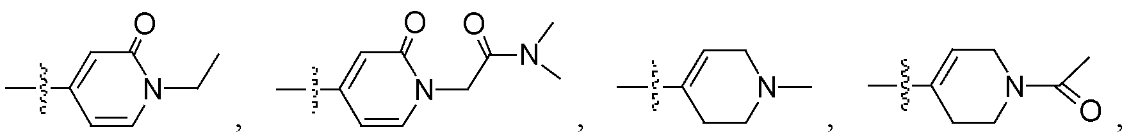
, , , ,

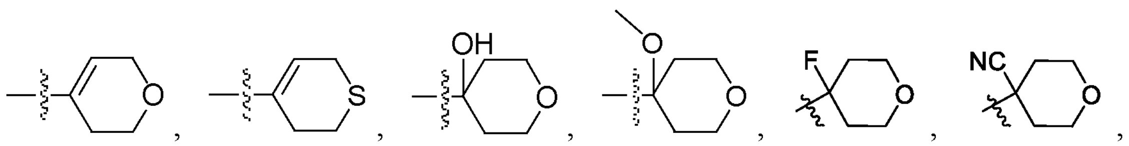
, , , , , ,

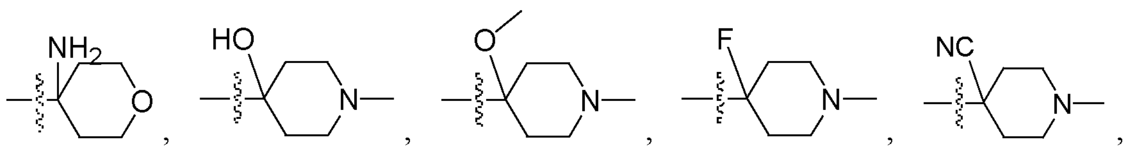
, , , , ,

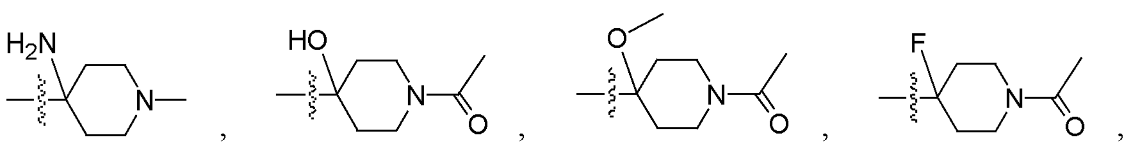
, , , ,

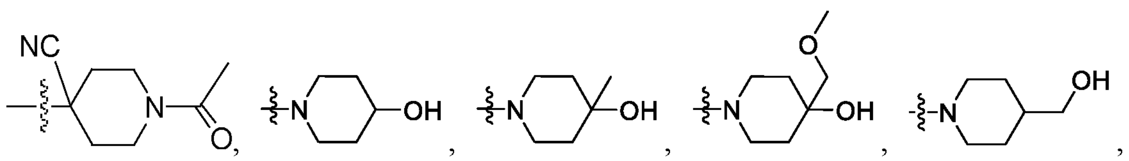
, , , , ,

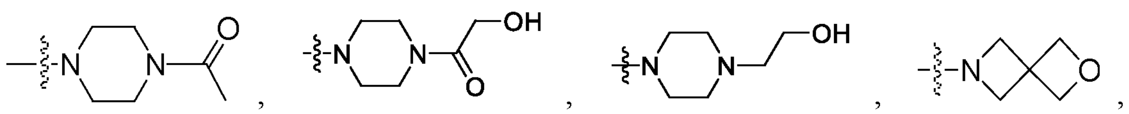
, , , ,

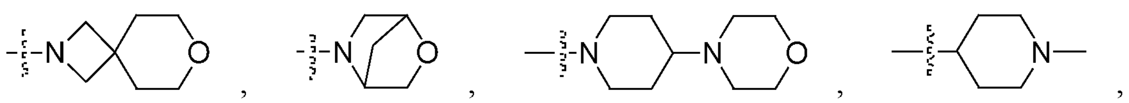
, , , ,

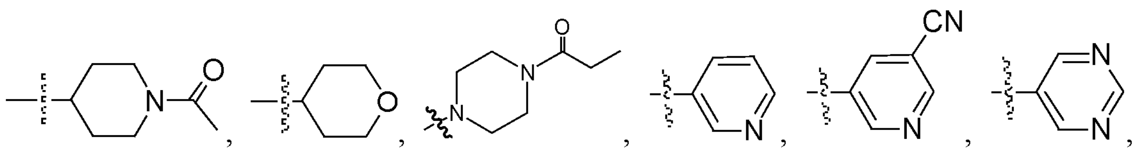
, , , , , ,

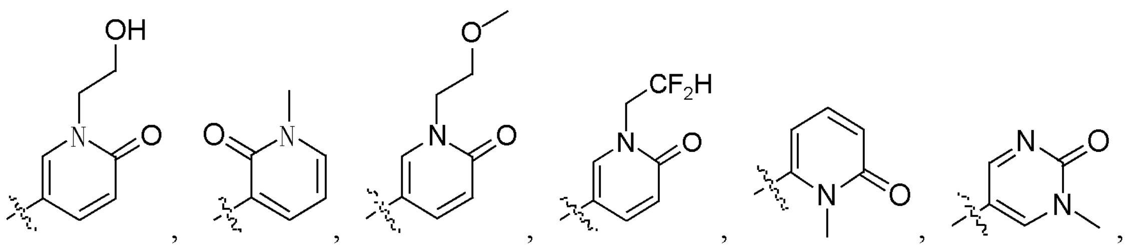
, , , , , ,

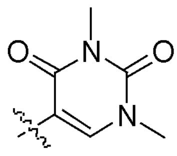, 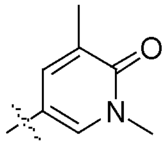, 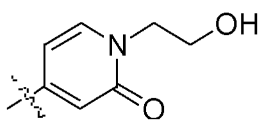, 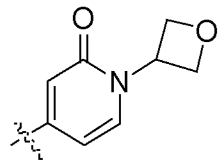, 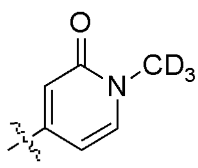,

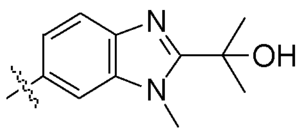, 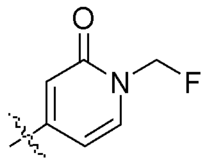, 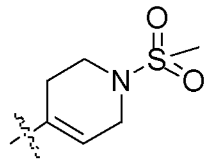, 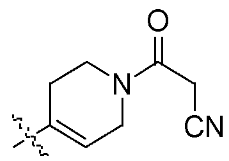,

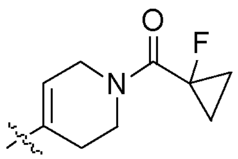 or 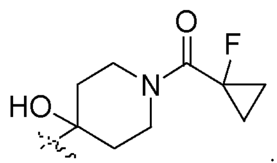.

In one preferred embodiment of the present disclosure, there is one or two $R^2$, each of which at each occurrence is independently hydrogen, halogen, hydroxyl, cyano, amino, nitro, formyl, oxo, methoxy, methyl, ethyl, n-propyl or isopropyl.

Further preferably, there is one or two $R^2$, each of which at each occurrence is independently hydrogen, halogen, hydroxyl, cyano, amino, nitro, methoxy or methyl.

Even further preferably, there is one or two $R^2$, each of which at each occurrence is independently hydrogen or methyl.

Even further preferably, $R^2$ is hydrogen.

In one preferred embodiment of the present disclosure, $R^3$ is hydrogen, halogen, hydroxyl, amino, cyano, methyl, ethyl, n-propyl, isopropyl, cyclopropyl, cyclobutyl, cyclopentyl, cyclohexyl or methoxy.

Further preferably, $R^3$ is hydrogen, halogen, hydroxyl, amino, cyano, methyl, ethyl, n-propyl, isopropyl or cyclopropyl.

Even further preferably, $R^3$ is hydrogen, F, Cl, Br, amino, methyl, ethyl or cyclopropyl.

In one preferred embodiment of the present disclosure, ring B is $C_{4-12}$ cycloalkenyl, 4- to 12-membered heterocyclyl, $C_{6-12}$ aryl, $C_{6-8}$ aryl-fused $C_{4-6}$ cycloalkyl, $C_{6-8}$ aryl-fused 4- to 6-membered heterocyclyl or 5- to 12-membered heteroaryl.

Further preferably, ring B is $C_{6-10}$ aryl, 5- to 10-membered heteroaryl or $C_{6-8}$ aryl-fused 4- to 6-membered heterocyclyl.

Further preferably, ring B is phenyl, pyridinyl or benzodihydrofuranyl.

In one preferred embodiment of the present disclosure, each of $R^4$, if present, is independently hydrogen, cyano, halogen, amino, nitro, $C_{1-4}$ alkyl, $C_{1-4}$ haloalkyl, $C_{1-4}$ hydroxyalkyl, $C_{3-6}$ cycloalkyl, $C_{3-6}$ halocycloalkyl or 3- to 6-membered heterocyclyl, wherein the $C_{1-4}$ alkyl, $C_{1-4}$ haloalkyl, $C_{1-4}$ hydroxyalkyl, $C_{3-6}$ cycloalkyl, $C_{3-6}$ halocycloalkyl, and 3- to 6-membered heterocyclyl are all optionally substituted with one or more of the following substituents: halogen, hydroxyl, amino, $-SO_2-C_{1-4}$ alkyl or oxo; w is 0, 1, 2 or 3.

Further preferably, each of $R^4$ is independently hydrogen, cyano, halogen, amino, nitro, $C_{1-4}$ alkyl, $C_{1-4}$ haloalkyl or $C_{1-4}$ hydroxyalkyl, wherein the $C_{1-4}$ alkyl, $C_{1-4}$ haloalkyl, and $C_{1-4}$ hydroxyalkyl are all optionally substituted with one or more of the following substituents: halogen, hydroxyl or amino; w is 1, 2 or 3.

Further preferably, each of $R^4$ is independently hydrogen, cyano, halogen, amino, nitro, methyl, ethyl, n-propyl or isopropyl, wherein the methyl, ethyl, n-propyl, and isopropyl are all optionally substituted with one or more of the following substituents: halogen or hydroxyl; w is 1, 2 or 3.

Further preferably, each of $R^4$ is independently hydrogen, halogen, amino, cyano, methyl, ethyl or isopropyl, wherein the methyl, ethyl, and isopropyl are all optionally substituted with one or more of the following substituents: halogen or hydroxyl; w is 1, 2 or 3.

Further preferably, each of $R^4$ is independently hydrogen, fluorine, amino, cyano, methyl, trifluoromethyl, difluoromethyl, monofluoromethyl, $-CF_2CH_2OH$, $-C(CH_3)_2OH$, $-CF_2CH_3$ or $-CH_2CHF_2$; w is 1, 2 or 3.

The present disclosure provides a compound, or a stereoisomer, optical isomer, pharmaceutical salt, prodrug, solvate or isotope derivative thereof, wherein the compound has a structure represented by formula (C):

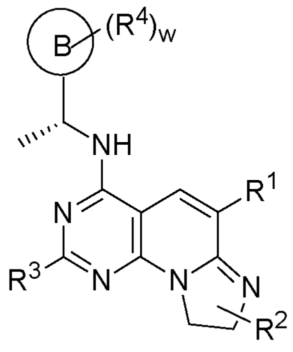

(C)

the substituents in formula (C) are as defined in formula (B).

The present disclosure further provides a compound, or a stereoisomer, optical isomer, pharmaceutical salt, prodrug, solvate or isotope derivative thereof, wherein the compound has a structure represented by formula (D):

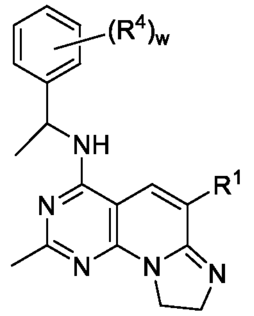

(D)

wherein $R^1$, $R^4$, and w are as defined in formula (A), (A'), (I), (II), (III), (IV), (B) or (C).

Preferably, each of $R^4$, if present, is independently cyano, halogen, amino, $C_{1-6}$ alkyl or $C_{1-6}$ haloalkyl, wherein the $C_{1-6}$ alkyl and $C_{1-6}$ haloalkyl are both optionally substituted with one or more hydroxyl; w is 1 or 2;

$R^1$ is $-O-R^A$, $-N(R^D)R^B$ or $R^C$;
when $R^1$ is $-O-R^A$, $R^A$ is 3- to 10-membered heterocyclyl, wherein the 3- to 10-membered heterocyclyl is optionally substituted with 1 to 3 identical or different $R^{a1}$;
each of $R^{a1}$, if present, is independently $C_{1-6}$ alkyl or $-COC_{1-6}$ alkyl;
when $R^1$ is $-N(R^D)R^B$, $R^B$ is $C_{1-6}$ alkyl or 3- to 10-membered heterocyclyl, wherein the $C_{1-6}$ alkyl and 3- to 10-membered heterocyclyl are both optionally substituted with 1 to 3 identical or different $R^{b1}$;
each of $R^{b1}$, if present, is independently $-OC_{1-6}$ alkyl;
$R^D$ is hydrogen;
when $R^1$ is $R^C$, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$;
each of $R^{c1}$, if present, is independently halogen, hydroxyl, cyano, amino, methylsulfonyl, $C_{1-6}$ alkyl, $-OC_{1-6}$ alkyl, $-COC_{1-6}$ alkyl, $-COC_{3-6}$ cycloalkyl, $-CH_2CON(C_{1-6}$ alkyl$)_2$ or 3- to 10-membered heterocyclyl, wherein the $C_{1-6}$ alkyl, $-OC_{1-6}$ alkyl, $-COC_{1-6}$ alkyl, $-COC_{3-6}$ cycloalkyl, $-CH_2CON(C_{1-6}$ alkyl$)_2$, and 3- to 10-membered heterocyclyl are all optionally substituted with one or more substituents selected from deuterium, hydroxyl, cyano, $C_{1-3}$ alkoxy or halogen.

Unless otherwise stated, heteroatoms in the heteroaryl and heterocyclyl are each independently O, N or S, and the number of heteroatoms is 1, 2, 3 or 4.

In one preferred embodiment of the present disclosure, $R^1$ is $-O-R^A$, $R^A$ is 3- to 6-membered heterocyclyl, wherein the 3- to 6-membered heterocyclyl is optionally substituted with 1 to 3 identical or different $R^{a1}$;
each of $R^{a1}$, if present, is independently $C_{1-6}$ alkyl or $-COC_{1-6}$ alkyl.

Further preferably, $R^A$ is 5- to 6-membered heterocyclyl, wherein the 5- to 6-membered heterocyclyl is optionally substituted with one or two identical or different $R^{a1}$;
each of $R^{a1}$, if present, is independently acetyl, methyl, ethyl, n-propyl or isopropyl.

Further preferably, $R^A$ is 5- to 6-membered monocyclic heterocyclyl, wherein the 5- to 6-membered monocyclic

heterocyclyl is optionally substituted with one or two identical or different $R^{a1}$; heteroatoms in the 5- to 6-membered monocyclic heterocyclyl are each independently O, N or S, and the number of heteroatoms is 1;
each of $R^{a1}$, if present, is independently acetyl, methyl or ethyl.

Further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;
each of $R^{a1}$, if present, is independently acetyl, methyl or ethyl.

Even further preferably, $R^A$ is the following group:

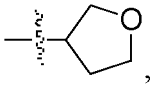, 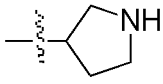,

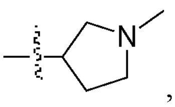, 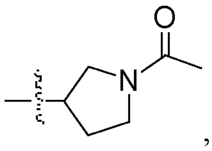, 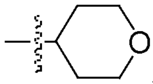, 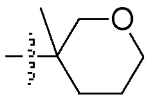 or 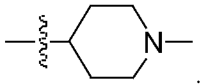.

In one preferred embodiment of the present disclosure, $R^1$ is $-N(R^D)R^B$, $R^B$ is $C_{1-6}$ alkyl or 3- to 6-membered monocyclic heterocyclyl, wherein the $C_{1-6}$ alkyl and 3- to 6-membered monocyclic heterocyclyl are both optionally substituted with one or two identical or different $R^{b1}$; and heteroatoms in the 3- to 6-membered monocyclic heterocyclyl are each independently O, N or S, and the number of heteroatoms is 1;
each of $R^{b1}$, if present, is independently $-OC_{1-4}$ alkyl.

Further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;
each of $R^{b1}$, if present, is independently methoxy or ethoxy.

Even further preferably, $R^B$ is the following group:

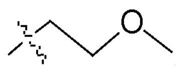, 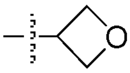 or

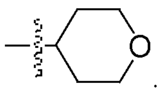.

In one preferred embodiment of the present disclosure, $R^1$ is $R^C$, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$;
each of $R^{c1}$, if present, is independently halogen, hydroxyl, cyano, amino, methylsulfonyl, $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$ or 3- to 6-membered heterocyclyl, wherein the $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$, and 3- to 6-membered heterocyclyl are all optionally substituted with one or more substituents selected from deuterium, hydroxyl, cyano, methoxy or halogen.

Further preferably, $R^C$ is 5- to 6-membered monocyclic heterocyclyl, 6- to 10-membered spiro heterocyclyl, 6- to 8-membered bridged heterocyclyl, 8- to 10-membered fused heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, 8- to 10-membered fused heterocyclyl, and 5-to 6-membered monocyclic heteroaryl are all optionally substituted with 1 to 4 identical or different $R^{c1}$;
each of $R^{c1}$, if present, is independently halogen, hydroxyl, cyano, amino, methylsulfonyl, methyl, ethyl, n-propyl, isopropyl, methoxy, ethoxy, acetyl, propionyl, -CO-cyclopropyl, -CO-cyclobutyl, $-CH_2CON(CH_3)_2$, 4-membered heterocyclyl, 5-membered heterocyclyl or 6-membered heterocyclyl, wherein the methylsulfonyl, methyl, ethyl, n-propyl, isopropyl, methoxy, ethoxy, acetyl, propionyl, -CO-cyclopropyl, -CO-cyclobutyl, $-CH_2CON(CH_3)_2$, 4-membered heterocyclyl, 5-membered heterocyclyl, and 6-membered heterocyclyl are all optionally substituted with one or more substituents selected from deuterium, hydroxyl, cyano, methoxy or halogen.

Even further preferably, $R^C$ is 6-membered monocyclic heterocyclyl, 4-membered/6-membered spiro heterocyclyl, 4-membered/4-membered spiro heterocyclyl, 7-membered bridged heterocyclyl, 6-membered/5-membered fused heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 7-membered bridged heterocyclyl, 6-membered/4-membered fused heterocyclyl, and 6-membered monocyclic heteroaryl are all optionally substituted with 1 to 4 identical or different $R^{c1}$;

each of $R^{c1}$, if present, is independently halogen, hydroxyl, cyano, amino, methylsulfonyl, methyl, ethyl, n-propyl, isopropyl, methoxy, ethoxy, acetyl, propionyl, -CO-cyclopropyl, $-CH_2CON(CH_3)_2$, 4-membered heterocyclyl, 5-membered heterocyclyl or 6-membered heterocyclyl, wherein the methylsulfonyl, methyl, ethyl, n-propyl, isopropyl, methoxy, ethoxy, acetyl, propionyl, -CO-cyclopropyl, 4-membered heterocyclyl, 5-membered heterocyclyl, and 6-membered heterocyclyl are all optionally substituted with one or more substituents selected from deuterium, hydroxyl, cyano, methoxy or halogen.

Further preferably, $R^C$ is

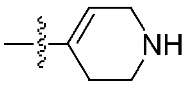, 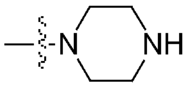, 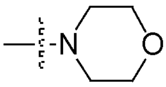,

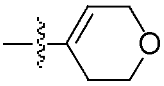, 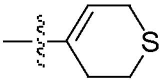, 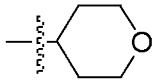, 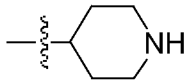, 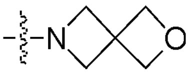,

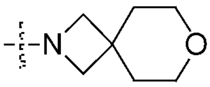, 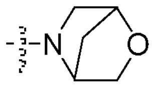, 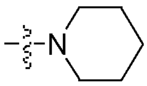, 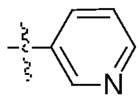, 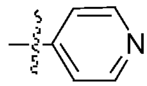, 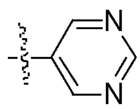

or

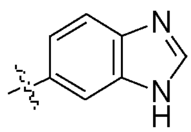,

and the $R^C$ are all optionally substituted with 1 to 4 identical or different $R^{c1}$;

each of $R^{c1}$, if present, is independently F, Cl, Br, hydroxyl, cyano, amino, methylsulfonyl, methyl, ethyl, isopropyl, $CD_3$, hydroxymethyl, hydroxyethyl (for example, 2-hydroxyethyl), $-CH(OH)(CH_3)_2$, $-CH_2OCH_3$, $-CH_2CH_2OCH_3$, monofluoromethyl, difluoromethyl, trifluoromethyl, monofluoroethyl (for example, 2-fluoroethyl), difluoroethyl (for example, 2,2-difluoroethyl), trifluoroethyl (for example, 2,2,2-trifluoroethyl), methoxy, acetyl, $-COCH_2CH_3$, $-COCH_2OH$, $-COCH_2CN$,

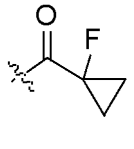,

$-CH_2CON(CH_3)_2$, oxetanyl (for example,

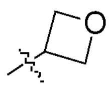

) or morpholinyl (for example, morpholin-4-yl).

Even further preferably, $R^C$ optionally substituted with $R^{c1}$ is the following group:

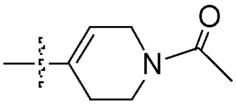 , 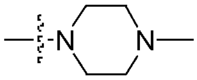 , 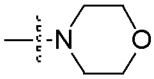 , 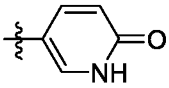 , 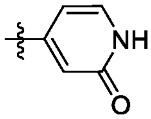 ,

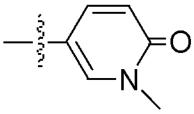 , 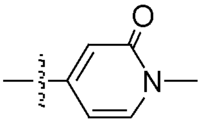 , 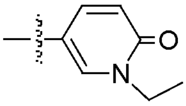 , 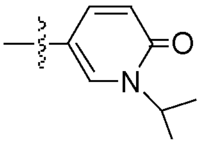 , 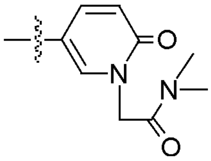 ,

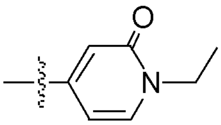 , 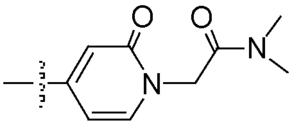 , 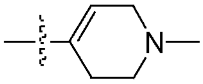 , 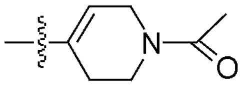 ,

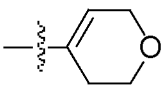 , 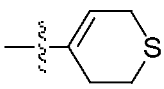 , 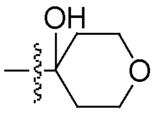 , 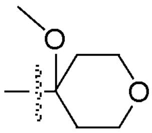 , 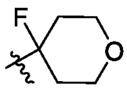 , 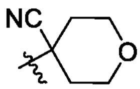 ,

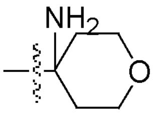 , 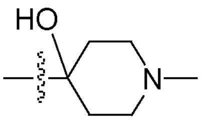 , 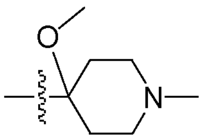 , 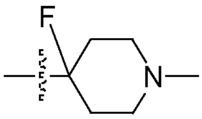 , 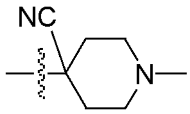 ,

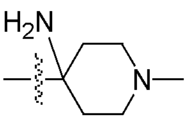 , 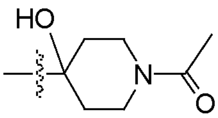 , 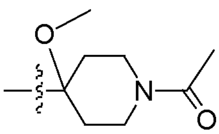 , 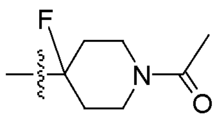 ,

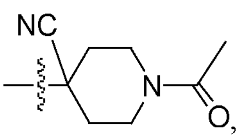 , 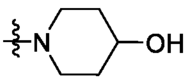 , 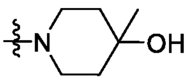 , 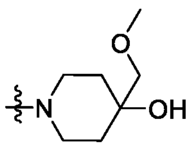 , 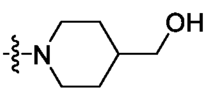 ,

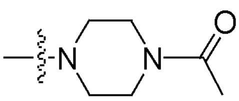 , 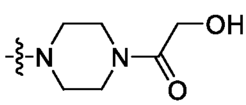 , 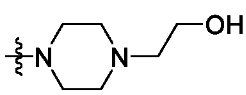 , 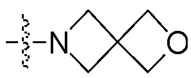 ,

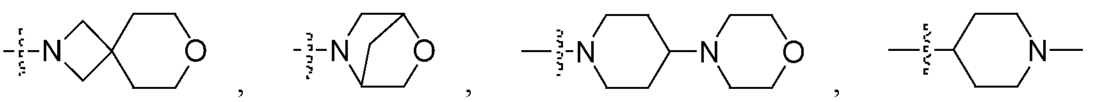 , , , ,

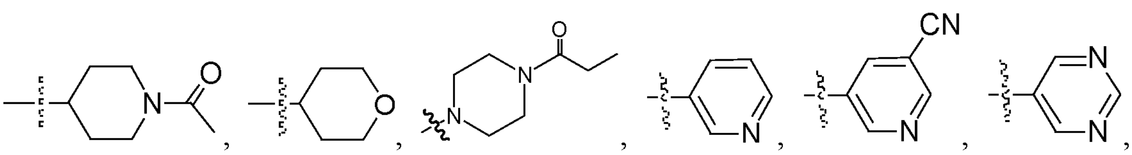 , , , , , ,

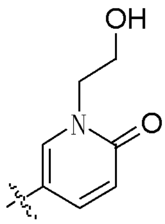, 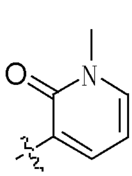, 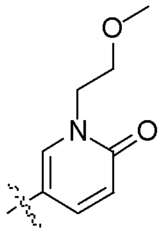, 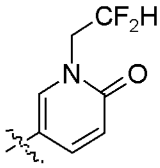, 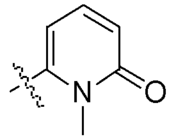, 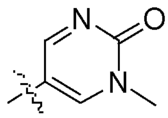,

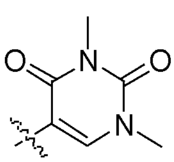, 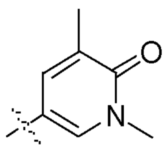, 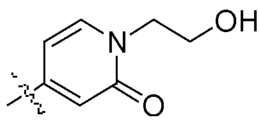, 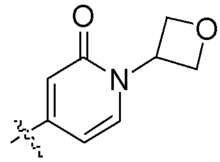, 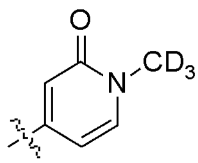,

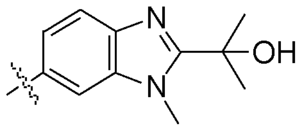, 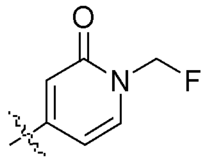, 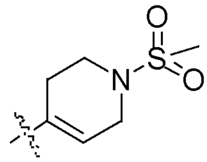, 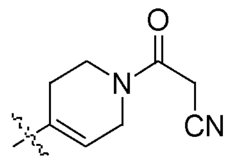,

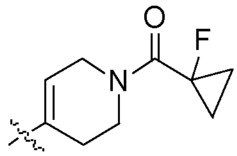 or 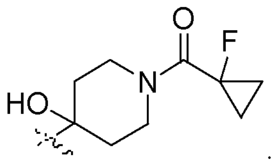.

In one preferred embodiment of the present disclosure, each of $R^4$, if present, is independently cyano, halogen, amino, $C_{1-4}$ alkyl or $C_{1-4}$ haloalkyl, wherein the $C_{1-4}$ alkyl and $C_{1-4}$ haloalkyl are both optionally substituted with one or more hydroxyl; w is 1 or 2.

Further preferably, each of $R^4$ is independently cyano, fluorine, amino, methyl, trifluoromethyl, difluoromethyl, monofluoromethyl, $-CF_2CH_2OH$, $-CF_2C(CH_3)_2OH$, $-CF_2CH_3$ or $-CH_2CHF_2$; w is 1 or 2.

The present disclosure provides a compound, or a stereoisomer, optical isomer, pharmaceutical salt, prodrug, solvate or isotope derivative thereof, wherein the compound has a structure represented by formula (E):

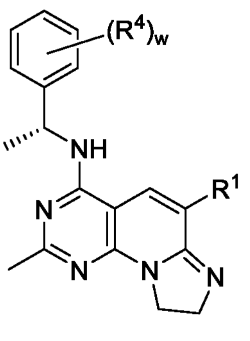

(E)

the substituents in formula (E) are as defined in formula (D).

The present disclosure further provides a compound, or a stereoisomer, optical isomer, pharmaceutical salt, prodrug, solvate or isotope derivative thereof, wherein the compound has a structure represented by formula (F) or formula (F'):

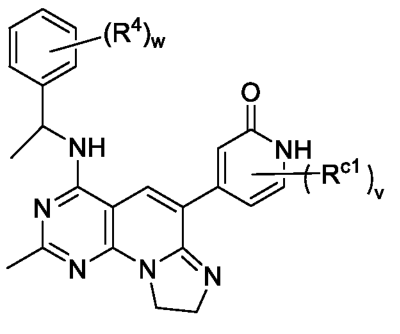

F (F)

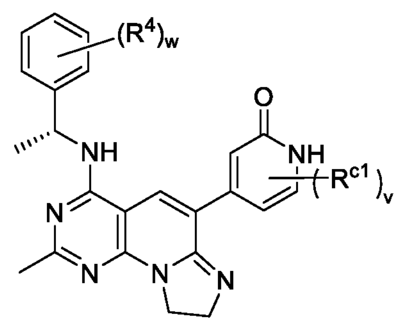

F' (F')

wherein $R^{c1}$, $R^4$, and w are as defined in formula (D) or (E); v is 0 or 1, and when v is 1, $R^{c1}$ is preferably linked to the N atom.

Preferably, $R^{c1}$ is methyl, ethyl, isopropyl, $CD_3$, hydroxymethyl, hydroxyethyl (for example, 2-hydroxyethyl), monofluoromethyl, difluoromethyl, trifluoromethyl, monofluoroethyl (for example, 2-fluoroethyl), difluoroethyl (for example, 2,2-difluoroethyl), trifluoroethyl (for example, 2,2,2-trifluoroethyl), $-CH_2CON(CH_3)_2$ or oxetanyl (for example,

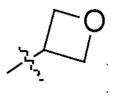

).

More preferably,

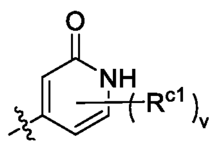

is the following group:

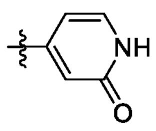

,

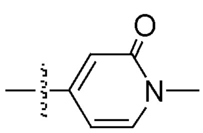

,

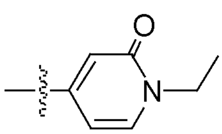

,

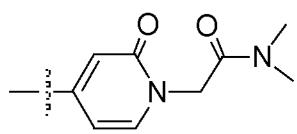

,

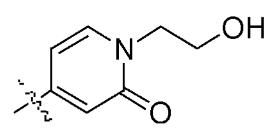

,

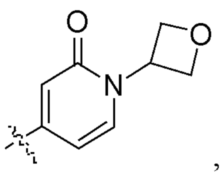

,

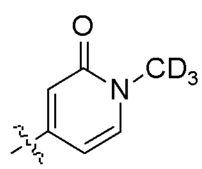

or

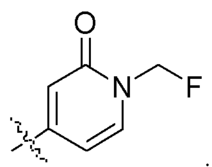

.

The present disclosure further provides a compound, or a stereoisomer, optical isomer, pharmaceutical salt, prodrug, solvate or isotope derivative thereof, wherein the compound has a structure represented by formula (G) or formula (G'):

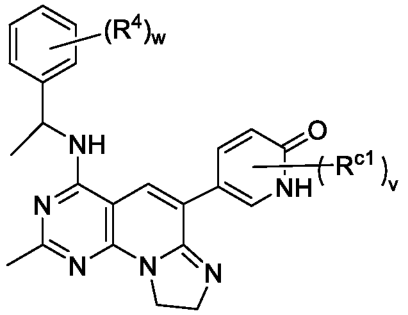 (G) 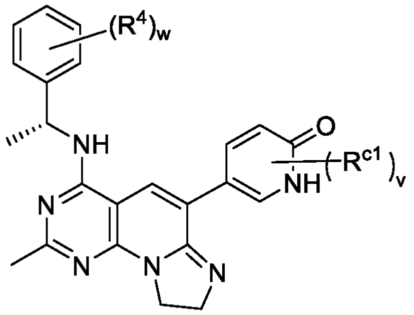 (G')

wherein $R^{c1}$, $R^4$, and w are as defined in formula (D) or (E); v is 0 or 1, and when v is 1, $R^{c1}$ is preferably linked to the N atom.

Preferably, $R^{c1}$ is methyl, ethyl, isopropyl, hydroxymethyl, hydroxyethyl (for example, 2-hydroxyethyl), $-CH_2OCH_3$, $-CH_2CH_2OCH_3$, monofluoromethyl, difluoromethyl, trifluoromethyl, monofluoroethyl (for example, 2-fluoroethyl), difluoroethyl (for example, 2,2-difluoroethyl), trifluoroethyl (for example, 2,2,2-trifluoroethyl) or $-CH_2CON(CH_3)_2$.

More preferably,

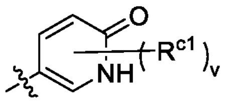

is the following group:

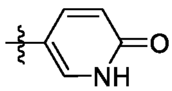,

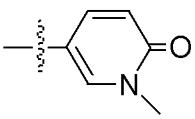, 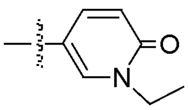, 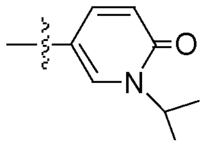, 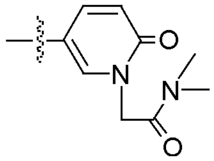, 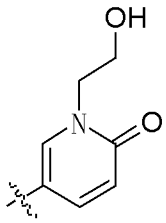,

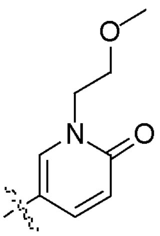 or 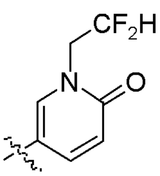.

Preferably, the present disclosure further provides a compound, or a stereoisomer, optical isomer, pharmaceutical salt, prodrug, solvate or isotope derivative thereof, wherein the compound has one of the following structures:

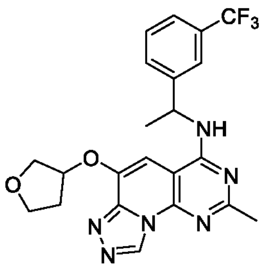
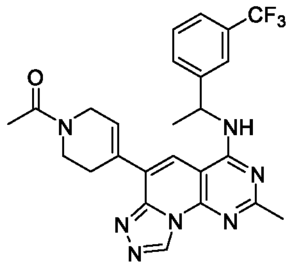
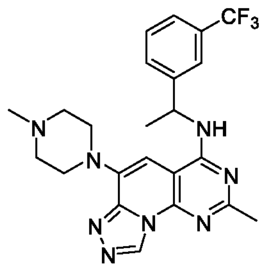
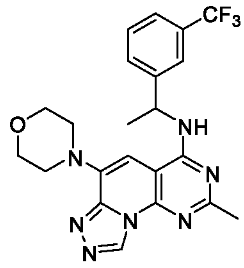

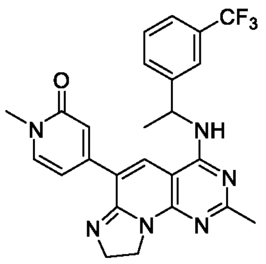
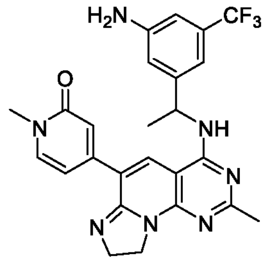
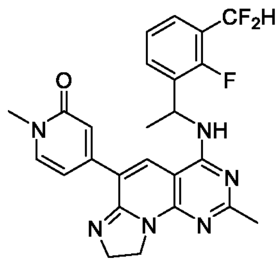
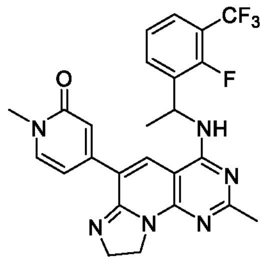

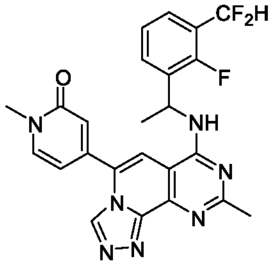
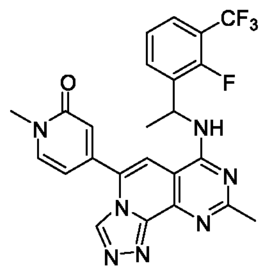
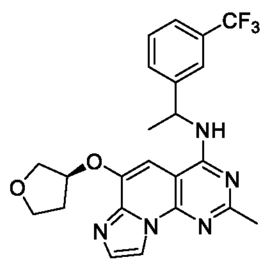
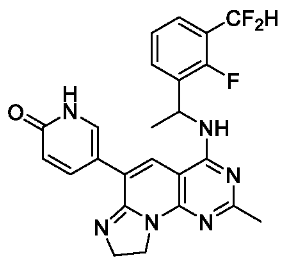

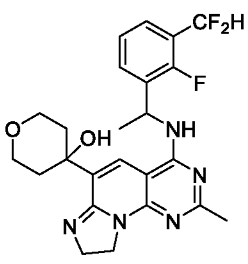
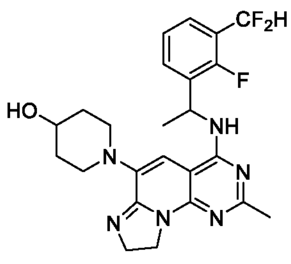
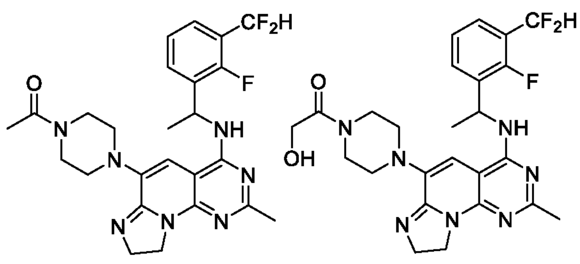

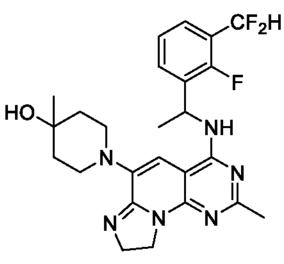
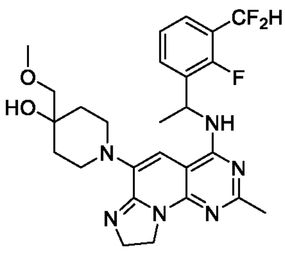
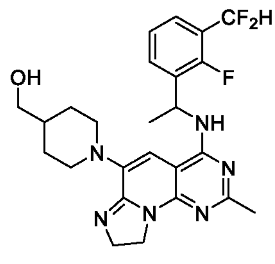
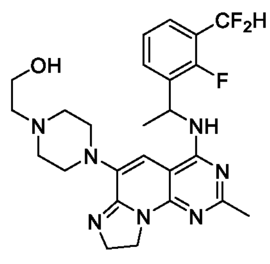

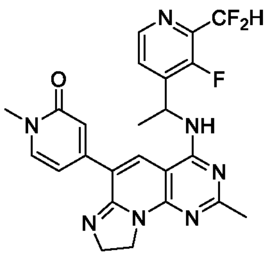
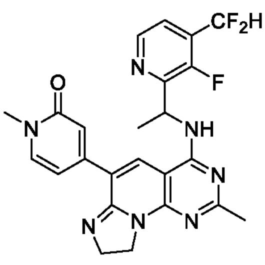
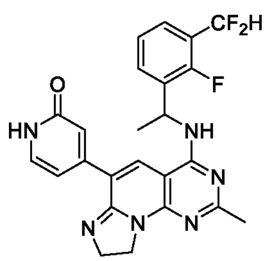
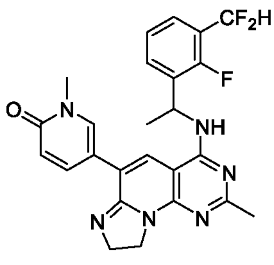

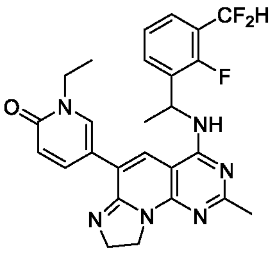
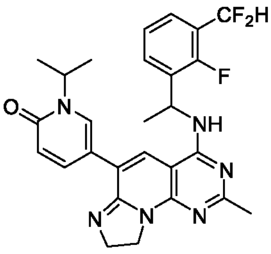
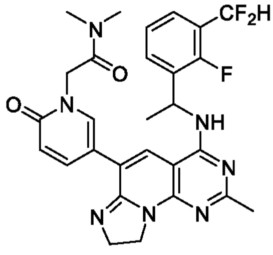
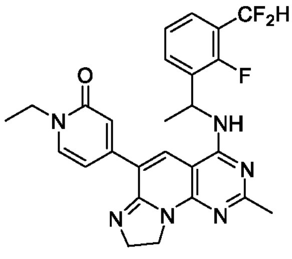

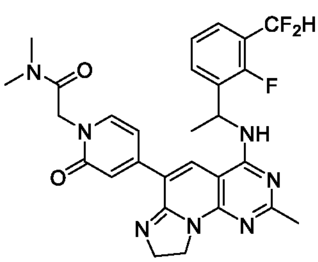
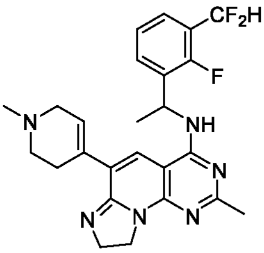
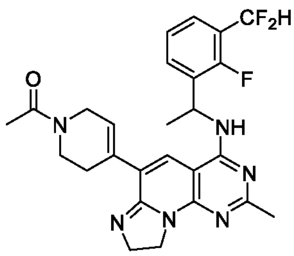
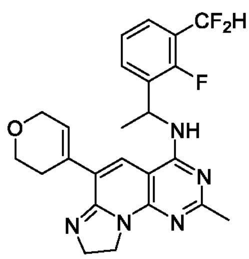

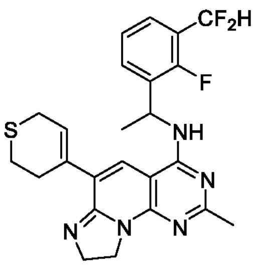
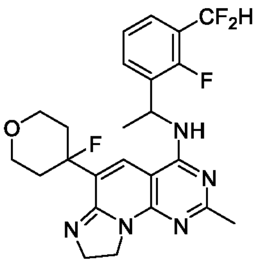
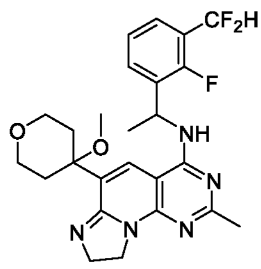
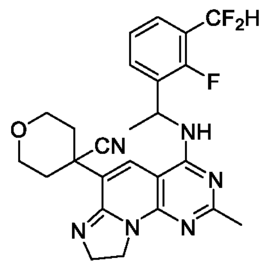

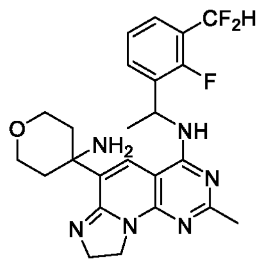
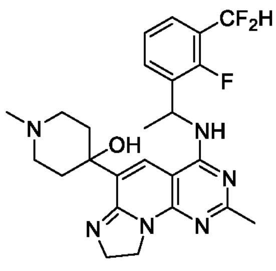
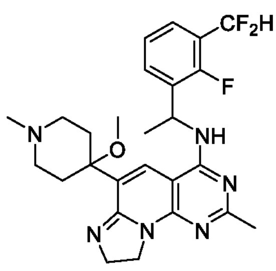
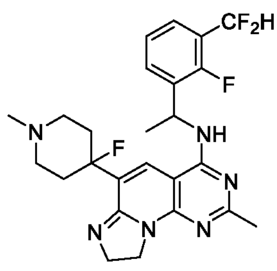

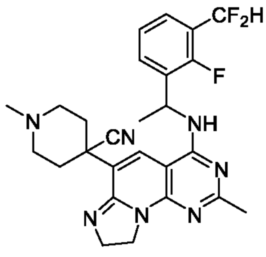
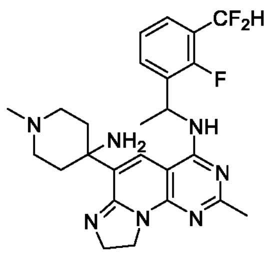
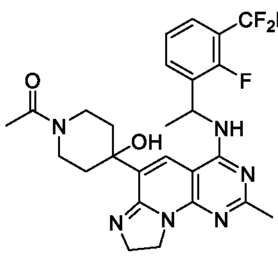
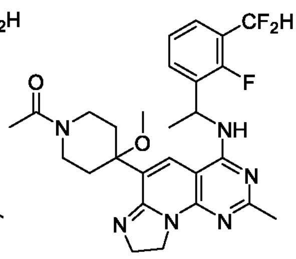

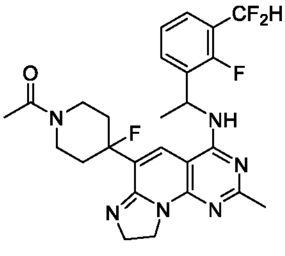
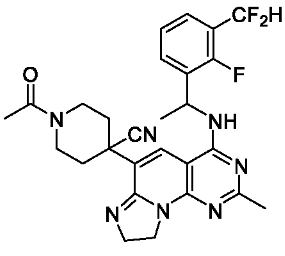
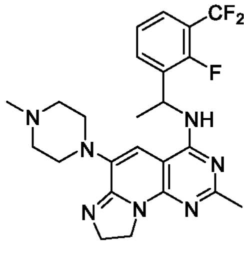
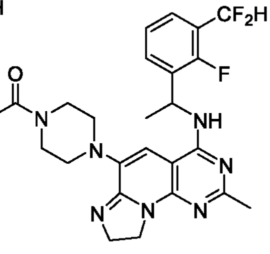

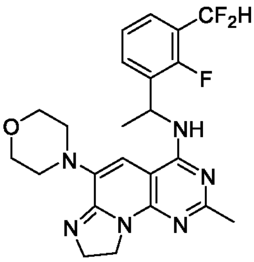
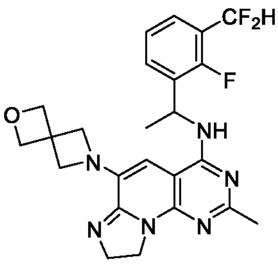
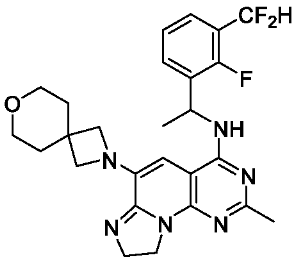
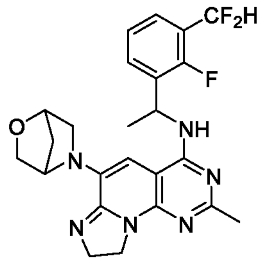

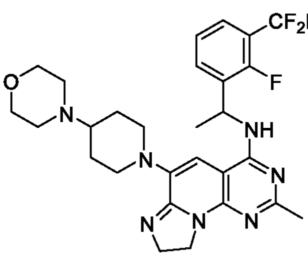
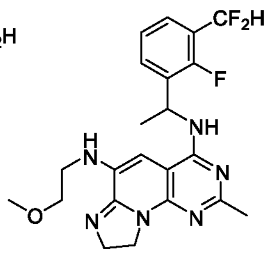
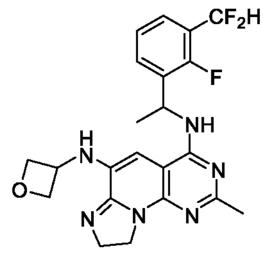
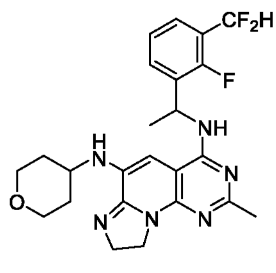

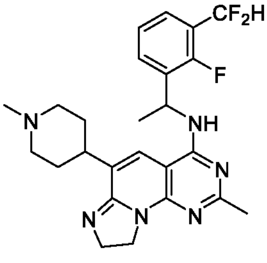
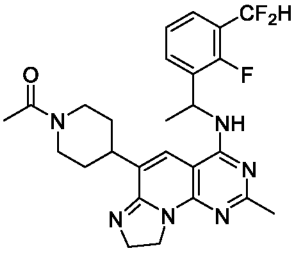
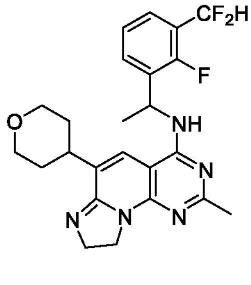
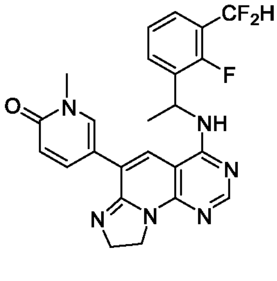

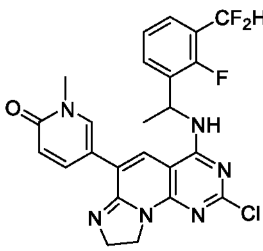
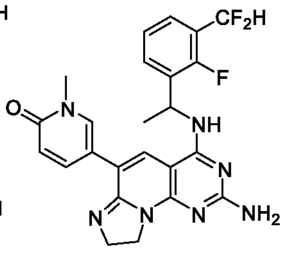
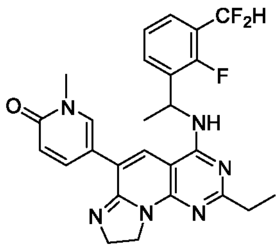
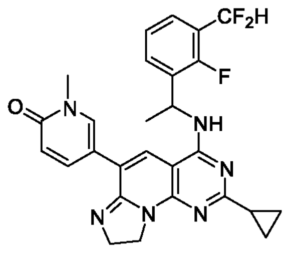

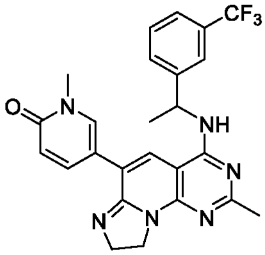
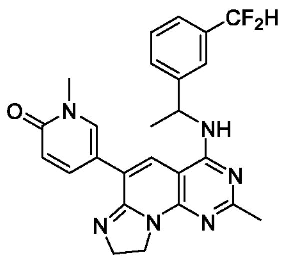
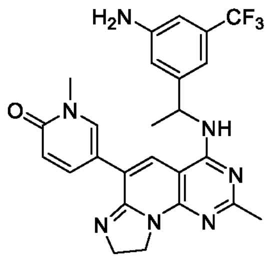
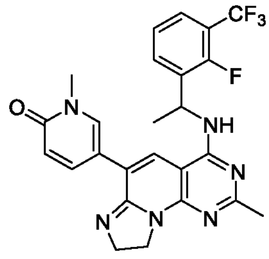

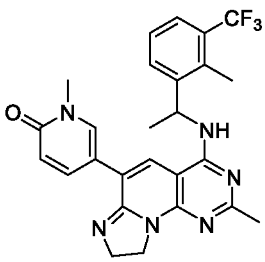
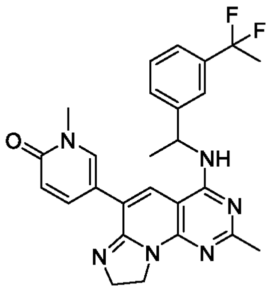
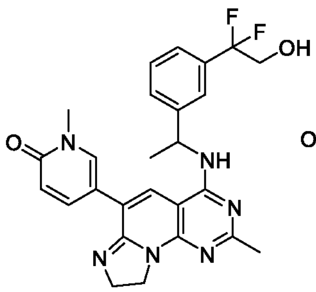
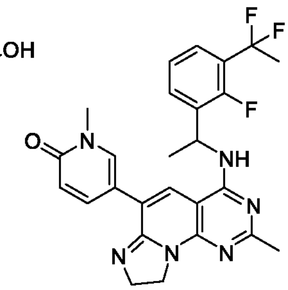

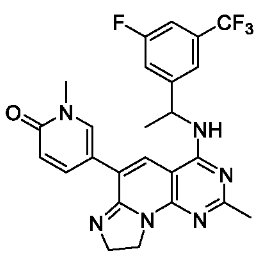
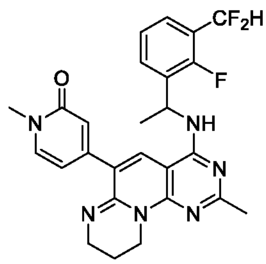
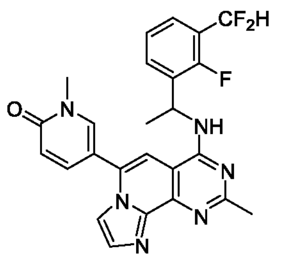
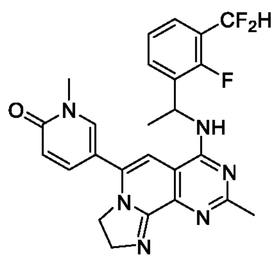

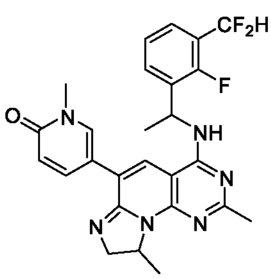
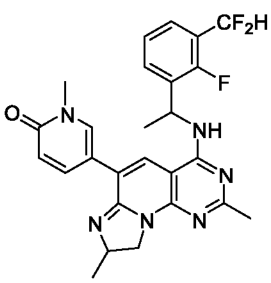
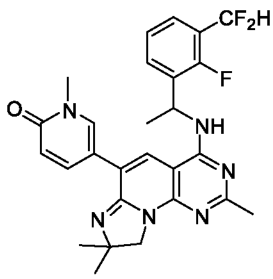
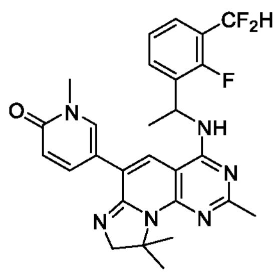

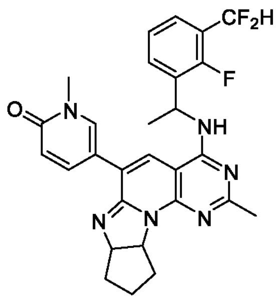
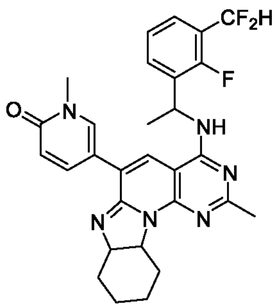
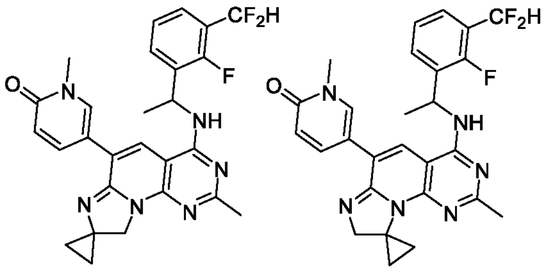

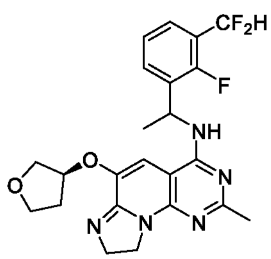
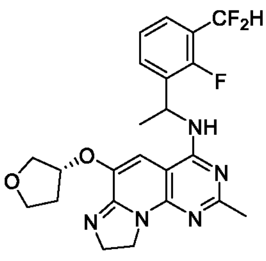
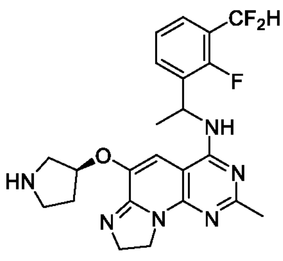
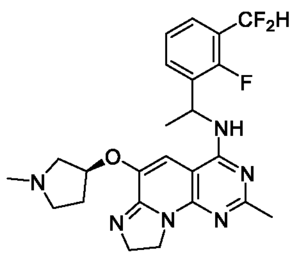

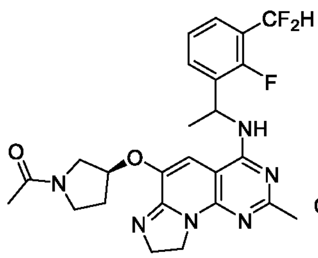
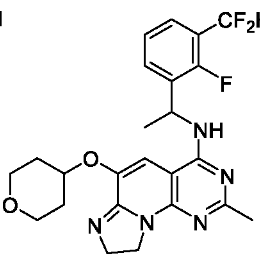
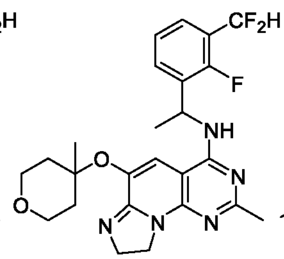
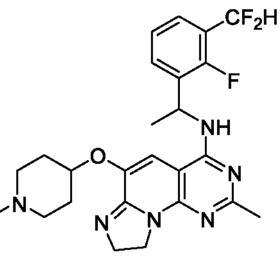

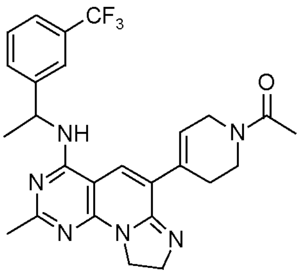
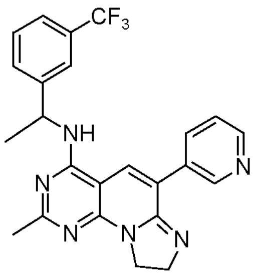
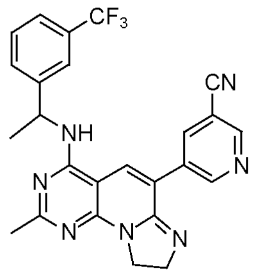
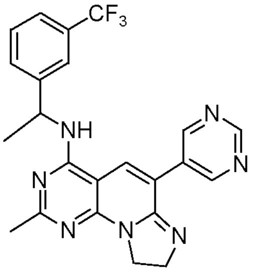

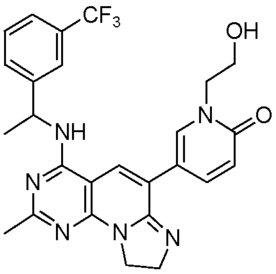
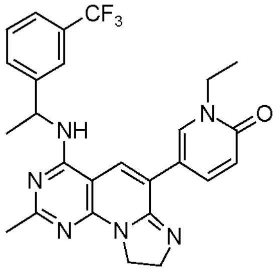
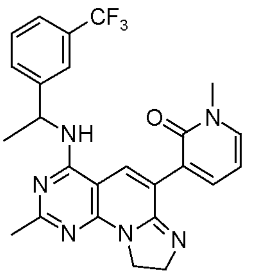
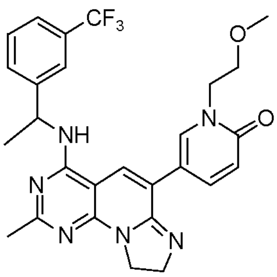

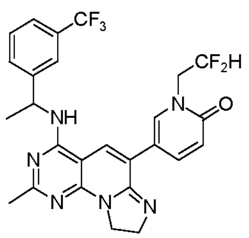
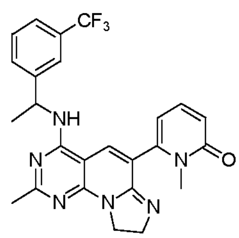
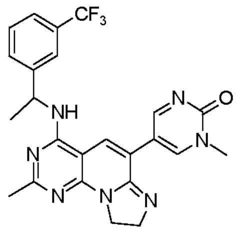
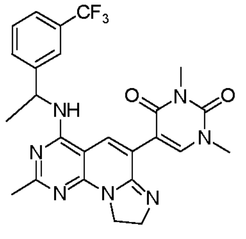

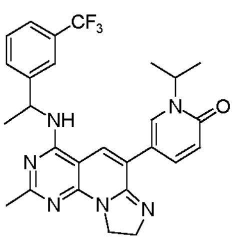
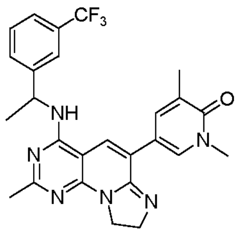
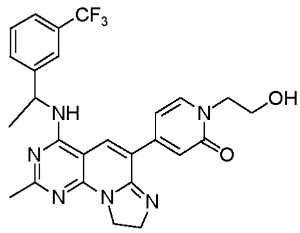
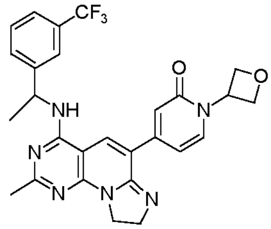

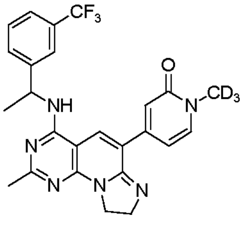
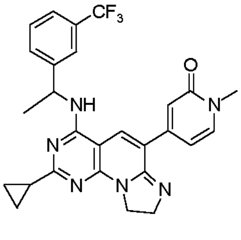
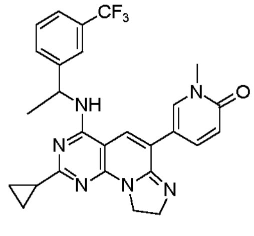
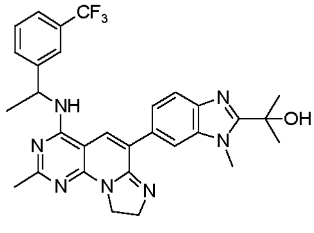

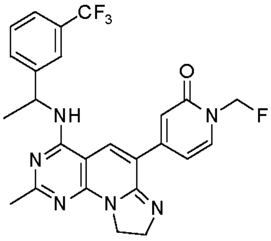
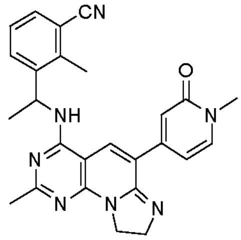
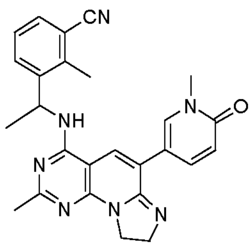
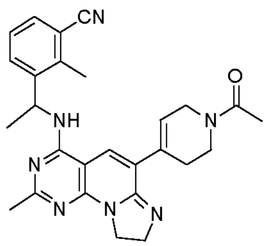

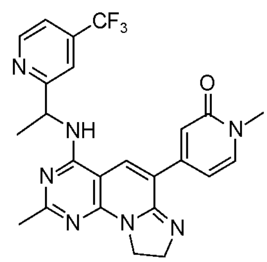
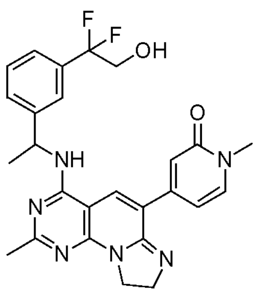
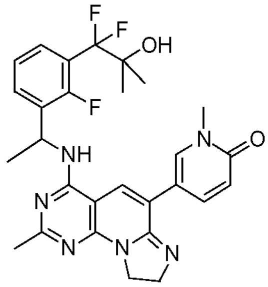
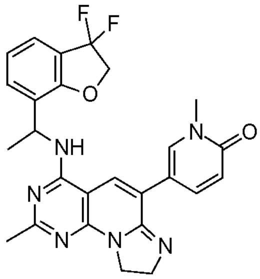

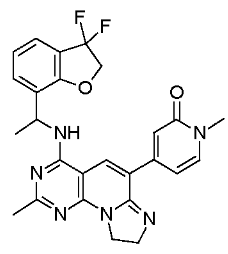
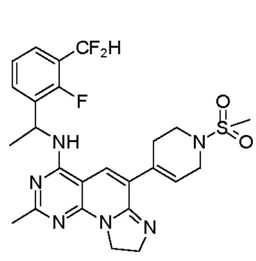
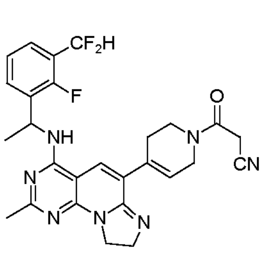
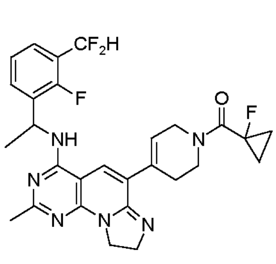

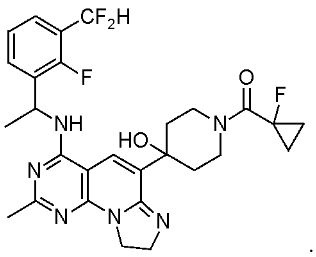

.

Preferably, the present disclosure provides a compound, or a stereoisomer, optical isomer, pharmaceutical salt, prodrug, solvate or isotope derivative thereof, wherein the compound has one of the following structures:

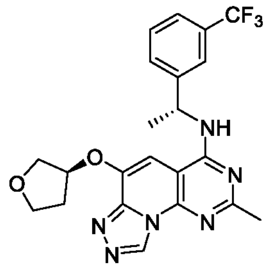

1

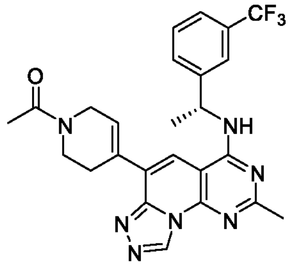

2

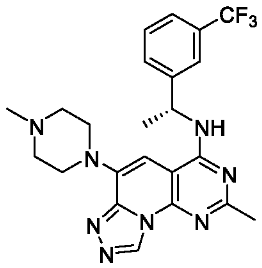

3

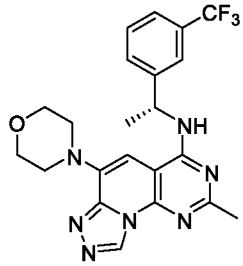

4

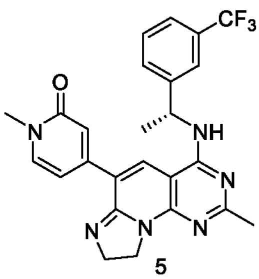

5

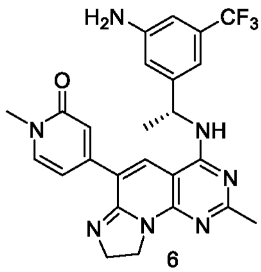

6

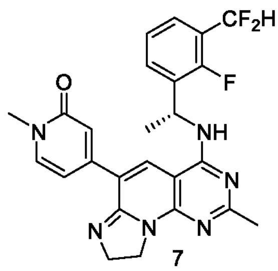

7

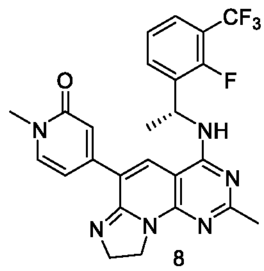

8

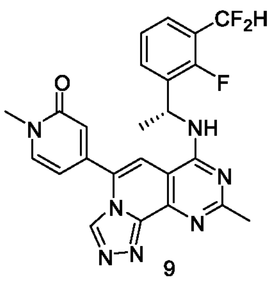

9

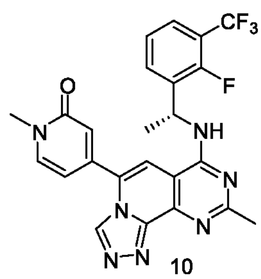

10

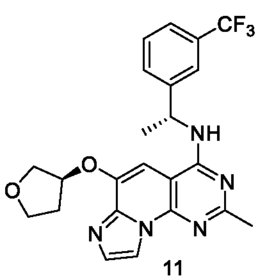

11

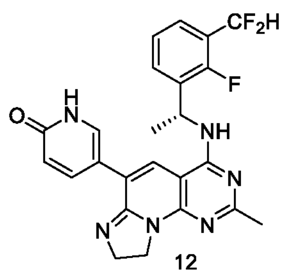

12

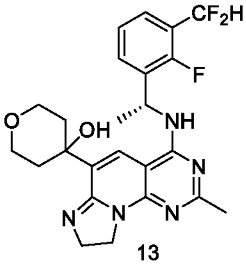

13

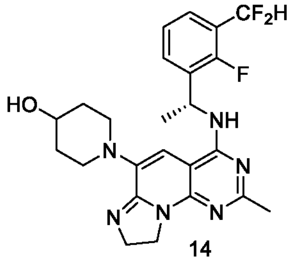

14

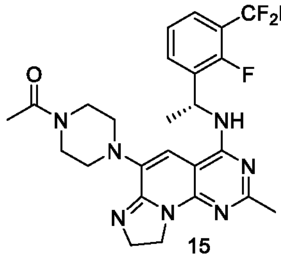

15

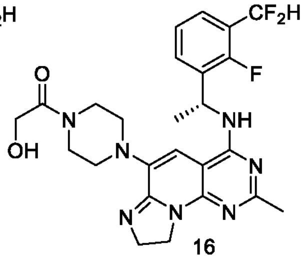

16

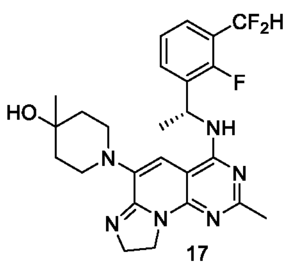
17

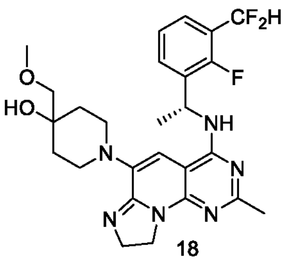
18

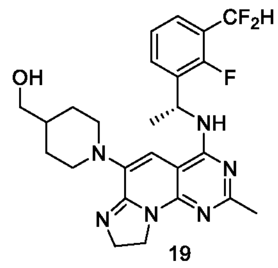
19

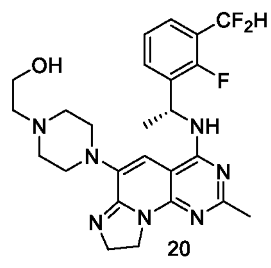
20

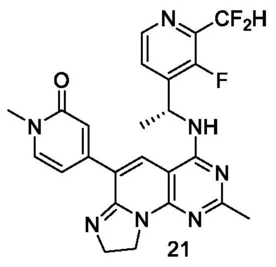
21

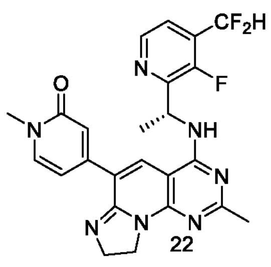
22

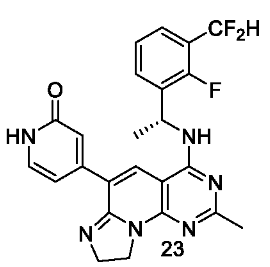
23

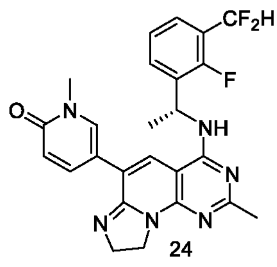
24

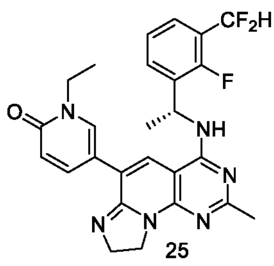
25

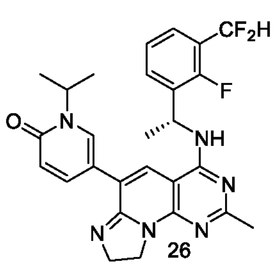
26

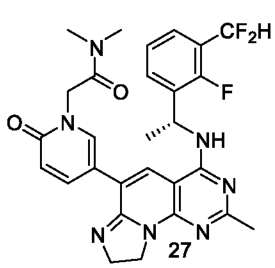
27

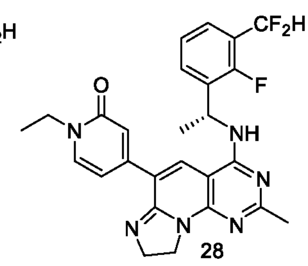
28

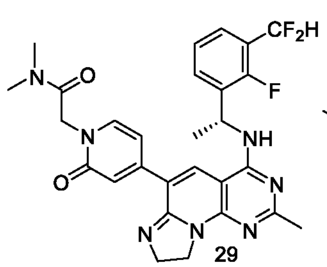
29

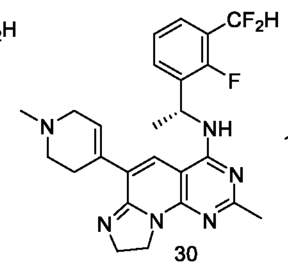
30

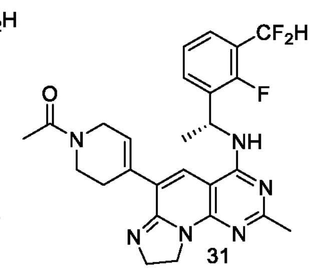
31

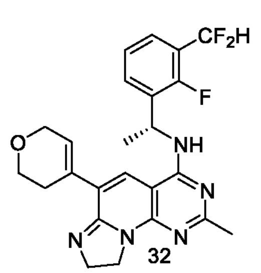
32

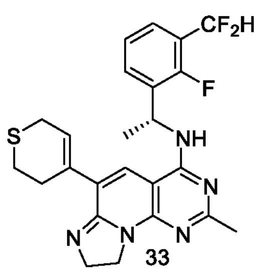
33

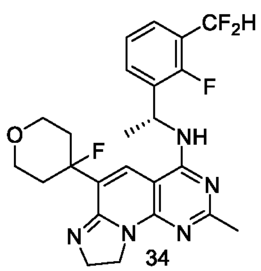
34

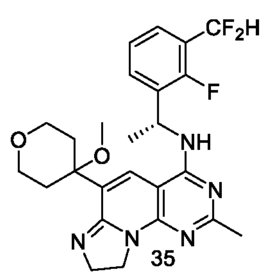
35

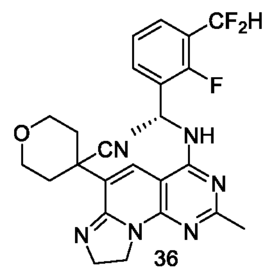
36

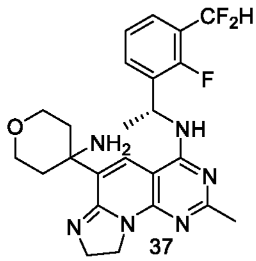
37

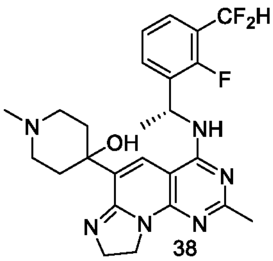
38

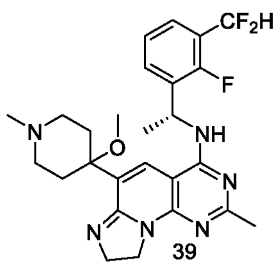
39

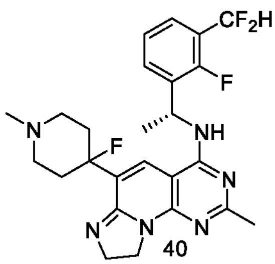
40

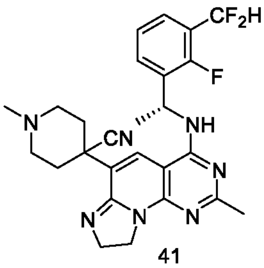
41

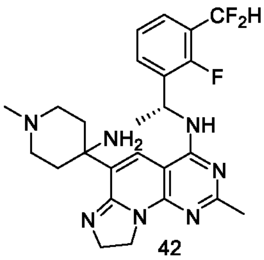
42

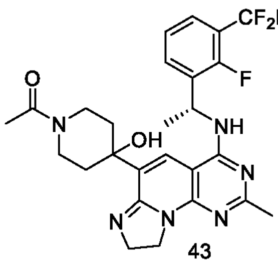
43

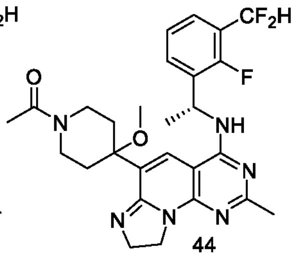
44

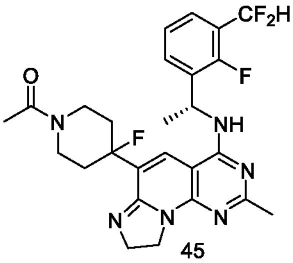
45

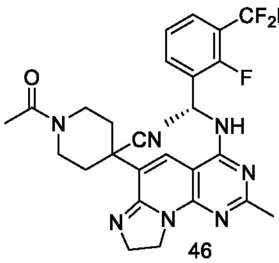
46

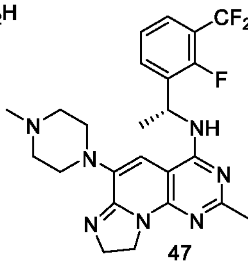
47

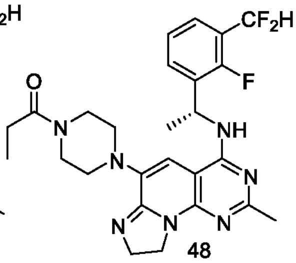
48

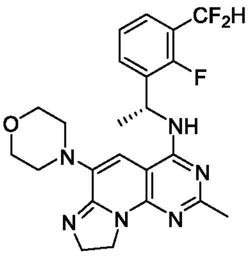
49

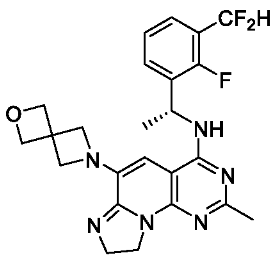
50

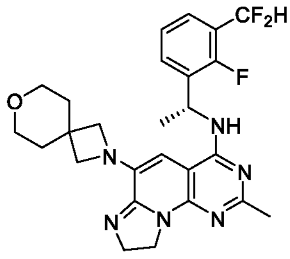
51

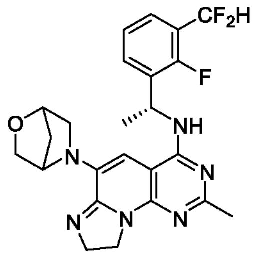
52

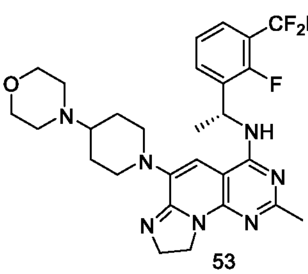
53

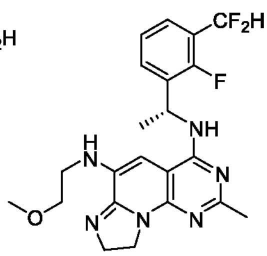
54

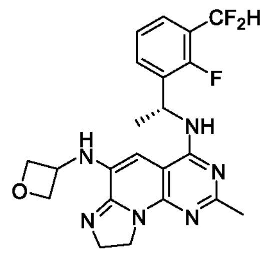
55

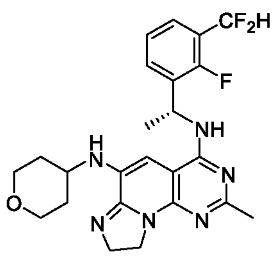
56

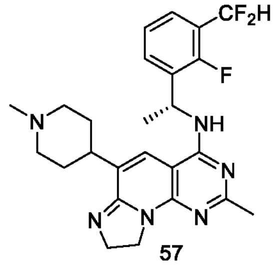
57

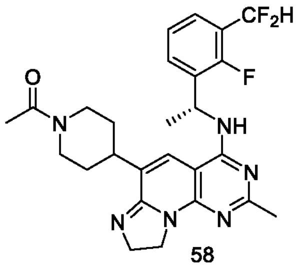
58

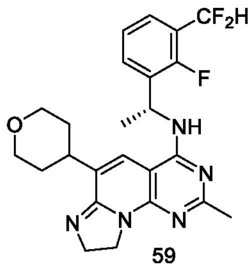
59

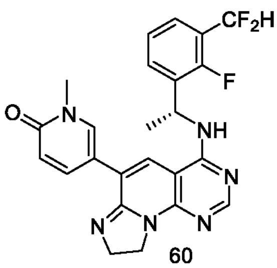
60

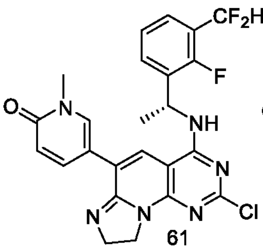
61

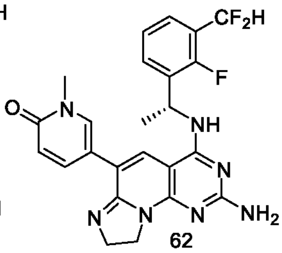
62

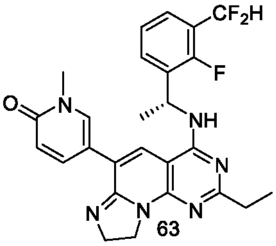
63

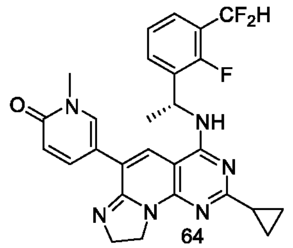
64

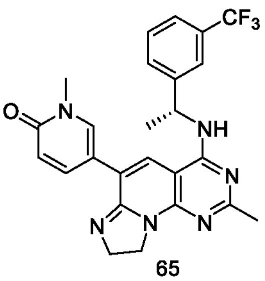
65

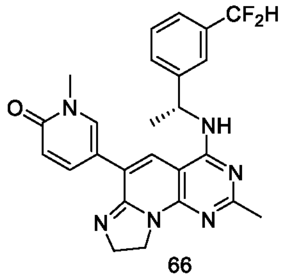
66

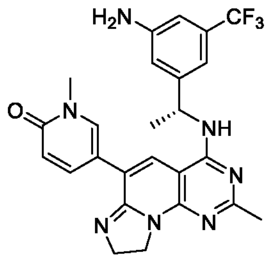
67

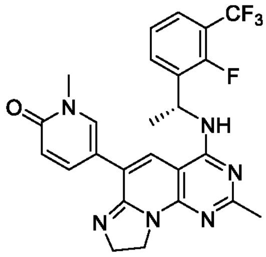
68

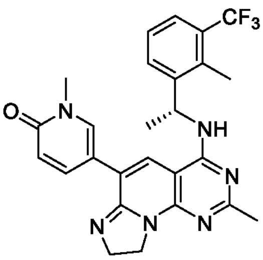
69

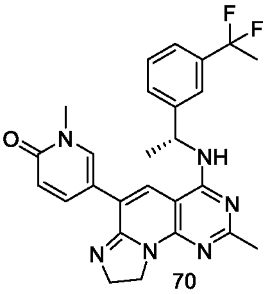
70

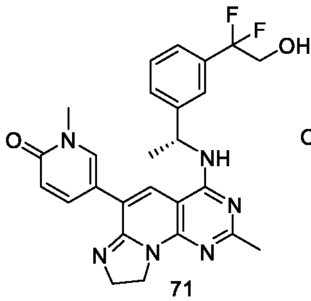
71

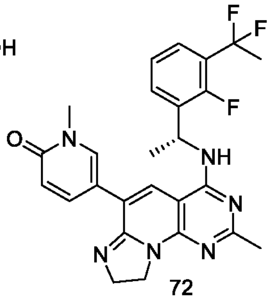
72

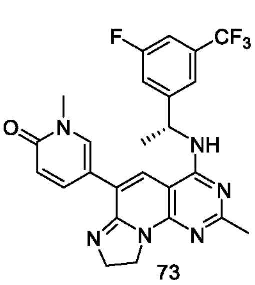
73

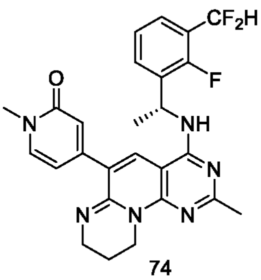
74

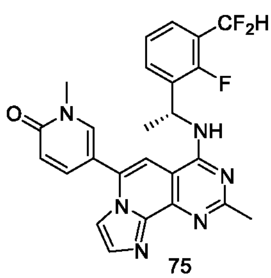
75

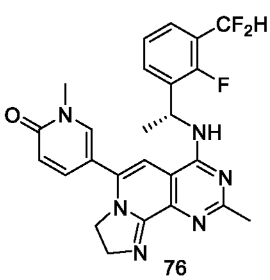
76

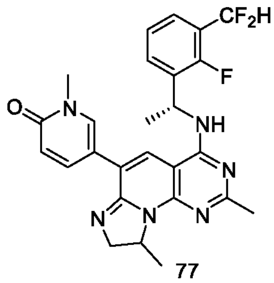
77

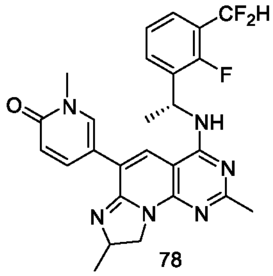
78

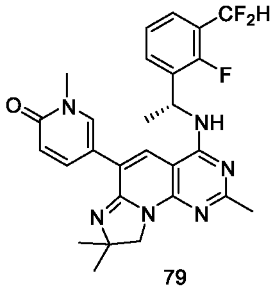
79

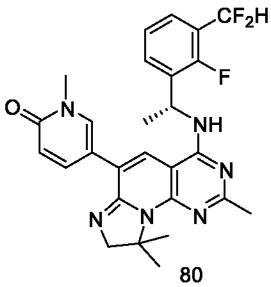
80

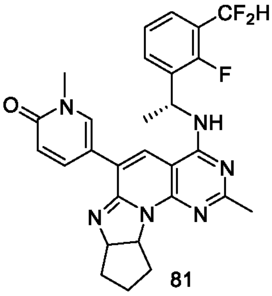
81

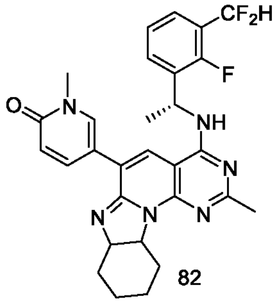
82

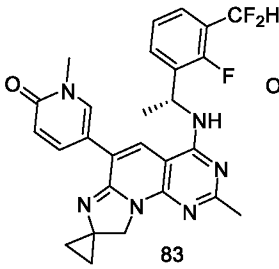
83

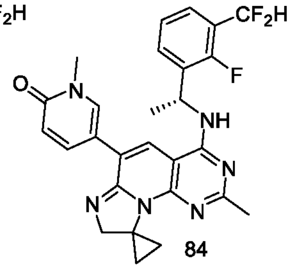
84

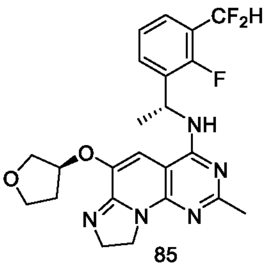
85

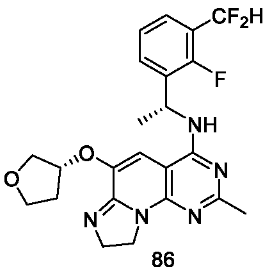
86

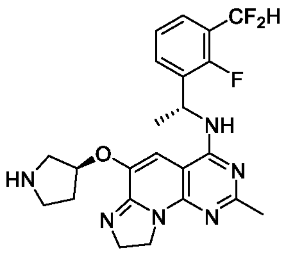
87

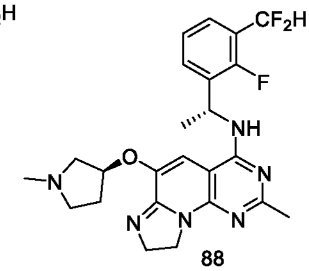
88

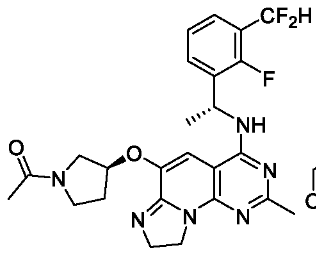
89

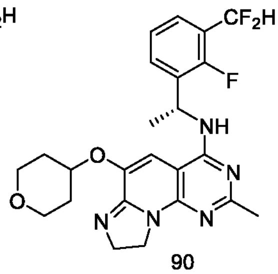
90

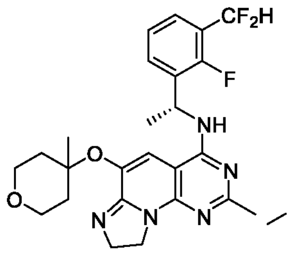
91

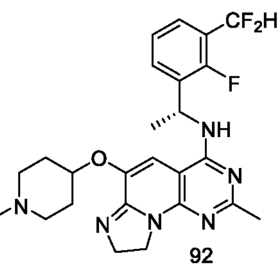
92

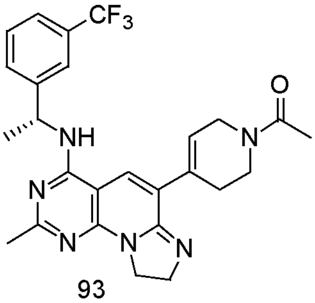
93

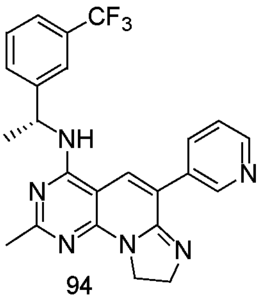
94

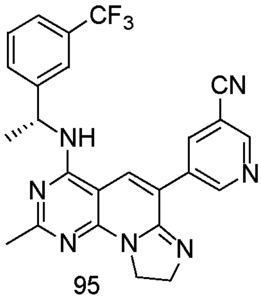
95

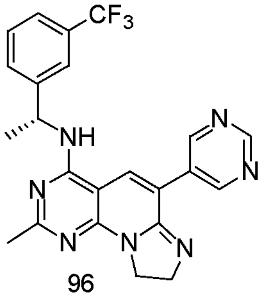
96

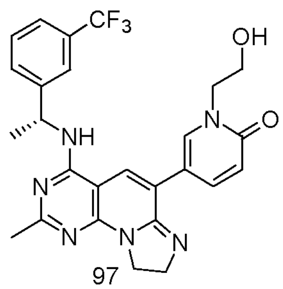
97

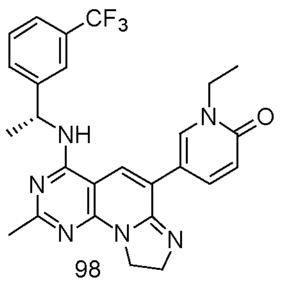
98

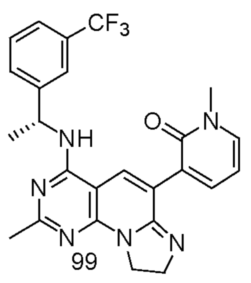
99

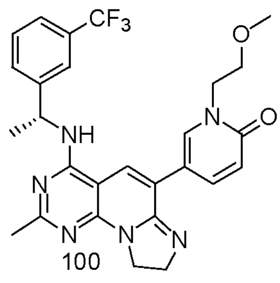
100

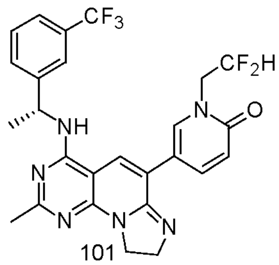
101

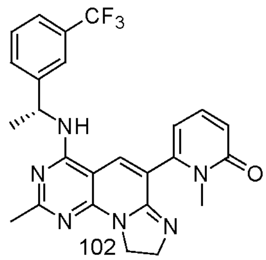
102

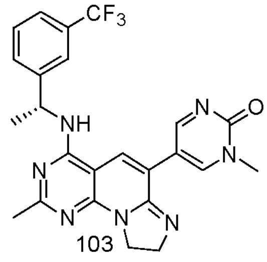
103

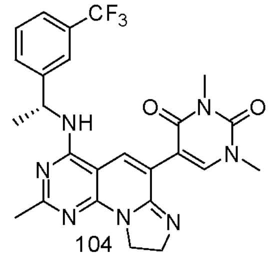
104

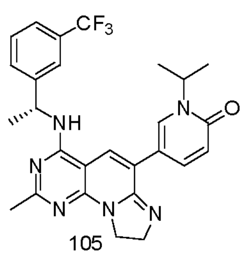
105

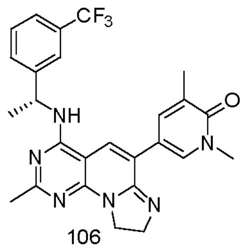
106

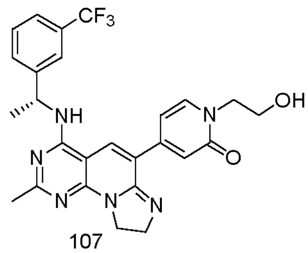
107

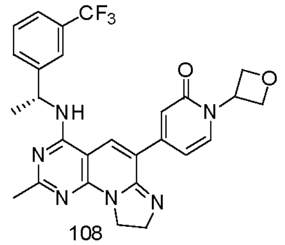
108

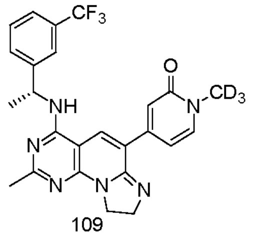
109

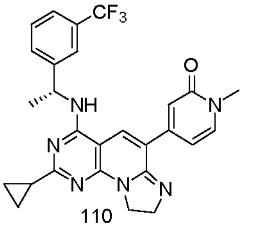
110

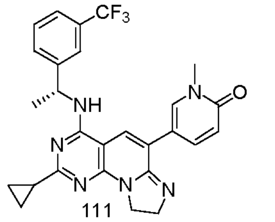
111

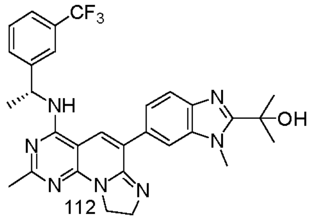
112

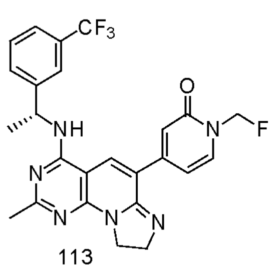
113

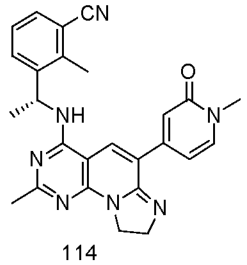
114

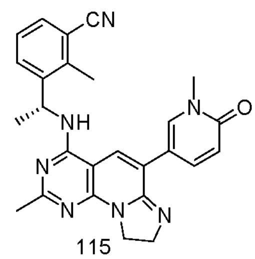
115

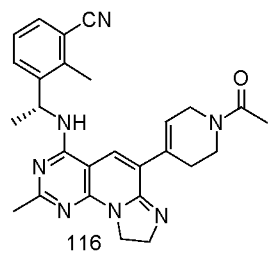
116

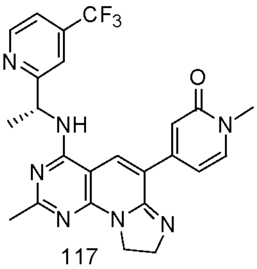

117

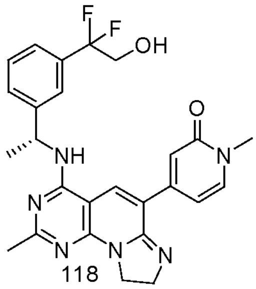

118

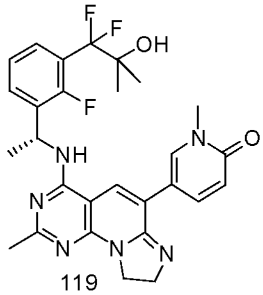

119

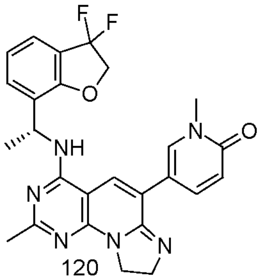

120

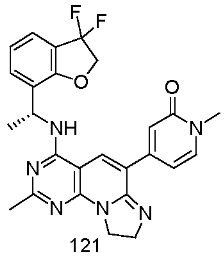

121

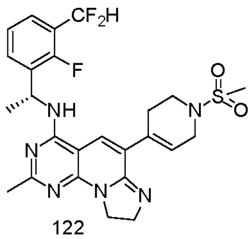

122

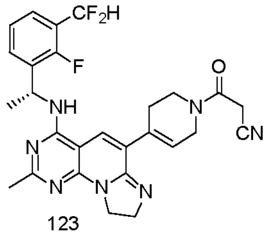

123

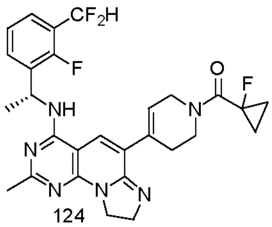

124

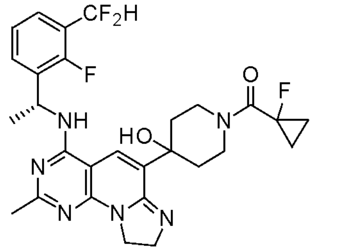

125

.

The present disclosure further aims to provide an intermediate for use in the manufacture of the compound or the stereoisomer, optical isomer, pharmaceutical salt, prodrug, solvate or isotope derivative thereof according to the present disclosure, wherein the intermediate is represented by formula (V):

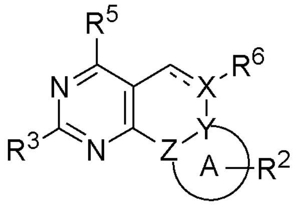

(V)

wherein $R^2$, $R^3$, ring A, X, Y, and Z are as defined in formula (A), (I) or (II);

$R^5$ is halogen, hydroxyl, -O-methylsulfonyl, -O-p-toluenesulfonyl or -O-trifluoromethylsulfonyl, preferably chlorine or hydroxyl.

$R^6$ is halogen, preferably bromine or iodine.

The present disclosure further aims to provide an intermediate for use in the manufacture of the compound or the stereoisomer, optical isomer, pharmaceutical salt, prodrug, solvate or isotope derivative thereof according to the present disclosure, wherein the intermediate is represented by formula (VI):

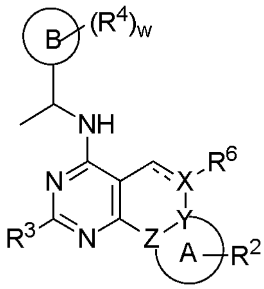

(VI)

wherein $R^2$, $R^3$, $R^4$, w, ring A, X, Y, and Z are as defined in formula (A) or (I);
$R^6$ is halogen, preferably bromine or iodine.

Furthermore, the compound or the stereoisomer, optical isomer, pharmaceutical salt, prodrug, solvate or isotope derivative thereof according to the present disclosure has a structure represented by formula (VII):

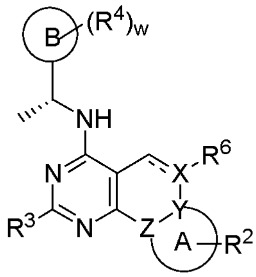

(VII)

wherein $R^2$, $R^3$, $R^4$, w, ring A, X, Y, and Z are as defined in formula (II);
$R^6$ is halogen, preferably bromine or iodine.

Furthermore, the compound or the stereoisomer, optical isomer, pharmaceutical salt, prodrug, solvate or isotope derivative thereof according to the present disclosure has a structure represented by formula (VIII):

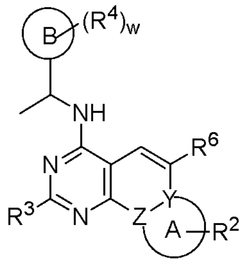

(VIII)

wherein $R^2$, $R^3$, $R^4$, w, ring A, Y, and Z are as defined in formula (III);
$R^6$ is halogen, preferably bromine or iodine.

Furthermore, the compound or the stereoisomer, optical isomer, pharmaceutical salt, prodrug, solvate or isotope derivative thereof according to the present disclosure has a structure represented by formula (IX):

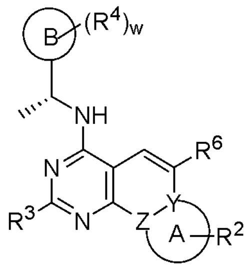
(IX)

wherein $R^2$, $R^3$, $R^4$, w, ring A, Y, and Z are as defined in formula (IV);
$R^6$ is halogen, preferably bromine or iodine.

The present disclosure further provides a pharmaceutical composition, comprising the compound or the stereoisomer, optical isomer, pharmaceutical salt, prodrug, solvate or isotope derivative thereof according to the present disclosure.

The present disclosure further provides a pharmaceutical composition, comprising the compound or the stereoisomer, optical isomer, pharmaceutical salt, prodrug, solvate or isotope derivative thereof according to the present disclosure, and a pharmaceutically acceptable excipient.

The present disclosure further aims to provide the compound or the stereoisomer, optical isomer, pharmaceutical salt, prodrug, solvate or isotope derivative thereof according to the present disclosure, or the pharmaceutical composition according to the present disclosure, for use as a medicament.

The present disclosure further aims to provide the compound or the stereoisomer, optical isomer, pharmaceutical salt, prodrug, solvate or isotope derivative thereof according to the present disclosure, or the pharmaceutical composition according to the present disclosure, for use in the prevention and/or treatment of a disease mediated by SOS1.

The present disclosure further aims to provide the compound or the stereoisomer, optical isomer, pharmaceutical salt, prodrug, solvate or isotope derivative thereof according to the present disclosure, or the pharmaceutical composition according to the present disclosure, for use in the prevention and/or treatment a disease caused by RAS mutations.

The present disclosure further aims to provide use of the compound or the stereoisomer, optical isomer, pharmaceutical salt, prodrug, solvate or isotope derivative thereof according to the present disclosure, or the pharmaceutical composition according to the present disclosure in the manufacture of a medicament for preventing and/or treating a disease mediated by SOS1.

In some examples, the disease mediated by SOS1 is cancer or a tumor, and a disease associated thereto.

In some examples, the disease mediated by SOS1 is lung cancer (for example, non-small cell lung cancer/NSCLC), and a disease associated thereto.

The present disclosure further aims to provide use of the compound or the stereoisomer, optical isomer, pharmaceutical salt, prodrug, solvate or isotope derivative thereof according to the present disclosure, or the pharmaceutical composition according to the present disclosure in the manufacture of a medicament for preventing and/or treating a disease caused by RAS mutations.

In some examples, the disease caused by the RAS mutations is cancer or a tumor, and a disease associated thereto.

In some examples, the disease caused by the RAS mutations is lung cancer (for example, non-small cell lung cancer/NSCLC), and a disease associated thereto.

The present disclosure further aims to provide a method for preventing and/or treating a disease mediated by SOS1, comprising administering, to a subject, a prophylactically and/or therapeutically effective dose of the compound or the stereoisomer, optical isomer, pharmaceutical salt, prodrug, solvate or isotope derivative thereof according to the present disclosure, or the pharmaceutical composition according to the present disclosure.

The present disclosure further aims to provide a method for preventing and/or treating a disease caused by RAS mutations, comprising administering, to a subject, a prophylactically and/or therapeutically effective dose of the compound or the stereoisomer, optical isomer, pharmaceutical salt, prodrug, solvate or isotope derivative thereof according to the present disclosure, or the pharmaceutical composition according to the present disclosure.

The present disclosure further aims to provide use of the compound or the stereoisomer, optical isomer, pharmaceutical salt, prodrug, solvate or isotope derivative thereof according to the present disclosure or the pharmaceutical composition according to the present disclosure in the non-diagnostic and non-therapeutic *in vitro* inhibition of the activity of guanine nucleotide exchange factors (GEFs), wherein the guanine nucleotide exchange factor is SOS1.

The present disclosure further aims to provide a method for non-diagnostically and non-therapeutically inhibiting the activity of guanine nucleotide exchange factors *in vitro,* comprising administering, to a subject, an inhibitory effective dose of the compound or the stereoisomer, optical isomer, pharmaceutical salt, prodrug, solvate or isotope derivative

thereof according to the present disclosure or the pharmaceutical composition according to the present disclosure, wherein the guanine nucleotide exchange factor is SOS1.

Advantageous Effects of Invention

The present disclosure designs a class of structurally novel compounds, providing a new direction for the development of SOS1 inhibitor drugs. *In vitro* enzymatic inhibition assay studies show that all these compounds exhibit strong inhibitory effects on SOS1 and may be used as promising compounds for preventing and/or treating the diseases mediated by SOS1. Moreover, these compounds further exhibit a significant inhibitory activity against the proliferation of NCI-H358 cells. Additionally, the present disclosure focuses on a particular synthesis method that is simple in process, convenient in operation, and conducive to large-scale industrial production and application.

## DETAILED DESCRIPTION

[Definitions of Terms]

The term "optional", "alternative", "optionally" or "alternatively" means that the event or condition described subsequently may, but not necessarily, occur and the description involves the case where the event or condition occurs and the case where the event or condition does not occur.

Unless otherwise specified, the term "alkyl" refers to a monovalent, linear or branched, saturated aliphatic hydrocarbyl group, typically containing 1 to 20 carbon atoms, preferably 1 to 10 carbon atoms (*i.e.,* $C_{1-10}$ alkyl), further preferably 1 to 8 carbon atoms (*i.e.,* $C_{1-8}$ alkyl), more preferably 1 to 6 carbon atoms (*i.e.,* $C_{1-6}$ alkyl). For example, "$C_{1-6}$ alkyl" means that this group is alkyl and the number of carbon atoms of the carbon chain is between 1 and 6 (specifically, 1, 2, 3, 4, 5 or 6). Non-limiting examples of alkyl include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl and the like.

Unless otherwise specified, the term "alkenyl" refers to a monovalent, linear or branched, unsaturated aliphatic hydrocarbyl group having at least one double bond, typically containing 2 to 20 carbon atoms, preferably 2 to 10 carbon atoms (*i.e.,* $C_{2-10}$ alkenyl), further preferably 2 to 8 carbon atoms (*i.e.*, $C_{2-8}$ alkenyl), more preferably 2 to 6 carbon atoms (*i.e.,* $C_{2-6}$ alkenyl). For example, "$C_{2-6}$ alkenyl" means that this group is alkenyl and the number of carbon atoms of the carbon chain is between 2 and 6 (specifically, 2, 3, 4, 5 or 6). Non-limiting examples of alkenyl include, but are not limited to, vinyl, 1-propenyl, 2-propenyl, 1-butenyl, isobutenyl, 1,3-butadienyl and the like.

Unless otherwise specified, the term "alkynyl" refers to a monovalent, linear or branched, unsaturated aliphatic hydrocarbyl group having at least one triple bond, typically containing 2 to 20 carbon atoms, preferably 2 to 10 carbon atoms (*i.e.,* $C_{2-10}$ alkynyl), further preferably 2 to 8 carbon atoms (*i.e.*, $C_{2-8}$ alkynyl), more preferably 2 to 6 carbon atoms (*i.e.,* $C_{2-6}$ alkynyl). For example, "$C_{2-6}$ alkynyl" means that this group is alkynyl and the number of carbon atoms of the carbon chain is between 2 and 6 (specifically, 2, 3, 4, 5 or 6). Non-limiting examples of alkynyl include, but are not limited to, ethynyl, 1-propynyl, 2-propynyl, 1-butynyl and the like.

Unless otherwise specified, the term "cycloalkyl" refers to a monovalent, monocyclic or polycyclic (*e.g.*, fused cyclic, bridged cyclic or spirocyclic) aliphatic hydrocarbyl group free of unsaturated bonds, typically containing 3 to 12 carbon atoms (*i.e.,* $C_{3-12}$ cycloalkyl), more preferably 3 to 10 carbon atoms (*i.e.,* $C_{3-10}$ cycloalkyl), further preferably 3 to 6 carbon atoms (*i.e.,* $C_{3-6}$ cycloalkyl), 4 to 6 carbon atoms (*i.e.*, $C_{4-6}$ cycloalkyl), or 5 to 6 carbon atoms (*i.e.,* $C_{5-6}$ cycloalkyl). Non-limiting examples of monocyclic cycloalkyl include, but are not limited to, cyclopropyl, cyclobutyl, cyclopentyl, cyclohexyl, methylcyclopropyl, 2-ethylcyclopentyl, dimethylcyclobutyl and the like. Non-limiting examples of fused cycloalkyl include, but are not limited to, decahydronaphthyl, octahydroindenyl, octahydropentalenyl and the like. Non-limiting examples of bridged cycloalkyl include, but are not limited to,

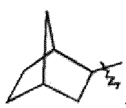 , 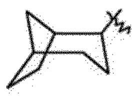 ,

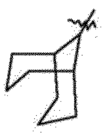 , 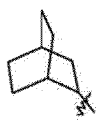 , 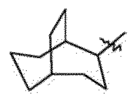 , 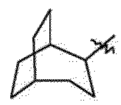 , 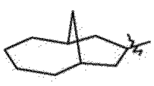 ,

and

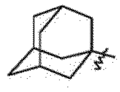

. Non-limiting examples of spiro cycloalkyl include, but are not limited to,

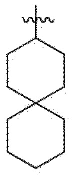 , 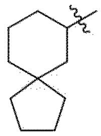 , 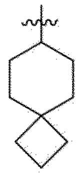 ,

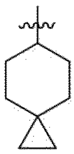 , 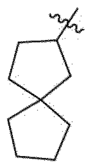 , 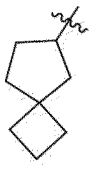 , 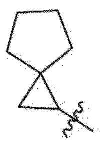 , 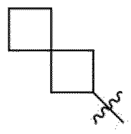

and the like.

Unless otherwise specified, the term "cycloalkenyl" refers to a monovalent, monocyclic or polycyclic aliphatic hydrocarbyl group having at least one double bond, typically containing 3 to 12 carbon atoms (*i.e.,* $C_{3-12}$ cycloalkenyl), more preferably 4 to 12 carbon atoms (*i.e.,* $C_{4-12}$ cycloalkenyl), or 3 to 10 carbon atoms (*i.e.,* $C_{3-10}$ cycloalkenyl), further preferably 3 to 6 carbon atoms (*i.e.,* $C_{3-6}$ cycloalkenyl), 4 to 6 carbon atoms (*i.e.,* $C_{4-6}$ cycloalkenyl), or 5 to 6 carbon atoms (*i.e.,* $C_{5-6}$ cycloalkenyl). Non-limiting monocyclic cycloalkenyl include, but are not limited to, cyclopropenyl, cyclobutenyl, cyclopentenyl, cyclohexenyl, cyclopentadienyl, cyclohexadienyl, cycloheptatrienyl, cyclooctatrienyl and the like.

Unless otherwise specified, the term "alkoxy" refers to an "-O- alkyl" group, wherein the alkyl is as previously defined, i.e., containing 1 to 20 carbon atoms, preferably 1 to 10 carbon atoms, preferably 1 to 8 carbon atoms, more preferably 1 to 6 carbon atoms (specifically, 1, 2, 3, 4, 5 or 6). Typical examples include, but are not limited to, methoxy, ethoxy, propoxy, isopropoxy, butoxy, 1-methylpropoxy, 2-methylpropoxy, tert-butoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy and the like.

Unless otherwise specified, the term "halogen" or "halogenated" involves F, Cl, Br, and I. The term "haloalkyl" means that one, two or more hydrogen atoms or all hydrogen atoms of the alkyl as previously defined are substituted with halogen(s). Typical examples of haloalkyl include $CCl_3$, $CF_3$, $CHCl_2$, $CH_2Cl$, $CH_2Br$, $CH_2I$, $CH_2CF_3$, $CF_2CF_3$ and the like. Haloalkyl can be expressed in different ways, for example, "$C_{1-6}$ haloalkyl" can also be expressed as "halogenated $C_{1-6}$ alkyl". The term "haloalkoxy" means that one, two or more hydrogen atoms or all hydrogen atoms of the alkoxy as previously defined are substituted with halogen(s). Typical examples of haloalkoxy include $OCCl_3$, $OCF_3$, $OCHCl_2$, $OCH_2Cl$, $OCH_2Br$, $OCH_2I$, $OCH_2CF_3$, $OCF_2CF_3$ and the like. Haloalkoxy can also be expressed in different ways, for example, "$C_{1-6}$ haloalkoxy" can also be expressed as "halogenated $C_{1-6}$ alkoxy". The term "halocycloalkyl" means that one, two or more hydrogen atoms or all hydrogen atoms of the cycloalkyl as previously defined are substituted with halogen(s).

Unless otherwise specified, the term "heterocyclyl" refers to a monovalent, saturated or partially unsaturated, monocyclic or polycyclic (*e.g.*, fused cyclic, bridged cyclic or spirocyclic) aliphatic cyclic system with a non-aromatic structure as a whole, typically containing 3 to 20 ring atoms, where one, two, three or more ring atoms are N, O or S, and the remaining ring atoms are C, preferably containing 3 to 12 ring atoms, further preferably 4 to 12 ring atoms, or 5 to 12 ring atoms, or 3 to 10 ring atoms, or 3 to 8 ring atoms, or 3 to 6 ring atoms, or 4 to 6 ring atoms, or 5 to 6 ring atoms. The number of heteroatoms is preferably 1 to 4, more preferably 1 to 3 (*i.e.,* 1, 2 or 3). Non-limiting examples of monocyclic heterocyclyl include, but are not limited to, pyrrolidinyl, imidazolidinyl, tetrahydrofuranyl, dihydropyrrolyl, piperidinyl, piperazinyl, pyranyl, 2,5-dihydrofuranyl, and pyridinonyl. Polycyclic heterocyclyl includes spirocyclic, fused cyclic, and bridged cyclic heterocyclyl. Non-limiting examples of fused cyclic heterocyclyl include, but are not limited to, decahydroquinolinyl, octahydroindolyl and the like. Non-limiting examples of bridged cyclic heterocyclyl include, but are

not limited to,

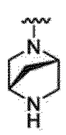, 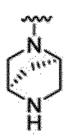, 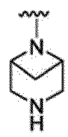, 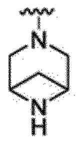,

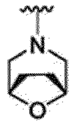, 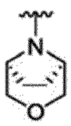, 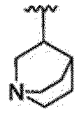, 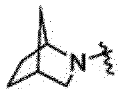, 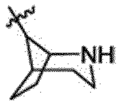, 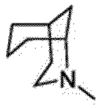, 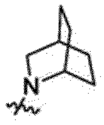, 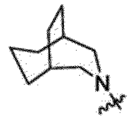,

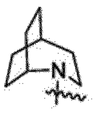, 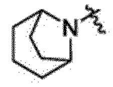, 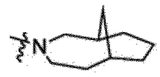

and the like. Non-limiting examples of spirocyclic heterocyclyl include, but are not limited to,

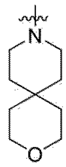, 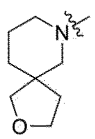, 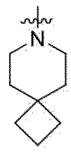, 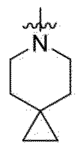, 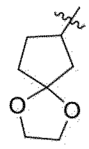,

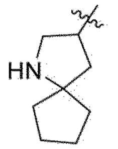, 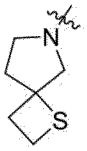, 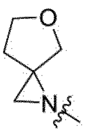, 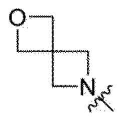

and the like.

Unless otherwise specified, the term "aryl" refers to a monovalent, monocyclic or polycyclic (*e.g.*, fused cyclic) aromatic cyclic system, typically containing 6 to 16 carbon atoms, or 6 to 14 carbon atoms, or 6 to 12 carbon atoms, preferably 6 to 10 carbon atoms. The term "aryl" can be used interchangeably with the term "aromatic ring". Non-limiting examples of aryl include, but are not limited to, phenyl, naphthyl, anthracenyl, phenanthryl, pyrenyl and the like.

Unless otherwise specified, the term "heteroaryl" refers to a monovalent, monocyclic or polycyclic (*e.g.*, fused cyclic) aromatic cyclic system, typically containing a 5 to 12-membered structure, or preferably 5- to 10-membered structure, 5-to 8-membered structure, more preferably 5- to 6-membered structure, wherein one, two, three or more ring atoms are heteroatoms and the remaining atoms are carbon atoms, the heteroatoms are each independently O, N or S, and the number of heteroatoms is preferably 1, 2 or 3. The term "heteroaryl" can be used interchangeably with the term "heteroaromatic ring". Non-limiting examples of heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiodiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl and the like.

Unless otherwise specified, the term "hydroxyl" refers to "-OH" group.

Unless otherwise specified, the term "cyano" refers to "-CN" group.

Unless otherwise specified, the term "amino" refers to "$-NH_2$" group. In some cases, the term "amino" also refers to a group in which one or two hydrogen atoms are substituted with alkyl (*e.g.*, $C_{1-6}$ alkyl, preferably $C_{1-4}$ alkyl).

Unless otherwise specified, the term "nitro" refers to "$-NO_2$" group.

Unless otherwise specified, the term "formyl" or "aldehydyl" refers to "-C(=O)H" group.

Unless otherwise specified, the term "oxo" refers to "=O" group linked to the carbon atom, while the term "oxido" refers to "=O" group linked to a heteroatom (*e.g.*, sulfur atom).

Unless otherwise specified, the term "pharmaceutically acceptable salt", "pharmaceutical salt" or "medicinal salt" refers to a salt suitable, within reasonable medical judgment, for use in contact with tissues of mammals, in particular humans, without undue toxicity, irritation, allergic response, etc., and commensurate with a reasonable benefit/risk ratio. The salt can be prepared *in situ* during the final separation and purification of a compound of the present disclosure, or individually prepared by reacting a free base or free acid with a suitable reagent. For example, the free base can react with a suitable acid.

Unless otherwise specified, the term "solvate" or "solvated compound" refers to a physical association of a compound of the present disclosure with one or more solvent molecules, whether it is organic or inorganic. The physical association involves hydrogen bonds. In some cases, for example, when one or more solvent molecules are accommodated in the lattice of a crystalline solid, a solvate will be able to be separated. The solvent molecules in the solvate can be regularly and/or randomly arranged. The solvate can comprise a stoichiometric or non-stoichiometric amount of solvent molecules. "Solvate" encompasses that in the solution phase and that which can be separated. Exemplary solvates include, but are not limited to, hydrates, ethanol-solvated complexes, methanol-solvated complexes, and isopropanol-solvated complexes. Solvation methods are well-known in the art.

Unless otherwise specified, the compound of the present disclosure further includes its "isotope derivative" (such as deuterate). The term "isotope derivative" means that the compound of the present disclosure can exist at an isotope trace level or an isotopically enriched level and contain one or more atoms whose atomic weight or mass number differs from that of the most abundant atom found in nature. The isotope can be radioactive or non-radioactive. The isotopes commonly used for isotope labeling are: hydrogen isotopes: $^{2}H$ (D) and $^{3}H$ (T); carbon isotopes: $^{13}C$ and $^{14}C$; chlorine isotopes: $^{35}Cl$ and $^{37}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$, and $^{18}O$; and sulfur isotope: $^{35}S$. These isotopically labelled compounds can be used to study the distribution of pharmaceutical molecules in tissues. In particular, $^{3}H$ and $^{13}C$ are more widely used because they are easy to label and convenient to detect. Replacements with certain heavy isotopes, such as heavy hydrogen ($^{2}H$), can enhance metabolic the stability and prolong the half-life of compound, thereby fulfilling the purpose of dosage reduction and providing therapeutic advantages. The isotopically labelled compounds are generally synthesized starting from the labeled starting materials by using known synthetic techniques just like the synthesis of non-isotopically labelled compounds.

Unless otherwise specified, the term "prodrug" refers to a drug transformed into the parent drug *in vivo.* The prodrugs are often useful because, in some cases, they can be easier to administer than their parent drugs. For example, they can be bioavailable via oral administration, while the parent drugs cannot. The solubility of the prodrugs is also somewhat improved in the pharmaceutical composition as compared to the parent drugs.

Unless otherwise specified, the term "optical isomer" refers to the substances that share exactly the same molecular structure and similar physical and chemical properties but have different optical activities.

Unless otherwise specified, the term "stereoisomer" refers to the compounds that share the same chemical structure but have different spatial arrangements of atoms or groups. The stereoisomer includes enantiomer, diastereomer, conformational isomer (rotational isomer), geometric (cis/trans) isomer, atropisomer, etc. Any resulting mixture of stereoisomers can be separated into pure or substantially pure geometrical isomers, enantiomers or diastereomers by, for example, chromatography and/or fractional crystallization depending on the differences in the physical and chemical properties of the components.

Unless otherwise indicated, the structural formula described herein includes all isomeric forms (e.g., enantiomeric, diastereomeric, geometric (or conformational) isomeric and the like): for example, isomers with asymmetric centers having R and S configurations, isomers with (Z) and (E) double bonds, and (Z) and (E) conformational isomers. Thus, both an individual stereoisomer of the compound of the present disclosure and its mixture with an enantiomer, diastereomer, or geometric isomer (or conformational isomer) thereof fall within the scope of the present disclosure.

Unless otherwise specified, the term "optional substitution", "optionally substituted with ...", or "optionally substituted ..." means that the hydrogen at a substitutable site of the group as described is replaced or not by one or more substituent(s) preferably selected from the group consisting of halogen, hydroxyl, mercapto, cyano, nitro, amino, azido, oxo, carboxyl, $C_{2-6}$ alkenyl, $C_{2-6}$ alkynyl, $C_{1-6}$ alkyl, $C_{1-6}$ alkoxy, $C_{3-10}$ cycloalkyl, 3- to 10-membered heterocyclyl, $C_{6-14}$ aryl, and 5-to 10-membered heteroaryl, wherein the $C_{2-6}$ alkenyl, $C_{2-6}$ alkynyl, $C_{1-6}$ alkyl, $C_{1-6}$ alkoxy, $C_{3-10}$ cycloalkyl, 3- to 10-membered heterocyclyl, $C_{6-14}$ aryl, and 5- to 10-membered heteroaryl are all optionally substituted with one or more substituents selected from halogen, hydroxyl, amino, cyno, $C_{1-6}$ alkyl or $C_{1-6}$ alkoxy.

The present disclosure will be further illustrated below with reference to specific examples. It shall be appreciated that these examples are intended only to illustrate the present disclosure and not to limit the scope of the present disclosure. Experimental methods for which no specific conditions are indicated in the following examples are generally carried out in accordance with conventional conditions or those recommended by the manufacturers. Unless otherwise

defined, all professional and scientific terms used herein have the same meanings as those familiar to a person skilled in the art. In addition, any methods and materials similar or equivalent to those described herein can be applied to the methods of the present disclosure. The preferred embodiments and materials described herein are for exemplary purposes only.

The structures of the compounds of the present disclosure are determined by nuclear magnetic resonance (NMR) and/or liquid chromatography - mass spectrometry (LC-MS) and/or high performance liquid chromatography (HPLC). The instrument for the NMR determination is Bruker AVANCE III 600 MHz; the instrument for LC-MS is WATERS ACQUITY UPLC H-Class PLUS and/or SQD2; and the instrument for HPLC is WATERS e2695_2998 and/or Agilent 1100.

The starting materials used in the examples of the present disclosure are known and commercially available, or can be synthesized by the methods known in the art.

Preparation Example 1: Synthesis of Intermediate A

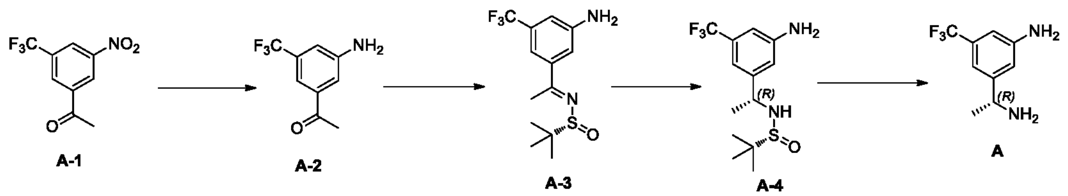

Synthesis of Intermediate A-2

1-(3-Nitro-5-(trifluoromethyl)phenyl)ethenone (10.00 g, 43.0 mmol) was dissolved in ethanol (200 mL), and then iron powder (7.22 g, 129.0 mmol) and concentrated hydrochloric acid (50 mL) were added. The system reacted at 80°C for 2 h. LC-MS analysis showed that no raw material was left. The system was cooled and filtered. The filtrate was evaporated under reduced pressure to remove the solvent. The residue was separated and purified by column chromatography (dichloromethane:methanol = 50:1-20:1) to afford A-2 (8.12 g, 40.0 mmol, 92%). ESI-MS: m/z 204.12 $[M+H]^+$.

Synthesis of Intermediate A-3

A-2 (27.02 g, 133.0 mmol) was dissolved in tetrahydrofuran (50 mL), and then (R)-(+)-2-methyl-2-propanesulfinamide (24.24 g, 200.0 mmol) and $Ti(OEt)_4$ (91.02 g, 399 mmol) were added. The system reacted at 80°C for 2 h. LC-MS analysis showed that no raw material was left. The reaction solution was quenched with ice water. The precipitate was dissolved in ethyl acetate and filtered. The filtrate was evaporated under reduced pressure to remove the solvent. The residue was separated and purified by column chromatography (dichloromethane:methanol = 50:1-20:1) to afford the product A-3 (34.65 g, 113.1 mmol, 85%). ESI-MS: m/z 307.12 $[M+H]^+$.

Synthesis of Intermediate A-4

A-3 (34.65 g, 113.1 mmol) was dissolved in tetrahydrofuran (50 mL), and sodium borohydride (6.42 g, 169.7 mmol) was added at -78°C. The temperature of the reaction system was raised slowly to room temperature. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (dichloromethane:methanol = 50:1-20:1) to afford A-4 (28.43 g, 92.2 mmol, 82%). ESI-MS: m/z 309.10 $[M+H]^+$.

Synthesis of Intermediate A

A-4 (28.43 g, 92.2 mmol) was dissolved in a solution of hydrochloric acid in dioxane (50 mL). The system reacted at room temperature for 2 h. LC-MS analysis showed that no raw material was left. The solvent was removed by evaporation under reduced pressure to afford Intermediate A in the form of hydrochloride salt, which was used directly in the next step. ESI-MS: m/z 205.22 $[M+H]^+$.

The following intermediates could be available by reference to the synthetic method for Intermediate A or the relevant synthetic methods described in CN110167928A. If required, the crude products were purified by chromatography.

| No. | Structure | [M+H]+ | No. | Structure | [M+H]+ |
|---|---|---|---|---|---|
| B | 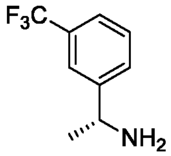 | 190.02 | J | 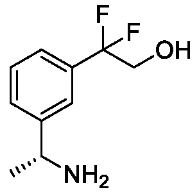 | 202.09 |
| C | 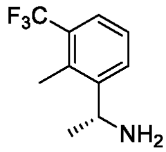 | 204.09 | K | 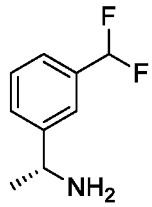 | 172.06 |
| D | 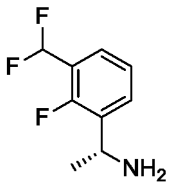 | 190.08 | L | 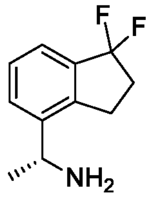 | 198.20 |
| E | 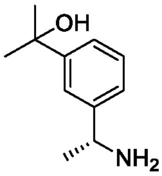 | 180.29 | M | 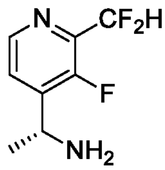 | 191.15 |
| F | 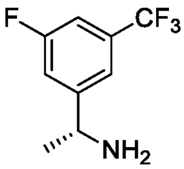 | 208.15 | N | 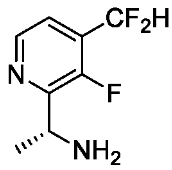 | 191.17 |
| G | 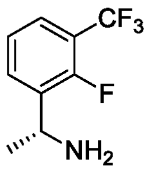 | 208.24 | O | 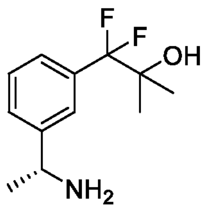 | 230.21 |
| H | 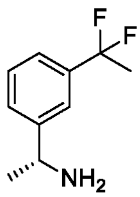 | 186.17 | P | 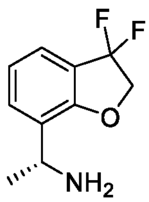 | 200.13 |

(continued)

| No. | Structure | [M+H]+ | No. | Structure | [M+H]+ |
|---|---|---|---|---|---|
| I | 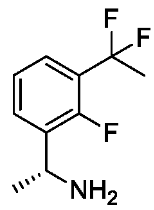 | 203.97 | Q | 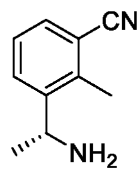 | 161.12 |

**Example** 1: Synthesis of Compound 1

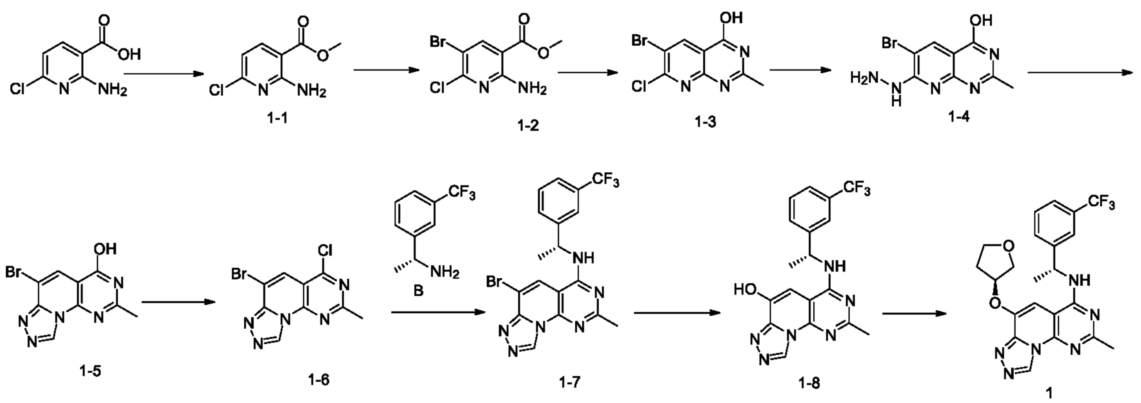

1-1 1-2 1-3 1-4 1-5 1-6 B 1-7 1-8 1

Synthesis of Intermediate 1-1

2-Amino-6-chloronicotinic acid (5.00 g, 29.0 mmol) was dissolved in 80 mL of methanol. The system was cooled to 0°C, and then 16 mL of concentrated sulfuric acid was added. The system reacted at 80°C for 5 h. LC-MS analysis showed that no raw material was left. The reaction solution was added with 100 mL of water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:1) to afford 1-1 (5.03 g, yield: 93%). ESI-MS: m/z 187.02 [M+H]+.

Synthesis of Intermediate 1-2

1-1 (5.03 g, 26.9 mmol) was dissolved in acetonitrile (80 mL), and then N-bromosuccinimide (7.17 g, 40.3 mmol) was added. The system reacted at room temperature for 1.5 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:3) to afford 1-2 (6.85 g, yield: 96%). ESI-MS: m/z 265.10/267.13 [M+H]+.

Synthesis of Intermediate 1-3

1-2 (6.85 g, 25.8 mmol) was dissolved in 80 mL of acetonitrile, and then 15 mL of methanesulfonic acid was added. The system reacted at 130°C for 8 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:3) to afford 1-3 (1.51 g, yield: 21%). ESI-MS: m/z 274.02/275.98 [M+H]+.

Synthesis of Intermediate 1-4

1-3 (1.51 g, 5.50 mmol) was dissolved in 50 mL of ethanol, and then hydrazine hydrate (1.76 g, 55.0 mmol) was added. The system reacted at 80°C for 2 h. LC-MS analysis showed that no raw material was left. The solvent was removed by evaporation under reduced pressure. The residue was used directly in the next step. ESI-MS: m/z 270.01/272.03 $[M+H]^+$.

Synthesis of Intermediate 1-5

1-4 was dissolved in 30 mL of chloroform, and then 10 mL of triethyl orthoformate was added. The system reacted at 80°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5: 1-1:3) to afford 1-5 (1.00 g, yield for two steps: 65%). ESI-MS: m/z 280.00/282.02 $[M+H]^+$.

Synthesis of Intermediate 1-6

1-5 (1.00 g, 3.60 mmol) was dissolved in 50 mL of toluene, and then diisopropylethylamine (464 mg, 3.60 mmol) and phosphoryl chloride (2.76 g, 18.0 mmol) were added. The system reacted at 100°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:1) to afford 1-6 (800 mg, yield: 75%). ESI-MS: m/z 298.03/300.02 $[M+H]^+$.

Synthesis of Intermediate 1-7

1-6 (800 mg, 2.68 mmol) was dissolved in 30 mL of N,N-dimethylformamide, and then diisopropylethylamine (697 mg, 5.40 mmol) and Intermediate B (612 mg, 3.24 mmol) were added. The system reacted at 120°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:2) to afford 1-7 (798 mg, yield: 66%). ESI-MS: m/z 451.02/453.07 $[M+H]^+$.

Synthesis of Intermediate 1-8

1-7 (100 mg, 0.22 mmol) was dissolved in a solution of dioxane (40 mL) and water (10 mL), and then potassium hydroxide (25 mg, 0.44 mmol), tris(dibenzylideneacetone)dipalladium (18 mg, 0.02 mmol), and 2-dicyclohexylphosphino-2',4',6'-triisopropylbiphenyl (10 mg, 0.02 mmol) were added. The whole system was reacted with stirring at 100°C for 3 h. TLC analysis showed that no raw material was left. The reaction solution was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford 1-8 (40 mg, yield: 47%). ESI-MS: m/z 389.15 $[M+H]^+$.

Synthesis of Compound 1

1-8 (40 mg, 0.10 mmol) was dissolved in N,N-dimethylformamide (15 mL), and then (R)-tetrahydrofuran-3-yl p-toluenesulfonate (30 mg, 0.12 mmol) and cesium carbonate (65 mg, 0.20 mmol) were added. The whole system was reacted with stirring at 80°C for 3 h. TLC analysis showed that no raw material was left. The reaction solution was added with 30 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 1 (30 mg, yield: 65%). ESI-MS: m/z 459.25 $[M+H]^+$. $^1H$ NMR (600 MHz, DMSO-$d_6$): δ 9.59 (s, 1H), 8.40 (d, *J*=7.2 Hz, 1H), 7.82 (s, 1H), 7.77 (d, *J*=7.2 Hz, 1H), 7.63-7.58 (m, 2H), 7.46 (s, 1H), 5.71-5.64 (m, 1H), 5.47-5.45 (m, 1H), 4.05-4.03(m, 1H), 3.98-3.94 (m, 2H), 3.88-3.84 (m, 1H), 2.46 (s, 3H), 2.42-2.37 (m, 2H), 1.66

(d, *J*=7.2 Hz, 3H).

**Example 2:** Synthesis of Compound 2

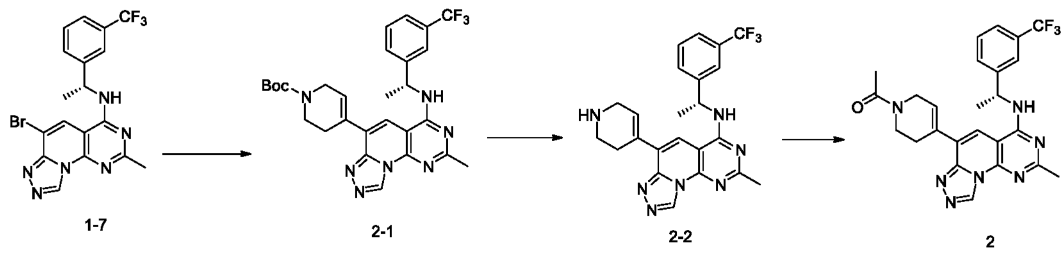

Synthesis of Intermediate 2-1

1-7 (100 mg, 0.22 mmol) was dissolved in dioxane (30 mL), and then N-Boc-1,2,5,6-tetrahydropyridine-4-boronic acid pinacol ester (84 mg, 0.27 mmol), tetrakis(triphenylphosphine)palladium (23 mg, 0.02 mmol), and cesium carbonate (143 mg, 0.44 mmol) were added. The whole system was reacted with stirring at 100°C for 3 h. TLC analysis showed that no raw material was left. The reaction solution was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford 2-1 (100 mg, yield: 82%). ESI-MS: m/z 554.19 $[M+H]^+$.

Synthesis of Intermediate 2-2

2-1 (100 mg, 0.18 mmol) was dissolved in a hydrochloric acid 4M solution in ethyl acetate (10 mL), and reacted with stirring at room temperature for 2 h. TLC analysis showed that no raw material was left. The solvent was removed under reduced pressure to afford 2-2 as a residue, which was used directly in the next step. ESI-MS: m/z 454.16 $[M+H]^+$.

Synthesis of Compound 2

2-2 was dissolved in dichloromethane (10 mL), and then triethylamine (36 mg, 0.36 mmol) and acetic anhydride (22 mg, 0.22 mmol) were added. The system was reacted with stirring at room temperature for 2 h. TLC analysis showed that no raw material was left. The reaction solution was quenched with water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-15:1) to afford Compound 2 (50 mg, yield: 56%). ESI-MS: m/z 496.17 $[M+H]^+$. $^1H$ NMR (600 MHz, DMSO-$d_6$): δ 9.59 (d, *J*=3.6 Hz, 1H), 8.75 (dd, *J*=7.8 Hz, *J*=7.8 Hz, 1H), 8.03(d, *J*=5.4 Hz, 1H), 7.83(s, 1H), 7.78 (d, *J*=7.2 Hz, 1H), 7.63-7.58 (m, 2H), 7.49-7.47 (m, 1H), 5.72-5.68 (m, 1H), 4.33-4.26 (m, 2H), 3.77-3.73(m, 2H), 2.87-2.63(m, 2H), 2.48 (s, 3H), 2.13-2.08 (m, 3H), 1.66 (d, *J*=7.2 Hz, 3H).

Example 3: Synthesis of Compound 3

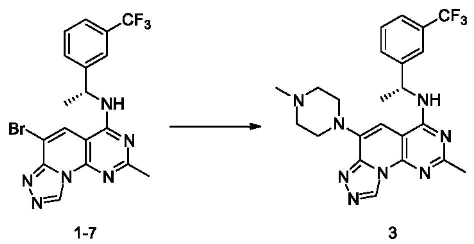

1-7 (100 mg, 0.22 mmol) was dissolved in N-methylpyrrolidone (30 mL), and then N-methyl piperazine (27 mg,

0.27 mmol), tris(dibenzylideneacetone)dipalladium (18 mg, 0.02 mmol), and sodium tert-butoxide (42 mg, 0.44 mmol) were added. The whole system was reacted with stirring at 120°C for 3 h. TLC analysis showed that no raw material was left. The reaction was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 3 (41 mg, yield 40%). ESI-MS: m/z 471.18 $[M+H]^+$. $^1H$ NMR (600 MHz, DMSO-$d_6$): δ 9.53(s, 1H), 8.42 (d, *J*=7.8 Hz, 1H), 7.82 (s, 1H), 7.77 (d, *J*=7.2Hz, 1H), 7.61-7.57 (m, 2H), 7.10 (s, 1H), 5.68-5.65 (m, 1H), 3.65-3.60 (m, 4H), 2.63-2.59 (m, 4H), 2.43(s, 3H), 2.30 (s, 3H), 1.65 (d, *J*=7.2 Hz, 3H).

**Example 4:** Synthesis of Compound 4

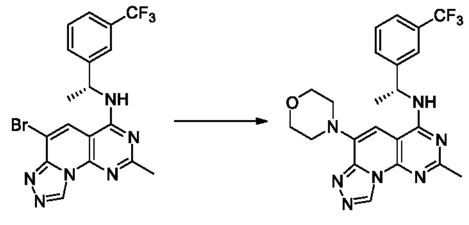

1-7 4

1-7 (100 mg, 0.22 mmol) was dissolved in N-methylpyrrolidone (30 mL), and then morpholine (24 mg, 0.27 mmol), tris(dibenzylideneacetone)dipalladium (18 mg, 0.02 mmol), and sodium tert-butoxide (42 mg, 0.44 mmol) were added. The whole system was reacted with stirring at 120°C for 3 h. TLC analysis showed that no raw material was left. The reaction was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 4 (50 mg, yield: 50%). ESI-MS: m/z 458.16 $[M+H]^+$. $^1H$ NMR (600 MHz, DMSO-$d_6$): δ 9.53(s, 1H), 8.43(d, *J*=7.8 Hz, 1H), 7.82 (s, 1H), 7.77 (d, *J*=6.6 Hz, 1H), 7.62-7.58 (m, 2H), 7.13(s, 1H), 5.70-5.65 (m, 1H), 3.88-3.87 (m, 4H), 3.61-3.59 (m, 4H), 2.43(s, 3H), 1.65 (d, *J*=7.2 Hz, 3H).

**Example 5:** Synthesis of Compound 5

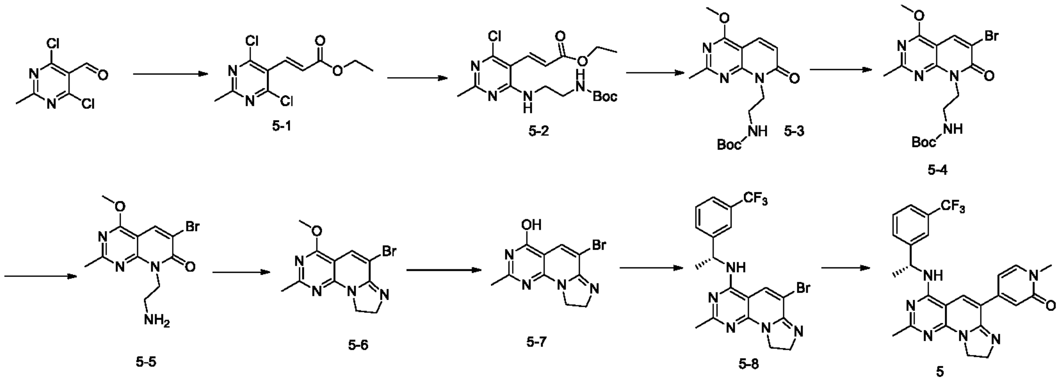

5-1 5-2 5-3 5-4 5-5 5-6 5-7 5-8 5

Synthesis of Intermediate 5-1

4,6-Dichloro-2-methylpyrimidine-5-carbaldehyde (2.00 g, 10.47 mmol) was dissolved in tetrahydrofuran (20 mL), and then (carbethoxymethylene)triphenylphosphorane (5.47 g, 15.71 mmol) and triethylamine (2.12 g, 20.94 mmol) were added. The system reacted at 80°C for 6 h. LC-MS analysis showed that no raw material was left. The reaction system was directly separated and purified by column chromatography (n-hexane:ethyl acetate = 20:1-10:1) to afford

5-1 (1.71 g, 6.54 mmol, yield: 63%). ESI-MS: m/z 260.85 $[M+H]^+$.

Synthesis of Intermediate 5-2

5-1 (1.71 g, 6.54 mmol) was dissolved in N,N-dimethylformamide (20 mL), and then tert-butyl (2-aminoethyl)carbamate (1.26 g, 7.85 mmol) and triethylamine (1.32 g, 13.08 mmol) were added. The system reacted at room temperature for 12 h. LC-MS analysis showed that no raw material was left. The reaction solution was added with water (30 mL), and extracted with ethyl acetate (30 mL×3). The organic phases were combined, washed with a saturated solution of sodium chloride (30 mL×2), and dried over anhydrous sodium sulfate. The solvent was removed by evaporation under reduced pressure. The residue was purified by thin layer chromatography (n-hexane:ethyl acetate = 10:1-3:1) to afford 5-2 (2.30 g, 5.99 mmol, yield: 92%). ESI-MS: m/z 385.15 $[M+H]^+$.

Synthesis of Intermediate 5-3

5-2 (2.30 g, 5.99 mmol) was dissolved in methanol (30 mL), and then sodium methoxide (3.6 mL, 17.97 mmol, 30% w/w in methanol) was added. The system reacted at room temperature for 12 h. A solid precipitated from the reaction solution. LC-MS analysis showed that no raw material was left. The reaction solution was added with water (10 mL), and filtered to afford 5-3 as a white solid (1.60 g, 4.79 mmol, yield: 80%). ESI-MS: m/z 335.07 $[M+H]^+$.

Synthesis of Intermediate 5-4

5-3 (1.60 g, 4.79 mmol) was dissolved in dichloromethane (30 mL), and then bromine (1.15 g, 7.19 mmol) was added. The system reacted at room temperature for 12 h. LC-MS analysis showed that no raw material was left. The reaction system was directly separated and purified by column chromatography (n-hexane:ethyl acetate = 5:1-3:1) to afford 5-4 (1.80 g, 4.36 mmol, yield: 91%). ESI-MS: m/z 412.98/415.00 $[M+H]^+$.

Synthesis of Intermediate 5-5

5-4 (1.80 g, 4.36 mmol) was dissolved in dichloromethane (20 mL), and then 5 mL of trifluoroacetic acid was added. The system reacted at room temperature for 4 h. LC-MS analysis showed that no raw material was left. The solvent was removed by evaporation under reduced pressure. The residue was added with 15 mL of water, and the pH value of the mixture was adjusted to 8 to 9 with a saturated aqueous sodium carbonate solution. The resultant was extracted with ethyl acetate (20 mL×3). The organic phases were combined, washed with a saturated solution of sodium chloride (20 mL×2), and dried over anhydrous sodium sulfate. The solvent was removed by evaporation under reduced pressure to afford 5-5 (0.90 g, 2.88 mmol, yield: 66%). ESI-MS: m/z 312.92/314.91 $[M+H]^+$.

Synthesis of Intermediate 5-6

5-5 (0.90 g, 2.88 mmol) was dissolved in toluene (20 mL), and then trimethylaluminum (1.9 mL, 3.75 mmol, 2.0 M in toluene) was slowly added. The system reacted at 120°C for 5 h. LC-MS analysis showed that no raw material was left. The reaction solution was quenched with a saturated aqueous ammonium chloride solution. The toluene was removed by evaporation under reduced pressure. The residue was added with 15 mL of water, extracted with ethyl acetate (20 mL×3). The organic phases were combined, washed with a saturated solution of sodium chloride (20 mL×2), and dried over anhydrous sodium sulfate. The solvent was removed by evaporation under reduced pressure to afford 5-6 (0.59 g, 2.01 mmol, yield: 70%). ESI-MS: m/z 294.87/296.83 $[M+H]^+$.

Synthesis of Intermediate 5-7

5-6 (0.59 g, 2.01 mmol) was dissolved in dichloromethane (10 mL). Boron tribromide (2.52 g, 10.05 mmol) was slowly added in an ice bath, and after addition, the ice bath was removed. The system reacted at room temperature for 72 h. LC-MS analysis showed that no raw material was left. Aqueous sodium carbonate solution was slowly added in an ice bath to quench the reaction. A solid precipitated and was filtered to afford 5-7 (0.46 g, 1.64 mmol, yield: 82%). ESI-MS: m/z 280.82/282.84 $[M+H]^+$.

Synthesis of Intermediate 5-8

5-7 (50 mg, 0.18 mmol) was dissolved in N,N-dimethylformamide (15 mL), and then (R)-1-(3-(trifluoromethyl)phenyl)ethylamine hydrochloride (61 mg, 0.27 mmol), tri(dimethylamino)benzotriazol-1-yloxyphosphonium hexafluorophosphate (102 mg, 0.23 mmol), and 1,8-diazabicycloundec-7-ene (82 mg, 0.54 mmol) were added. The system reacted at room temperature for 8 h. LC-MS analysis showed that no raw material was left. The reaction solution was added with water (30 mL), and extracted with ethyl acetate (30 mL×3). The organic phases were combined, washed with a saturated solution of sodium chloride (30 mL×2), and dried over anhydrous sodium sulfate. The solvent was removed by evaporation under reduced pressure. The residue was purified by thin layer chromatography (dichloromethane:methanol = 20:1) to afford 5-8 (40 mg, 0.089 mmol, yield: 49%). ESI-MS: m/z 451.94/453.94 $[M+H]^+$.

Synthesis of Compound 5

5-8 (40 mg, 0.089 mmol) was dissolved in a solution of dioxane (10 mL) and water (2 mL), and then 1-methyl-4-(4,4,5,5-tetramethyl-1,3,2-dioxaborolan-2-yl)pyridin-2(1H)-one (31 mg, 0.133 mmol), [1,1'-bis(diphenylphosphino)ferrocene]dichloropalladium(II) (13 mg, 0.018 mmol), and cesium carbonate (58 mg, 0.178 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:triethylamine = 100:2:1) to afford Compound 5 (17 mg, 0.035 mmol, yield: 40%). ESI-MS: m/z 481.11 $[M+H]^+$. $^1$H NMR (600 MHz, DMSO-$d_6$): δ 8.37 (s, 1H), 8.32 (s, 1H), 7.78-7.73(m, 3H), 7.60-7.59 (m, 2H), 7.21 (s, 1H), 6.81 (d, *J*=12.0 Hz, 1H), 5.61-5.59 (m, 1H), 4.12 (t, *J*=6.0 Hz, 2H), 3.97 (t, *J*=6.0 Hz, 2H), 3.46 (s, 3H), 2.31 (s, 3H), 1.59 (d, *J*=6.0 Hz, 3H).

**Example 6:** Synthesis of Compound 6

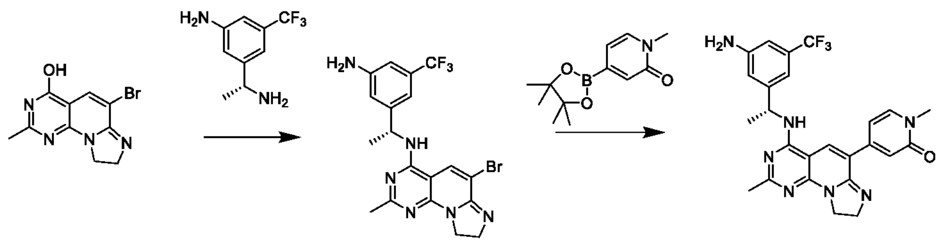

5-7 6-1 6

Synthesis of Intermediate 6-1

5-7 (100 mg, 0.36 mmol) was dissolved in N,N-dimethylformamide (15 mL), and then (R)-3-(1-aminoethyl)-5-(trifluoromethyl)aniline (110 mg, 0.54 mmol), tri(dimethylamino)benzotriazol-1-yloxyphosphonium hexafluorophosphate (207 mg, 0.47 mmol), and 1,8-diazabicycloundec-7-ene (164 mg, 1.08 mmol) were added. The system reacted at room temperature for 8 h. LC-MS analysis showed that no raw material was left. The reaction solution was added with water (30 mL), and extracted with ethyl acetate (30 mL×3). The organic phases were combined, washed with a saturated solution of sodium chloride (30 mL×2), and dried over anhydrous sodium sulfate. The solvent was removed by evaporation under reduced pressure. The residue was purified by thin layer chromatography (dichloromethane:methanol = 20:1) to afford 6-1 (60 mg, yield: 36%). ESI-MS: m/z 466.91/469.12 $[M+H]^+$.

Synthesis of Compound 6

6-1 (60 mg, 0.128 mmol) was dissolved in a solution of dioxane (10 mL) and water (2 mL), and then 1-methyl-4-(4,4,5,5-tetramethyl-1,3,2-dioxaborolan-2-yl)pyridin-2(1H)-one (45 mg, 0.191 mmol), [1,1'-bis(diphenylphosphino)ferrocene]dichloropalladium(II) (19 mg, 0.026 mmol), and cesium carbonate (84 mg, 0.258 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:triethylamine = 50:2:1) to afford Compound 6 (20 mg, yield: 31%). ESI-MS: m/z 496.08 $[M+H]^+$. $^1$H NMR (600 MHz, DMSO-$d_6$): δ 8.30 (s, 1H), 8.13(d, *J*=6.0 Hz, 1H), 7.72 (d, *J*=6.0 Hz, 1H), 7.32 (s, 1H), 6.88 (d, *J*=6.0 Hz, 1H), 6.84-6.82 (m, 2H), 6.71 (s, 1H), 5.57 (s, 2H), 5.50-5.47 (m, 1H), 4.07-4.05 (m, 2H), 3.98-3.95 (m, 2H), 3.45(s,

3H), 2.31 (s, 3H), 1.52 (d, *J*=6.0 Hz, 3H).

**Example 7:** Synthesis of Compound 7

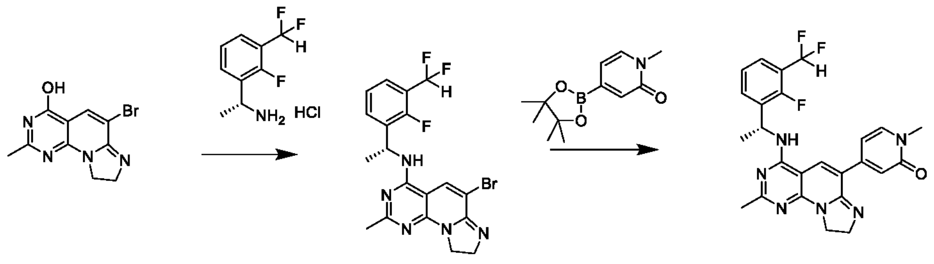


5-7 7-1 7

Synthesis of Intermediate 7-1

5-7 (100 mg, 0.36 mmol) was dissolved in N,N-dimethylformamide (15 mL), and then (R)-1-(3-(difluoromethyl)-2-fluorophenyl)ethylamine hydrochloride (121 mg, 0.54 mmol), tri(dimethylamino)benzotriazol-1-yloxyphosphonium hexafluorophosphate (205 mg, 0.47 mmol), and 1,8-diazabicycloundec-7-ene (163 mg, 1.08 mmol) were added. The system reacted at room temperature for 8 h. LC-MS analysis showed that no raw material was left. The reaction solution was added with water (30 mL), and extracted with ethyl acetate (30 mL×3). The organic phases were combined, washed with a saturated solution of sodium chloride (30 mL×2), and dried over anhydrous sodium sulfate. The solvent was removed by evaporation under reduced pressure. The residue was purified by thin layer chromatography (dichloromethane:methanol = 20:1) to afford 7-1 (110 mg, yield 68%). ESI-MS: m/z 451.93/453.93 $[M+H]^+$.

Synthesis of Compound 7

7-1 (110 mg, 0.243 mmol) was dissolved in a solution of dioxane (10 mL) and water (2 mL), and then 1-methyl-4-(4,4,5,5-tetramethyl-1,3,2-dioxaborolan-2-yl)pyridin-2(1H)-one (86 mg, 0.366 mmol), [1,1'-bis(diphenylphosphino)ferrocene]dichloropalladium(II) (36 mg, 0.049 mmol), and cesium carbonate (159 mg, 0.488 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:triethylamine = 100:2:1) to afford Compound 7 (50 mg, yield: 43%). ESI-MS: m/z 481.09 $[M+H]^+$. $^1H$ NMR (600 MHz, DMSO-$d_6$): δ 8.33(s, 1H), 8.18 (d, *J*=6.0 Hz, 1H), 7.72 (d, *J*=6.0 Hz, 1H), 7.66-7.64 (m, 1H), 7.52-7.50 (m, 1H), 7.33-7.32 (m, 2H), 7.24 (t, J=54.0 Hz, 1H), 6.91-6.89 (m, 1H), 5.75-5.70 (m, 1H), 4.02-3.95 (m, 4H), 3.45(s, 3H), 3.25 (s, 3H), 1.58 (d, *J*=6.0 Hz, 3H).

**Example 8:** Synthesis of Compound 8

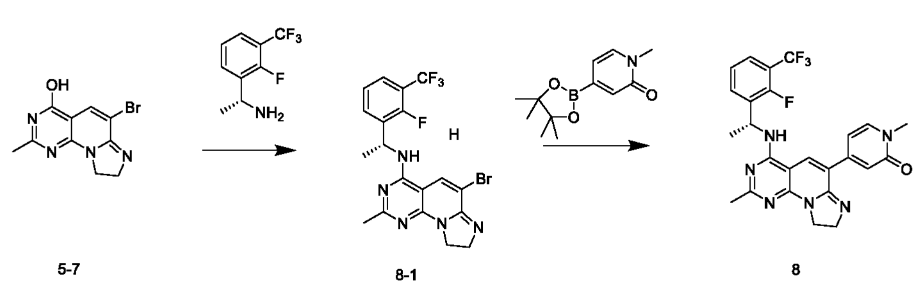

5-7 8-1 8

Synthesis of Intermediate 8-1

5-7 (100 mg, 0.36 mmol) was dissolved in N,N-dimethylformamide (15 mL), and then Intermediate G (112 mg, 0.54 mmol), tri(dimethylamino)benzotriazol-1-yloxyphosphonium hexafluorophosphate (205 mg, 0.47 mmol), and 1,8-diazabicycloundec-7-ene (163 mg, 1.08 mmol) were added. The system reacted at room temperature for 8 h. LC-MS analysis showed that no raw material was left. The reaction solution was added with water (30 mL), and extracted with ethyl acetate (30 mL×3). The organic phases were combined, washed with a saturated solution of sodium chloride (30 mL×2), and dried over anhydrous sodium sulfate. The solvent was removed by evaporation under reduced pressure. The residue was purified by thin layer chromatography (dichloromethane:methanol = 20:1) to afford 8-1 (127 mg, yield: 75%). ESI-MS: m/z 470.98/472.98 $[M+H]^+$.

Synthesis of Compound 8

8-1 (113 mg, 0.24 mmol) was dissolved in a solution of dioxane (10 mL) and water (2 mL), and then 1-methyl-4-(4,4,5,5-tetramethyl-1,3,2-dioxaborolan-2-yl)pyridin-2(1H)-one (86 mg, 0.366 mmol), [1,1'-bis(diphenylphosphino)ferrocene]dichloropalladium(II) (36 mg, 0.049 mmol), and cesium carbonate (159 mg, 0.488 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:triethylamine = 100:2:1) to afford Compound 8 (55 mg, yield: 46%). ESI-MS: m/z 499.11 $[M+H]^+$. $^1$H NMR (600 MHz, DMSO-$d_6$): δ 8.95 (s, 1H), 8.80 (s, 1H), 7.87-7.85 (m, 2H), 7.67 (dd, *J*=6.6 Hz, *J*=6.6 Hz, 1H), 7.39 (dd, *J*=7.8 Hz, *J*=7.8 Hz, 1H), 6.91 (s, 1H), 6.65 (d, *J*=6.6 Hz, 1H), 5.77-5.71 (m, 1H), 4.41-4.34 (m, 4H), 3.50 (s, 3H), 2.34 (s, 3H), 1.62 (d, *J*=6.0 Hz, 3H).

**Example 9:** Synthesis of Compound 9

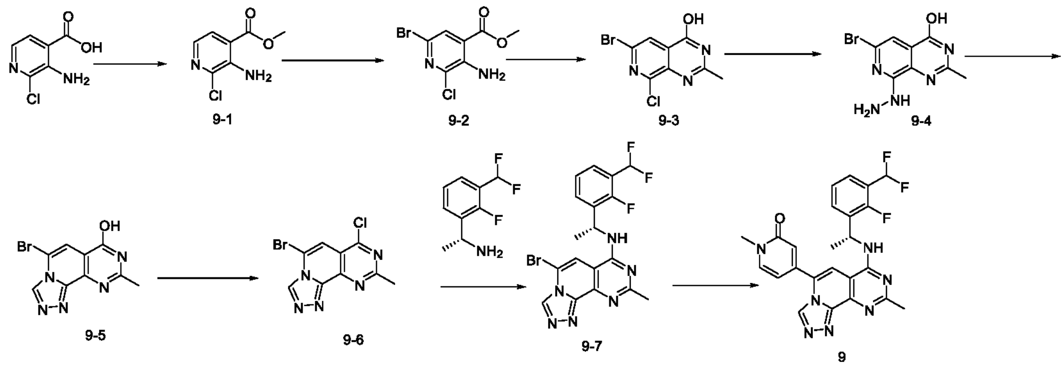

9-1 9-2 9-3 9-4 9-5 9-6 9-7 9

Synthesis of Intermediate 9-1

3-Amino-2-chloroisonicotinic acid (5.00 g, 29.0 mmol) was dissolved in methanol (80 mL). The system was cooled to 0°C, and then concentrated sulfuric acid (16 mL) was added. The system reacted at 80°C for 5 h. LC-MS analysis showed that no raw material was left. The reaction solution was added with water (100 mL), and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:1) to afford 9-1 (5.00 g, yield: 92%). ESI-MS: m/z 187.02 $[M+H]^+$.

Synthesis of Intermediate 9-2

9-1 (5.00 g, 26.8 mmol) was dissolved in acetonitrile (80 mL), and then N-bromosuccinimide (7.17 g, 40.3 mmol) was added. The system reacted at room temperature for 1.5 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous

sodium sulfate. The solvent was removed by evaporation under reduced pressure. The residue was separated and purified by column chromatography (petroleum ether:ethyl acetate = 5:1-1:3) to afford 9-2 (6.85 g, yield: 96%). ESI-MS: m/z 265.10 /267.12 $[M+H]^+$.

Synthesis of Intermediate 9-3

9-2 (6.85 g, 25.8 mmol) was dissolved in acetonitrile (80 mL), and then methanesulfonic acid (15 mL) was added. The system reacted at 130°C for 8 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:3) to afford 9-3 (1.51 g, yield: 21%). ESI-MS: m/z 274.02/276.11 $[M+H]^+$.

Synthesis of Intermediate 9-4

9-3 (1.51 g, 5.50 mmol) was dissolved in ethanol (50 mL), and then hydrazine hydrate (1.76 g, 55.0 mmol) was added. The system reacted at 80°C for 2 h. LC-MS analysis showed that no raw material was left. The solvent was removed by evaporation under reduced pressure. The residue was used directly in the next step. ESI-MS: m/z 270.02/271.06 $[M+H]^+$.

Synthesis of Intermediate 9-5

9-4 was dissolved in chloroform (30 mL), and then triethyl orthoformate (10 mL) was added. The system reacted at 80°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:3) to afford 9-5 (1.00 g, yield: 65%). ESI-MS: m/z 280.02/282.14 $[M+H]^+$.

Synthesis of Intermediate 9-6

9-5 (1.00 g, 3.60 mmol) was dissolved in toluene (50 mL), and then diisopropylethylamine (465 mg, 3.60 mmol) and phosphoryl chloride (2.76 g, 18.0 mmol) were added. The system reacted at 100°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:1) to afford 9-6 (800 mg, yield: 74%). ESI-MS: m/z 298.02/300.20 $[M+H]^+$.

Synthesis of Intermediate 9-7

9-6 (400 mg, 1.34 mmol) was dissolved in N,N-dimethylformamide (30 mL), and then diisopropylethylamine (349 mg, 2.70 mmol) and Intermediate D (384 mg, 2.02 mmol) were added. The system reacted at 120°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:2) to afford 9-7 (397 mg, yield: 66%). ESI-MS: m/z 451.02/453.09 $[M+H]^+$.

Synthesis of Compound 9

9-7 (397 mg, 0.88 mmol) was dissolved in dioxane (30 mL), and then 1-methyl-4-(4,4,5,5-tetramethyl-1,3,2-dioxaborolan-2-yl)pyridin-2(1H)-one (249 mg, 1.06 mmol), tetrakis(triphenylphosphine)palladium (104 mg, 0.09 mmol), and cesium carbonate (574 mg, 1.76 mmol) were added. The whole system was reacted with stirring at 100°C for 3 h. TLC analysis showed that no raw material was left. The reaction solution was added with water (50 mL), and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 9

(253 mg, yield: 60%). ESI-MS: m/z 480.11 $[M+H]^+$. $^1H$ NMR (600 MHz, DMSO-$d_6$): δ 9.34 (s, 1H), 8.58 (d, J=7.2 Hz, 1H), 7.98 (d, J=6.6 Hz, 1H), 7.94 (s, 1H), 7.70 (dd, *J*=7.2 Hz, *J*=7.2 Hz, 1H), 7.53(dd, J=6.6 Hz, J=7.2 Hz, 1H), 7.32 (dd, J=7.8 Hz, J=7.8 Hz, 1H), 7.26 (t, *J*=54.6 Hz, 1H), 6.87 (d, *J*=1.8 Hz, 1H), 6.66 (dd, *J*=1.8 Hz, *J*=2.4 Hz, 1H), 5.81-5.79 (m, 1H), 3.56 (s, 3H), 2.49 (s, 3H), 1.63 (d, *J*=6.6 Hz, 3H).

**Example 10:** Synthesis of Compound 10

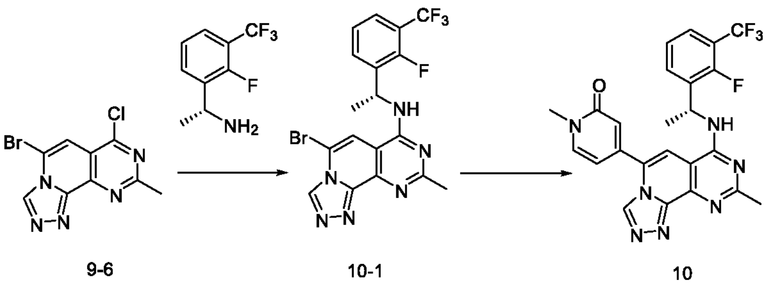

Synthesis of Intermediate 10-1

9-6 (400 mg, 1.34 mmol) was dissolved in N,N-dimethylformamide (30 mL), and then diisopropylethylamine (349 mg, 2.70 mmol) and Intermediate G (418 mg, 2.02 mmol) were added. The system reacted at 120°C for 2 h. LC-MS analysis showed that no raw material was left. The reaction solution was quenched with ice water, and extracted with ethyl acetate (100 mL×3). The organic phases were combined, washed with a saturated solution of sodium chloride (100 mL×2), and dried over anhydrous sodium sulfate. The solvent was removed by evaporation under reduced pressure. The residue was separated and purified by column chromatography (petroleum ether:ethyl acetate = 5:1-1:2) to afford 10-1 (415 mg, yield: 66%). ESI-MS: m/z 469.02/471.09 $[M+H]^+$.

Synthesis of Compound 10

10-1 (415 mg, 0.88 mmol) was dissolved in dioxane (30 mL), and then 1-methyl-4-(4,4,5,5-tetramethyl-1,3,2-dioxaborolan-2-yl)pyridin-2(1H)-one (249 mg, 1.06 mmol), tetrakis(triphenylphosphine)palladium (104 mg, 0.09 mmol), and cesium carbonate (574 mg, 1.76 mmol) were added. The whole system was reacted with stirring at 100°C for 3 h. TLC analysis showed that no raw material was left. The reaction solution was added with water (50 mL), and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 10 (250 mg, yield: 57%). ESI-MS: m/z 498.11 $[M+H]^+$. $^1H$ NMR (600 MHz, DMSO-$d_6$): δ 9.34 (s, 1H), 8.64 (d, *J*=6.6 Hz, 1H), 7.98 (d, *J*=6.6 Hz, 1H), 7.94 (s, 1H), 7.83(dd, *J*=7.2 Hz, *J*=7.2 Hz, 1H), 7.67 (dd, *J*=6.6 Hz, *J*=7.2 Hz, 1H), 7.38 (dd, *J*=7.8 Hz, *J*=7.8 Hz, 1H), 6.87 (d, *J*=1.8 Hz, 1H), 6.67 (dd, *J*=1.8 Hz, *J*=1.8 Hz, 1H), 5.78-5.74 (m, 1H), 3.56 (s, 3H), 2.47 (s, 3H), 1.64 (d, *J*=6.6 Hz, 3H).

**Example 11:** Synthesis of Compound 11

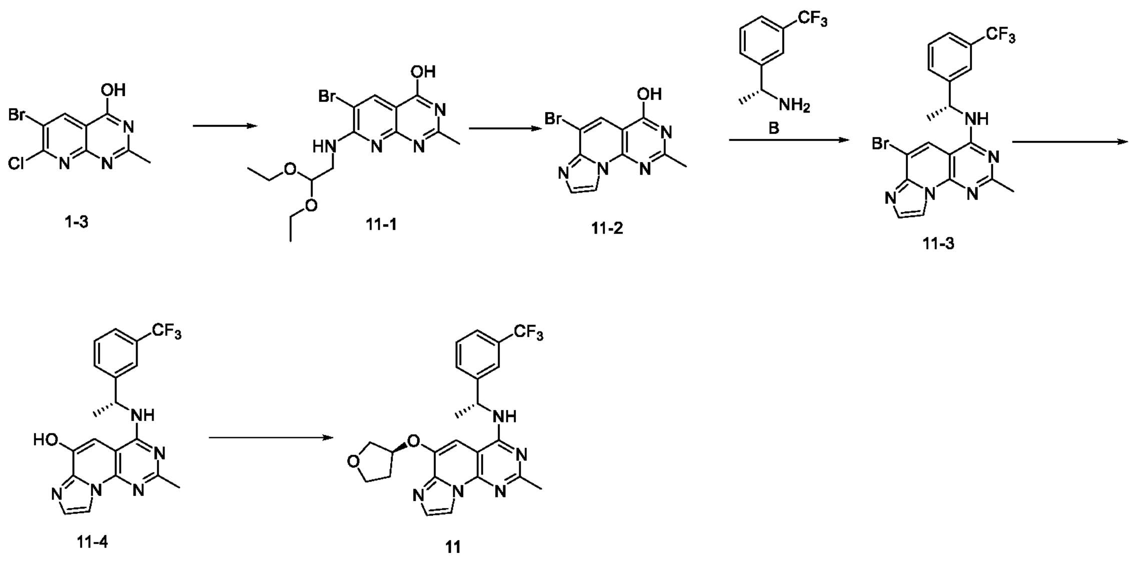


1-3 11-1 11-2 11-3 11-4 11

Synthesis of Intermediate 11-1

Intermediate 1-3 (0.68 g, 2.48 mmol) was dissolved in anhydrous ethanol (10 mL). Aminoacetaldehyde diethyl acetal (0.49 g, 3.72 mmol) and *N*,*N*-diisopropylethylamine (0.64 g, 4.96 mmol) were added, and reacted under reflux condition for 5 h. LC-MS analysis showed that no raw material was left. The reaction system was cooled, added with a saturated saline solution, and extracted with ethyl acetate. The organic phase was washed with a saturated saline solution, and dried over anhydrous sodium sulfate. The solvent was removed by evaporation under reduced pressure to afford 11-1 (0.83 g, yield: 90%). ESI-MS: m/z 371.58/373.32 $[M+H]^+$.

Synthesis of Intermediate 11-2

Intermediate 11-1 (0.83 g, 2.24 mmol) was dissolved in concentrated sulfuric acid, heated to 65°C and reacted for 2 h. LC-MS analysis showed that no raw material was left. The reaction solution was slowly dripped into a saturated sodium bicarbonate solution to quench the reaction. The resultant was extracted with ethyl acetate, and the organic phase was washed with a saturated saline solution, and dried over anhydrous sodium sulfate. The solvent was removed by evaporation under reduced pressure to afford 11-2 (0.40 g, yield: 63%). ESI-MS: m/z 279.10/301.14 $[M+H]^+$.

Synthesis of Intermediate 11-3

Intermediate 11-2 (0.26 g, 0.94 mmol) was dissolved in *N*,*N*-dimethylformamide (5 mL), and Intermediate B (0.27 g, 1.22 mmol) and benzotriazol-1-yl-oxytripyrrolidinophosphonium hexafluorophosphate (0.74 g, 1.41 mmol) were added, and reacted at room temperature for 2 h. LC-MS analysis showed that no raw material was left. The reaction system was added with a saturated saline solution, and extracted with ethyl acetate. The organic phase was washed with saturated saline solution, and dried over anhydrous sodium sulfate. The solvent was removed by evaporation under reduced pressure to afford 11-3 (0.18 g, yield: 43%). ESI-MS: m/z 449.90/451.91 $[M+H]^+$.

Synthesis of Intermediate 11-4

11-3 (180 mg, 0.40 mmol) was dissolved in a solution of dioxane (40 mL) and water (10 mL), and then potassium hydroxide (67 mg, 1.2 mmol), tris(dibenzylideneacetone)dipalladium (18 mg, 0.02 mmol), and 2-dicyclohexylphosphino-2',4',6'-triisopropylbiphenyl (10 mg, 0.02 mmol) were added. The whole system was reacted with stirring at 100°C for 3 h. TLC analysis showed that no raw material was left. The reaction solution was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford 11-4 (71 mg, yield: 46%). ESI-MS: m/z 388.07 $[M+H]^+$.

Synthesis of Compound 11

11-4 (71 mg, 0.16 mmol) was dissolved in *N*,*N*-dimethylformamide (15 mL), and then (*R*)-tetrahydrofuran-3-yl p-toluenesulfonate (47 mg, 0.19 mmol) and cesium carbonate (104 mg, 0.32 mmol) were added. The whole system was reacted with stirring at 80°C for 3 h. TLC analysis showed that no raw material was left. The reaction solution was added with 30 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-20:1) to afford Compound 11 (48 mg, yield: 66%). ESI-MS: m/z 458.32 $[M+H]^+$. $^1H$ NMR (600 MHz, DMSO-$d_6$): δ 8.29 (s, 1H), 7.87 (d, *J*=4.8 Hz, 1H), 7.82 (d, *J*=6.3 Hz, 1H), 7.67-7.53(m, 4H), 5.69 (p, *J*=7.2 Hz, 1H), 5.51 (d, *J*=6.5 Hz, 1H), 4.05 (dt, *J*=10.8, 3.6 Hz, 1H), 3.97-3.91 (m, 2H), 3.84 (td, *J*=8.3, 4.5 Hz, 1H), 2.45 (s, 3H), 1.68 (d, *J*=7.1 Hz, 3H), 1.23 (m, 2H).

**Example 12 to Example 30:**

The following compounds of Example 12 to Example 30 could be available by reference to the synthetic methods of Example 1 to Example 11.

| Example No. | Structure | ESI-MS / $[M+H]^+$ | $^1H$ NMR |
|---|---|---|---|
| 12 | 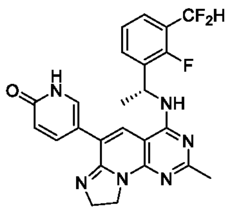 | 467.23 | $^1H$ NMR (600 MHz, DMSO-$d_6$): 811.75 (s, 1H), 8.68(s, 1H), 8.11-8.07 (m, 2H), 7.97 (d, *J*=7.2 Hz, 1H), 7.66-7.64 (m, 1H), 7.52-7.49 (m, 1H), 7.33-7.30 (m, 1H), 7.24 (t, *J*=54.0 Hz, 1H), 6.48 (d, *J*=9.6 Hz, 1H), 5.75-5.70 (m, 1H), 4.07-3.94 (m, 4H), 2.24 (s, 3H), 1.57 (d, *J*=6.6 Hz, 3H). |
| 13 | 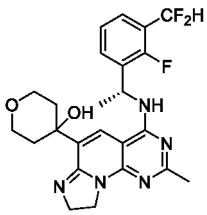 | 474.09 | |
| 14 | 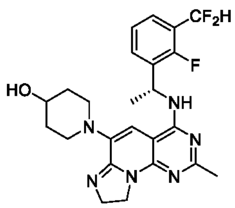 | 473.38 | |
| 15 | 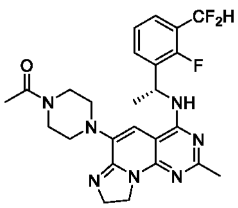 | 500.29 | |
| 16 | 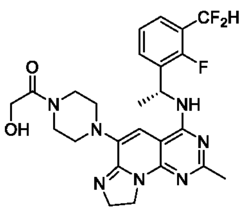 | 516.17 | |

(continued)

| Example No. | Structure | ESI-MS / [M+H]+ | 1H NMR |
|---|---|---|---|
| 17 | 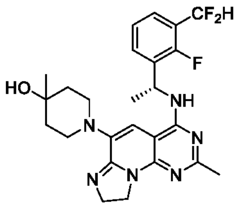 | 487.24 | |
| 18 | 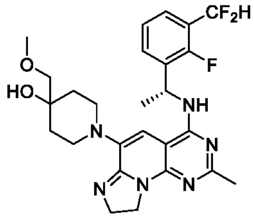 | 517.27 | |
| 19 | 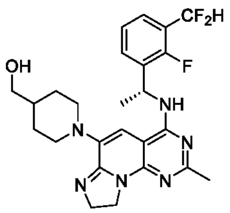 | 487.17 | |
| 20 | 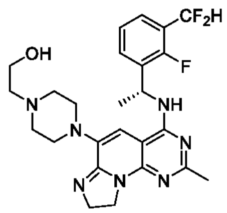 | 502.09 | |
| 21 | 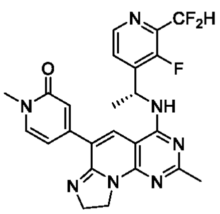 | 482.36 | |
| 22 | 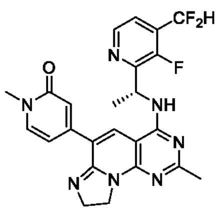 | 482.19 | |
| 23 | 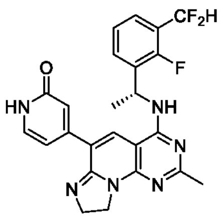 | 467.26 | |

(continued)

| Example No. | Structure | ESI-MS / [M+H]+ | 1H NMR |
|---|---|---|---|
| 24 | 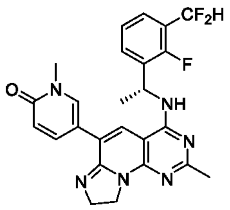 | 481.07 | |
| 25 | 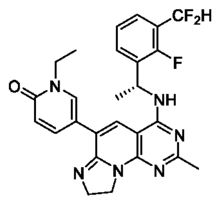 | 495.20 | |
| 26 | 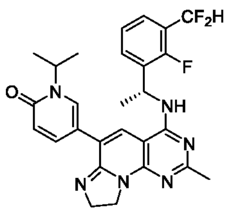 | 509.31 | |
| 27 | 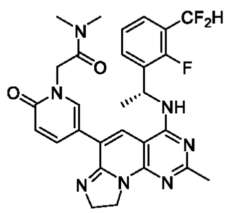 | 552.19 | |
| 28 | 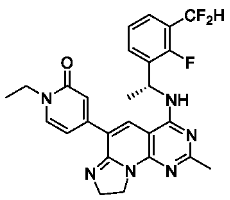 | 495.27 | |
| 29 | 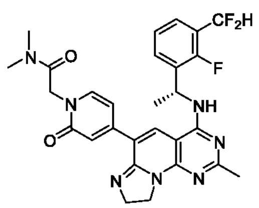 | 552.27 | |
| 30 | 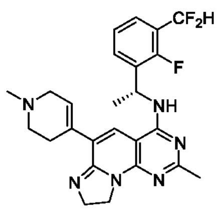 | 469.34 | |

**Example 31:** Synthesis of Compound 31

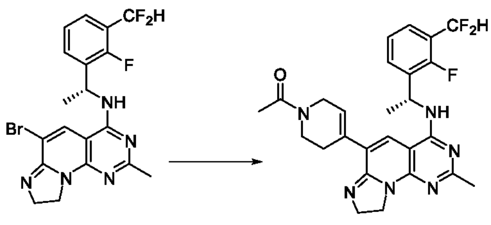

Synthesis of Compound 31

Intermediate 7-1 (99 mg, 0.22 mmol) in Example 7 was dissolved in dioxane (30 mL), and then 1-acetyl-5,6-dihydro-2H-pyridine-4-boronic acid pinacol ester (68 mg, 0.27 mmol), tetrakis(triphenylphosphine)palladium (23 mg, 0.02 mmol), and cesium carbonate (143 mg, 0.44 mmol) were added. The whole system was reacted with stirring at 100°C for 3 h. TLC analysis showed that no raw material was left. The reaction solution was added with 50 mL of water, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-15:1) to afford Compound 31 (69 mg, 0.14 mmol, yield: 64%). ESI-MS: m/z 497.24 $[M+H]^+$. $^1H$ NMR (600 MHz, DMSO-$d_6$): δ 8.23(s, 1H), 8.00 (s, 1H), 7.64 (dd, J=7.2 Hz, J=7.2 Hz, 1H), 7.50 (dd, *J*=7.2 Hz, *J*=7.2 Hz, 1H), 7.32-7.29 (m, 1H), 7.23(t, *J*=54.0 Hz, 1H), 6.82 (d, *J*=87.6 Hz, 1H), 5.75-5.70 (m, 1H), 4.22-4.02 (m, 4H), 3.95-3.92 (m, 2H), 3.67-3.62 (m, 2H), 2.50 (s, 3H), 2.26 (s, 3H), 2.06 (d, *J*=24.6 Hz, 2H), 1.56 (d, *J*=6.6 Hz, 3H).

**Example 32 to Example 92:**

The following compounds of Example 32 to Example 92 could be available by reference to the synthetic methods of Example 1 to Example 11.

| Example No. | Structure | ESI-MS / $[M+H]^+$ | $^1H$ NMR |
|---|---|---|---|
| 32 | 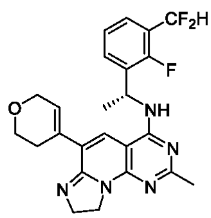 | 456.15 | |
| 33 | 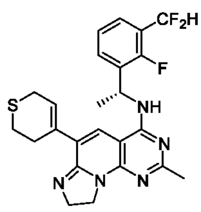 | 472.11 | |
| 34 | 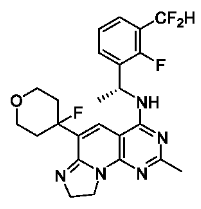 | 476.33 | |

(continued)

| Example No. | Structure | ESI-MS / [M+H]+ | 1H NMR |
|---|---|---|---|
| 35 | 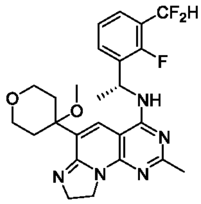 | 488.12 | |
| 36 | 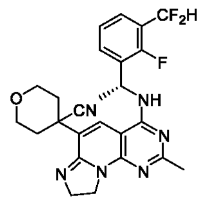 | 483.24 | |
| 37 | 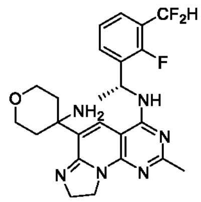 | 473.37 | |
| 38 | 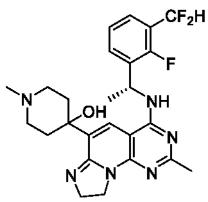 | 487.26 | |
| 39 | 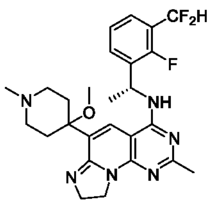 | 501.14 | |
| 40 | 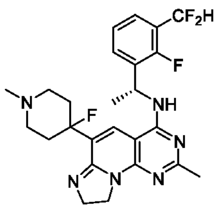 | 489.21 | |
| 41 | 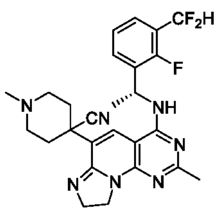 | 496.35 | |

(continued)

| Example No. | Structure | ESI-MS / [M+H]+ | 1H NMR |
|---|---|---|---|
| 42 | 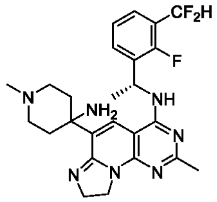 | 486.29 | |
| 43 | 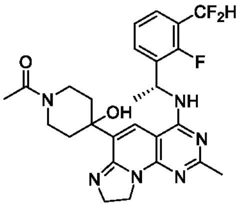 | 515.18 | |
| 44 | 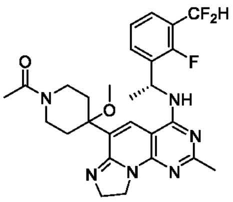 | 529.21 | |
| 45 | 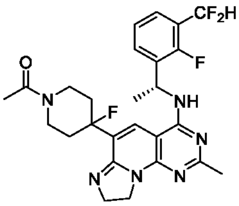 | 517.14 | |
| 46 | 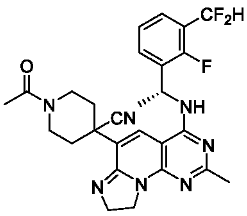 | 524.19 | |
| 47 | 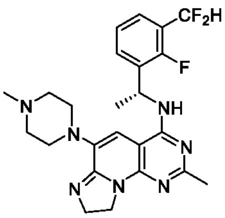 | 472.34 | |
| 48 | 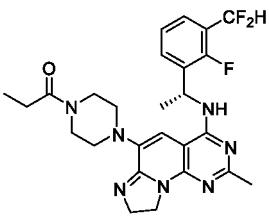 | 500.29 | |

(continued)

| Example No. | Structure | ESI-MS / [M+H]+ | 1H NMR |
|---|---|---|---|
| 49 | 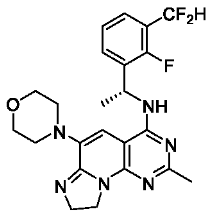 | 459.28 | |
| 50 | 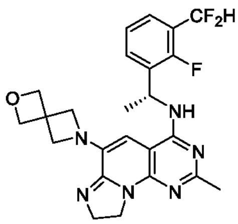 | 471.23 | |
| 51 | 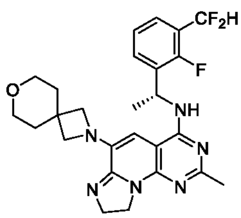 | 499.31 | |
| 52 | 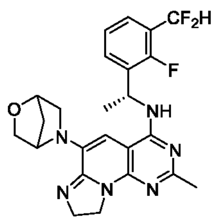 | 471.22 | |
| 53 | 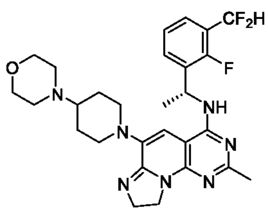 | 542.36 | |
| 54 | 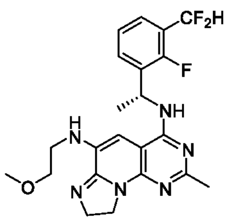 | 447.14 | |
| 55 | 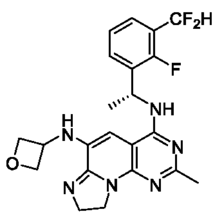 | 445.17 | |

(continued)

| Example No. | Structure | ESI-MS / [M+H]+ | 1H NMR |
|---|---|---|---|
| 56 | 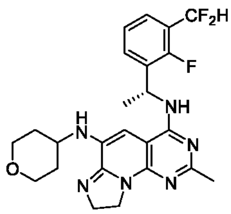 | 473.32 | |
| 57 | 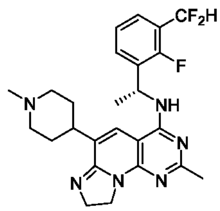 | 471.13 | |
| 58 | 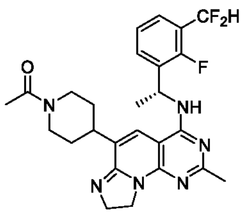 | 499.33 | |
| 59 | 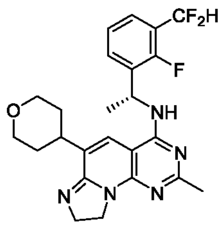 | 458.19 | |
| 60 | 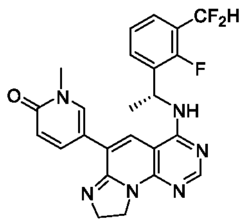 | 467.18 | |
| 61 | 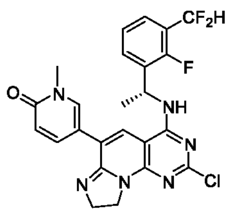 | 501.24 | |
| 62 | 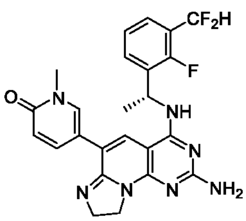 | 482.18 | |

(continued)

| Example No. | Structure | ESI-MS / [M+H]+ | 1H NMR |
|---|---|---|---|
| 63 | 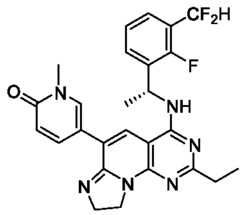 | 495.27 | |
| 64 | 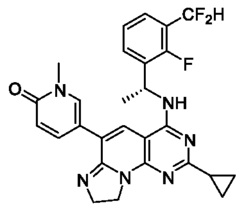 | 507.25 | |
| 65 | 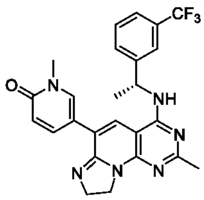 | 481.14 | 1H NMR (600 MHz, DMSO-$d_6$): δ 8.85 (s, 1H), 8.02 (d, *J*=14.2 Hz, 2H), 7.97 (d, *J*=6.8 Hz, 1H), 7.77 (s, 1H), 7.73(d, *J*=6.8 Hz, 1H), 7.65-7.52 (m, 2H), 6.53(d, *J*=9.5 Hz, 1H), 5.62-5.49 (m, 1H), 4.11-3.89 (m, 4H), 3.48 (s, 3H), 2.28 (s, 3H), 1.59 (d, *J*=7.0 Hz, 3H). |
| 66 | 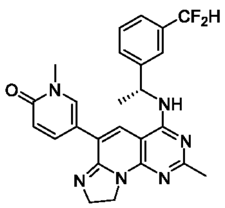 | 463.31 | |
| 67 | 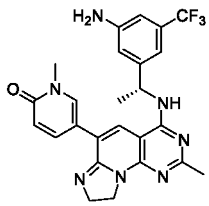 | 496.17 | |
| 68 | 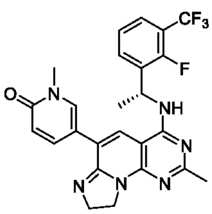 | 499.19 | |
| 69 | 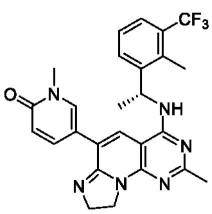 | 495.17 | |

(continued)

| Example No. | Structure | ESI-MS / $[M+H]^+$ | $^1H$ NMR |
|---|---|---|---|
| 70 | 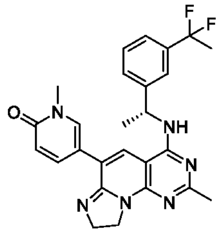 | 477.34 | |
| 71 | 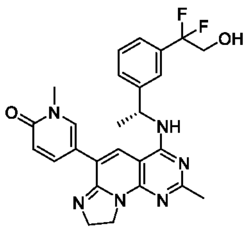 | 493.32 | $^1H$ NMR (600 MHz, $CD_3OD$): δ 8.29 (s, 1H), 8.01 (s, 1H), 7.92 (d, *J*=9.6 Hz, 1H), 7.63(s, 1H), 7.55 (d, *J*=7.2 Hz, 1H), 7.45-7.40 (m, 2H), 6.64 (d, *J*=9.6 Hz, 1H), 5.64-5.61 (m, 1H), 4.32-4.24 (m, 2H), 4.07 (t, *J*=9.6 Hz, 2H), 3.89 (t, *J*=13.2 Hz, 2H), 3.65 (s, 3H), 2.41 (s, 3H), 1.64 (d, *J*=7.2 Hz, 3H). |
| 72 | 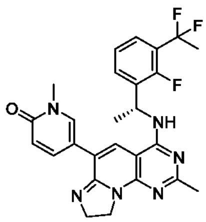 | 495.10 | |
| 73 | 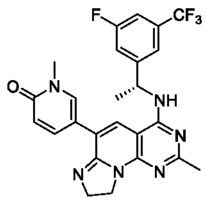 | 499.24 | |
| 74 | 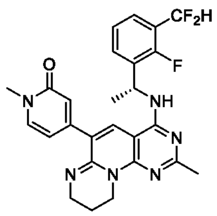 | 495.12 | |
| 75 | 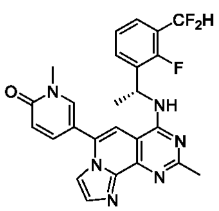 | 479.11 | |
| 76 | 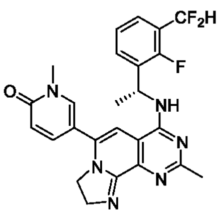 | 481.27 | |

(continued)

| Example No. | Structure | ESI-MS / [M+H]+ | $^1$H NMR |
|---|---|---|---|
| 77 | 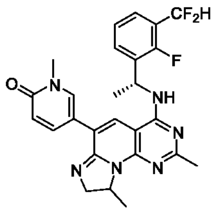 | 495.12 | |
| 78 | 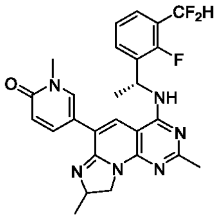 | 495.29 | |
| 79 | 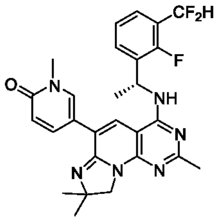 | 509.37 | |
| 80 | 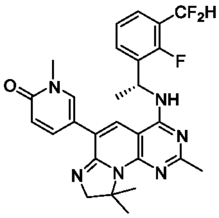 | 509.24 | |
| 81 | 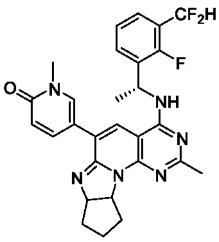 | 521.12 | |
| 82 | 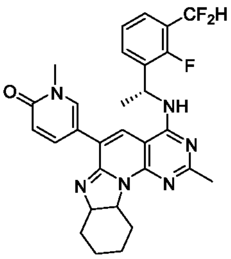 | 535.33 | |

(continued)

| Example No. | Structure | ESI-MS / [M+H]+ | 1H NMR |
|---|---|---|---|
| 83 | 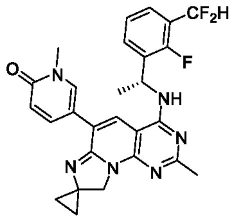 | 507.12 | |
| 84 | 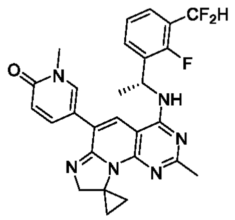 | 507.29 | |
| 85 | 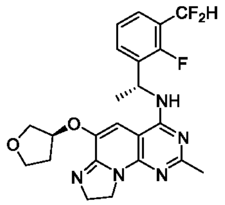 | 460.30 | |
| 86 | 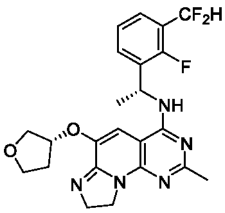 | 460.24 | |
| 87 | 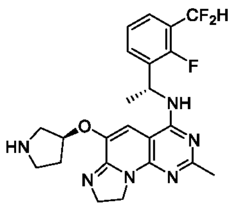 | 459.11 | |
| 88 | 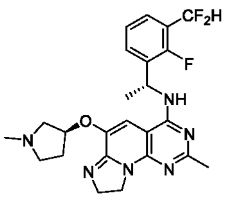 | 473.34 | |
| 89 | 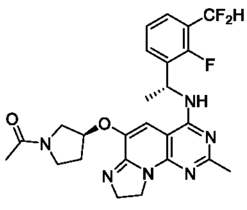 | 501.13 | |

(continued)

| Example No. | Structure | ESI-MS / [M+H]+ | $^1$H NMR |
|---|---|---|---|
| 90 | 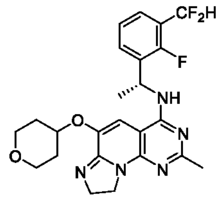 | 474.17 | |
| 91 | 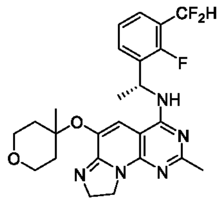 | 488.38 | |
| 92 | 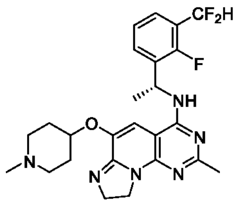 | 487.19 | |

**Example 93:** Synthesis of Compound 93

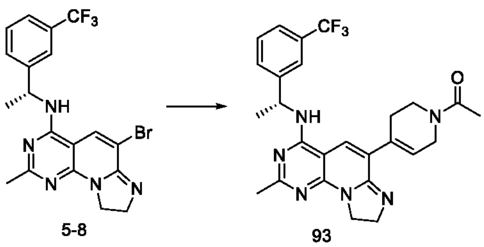

Synthesis of Compound 93

Intermediate 5-8 (40 mg, 0.089 mmol) in Example 5 was dissolved in a solution of dioxane (10 mL) and water (2 mL), and then 1-acetyl-5,6-dihydro-2H-pyridine-4-boronic acid pinacol ester (34 mg, 0.133 mmol), [1,1'-bis(diphenylphosphino)ferrocene]dichloropalladium(II) (13 mg, 0.018 mmol), and cesium carbonate (58 mg, 0.178 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:trimethylamine = 100:2:1) to afford Compound 93 (29 mg, 0.058 mmol, yield: 65%). ESI-MS: m/z 497.27 [M+H]$^+$. $^1$H NMR (600 MHz, DMSO-$d_6$): δ 7.97 (s, 1H), 7.76 (s, 1H), 7.72-7.71 (m, 2H), 7.62-7.56 (m, 2H), 7.06 (d, $J$=96.0 Hz, 1H), 5.60-5.55 (m, 1H), 4.16-4.10 (m, 2H), 4.03-3.90 (m, 4H), 3.66-3.62 (m, 2H), 2.51 (s, 3H), 2.27 (s, 3H), 2.08-2.04 (m, 2H), 1.57 (d, $J$=6.6 Hz, 3H).

**Example 94 to Example 108:**

The following compounds of Example 94 to Example 108 could be available by reference to the synthetic method of Example 93.

| Example No. | Structure | ESI-MS / [M+H] + | 1H NMR |
|---|---|---|---|
| 94 | 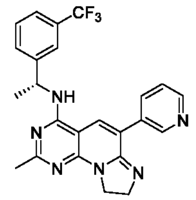 | 451.23 | $^1$H NMR (600 MHz, DMSO-$d_6$): δ 9.09 (s, 1H), 8.54 (d, *J*=3.6 Hz, 1H), 8.38 (d, *J*=8.0 Hz, 1H), 8.17 (s, 1H), 8.00 (d, *J*=7.6 Hz, 1H), 7.77 (s, 1H), 7.73(d, *J*=7.0 Hz, 1H), 7.60-7.56 (m, 2H), 7.49-7.40 (m, 1H), 5.60-5.54 (m, 1H), 4.08-3.87 (m, 4H), 2.28 (s, 3H), 1.57 (d, *J*=7.0 Hz, 3H). |
| 95 | 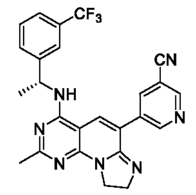 | 476.09 | $^1$H NMR (600 MHz, DMSO-$d_6$): δ 9.45 (s, 1H), 8.99 (s, 1H), 8.93(s, 1H), 8.33(s, 1H), 8.04 (d, *J*=7.0 Hz, 1H), 7.78 (s, 1H), 7.74 (d, *J*=6.9 Hz, 1H), 7.61-7.57 (m, 2H), 5.63-5.54 (m, 1H), 4.08-3.94 (m, 4H), 2.29 (s, 3H), 1.59 (d, *J*=6.9 Hz, 3H). |
| 96 | 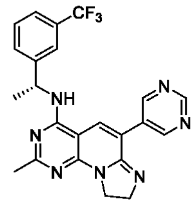 | 452.19 | $^1$H NMR (600 MHz, DMSO-$d_6$): δ 9.39 (s, 2H), 9.15 (s, 1H), 8.28 (s, 1H), 7.99 (d, *J*=6.4 Hz, 1H), 7.77 (s, 1H), 7.73(d, *J*=6.3 Hz, 1H), 7.63-7.57 (m, 2H), 5.61-5.54 (m, 1H), 4.08-3.91 (m, 4H), 2.29 (s, 3H), 1.58 (d, *J*=6.3 Hz, 3H). |
| 97 | 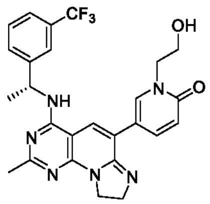 | 511.21 | $^1$H NMR (600 MHz, DMSO-$d_6$): δ 8.92 (s, 1H), 8.05 (dd, *J*=9.6 Hz, 2.6 Hz, 1H), 8.01 (s, 1H), 7.92 (d, *J*=7.6 Hz, 1H), 7.77 (s, 1H), 7.73(d, *J*=7.2 Hz, 1H), 7.63-7.54 (m, 2H), 6.53(d, J=9.6 Hz, 1H), 5.60-5.57 (m, 1H), 4.92 (t, *J*=5.3 Hz, 1H), 4.01-3.96 (m, 6H), 3.67-3.64 (m, 2H), 2.28 (s, 3H), 1.59 (d, *J*=7.2 Hz, 3H). |
| 98 | 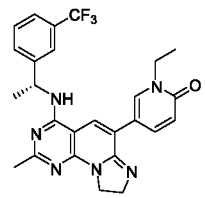 | 495.21 | $^1$H NMR (600 MHz, DMSO-$d_6$): δ 8.89 (s, 1H), 8.05 (s, 1H), 8.03-.95 (m, 2H), 7.78 (s, 1H), 7.74 (d, *J*=7.0 Hz, 1H), 7.65-7.53 (m, 2H), 6.52 (d, *J*=9.5 Hz, 1H), 5.60-5.55 (m, 1H), 4.19-3.76 (m, 6H), 2.27 (s, 3H), 1.59 (d, *J*=7.1 Hz, 3H), 1.26 (t, *J*=7.1 Hz, 3H). |
| 99 | 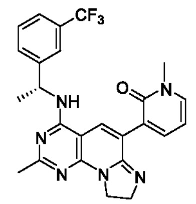 | 481.23 | $^1$H NMR (600 MHz, $CD_3OD$): δ 8.28 (s, 1H), 7.82-7.80 (m, 2H), 7.74 (s, 1H), 7.70 (d, *J*=7.4 Hz, 1H), 7.57-7.48 (m, 2H), 6.51 (t, *J*=6.8 Hz, 1H), 5.65-5.61 (m, 1H), 4.53-4.45 (m, 2H), 4.06 (t, *J*=10.2 Hz, 2H), 3.67 (s, 3H), 2.44 (s, 3H), 1.64 (d, *J*=7.1 Hz, 3H). |
| 100 | 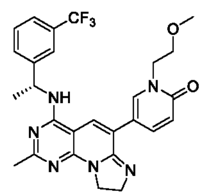 | 525.26 | $^1$H NMR (600 MHz, DMSO-$d_6$): δ 8.82 (s, 1H), 8.20-7.90 (m, 3H), 7.78 (s, 1H), 7.73(d, *J*=7.2 Hz, 1H), 7.62-7.56 (m, 2H), 6.55 (d, *J*=9.6 Hz, 1H), 5.63-5.55 (m, 1H), 4.14-4.04 (m, 4H), 4.00-3.97 (m, 2H), 3.62 (t, *J*=5.3 Hz, 2H), 3.26 (s, 3H), 2.29 (s, 3H), 1.59 (d, *J*=7.1 Hz, 3H). |

(continued)

| Example No. | Structure | ESI-MS / [M+H]+ | $^1$H NMR |
|---|---|---|---|
| 101 | 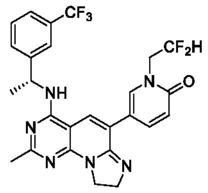 | 531.23 | $^1$H NMR (600 MHz, DMSO-$d_6$): δ 8.90 (s, 1H), 8.30-7.89 (m, 3H), 7.77 (s, 1H), 7.73(d, *J*=7.2 Hz, 1H), 7.61-7.57 (m, 2H), 6.62 (d, *J*=9.6 Hz, 1H), 6.35 (tt, *J*=55.4 Hz, 3.7 Hz, 1H), 5.67-5.51 (m, 1H), 4.43(td, *J*=14.8 Hz, 3.4 Hz, 2H), 4.17-3.83 (m, 4H), 2.29 (s, 3H), 1.59 (d, *J*=7.1 Hz, 3H). |
| 102 | 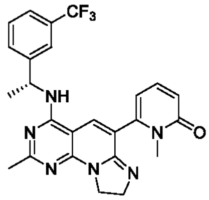 | 481.19 | $^1$H NMR (600 MHz, $CD_3OD$): δ 8.00 (s, 1H), 7.78-7.65 (m, 2H), 7.61-7.48 (m, 3H), 6.65 (dd, *J*=9.1, 1.1 Hz, 1H), 6.46 (dd, *J*=6.8, 1.2 Hz, 1H), 5.61 (q, *J*=7.0 Hz, 1H), 4.36-4.18 (m, 2H), 4.13-3.92 (m, 2H), 3.52 (s, 3H), 2.40 (s, 3H), 1.62 (d, *J*=7.1 Hz, 3H). |
| 103 | 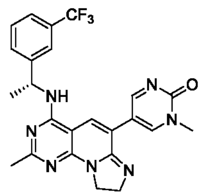 | 482.24 | $^1$H NMR (600 MHz, DMSO-$d_6$): δ 9.19 (s, 1H), 8.21 (s, 1H), 8.11-7.89 (m, 2H), 7.85-7.67 (m, 2H), 7.59 (s, 2H), 5.69-5.46 (m, 1H), 4.20-3.75 (m, 4H), 3.51 (s, 3H), 2.28 (s, 3H), 1.56 (d, *J*=7.2 Hz, 3H). |
| 104 | 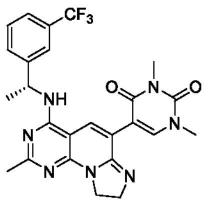 | 512.28 | $^1$H NMR (600 MHz, $CD_3OD$): δ 8.22 (s, 1H), 8.01 (s, 1H), 7.75 (s, 1H), 7.71 (d, *J*=7.1 Hz, 1H), 7.61-7.48 (m, 2H), 5.63(q, *J*=7.0 Hz, 1H), 4.45 (t, *J*=9.5 Hz, 2H), 4.07 (t, *J*=10.2 Hz, 2H), 3.49 (s, 3H), 3.40 (s, 3H), 2.43(s, 3H), 1.65 (d, *J*=7.1 Hz, 3H). |
| 105 | 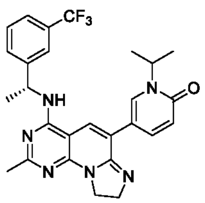 | 509.22 | $^1$H NMR (600 MHz, DMSO-$d_6$): δ 8.19-7.90 (m, 4H), 7.77 (s, 1H), 7.73(d, *J*=7.1 Hz, 1H), 7.64-7.52 (m, 2H), 6.54 (d, *J*=9.6 Hz, 1H), 5.63-5.55 (m, 1H), 5.14-5.03(m, 1H), 4.15-3.91 (m, 4H), 2.29 (s, 3H), 1.59 (d, *J*=7.1 Hz, 3H), 1.33(d, *J*=2.9 Hz, 3H), 1.31 (d, *J*=2.9 Hz, 3H). |
| 106 | 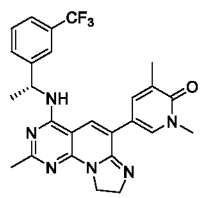 | 495.24 | $^1$H NMR (600 MHz, DMSO-$d_6$): δ 8.63(s, 1H), 8.01 (s, 1H), 7.98 (d, *J*=5.4 Hz, 1H), 7.85 (s, 1H), 7.78 (s, 1H), 7.74 (d, *J*=7.1 Hz, 1H), 7.65-7.54 (m, 2H), 5.64-5.51 (m, 1H), 4.16-3.91 (m, 4H), 3.50 (s, 3H), 2.28 (s, 3H), 2.11 (s, 3H), 1.60 (d, *J*=7.1 Hz, 3H). |
| 107 | 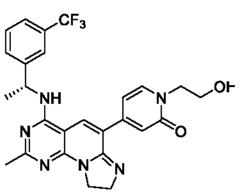 | 511.24 | $^1$H NMR (600 MHz, DMSO-$d_6$): δ 8.20 (s, 1H), 8.10 (d, *J*=7.7 Hz, 1H), 7.75 (s, 1H), 7.71 (d, *J*=7.2 Hz, 1H), 7.63-7.53(m, 3H), 7.25 (d, *J*=1.8 Hz, 1H), 6.83(dd, *J*=7.2 Hz, 2.0 Hz, 1H), 5.63-5.51 (m, 1H), 4.89 (t, *J*=5.3 Hz, 1H), 4.10-3.83(m, 6H), 3.63(dd, *J*=10.9 Hz, 5.5 Hz, 2H), 2.26 (s, 3H), 1.56 (d, *J*=7.1 Hz, 3H). |

(continued)

| Example No. | Structure | ESI-MS / [M+H]+ | $^1$H NMR |
|---|---|---|---|
| 108 | 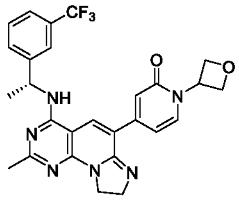 | 523.27 | $^1$H NMR (600 MHz, DMSO-$d_6$): δ 8.38 (s, 1H), 8.32 (s, 1H), 7.85 (d, *J*=7.3 Hz, 1H), 7.78 (s, 1H), 7.73(d, *J*=7.3 Hz, 1H), 7.66-7.56 (m, 2H), 7.19 (s, 1H), 6.94 (d, *J*=6.8 Hz, 1H), 5.69-5.49 (m, 2H), 4.91 (t, *J*=7.4 Hz, 2H), 4.77 (t, *J*=7.0 Hz, 2H), 4.21- 3.93(m, 4H), 2.31 (s, 3H), 1.59 (d, *J*=7.1 Hz, 3H). |

**Example 109:** Synthesis of Compound 109

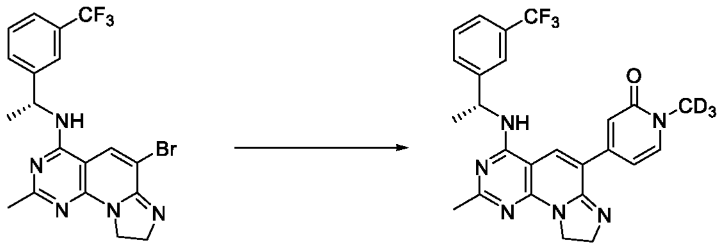

**5-8** **109**

Intermediate 5-8 (40 mg, 0.089 mmol) in Example 5 was dissolved in a solution of dioxane (10 mL) and water (2 mL), and then (1-(methyl-$d_3$)-2-oxo-1,2-dihydropyridin-4-yl)boronic acid pinacol ester (32 mg, 0.133 mmol), [1,1'-bis(diphenylphosphino)ferrocene]dichloropalladium(II) (13 mg, 0.018 mmol), and cesium carbonate (58 mg, 0.178 mmol) were added. The system reacted at 100°C for 4 h. LC-MS analysis showed that no raw material was left. The solvent was removed by evaporation under reduced pressure. The residue was extracted with ethyl acetate (10 mL×3). The organic phases were combined, washed with a saturated solution of sodium chloride (10 mL×2), and dried over anhydrous sodium sulfate. The solvent was removed by evaporation under reduced pressure. The residue was purified by thin layer chromatography (dichloromethane:methanol:trimethylamine = 100:2:1) to afford Compound 109 (25 mg, 0.051 mmol, yield: 57%). ESI-MS: m/z 484.24 [M+H]$^+$. $^1$H NMR (600 MHz, DMSO-$d_6$): δ 8.29 (s, 1H), 8.22 (d, *J*=7.2 Hz, 1H), 7.77 (s, 1H), 7.76-7.68 (m, 2H), 7.65-7.54 (m, 2H), 7.28 (s, 1H), 6.86 (d, *J*=7.0 Hz, 1H), 5.65-5.53(m, 1H), 4.22-3.87 (m, 4H), 2.29 (s, 3H), 1.59 (d, J=7.0 Hz, 3H).

**Example 110 to Example 121:**

The following compounds of Example 110 to Example 121 could be available by reference to the synthetic method of Example 93.

| Example No. | Structure | ESI-MS / [M+H]+ | $^1$H NMR |
|---|---|---|---|
| 110 | 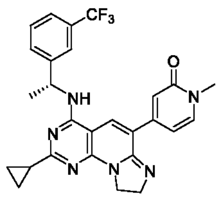 | 507.29 | $^1$H NMR (600 MHz, DMSO-$d_6$): δ 8.29 (s, 1H), 8.21 (d, *J*=6.7 Hz, 1H), 7.72 (dd, *J*=11.8, 7.6 Hz, 2H), 7.67 (d, *J*=7.9 Hz, 1H), 7.62-7.53(m, 2H), 7.32 (s, 1H), 6.89 (dd, *J*=7.2, 1.9 Hz, 1H), 5.38-5.33(m, 1H), 4.15-3.86 (m, 4H), 3.45 (s, 3H), 1.87-1.75 (m, 1H), 1.56 (d, *J*=7.1 Hz, 3H), 1.07- 0.99 (m, 1H), 0.91-0.85 (m, 1H), 0.81-0.75 (m, 1H), 0.55-0.51 (m, 1H). |

(continued)

| Example No. | Structure | ESI-MS / [M+H]+ | 1H NMR |
|---|---|---|---|
| 111 | 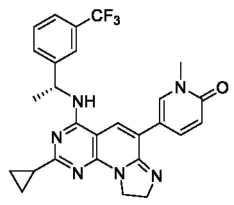 | 507.24 | $^1H$ NMR (600 MHz, DMSO-$d_6$): δ 8.77 (s, 1H), 8.15 (s, 2H), 8.01 (d, *J*=9.3 Hz, 1H), 7.72 (s, 1H), 7.69 (d, *J*=7.2 Hz, 1H), 7.63-7.52 (m, 2H), 6.54 (d, *J*=9.5 Hz, 1H), 5.42-5.31 (m, 1H), 4.22-3.92 (m, 4H), 3.50 (s, 3H), 1.82 (ddd, *J*=12.6, 8.2, 4.7 Hz, 1H), 1.57 (d, *J*=7.1 Hz, 3H), 1.08-0.99 (m, 1H), 0.92-0.83(m, 1H), 0.81-0.73(m, 1H), 0.57-0.47 (m, 1H). |
| 112 | 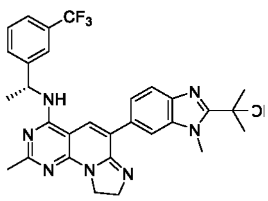 | 562.28 | $^1H$ NMR (600 MHz, DMSO-$d_6$): δ 8.56-8.30 (m, 2H), 8.15 (s, 1H), 7.82 (s, 1H), 7.77 (d, *J*=5.3 Hz, 1H), 7.67 (d, *J*=8.4 Hz, 1H), 7.64-7.50 (m, 3H), 5.69 (d, *J*=5.0 Hz, 1H), 5.65-5.56 (m, 1H), 4.35-4.14 (m, 2H), 4.09-3.90 (m, 5H), 2.34 (s, 3H), 1.77-1.53(m, 9H). |
| 113 | 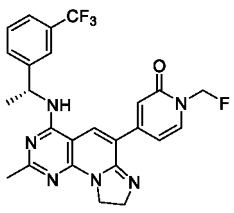 | 499.18 | $^1H$ NMR (600 MHz, DMSO-$d_6$): δ 8.30 (s, 1H), 8.17 (d, *J*=7.7 Hz, 1H), 7.84 (d, *J*=7.4 Hz, 1H), 7.77 (s, 1H), 7.73(d, *J*=7.2 Hz, 1H), 7.65-7.55 (m, 2H), 7.52 (s, 1H), 6.97 (dd, *J*=7.4, 1.8 Hz, 1H), 5.96 (d, *J*=51.3 Hz, 2H), 5.64-5.55 (m, 1H), 4.09-3.91 (m, 4H), 2.28 (s, 3H), 1.59 (d, *J*=7.1 Hz, 3H). |
| 114 | 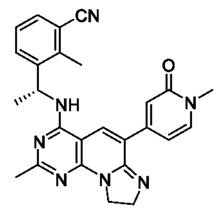 | 452.23 | $^1H$ NMR (600 MHz, DMSO-$d_6$): δ 8.27 (s, 1H), 8.24 (d, *J*=6.9 Hz, 1H), 7.74 (d, *J*=7.8 Hz, 1H), 7.71 (d, *J*=7.2 Hz, 1H), 7.64 (d, *J*=7.6 Hz, 1H), 7.40-7.37 (m, 1H), 7.31 (s, 1H), 6.89 (dd, *J*=7.1, 1.6 Hz, 1H), 5.57 (p, *J*=6.9 Hz, 1H), 4.15-3.83(m, 4H), 3.45 (s, 3H), 2.68 (s, 3H), 2.26 (s, 3H), 1.51 (d, *J*=7.0 Hz, 3H). |
| 115 | 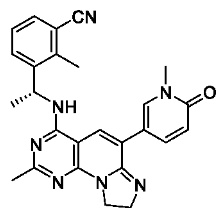 | 452.26 | $^1H$ NMR (600 MHz, DMSO-$d_6$): δ 8.74 (s, 1H), 8.17 (s, 2H), 7.99 (d, *J*=8.7 Hz, 1H), 7.77 (d, *J*=7.8 Hz, 1H), 7.64 (d, *J*=7.1 Hz, 1H), 7.40-7.37 (m, 1H), 6.55 (d, *J*=9.5 Hz, 1H), 5.68-5.53 (m, 1H), 4.24-3.85 (m, 4H), 3.50 (s, 3H), 2.68 (s, 3H), 2.28 (s, 3H), 1.52 (d, *J*=7.0 Hz, 3H). |
| 116 | 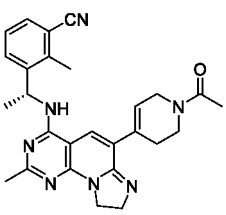 | 468.27 | $^1H$ NMR (600 MHz, DMSO-$d_6$): δ 8.12 (s, 1H), 7.81 (s, 1H), 7.74 (d, *J*=7.0 Hz, 1H), 7.62 (d, *J*=6.6 Hz, 1H), 7.38-7.35 (m, 1H), 6.97 (d, *J*=99.5 Hz, 1H), 5.62-5.49 (m, 1H), 4.36-3.81 (m, 6H), 3.65-3.61 (m, 2H), 2.66 (s, 3H), 2.24 (s, 3H), 2.05 (d, *J*=26.0 Hz, 2H), 1.49 (d, *J*=5.9 Hz, 3H). |
| 117 | 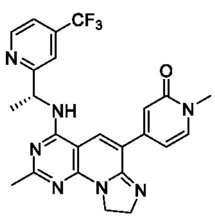 | 482.18 | $^1H$ NMR (600 MHz, $CD_3OD$): δ 8.77 (d, *J*=4.8 Hz, 1H), 8.74 (s, 1H), 7.85 (d, *J*=7.2 Hz, 1H), 7.80 (s, 1H), 7.59 (d, *J*=4.8 Hz, 1H), 6.87 (s, 1H), 6.69 (dd, *J*=7.2 Hz, *J*=1.8 Hz, 1H), 5.69-5.65 (m, 1H), 4.66-4.63(m, 2H), 4.17-4.13(m, 2H), 3.66 (s, 3H), 2.42 (s, 3H), 1.72 (d, *J*=7.2 Hz, 3H). |

(continued)

| Example No. | Structure | ESI-MS / [M+H]+ | $^1$H NMR |
|---|---|---|---|
| 118 | 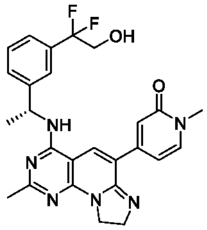 | 493.21 | $^1$H NMR (600 MHz, DMSO-$d_6$): δ 8.65-8.60 (m, 2H), 7.80 (d, *J*=6.6 Hz, 1H), 7.61 (s, 1H), 7.57 (d, *J*=7.8 Hz, 1H), 7.47-7.44 (m, 1H), 7.40 (d, *J*=7.2 Hz, 1H), 7.06 (s, 1H), 6.73(d, *J*=6.6 Hz, 1H), 5.64-5.59 (m, 1H), 4.30-4.20 (m, 2H), 3.98 (t, *J*=10.2 Hz, 2H), 3.87-3.82 (m, 2H), 3.47 (s, 3H), 2.36 (s, 3H), 1.59 (d, *J*=7.2 Hz, 3H). |
| 119 | 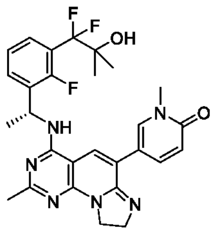 | 539.24 | $^1$H NMR (600 MHz, DMSO-$d_6$): δ 8.85 (s, 1H), 8.18-8.07 (m, 3H), 7.62-7.60 (m, 1H), 7.32-7.30 (m, 1H), 7.24-7.22 (m, 1H), 6.53(d, *J*=9.0 Hz, 1H), 5.77-5.71 (m, 1H), 5.34 (s, 1H), 4.10-3.96 (m, 4H), 3.49 (s, 3H), 2.24 (s, 3H), 1.55 (d, *J*=7.2 Hz, 3H), 1.24 (s, 3H), 1.18 (s, 3H). |
| 120 | 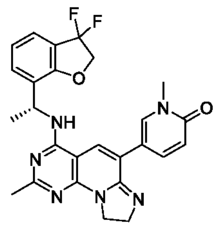 | 491.20 | $^1$H NMR (600 MHz, DMSO-$d_6$): δ 8.69 (s, 1H), 8.24 (s, 1H), 8.10 (s, 1H), 7.96 (d, *J*=9.0 Hz, 1H), 7.57 (d, *J*=7.8 Hz, 1H), 7.52 (d, *J*=7.2 Hz, 1H), 7.12-7.10 (m, 1H), 6.55 (d, *J*=9.0 Hz, 1H), 5.70-5.67 (m, 1H), 4.87 (d, *J*=16.8 Hz, 2H), 4.20-4.17 (m, 2H), 4.01-3.98 (m, 2H), 3.49 (s, 3H), 2.29 (s, 3H), 1.56 (d, *J*=6.6 Hz, 3H). |
| 121 | 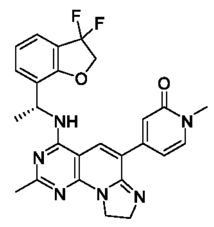 | 491.23 | $^1$H NMR (600 MHz, DMSO-$d_6$): δ 8.92 (s, 2H), 7.93(d, *J*=6.6 Hz, 1H), 7.60 (d, *J*=7.8 Hz, 1H), 7.55 (d, *J*=7.2 Hz, 1H), 7.13-7.10 (m, 1H), 6.71 (s, 1H), 6.48 (d, *J*=6.6 Hz, 1H), 5.74-5.69 (m, 1H), 4.88 (t, *J*=16.2 Hz, 2H), 4.60-4.53(m, 2H), 4.03(t, *J*=10.2 Hz, 2H), 3.51 (s, 3H), 2.42 (s, 3H), 1.59 (d, *J*=6.6 Hz, 3H). |

**Example 122:** Synthesis of Compound 122

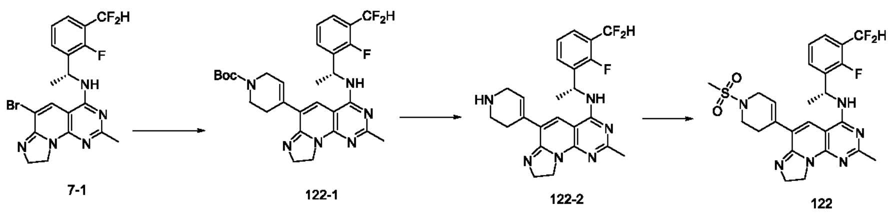

7-1 122-1 122-2 122

Synthesis of Intermediate 122-1

7-1 (99 mg, 0.22 mmol) was dissolved in dioxane (30 mL), and then N-Boc-1,2,5,6-tetrahydropyridine-4-boronic acid pinacol ester (84 mg, 0.27 mmol), tetrakis(triphenylphosphine)palladium (23 mg, 0.02 mmol), and cesium carbonate (143 mg, 0.44 mmol) were added. The whole system was reacted with stirring at 100°C for 3 h. TLC analysis showed that no raw material was left. The reaction solution was added with 50 mL of water, and extracted with ethyl acetate (30 mL×3). The organic phases were combined, washed with a saturated solution of sodium chloride (50 mL×2), and dried

over anhydrous sodium sulfate. The solvent was removed under reduced pressure. The residue was separated and purified by column chromatography (dichloromethane:methanol = 60:1-20:1) to afford 122-1 (100 mg, yield: 82%). ESI-MS: m/z 555.19 $[M+H]^+$.

Synthesis of Intermediate 122-2

122-1 (100 mg, 0.18 mmol) was dissolved in a hydrochloric acid 4M solution in ethyl acetate (10 mL), and reacted with stirring at room temperature for 2 h. TLC analysis showed that no raw material was left. The solvent was removed under reduced pressure to afford 122-2 as a residue, which was used directly in the next step. ESI-MS: m/z 455.18 $[M+H]^+$.

Synthesis of Compound 122

122-2 was dissolved in dichloromethane (10 mL), and then triethylamine (36 mg, 0.36 mmol) and methylsufonyl chloride (25 mg, 0.22 mmol) were added, and reacted with stirring at room temperature for 2 h. TLC analysis showed that no raw material was left. The reaction solution was added with water to quench the reaction, and extracted with ethyl acetate (30 mL×3). The organic phases were combined, washed with a saturated solution of sodium chloride (50 mL×2), and dried over anhydrous sodium sulfate. The solvent was removed under reduced pressure. The residue was separated and purified by column chromatography (dichloromethane:methanol = 60:1-15:1) to afford Compound 122 (50 mg, yield: 52%). ESI-MS: m/z 533.20 $[M+H]^+$. $^1$H NMR (600 MHz, DMSO-$d_6$): δ 8.00 (s, 1H), 7.80 (s, 1H), 7.64 (dd, *J*=7.2 Hz, *J*=7.2 Hz, 1H), 7.50 (dd, *J*=7.2 Hz, *J*=7.2 Hz,1H), 7.32-7.30 (m, 1H), 7.24 (t, *J*=54.0 Hz, 1H), 7.11 (s, 1H), 5.75-5.70 (m, 1H), 4.00-3.88 (m, 6H), 3.40-3.37 (m, 2H), 2.95 (s, 3H), 2.72-2.63(m, 2H), 2.24 (s, 3H), 1.56 (d, *J*=6.6 Hz, 3H).

**Example 123 and Example 124:**

The following compounds of Example 123 and Example 124 could be available by reference to the synthetic method of Example 93.

| Example No. | Structure | ESI-MS / [M+H] + | $^1$H NMR |
|---|---|---|---|
| 123 | 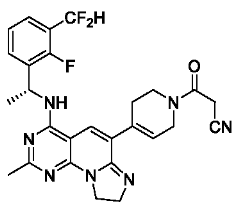 | 522.22 | $^1$H NMR (600 MHz, $CD_3OD$): δ 7.83(s, 1H), 7.57 (dd, *J*=7.2 Hz, *J*=7.2 Hz, 1H), 7.49 (dd, *J*=7.2 Hz, *J*=7.2 Hz,1H), 7.27-7.23 (m, 1H), 7.01 (t, *J*=55.2 Hz, 1H), 6.39 (d, *J*=87.6 Hz, 1H), 5.78-5.74 (m, 1H), 4.26-4.01 (m, 6H), 3.86-3.84 (m, 1H), 3.72-3.70 (m, 1H), 3.29 (s, 2H), 2.68-2.64 (m, 1H), 2.60-2.56 (m, 1H), 2.34 (s, 3H), 1.63(d, *J*=7.2 Hz, 3H). |
| 124 | 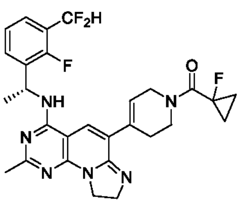 | 541.27 | $^1$H NMR (600 MHz, DMSO-$d_6$): δ 8.73(s, 1H), 8.53(s, 1H), 7.67 (dd, *J*=7.2 Hz, *J*=7.2 Hz, 1H), 7.53(dd, *J*=7.2 Hz, *J*=7.2 Hz, 1H), 7.34-7.31 (m, 1H), 7.24 (t, *J*=54.0 Hz, 1H), 6.29 (s, 1H), 5.79-5.74 (m, 1H), 4.53-4.47 (m, 2H), 4.21-3.89 (m, 6H), 3.50-3.34 (m, 2H), 2.38 (s, 3H), 1.61 (d, *J*=6.6 Hz, 3H), 1.37-1.31 (m, 2H), 1.27-1.21 (m, 2H). |

**Example 125:** Synthesis of Compound 125

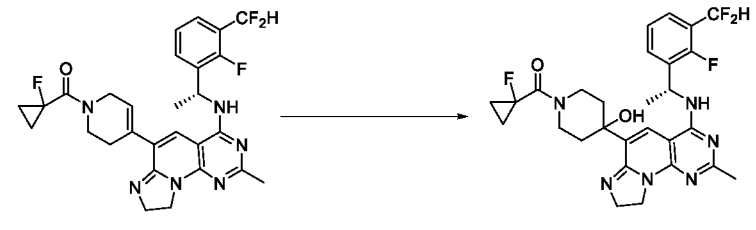

124 125

Compound 124 (100 mg, 0.18 mmol) was dissolved in dichloromethane/isopropanol (22 mL, $V_1/V_2$=1/10), and phenylsilane (19.5 mg, 0.18 mmol) and tris(2,2,6,6-tetramethyl-3,5-heptanedionato)manganese (7.6 mg, 0.01 mmol) were successively added at room temperature. The reaction solution was purged three times under an oxygen atmosphere, and stirred at room temperature for 16 h while being kept under an oxygen atmosphere (balloon). LC-MS showed that the raw material was completely consumed. The reaction solution was filtered, and the resulting filtrate was concentrated under reduced pressure to afford a crude product. The crude product was separated and purified by column chromatography (dichloromethane:methanol = 60:1-15:1) to afford Compound 125 (28 mg, yield: 28%). ESI-MS: m/z 559.24 $[M+H]^+$. $^1H$ NMR (400 MHz, $CD_3OD$): δ 8.32 (d, *J*=16.0 Hz, 1H), 7.57 (t, *J*=7.2 Hz, 1H), 7.47 (t, *J*=7.2 Hz, 1H), 7.23(t, *J*=8.0 Hz, 1H), 6.99 (t, *J*=54.8 Hz, 1H), 5.82-5.73(m, 1H), 4.56-4.24 (m, 4H), 4.10 (t, *J*=10.0 Hz, 2H), 3.85-3.45 (m, 2H), 2.39 (s, 3H), 2.10-2.06 (m, 4H), 1.64 (d, *J*=7.2 Hz, 3H), 1.28-1.24 (m, 4H).

**Bioactivity Assays:**

1. K-$Ras^{G12D}$ and hSOS1 binding assay

This assay could be used to examine the potency of the compounds to inhibit the protein-protein interaction between SOS1 and KRAS G12D. The binding of anti-GSK-Europium (FRET donor) -conjugated GST-$KRas^{G12D}$ to anti-6His-XL665-conjugated His-tagged hSOS1 (FRET donor) was detected by homogeneous time-resolved fluorescence (HTRF), and the inhibitory effects of the compounds on the binding of K-$Ras^{G12D}$ to hSOS1 were determined.

1.1 Reagents

Buffer (5 mM HEPES pH 7.4, 150 mM NaCl, 10 mM EDTA, 1 mM DTT, 0.05% BAS pH 7.0, 0.0025% Igepal, and 100 mM KF);
GST-tagged hK-RasG12D (produced in-house);
His-tagged hSOS1 (produced in-house);

Preparation of a liquid mixture of Ras:

10 nM (final concentration) GST-hK-RasG12D and 2 nM (final concentration) anti-GSK-Europium in assay buffer were mixed, and set aside at room temperature.

Preparation of a liquid mixture of SOS:

20 nM (final concentration) His-tagged hSOS1 and 10 nM (final concentration) anti-6His-XL665 in assay buffer were mixed, and set aside at room temperature.
The test compound was dissolved in DMSO at a concentration of 100 times the assay concentration. 50 nL of the solution of compound was pipetted by Hummingbird liquid handler or Echo acoustic system to a black microassay plate.

1.2 Experimental steps

All the experimental steps were conducted at 20°C. In the experiment, 2.5 μL of the liquid mixture of Ras was added to all wells of the assay plate using a Multidrop dispenser. After pre-incubation for 2 min, 2.5 μL of the liquid mixture of SOS was added to all the test wells other than the marginal wells, and 2.5 μL of the solution of compound, as a control, was added to the marginal wells. After incubation for 60 min, the assay plate passed through the HTRF

module of Pheraster (excitation light: 337 nm, emission light 1: 620 nm, emission light 2: 665 nm).

1.3 Data calculation

The $IC_{50}$ values were calculated and analyzed using a four-parameter logistic regression model.

1.4 SOS1 inhibitory activity results

The representative compounds of Examples were tested according to the above method. The data on SOS1 inhibitory activity were as shown in the table below.

| Compound of Example No. | Activity by $IC_{50}$ (nM) | Compound of Example No. | Activity by $IC_{50}$ (nM) |
|---|---|---|---|
| 1 | B | 2 | B |
| 3 | B | 4 | B |
| 5 | B | 6 | A |
| 7 | A | 8 | A |
| 9 | B | 10 | B |
| 11 | D | 12 | A |
| 31 | B | 65 | B |
| 71 | A | 97 | A |
| 98 | A | 99 | B |
| 109 | B | 118 | A |
| 122 | A | 123 | A |
| 124 | A | | |

In the table, A denoted $IC_{50}$ < 10 nM, B denoted 10 nM ≤ $IC_{50}$ < 50 nM, C denoted 50 nM ≤ $IC_{50}$ < 100 nM, D denoted 100 nM ≤ $IC_{50}$ < 300 nM.

2. 3D Cell Proliferation Inhibition Assay

The cell proliferation inhibition assay was used to determine the abilities of compounds to inhibit the proliferation and growth of SOS 1-mediated tumor cells at the *in vitro* 3D cellular level. The CellTiter-Glo® 3D Cell Viability Assay was employed.

2.1 Reagents and materials

NCI-H358: KRAS G12C-mutated non-small cell lung cancer (NSCLC);
CellTiter-Glo® 3D Cell Viability Assay, Promega, G9683;
RPMI 1640 medium, Gibco, A10491-01;
FBS, Gibco, 10099141C.

2.2 Experimental steps

2.2.1 Cell culture

Day 1: NCI-H358 cells were passaged into a T75 cell culture flask.

Day 3: the medium was removed; the cells were rinsed once with DPBS, and digested at room temperature or at 37°C using 2 mL of TrypLE™ Express Enzyme until the cells were detached; the resultant was added with 5 mL of fresh medium, and centrifuged at 1000 rpm for 5 min; the supernatant was discarded, and 5 mL of fresh medium was

added to resuspend the cells; the cells were counted and then seeded at 40 μL/well in a 3D cell culture plate (Echo Qualified 384-Well Polypropylene Microplate 2.0, Clear, Flat Bottom).

2.2.2 3D Cell proliferation inhibition

Day 1: the test compound was dissolved in DMSO to prepare a 10 mM stock solution; after a 1000-fold dilution with DMSO, the test compound solution was subjected to ten 3-fold serial dilutions starting from a concentration of 10 μM; 200 nL of the compound solution was added to the culture plate.
Day 8: CTG 3D reagent was added at 40 μL/well, and the signal was detected using Envision.

2.3 Data analysis

The $IC_{50}$ values of the compounds were fitted using the Graphpad Prism 8 nonlinear regression equation.

$$Y=Bottom + (Top-Bottom)/(1+10^{\wedge}((LogIC_{50}-X)*HillSlope));$$

X: Log of cpd concentration;
Y: Percent inhibition (% inh).

2.4 Results of inhibitory activity against NCI-H358 cell proliferation

The compounds of Examples were tested according to the above method. The data on inhibitory activity agaisnt NCI-H358 cell proliferation were as shown in the table below.

| Compound of Example No. | Activity by $IC_{50}$ (nM) | Compound of Example No. | Activity by $IC_{50}$ (nM) |
|---|---|---|---|
| 2 | C | 3 | C |
| 4 | C | 5 | A |
| 6 | A | 7 | A |
| 12 | C | 31 | A |
| 65 | C | 71 | C |
| 94 | C | 95 | C |
| 97 | C | 98 | C |
| 99 | C | 100 | C |
| 101 | C | 102 | C |
| 103 | C | 104 | C |
| 105 | C | 106 | C |
| 107 | C | 108 | B |
| 109 | B | 110 | C |
| 111 | C | 112 | C |
| 113 | C | 117 | C |
| 118 | C | 119 | B |
| 120 | C | 121 | C |
| 122 | B | 123 | B |
| 124 | B | 125 | B |

In the table, A denoted $IC_{50}$ < 50 nM, B denoted 50 nM ≤ $IC_{50}$ < 100 nM, C denoted 100 nM ≤ $IC_{50}$ < 500 nM.

## Claims

1. A compound represented by formula (A), or a stereoisomer, optical isomer, pharmaceutical salt, prodrug, solvate or isotope derivative thereof,

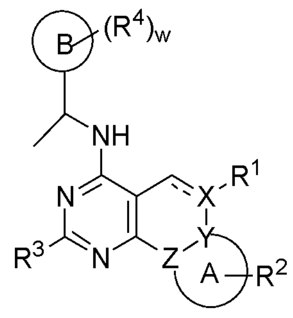

(A)

wherein ----- represents a single bond or a double bond;
Y and Z are both C or N, where Z is C when Y is N and Z is N when Y is C;
Y and Z together with the atoms to which they are linked form a ring A, where the ring A is 5- to 12-membered heterocyclyl or 5- to 12-membered heteroaryl;
there is one, two or three $R^2$, each of which at each occurrence is independently hydrogen, halogen, hydroxyl, cyano, amino, nitro, formyl, oxo, $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more cyano, hydroxyl or halogen;
$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;
ring B is $C_{4-12}$ cycloalkyl, $C_{4-12}$ cycloalkenyl, 4- to 12-membered heterocyclyl, $C_{6-12}$ aryl, 5- to 12-membered heteroaryl, $C_{6-12}$ aryl-fused $C_{4-12}$ cycloalkyl, $C_{6-12}$ aryl-fused 4- to 12-membered heterocyclyl or $C_{6-12}$ aryl-fused $C_{4-12}$ cycloalkenyl;
each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2, 3 or 4;
when ----- is a double bond, X is C, and $R^1$ linked thereto is -O-$R^A$, -N($R^D$)$R^B$ or $R^C$;
when $R^1$ is -O-$R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;
each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -O$C_{1-6}$ alkyl, -S$C_{1-6}$ alkyl, -CO$C_{1-6}$ alkyl, -$CH_2$CO$C_{1-6}$ alkyl, -$CH_2$CON($C_{1-6}$ alkyl)$_2$, -$CH_2$CONH$C_{1-6}$ alkyl, -NH$C_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;
when $R^1$ is -N($R^D$)$R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, -O$C_{1-6}$ alkyl, -S$C_{1-6}$ alkyl, -CO$C_{1-6}$ alkyl, -$CH_2$CO$C_{1-6}$ alkyl, -$CH_2$CON($C_{1-6}$ alkyl)$_2$, -$CH_2$CONH$C_{1-6}$ alkyl, -NH$C_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;
$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, -O$C_{1-6}$

alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl or $-N(C_{1-6}$ alkyl$)_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from deuterium, hydroxyl, cyano, $C_{1-3}$ alkyl, $C_{1-3}$ alkoxy or halogen;

when ----- is a single bond, X is N, and $R^1$ linked thereto is $C_{3-10}$ cycloalkyl, $C_{3-10}$ cycloalkenyl, $C_{6-10}$ aryl, 3- to 10-membered heterocyclyl or 5- to 10-membered heteroaryl, wherein the $C_{3-10}$ cycloalkyl, $C_{3-10}$ cycloalkenyl, $C_{6-10}$ aryl, 3- to 10-membered heterocyclyl, and 5- to 10-membered heteroaryl are all optionally substituted with one or more identical or different $R^{a4}$ and/or $R^{b4}$;

each of $R^{a4}$, if present, is independently $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{b4}$ and/or $R^{c4}$;

each of $R^{b4}$, if present, is independently $-OR^{c4}$, $-NR^{c4}R^{c4}$, halogen, -CN, $-C(O)R^{c4}$, $-C(O)OR^{c4}$, $-C(O)NR^{c4}R^{c4}$, $-OC(O)R^{c4}$, $-S(O)_2R^{c4}$, $-S(O)_2NR^{c4}R^{c4}$, $-NHC(O)R^{c4}$, $-N(C_{1-4}$ alkyl$)C(O)R^{c4}$, $-NHC(O)OR^{c4}$ or a divalent substituent =O or =NH, where the =O and =NH may only be substituents in a non-aromatic ring system;

each of $R^{c4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{d4}$ and/or $R^{e4}$;

each of $R^{d4}$, if present, is independently $-OR^{e4}$, $-NR^{e4}R^{e4}$, halogen, -CN, $-C(O)R^{e4}$, $-C(O)OR^{e4}$, $-C(O)NR^{e4}R^{e4}$, $-S(O)_2R^{e4}$, $-S(O)_2NR^{e4}R^{e4}$, $-NHC(O)R^{e4}$, $-N(C_{1-4}$ alkyl$)C(O)R^{e4}$ or a divalent substituent =O, where the =O may only be a substituent in a non-aromatic ring system;

each of $R^{e4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more hydrogen, cyano, hydroxyl or halogen;

wherein Z and Y are deemed as the atoms of ring A and counted in the number of the atoms of ring A;

unless otherwise stated, heteroatoms in the heteroaryl and heterocyclyl are each independently O, N or S, and the number of heteroatoms is 1, 2, 3 or 4.

2. The compound or the stereoisomer, optical isomer, pharmaceutical salt, prodrug or solvate thereof according to claim 1, wherein the compound has a structure represented by formula (A'):

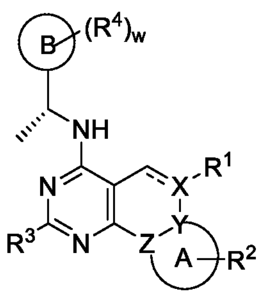

(A')

the substituents in formula (A') are as defined in formula (A).

3. A compound represented by formula (I), or a stereoisomer, optical isomer, pharmaceutical salt, prodrug or solvate

thereof,

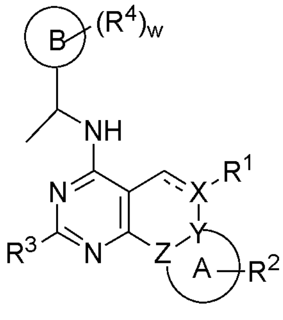

(I)

wherein ----- represents a single bond or a double bond;

Y and Z are both C or N, where Z is C when Y is N and Z is N when Y is C;

Y and Z together with the atoms to which they are linked form a ring A, where the ring A is 5- to 12-membered heterocyclyl or 5- to 12-membered heteroaryl;

there is one, two or three $R^2$, each of which at each occurrence is independently hydrogen, halogen, hydroxyl, cyano, amino, nitro, formyl, oxo, $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{1-6}$ alkoxy, -$C_{1-6}$ alkyl-NH($C_{1-6}$ alkyl), -$C_{1-6}$ alkyl-N($C_{1-6}$ alkyl)$_2$, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more cyano, hydroxyl or halogen;

$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;

ring B is $C_{4-12}$ cycloalkyl, $C_{4-12}$ cycloalkenyl, $C_{4-12}$ heterocyclyl, $C_{6-12}$ aryl, $C_{5-12}$ heteroaryl, $C_{6-12}$ aryl-fused $C_{4-12}$ cycloalkyl, $C_{6-12}$ aryl-fused $C_{4-12}$ heterocyclyl or $C_{6-12}$ aryl-fused $C_{4-12}$ cycloalkenyl;

each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, -$C_{0-6}$ alkyl-NH-$C_{1-6}$ alkyl, -$C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2, 3 or 4;

when ----- is a double bond, X is C, and $R^1$ linked thereto is -O-$R^A$, -N($R^D$)$R^B$ or $R^C$;

when $R^1$ is -O-$R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

when $R^1$ is -N($R^D$)$R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;

each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl, -N($C_{1-6}$ alkyl)$_2$, $C_{3-10}$ cycloalkyl, $C_{6-10}$ aryl, 3- to 10-membered heterocyclyl or 5- to 10-membered heteroaryl;

$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON$($C_{1-6}$ alkyl)$_2$, -$CH_2CONHC_{1-6}$ alkyl, -$NHC_{1-6}$ alkyl or -N($C_{1-6}$ alkyl)$_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl,

$C_{6-10}$ aryl, 3- to 10-membered heterocyclyl, and 5- to 10-membered heteroaryl are all optionally substituted with 1 to 3 identical or different $R^{c1}$;

each of $R^{c1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from hydroxyl, $C_{1-3}$ alkyl, $C_{1-3}$ alkoxy or halogen;

when $\overline{-----}$ is a single bond, X is N, and $R^1$ linked thereto is $C_{3-10}$ cycloalkyl, $C_{3-10}$ cycloalkenyl, $C_{6-10}$ aryl, 3- to 10-membered heterocyclyl or 5- to 10-membered heteroaryl, wherein the $C_{3-10}$ cycloalkyl, $C_{3-10}$ cycloalkenyl, $C_{6-10}$ aryl, 3- to 10-membered heterocyclyl, and 5- to 10-membered heteroaryl are all optionally substituted with one or more identical or different $R^{a4}$ and/or $R^{b4}$;

each of $R^{a4}$, if present, is independently $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{b4}$ and/or $R^{c4}$;

each of $R^{b4}$, if present, is independently $-OR^{c4}$, $-NR^{c4}R^{c4}$, halogen, -CN, $-C(O)R^{c4}$, $-C(O)OR^{c4}$, $-C(O)NR^{c4}R^{c4}$, $-OC(O)R^{c4}$, $-S(O)_2R^{c4}$, $-S(O)_2NR^{c4}R^{c4}$, $-NHC(O)R^{c4}$, $-N(C_{1-4}$ alkyl$)C(O)R^{c4}$, $-NHC(O)OR^{c4}$ or a divalent substituent =O or =NH, where the =O and =NH may only be substituents in a non-aromatic ring system;

each of $R^{c4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more identical or different $R^{d4}$ and/or $R^{e4}$;

each of $R^{d4}$, if present, is independently $-OR^{e4}$, $-NR^{e4}R^{e4}$, halogen, -CN, $-C(O)R^{e4}$, $-C(O)OR^{e4}$, $-C(O)NR^{e4}R^{e4}$, $-S(O)_2R^{e4}$, $-S(O)_2NR^{e4}R^{e4}$, $-NHC(O)R^{e4}$, $-N(C_{1-4}$ alkyl$)C(O)R^{e4}$ or a divalent substituent =O, where the =O may only be a substituent in a non-aromatic ring system;

each of $R^{e4}$, if present, is independently hydrogen, $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with one or more hydrogen, cyano, hydroxyl or halogen;

wherein Z and Y are deemed as the atoms of ring A and counted in the number of the atoms of ring A;

unless otherwise stated, heteroatoms in the heteroaryl and heterocyclyl are each independently O, N or S, and the number of heteroatoms is 1, 2, 3 or 4.

4. The compound or the stereoisomer, optical isomer, pharmaceutical salt, prodrug or solvate thereof according to claim 3, wherein the compound has a structure represented by formula (II):

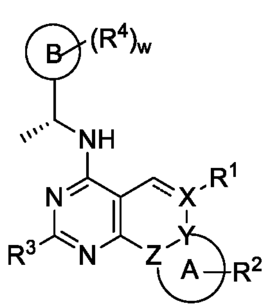

(II)

the substituents in formula (II) are as defined in formula (I).

5. The compound or the stereoisomer, optical isomer, pharmaceutical salt, prodrug or solvate thereof according to claim 3, wherein the compound has a structure represented by formula (III):

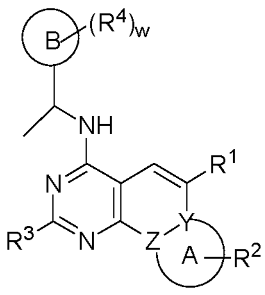

(III)

the substituents in formula (III) are as defined in formula (I).

6. The compound or the stereoisomer, optical isomer, pharmaceutical salt, prodrug or solvate thereof according to claim 3, wherein the compound has a structure represented by formula (IV):

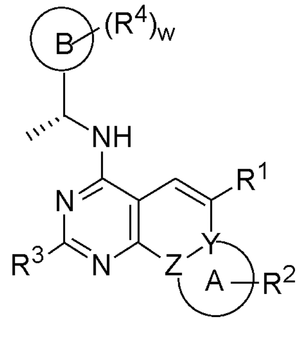

(IV)

the substituents in formula (IV) are as defined in formula (I).

7. The compound or the stereoisomer, optical isomer, pharmaceutical salt, prodrug or solvate thereof according to any one of claims 1 to 6, wherein



is a double bond, X is C, and $R^1$ linked thereto is -O-$R^A$;
preferably, $R^A$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;
each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, -$OC_{1-6}$ alkyl, -$SC_{1-6}$ alkyl, -$COC_{1-6}$ alkyl, -$CH_2COC_{1-6}$ alkyl, -$CH_2CON(C_{1-6}$ alkyl$)_2$, -$CH_2CONHC_{1-6}$ alkyl, -$NHC_{1-6}$ alkyl or -$N(C_{1-6}$ alkyl$)_2$;
further preferably, $R^A$ is $C_{3-6}$ cycloalkyl, $C_{6-10}$ aryl, 3- to 6-membered heterocyclyl or 5- to 6-membered heteroaryl, wherein the $C_{3-6}$ cycloalkyl, $C_{6-10}$ aryl, 3- to 6-membered heterocyclyl, and 5- to 6-membered heteroaryl are all optionally substituted with 1 to 3 identical or different $R^{a1}$;
each of $R^{a1}$, if present, is independently halogen, hydroxyl, cyano, amino, oxo, formyl, $C_{1-6}$ alkyl, -$OC_{1-6}$ alkyl, -$SC_{1-6}$ alkyl or -$COC_{1-6}$ alkyl;
further preferably, $R^A$ is 5- to 6-membered heterocyclyl or 5- to 6-membered heteroaryl, wherein the 5- to 6-membered heterocyclyl and 5- to 6-membered heteroaryl are both optionally substituted with 1 to 3 identical or different $R^{a1}$;
each of $R^{a1}$, if present, is independently halogen, hydroxyl, cyano, amino, oxo, formyl, $C_{1-4}$ alkyl, -$OC_{1-4}$ alkyl, -$SC_{1-4}$ alkyl or -$COC_{1-4}$ alkyl;
further preferably, $R^A$ is 5- to 6-membered heterocyclyl, wherein the 5- to 6-membered heterocyclyl is optionally substituted with one or two identical or different $R^{a1}$;
each of $R^{a1}$, if present, is independently halogen, oxo, formyl, acetyl, methyl, ethyl, n-propyl, isopropyl or methoxy;
further preferably, $R^A$ is 5- to 6-membered monocyclic heterocyclyl, wherein the 5- to 6-membered monocyclic heterocyclyl is optionally substituted with one or two identical or different $R^{a1}$; heteroatoms in the 5- to 6-membered monocyclic heterocyclyl are each independently O, N or S, and the number of heteroatoms is 1; each of $R^{a1}$, if present, is independently halogen, oxo, formyl, acetyl, methyl, ethyl, n-propyl, isopropyl or methoxy; further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;
each of $R^{a1}$, if present, is independently halogen, oxo, formyl, acetyl, methyl, ethyl, n-propyl, isopropyl or methoxy; even further preferably, $R^A$ is the following group:

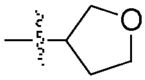, 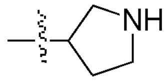,

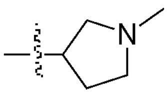, 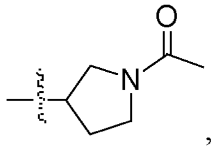, 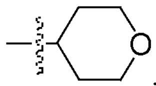, 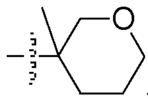, or 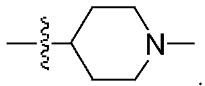.

**8.** The compound or the stereoisomer, optical isomer, pharmaceutical salt, prodrug or solvate thereof according to any one of claims 1 to 6, wherein



is a double bond, X is C, and $R^1$ linked thereto is $-N(R^D)R^B$;
preferably, $R^B$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl;
further preferably, $R^B$ is $C_{1-6}$ alkyl, 3- to 10-membered heterocyclyl or 5- to 10-membered heteroaryl, wherein the $C_{1-6}$ alkyl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl;
further preferably, $R^B$ is $C_{1-6}$ alkyl or 5- to 6-membered monocyclic heterocyclyl, wherein the $C_{1-6}$ alkyl and 5- to 6-membered monocyclic heterocyclyl are both optionally substituted with one or two identical or different $R^{b1}$; heteroatoms in the 5-to 6-membered monocyclic heterocyclyl are each independently O, N or S, and the number of heteroatoms is 1;
each of $R^{b1}$, if present, is independently halogen, oxo, formyl, $C_{1-4}$ alkyl, $-OC_{1-4}$ alkyl or $-COC_{1-4}$ alkyl;
further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;
each of $R^{b1}$, if present, is independently oxo, formyl, acetyl, methyl, ethyl, methoxy, or ethoxy;
even further preferably, $R^B$ is the following group:

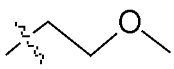, 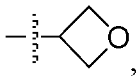, or

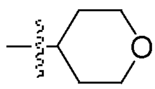;

preferably, $R^D$ is hydrogen, $C_{1-6}$ alkyl or $-OC_{1-6}$ alkyl; further preferably, $R^D$ is hydrogen or $C_{1-3}$ alkyl; even further preferably, $R^D$ is hydrogen or methyl; most preferably, $R^D$ is hydrogen.

**9.** The compound or the stereoisomer, optical isomer, pharmaceutical salt, prodrug or solvate thereof according to any one of claims 1 to 6, wherein



is a double bond, X is C, and $R^1$ linked thereto is $R^C$;

preferably, $R^C$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{c1}$;

each of $R^{c1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl or 3- to 10-membered heterocyclyl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, and 3- to 10-membered heterocyclyl are all optionally substituted with one or more substituents selected from hydroxyl, $C_{1-3}$ alkyl, $C_{1-3}$ alkoxy or halogen;

further preferably, $R^C$ is $C_{1-6}$ alkyl, $C_{3-10}$ cycloalkyl, 3- to 10-membered heterocyclyl or 5- to 10-membered heteroaryl, wherein the $C_{1-6}$ alkyl, $C_{3-10}$ cycloalkyl, 3- to 10-membered heterocyclyl, and 5- to 10-membered heteroaryl are all optionally substituted with 1 to 3 identical or different $R^{c1}$;

each of $R^{c1}$, if present, is independently halogen, hydroxyl, cyano, amino, nitro, oxo, formyl, $C_{1-4}$ alkyl, $-OC_{1-4}$ alkyl, $-SC_{1-4}$ alkyl, $-COC_{1-4}$ alkyl, $-CH_2CON(C_{1-4}$ alkyl$)_2$, $-CH_2CONHC_{1-4}$ alkyl or 3- to 6-membered heterocyclyl, wherein the $C_{1-4}$ alkyl, $-OC_{1-4}$ alkyl, $-SC_{1-4}$ alkyl, $-COC_{1-4}$ alkyl, $-CH_2CON(C_{1-4}$ alkyl$)_2$, $-CH_2CONHC_{1-4}$ alkyl, and 3- to 6-membered heterocyclyl are all optionally substituted with one or more substituents selected from hydroxyl, methyl, methoxy or halogen;

further preferably, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 3 identical or different $R^{c1}$;

each of $R^{c1}$, if present, is independently halogen, hydroxyl, cyano, amino, oxo, formyl, $C_{1-4}$ alkyl, $-OC_{1-4}$ alkyl, $-COC_{1-4}$ alkyl, $-CH_2CON(C_{1-4}$ alkyl$)_2$ or 3- to 6-membered heterocyclyl, wherein the $C_{1-4}$ alkyl, $-OC_{1-4}$ alkyl, $-COC_{1-4}$ alkyl, $-CH_2CON(C_{1-4}$ alkyl$)_2$, and 3- to 6-membered heterocyclyl are all optionally substituted with one or more substituents selected from hydroxyl, methyl, methoxy or halogen;

further preferably, $R^C$ is 5- to 6-membered monocyclic heterocyclyl, 6- to 10-membered spiro heterocyclyl, 6- to 8-membered bridged heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, and 5- to 6-membered monocyclic heteroaryl are all optionally substituted with 1 to 3 identical or different $R^{c1}$;

each of $R^{c1}$, if present, is independently halogen, hydroxyl, cyano, amino, oxo, formyl, acetyl, propionyl, methoxy, ethoxy, methyl, ethyl, n-propyl, isopropyl, $-CH_2CON(CH_3)_2$ or 6-membered heterocyclyl, wherein the acetyl, propionyl, methoxy, ethoxy, methyl, ethyl, n-propyl, isopropyl, $-CH_2CON(CH_3)_2$, and 6-membered heterocyclyl are all optionally substituted with one or more substituents selected from hydroxyl, methyl, methoxy or halogen;

even further preferably, $R^C$ is 6-membered monocyclic heterocyclyl, 4-membered/6-membered spiro heterocyclyl, 4-membered/4-membered spiro heterocyclyl, 7-membered bridged heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 7-membered bridged heterocyclyl, and 6-membered monocyclic heteroaryl are all optionally substituted with 1 to 3 identical or different $R^{c1}$;

each of $R^{c1}$, if present, is independently halogen, hydroxyl, cyano, amino, oxo, formyl, acetyl, $-COCH_2CH_3$, $-COCH_2OH$, hydroxymethyl, hydroxyethyl, $-CH_2OCH_3$, methoxy, ethoxy, methyl, ethyl, n-propyl, isopropyl, $-CH_2CON(CH_3)_2$ or 6-membered heterocyclyl;

further preferably, $R^C$ is

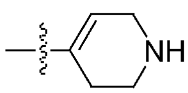 , 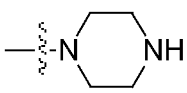 , 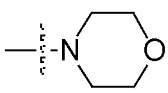 ,

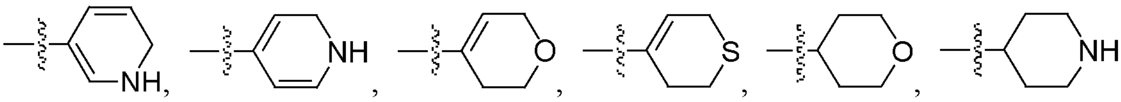,

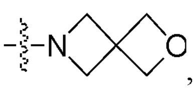, 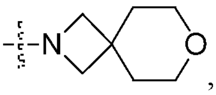, 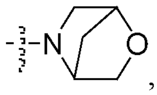, or 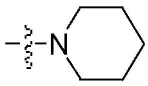,

wherein the $R^C$ are all optionally substituted with one or two identical or different $R^{c1}$;
each of $R^{c1}$, if present, is independently F, Cl, Br, hydroxyl, cyano, amino, oxo, acetyl, $-COCH_2CH_3$, $-COCH_2OH$, hydroxymethyl, hydroxyethyl, $-CH_2OCH_3$, methoxy, methyl, ethyl, isopropyl, $-CH_2CON(CH_3)_2$ or morpholinyl;
even further preferably, $R^C$ optionally substituted with $R^{c1}$ is the following group:

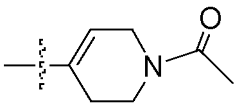, 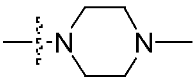, 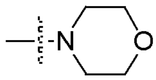, 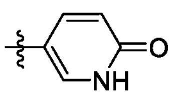, 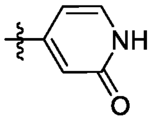,

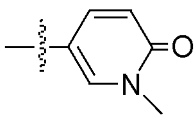, 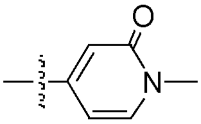, 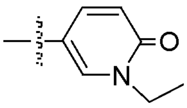, 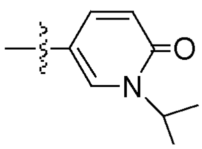, 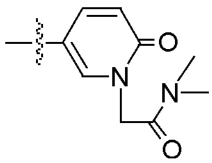,

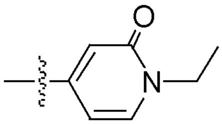, 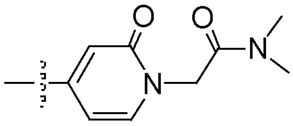, 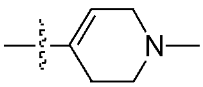, 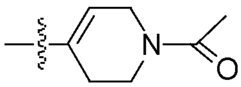,

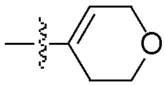, 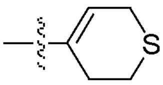, 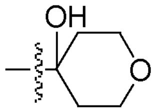, 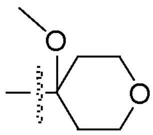, 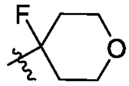, 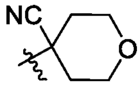,

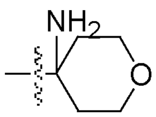, 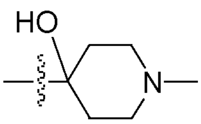, 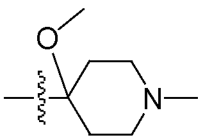, 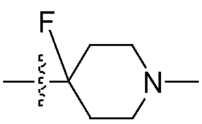, 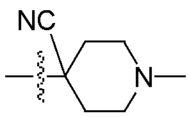,

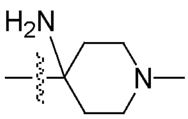, 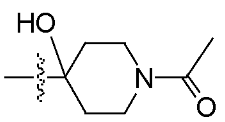, 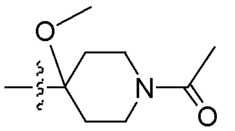, 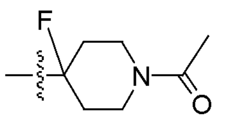,

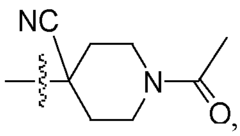, 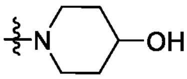, 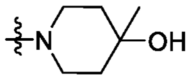, 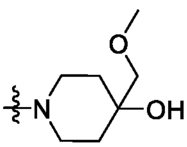, 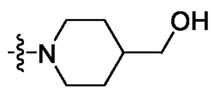,

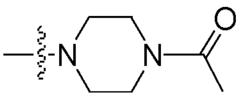, 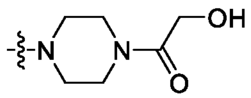, 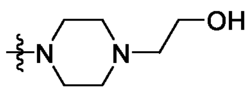, 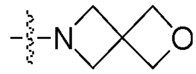,

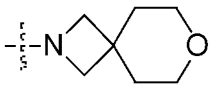, 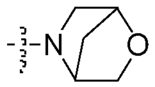, 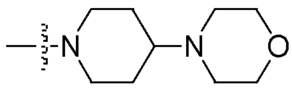, 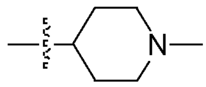,

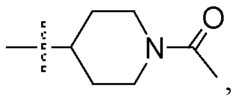, 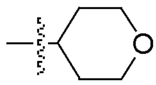 or 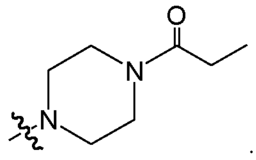.

**10.** The compound or the stereoisomer, optical isomer, pharmaceutical salt, prodrug or solvate thereof according to any one of claims 1 to 9, wherein

ring A is 5- to 10-membered heterocyclyl or 5- to 10-membered heteroaryl, wherein heteroatoms in the 5- to 10-membered heterocyclyl and 5- to 10-membered heteroaryl are each independently O or N, and the number of heteroatoms is 1 to 4;

further preferably, ring A is 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/4-membered fused heterocyclyl, 5-membered/6-membered fused heterocyclyl, 6-membered/5-membered fused heterocyclyl, 6-membered/4-membered fused heterocyclyl, 6-membered/6-membered fused heterocyclyl, 5-membered/3-membered spiro heterocyclyl, 5-membered/5-membered spiro heterocyclyl, 5-membered/4-membered spiro heterocyclyl, 5-membered/6-membered spiro heterocyclyl, 6-membered/3-membered spiro heterocyclyl, 6-membered/5-membered spiro heterocyclyl, 6-membered/4-membered spiro heterocyclyl or 6-membered/6-membered spiro heterocyclyl, wherein heteroatoms in the 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/4-membered fused heterocyclyl, 5-membered/6-membered fused heterocyclyl, 6-membered/5-membered fused heterocyclyl, 6-membered/4-membered fused heterocyclyl, 6-membered/6-membered fused heterocyclyl, 5-membered/3-membered spiro heterocyclyl, 5-membered/5-membered spiro heterocyclyl, 5-membered/4-membered spiro heterocyclyl, 5-membered/6-membered spiro heterocyclyl, 6-membered/3-membered spiro heterocyclyl, 6-membered/5-membered spiro heterocyclyl, 6-membered/4-membered spiro heterocyclyl, and 6-membered/6-membered spiro heterocyclyl are each independently N, and the number of heteroatoms is 1 to 4;

further preferably, ring A is 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/6-membered fused heterocyclyl or 5-membered/3-membered spiro heterocyclyl, wherein heteroatoms in the 5-membered monocyclic heterocyclyl, 6-membered monocyclic heterocyclyl, 5-membered monocyclic heteroaryl, 6-membered monocyclic heteroaryl, 5-membered/5-membered fused heterocyclyl, 5-membered/6-membered fused heterocyclyl, and 5-membered/3-membered spiro heterocyclyl are each independently N, and the number of heteroatoms is 1 to 3;

further preferably, ring A is 5-membered monocyclic heterocyclyl or 5-membered monocyclic heteroaryl, wherein heteroatoms in the 5-membered monocyclic heterocyclyl and 5-membered monocyclic heteroaryl are N, and the number of heteroatoms is 1 to 3;

even further preferably, ring A is the following group:

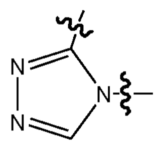, 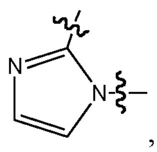,

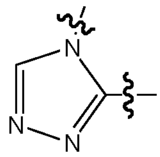, 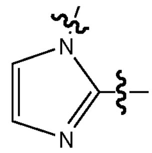, 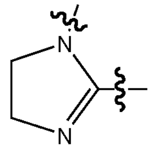, 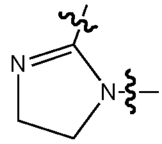, 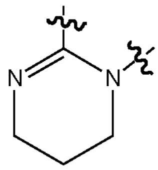, 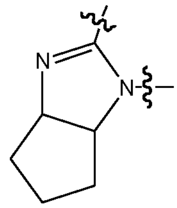,

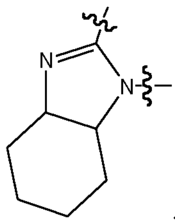, 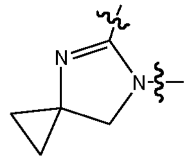 or 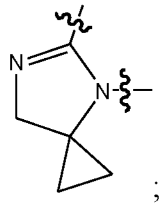;

even further preferably, ring A is the following group:

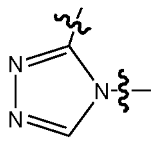, 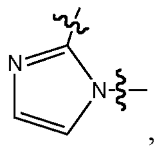,

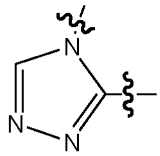, 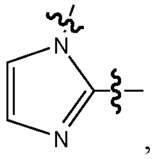, 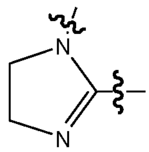 or 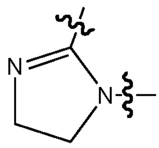.

**11.** The compound or the stereoisomer, optical isomer, pharmaceutical salt, prodrug or solvate thereof according to any one of claims 1 to 10, wherein

there is one, two or three $R^2$, each of which at each occurrence is independently hydrogen, halogen, hydroxyl, cyano, amino, nitro, formyl, oxo, $C_{1-3}$ alkyl, $C_{1-3}$ alkoxy, -$C_{1-3}$ alkyl-NH($C_{1-3}$ alkyl) or -$C_{1-3}$ alkyl-N($C_{1-3}$ alkyl)$_2$, wherein the $C_{1-3}$ alkyl, $C_{1-3}$ alkoxy, -$C_{1-3}$ alkyl-NH($C_{1-3}$ alkyl), and -$C_{1-3}$ alkyl-N($C_{1-3}$ alkyl)$_2$ are all optionally substituted with one or more hydroxyl or halogen;
further preferably, there is one, two or three $R^2$, each of which at each occurrence is independently hydrogen, halogen, hydroxyl, cyano, amino, nitro, formyl, oxo, methoxy, methyl, ethyl, n-propyl or isopropyl;
further preferably, there is one or two $R^2$, each of which at each occurrence is independently hydrogen, halogen, hydroxyl, cyano, amino, nitro, methoxy or methyl;
even further preferably, there is one or two $R^2$, each of which at each occurrence is independently hydrogen or methyl.

**12.** The compound or the stereoisomer, optical isomer, pharmaceutical salt, prodrug or solvate thereof according to any one of claims 1 to 11, wherein

$R^3$ is hydrogen, halogen, hydroxyl, amino, cyano, $C_{1-4}$ alkyl, $C_{1-4}$ alkoxy or $C_{3-6}$ cycloalkyl, wherein the $C_{1-4}$ alkyl, $C_{1-4}$ alkoxy, and $C_{3-6}$ cycloalkyl are all optionally substituted with one or more hydroxyl or halogen;
further preferably, $R^3$ is hydrogen, halogen, hydroxyl, amino, cyano, methyl, ethyl, n-propyl, isopropyl, cyclopropyl, cyclobutyl, cyclopentyl, cyclohexyl or methoxy;
further preferably, $R^3$ is hydrogen, halogen, hydroxyl, amino, cyano, methyl, ethyl, n-propyl, isopropyl or cyclopropyl;
even further preferably, $R^3$ is hydrogen, F, Cl, Br, amino, methyl, ethyl or cyclopropyl.

**13.** The compound or the stereoisomer, optical isomer, pharmaceutical salt, prodrug or solvate thereof according to any

one of claims 1 to 12, wherein

ring B is $C_{4-12}$ cycloalkenyl, $C_{4-12}$ heterocyclyl, $C_{6-12}$ aryl, $C_{6-8}$ aryl-fused $C_{4-6}$ cycloalkyl, $C_{6-8}$ aryl-fused $C_{4-6}$ heterocyclyl or $C_{5-12}$ heteroaryl;
further preferably, ring B is $C_{6-10}$ aryl or $C_{5-10}$ heteroaryl;
further preferably, ring B is phenyl or pyridinyl.

**14.** The compound or the stereoisomer, optical isomer, pharmaceutical salt, prodrug or solvate thereof according to any one of claims 1 to 13, wherein

each of $R^4$, if present, is independently hydrogen, cyano, halogen, amino, nitro, $C_{1-4}$ alkyl, $C_{1-4}$ haloalkyl, $C_{1-4}$ hydroxyalkyl, $C_{3-6}$ cycloalkyl, $C_{3-6}$ halocycloalkyl or 3-to 6-membered heterocyclyl, wherein the $C_{1-4}$ alkyl, $C_{1-4}$ haloalkyl, $C_{1-4}$ hydroxyalkyl, $C_{3-6}$ cycloalkyl, $C_{3-6}$ halocycloalkyl, and 3- to 6-membered heterocyclyl are all optionally substituted with one or more of the following substituents: halogen, hydroxyl, amino, $-SO_2-C_{1-4}$ alkyl or oxo; w is 0, 1, 2 or 3;
further preferably, each of $R^4$ is independently hydrogen, cyano, halogen, amino, nitro, $C_{1-4}$ alkyl, $C_{1-4}$ haloalkyl or $C_{1-4}$ hydroxyalkyl, wherein the $C_{1-4}$ alkyl, $C_{1-4}$ haloalkyl, and $C_{1-4}$ hydroxyalkyl are all optionally substituted with one or more of the following substituents: halogen, hydroxyl or amino; w is 1, 2 or 3;
further preferably, each of $R^4$ is independently hydrogen, cyano, halogen, amino, nitro, methyl, ethyl, n-propyl or isopropyl, wherein the methyl, ethyl, n-propyl, and isopropyl are all optionally substituted with one or more of the following substituents: halogen or hydroxyl; w is 1, 2 or 3;
further preferably, each of $R^4$ is independently hydrogen, halogen, amino, methyl, ethyl or isopropyl, wherein the methyl, ethyl, and isopropyl are all optionally substituted with one or more of the following substituents: halogen or hydroxyl; w is 1, 2 or 3;
further preferably, each of $R^4$ is independently hydrogen, halogen, amino, methyl, trifluoromethyl, difluoromethyl, monofluoromethyl, $-CF_2CH_2OH$, $-C(CH_3)_2OH$ or $-CF_2CH_3$; w is 1, 2 or 3.

**15.** A compound represented by formula (B), or a stereoisomer, optical isomer, pharmaceutical salt, prodrug, solvate or isotope derivative thereof,

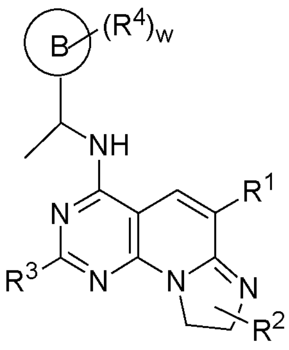
(B)

wherein $R^1$ is $-O-R^A$, $-N(R^D)R^B$ or $R^C$;
when $R^1$ is $-O-R^A$, $R^A$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;
each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}\ alkyl)_2$, $-CH_2CONHC_{1-6}$ alkyl, $-NHC_{1-6}$ alkyl, $-N(C_{1-6}\ alkyl)_2$, $C_{3-10}$ cycloalkyl, $C_{6-10}$ aryl, 3- to 10-membered heterocyclyl or 5- to 10-membered heteroaryl;
when $R^1$ is $-N(R^D)R^B$, $R^B$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}\ alkyl)_2$,

$-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl;

$R^D$ is hydrogen, halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl or $-N(C_{1-6}$ alkyl$)_2$;

when $R^1$ is $R^C$, $R^C$ is $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl or 5- to 10-membered heteroaryl, wherein the $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, $C_{6-10}$ aryl, 3- to 10-membered heterocyclyl, and 5- to 10-membered heteroaryl are all optionally substituted with one or more substituents selected from deuterium, hydroxyl, cyano, $C_{1-3}$ alkyl, $C_{1-3}$ alkoxy or halogen;

there is one or two $R^2$, each of which at each occurrence is independently hydrogen, halogen, hydroxyl, cyano, amino, nitro, formyl, oxo, $C_{1-6}$ alkyl, $C_{1-6}$ alkoxy, halogenated $C_{1-6}$ alkyl or halogenated $C_{1-6}$ alkoxy;

$R^3$ is hydrogen, halogen, hydroxyl, amino, cyano, $C_{1-6}$ alkyl, $C_{1-6}$ alkoxy, $C_{3-6}$ cycloalkyl or 3- to 6-membered heterocyclyl, wherein the $C_{1-6}$ alkyl, $C_{1-6}$ alkoxy, $C_{3-6}$ cycloalkyl, and 3- to 6-membered heterocyclyl are all optionally substituted with one or more hydroxyl or halogen;

ring B is $C_{4-12}$ cycloalkyl, $C_{4-12}$ cycloalkenyl, $C_{4-12}$ heterocyclyl, $C_{6-12}$ aryl, $C_{5-12}$ heteroaryl, $C_{6-12}$ aryl-fused $C_{4-12}$ cycloalkyl, $C_{6-12}$ aryl-fused $C_{4-12}$ heterocyclyl or $C_{6-12}$ aryl-fused $C_{4-12}$ cycloalkenyl;

each of $R^4$, if present, is independently hydrogen, cyano, halogen, amino, hydroxyl, oxo, nitro, $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, $-C_{0-6}$ alkyl-NH-$C_{1-6}$ alkyl, $-C_{0-6}$ alkyl-N($C_{1-6}$ alkyl)($C_{1-6}$ alkyl), $C_{3-6}$ cycloalkyl, $C_{3-6}$ halocycloalkyl or 3- to 6-membered heterocyclyl, wherein the $C_{1-6}$ alkyl, $C_{2-6}$ alkenyl, $C_{2-6}$ alkynyl, $C_{1-6}$ haloalkyl, $C_{1-6}$ hydroxyalkyl, $C_{1-6}$ alkoxy, $-C_{0-6}$ alkyl-NH-$C_{1-6}$ alkyl, $-C_{0-6}$ alkyl-N($C_{1-6}$ alkyl)($C_{1-6}$ alkyl), $C_{3-6}$ cycloalkyl, $C_{3-6}$ halocycloalkyl, and 3- to 6-membered heterocyclyl are all optionally substituted with one or more of the following substituents: halogen, hydroxyl, amino, $-SO_2$-$C_{1-4}$ alkyl or oxo; w is 0, 1, 2, 3 or 4;

unless otherwise stated, heteroatoms in the heteroaryl and heterocyclyl are each independently O, N or S, and the number of heteroatoms is 1, 2, 3 or 4.

**16.** The compound or the stereoisomer, optical isomer, pharmaceutical salt, prodrug, solvate or isotope derivative thereof according to claim 15, wherein

$R^1$ is -O-$R^A$, $R^A$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{a1}$;

each of $R^{a1}$, if present, is independently halogen, hydroxyl, cyano, amino, nitro, oxo, formyl, $C_{1-6}$ alkyl, $C_{2-6}$ alkenyl, $C_{2-6}$ alkynyl, $-OC_{1-6}$ alkyl, $-SC_{1-6}$ alkyl, $-COC_{1-6}$ alkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl or $-N(C_{1-6}$ alkyl$)_2$;

further preferably, $R^A$ is $C_{3-6}$ cycloalkyl, $C_{6-10}$ aryl, 3- to 6-membered heterocyclyl or 5- to 6-membered heteroaryl, wherein the $C_{3-6}$ cycloalkyl, $C_{6-10}$ aryl, 3- to 6-membered heterocyclyl, and 5- to 6-membered heteroaryl are all optionally substituted with 1 to 3 identical or different $R^{a1}$;

each of $R^{a1}$, if present, is independently halogen, hydroxyl, cyano, amino, oxo, formyl, $C_{1-6}$ alkyl, $-OC_{1-6}$ alkyl, $-SC_{1-6}$ alkyl or $-COC_{1-6}$ alkyl;

further preferably, $R^A$ is 5- to 6-membered heterocyclyl or 5- to 6-membered heteroaryl, wherein the 5- to 6-membered heterocyclyl and 5- to 6-membered heteroaryl are both optionally substituted with 1 to 3 identical or different $R^{a1}$;

each of $R^{a1}$, if present, is independently halogen, hydroxyl, cyano, amino, oxo, formyl, $C_{1-4}$ alkyl, $-OC_{1-4}$ alkyl, $-SC_{1-4}$ alkyl or $-COC_{1-4}$ alkyl;

further preferably, $R^A$ is 5- to 6-membered heterocyclyl, wherein the 5- to 6-membered heterocyclyl is optionally substituted with one or two identical or different $R^{a1}$;

each of $R^{a1}$, if present, is independently halogen, oxo, formyl, acetyl, methyl, ethyl, n-propyl, isopropyl or methoxy;

further preferably, $R^A$ is 5- to 6-membered monocyclic heterocyclyl, wherein the 5- to 6-membered monocyclic heterocyclyl is optionally substituted with one or two identical or different $R^{a1}$; heteroatoms in the 5- to 6-membered monocyclic heterocyclyl are each independently O, N or S, and the number of heteroatoms is 1; each of $R^{a1}$, if present, is independently halogen, oxo, formyl, acetyl, methyl, ethyl, n-propyl, isopropyl or methoxy; further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;
each of $R^{a1}$, if present, is independently halogen, oxo, formyl, acetyl, methyl, ethyl, n-propyl, isopropyl or methoxy;
even further preferably, $R^A$ is the following group:

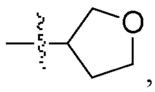, 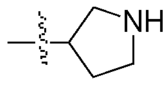,

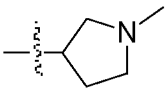, 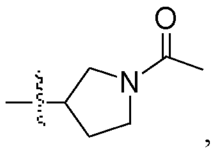, 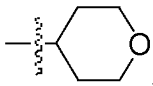, 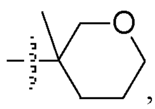, or 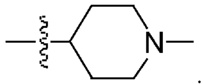.

**17.** The compound or the stereoisomer, optical isomer, pharmaceutical salt, prodrug, solvate or isotope derivative thereof according to claim 15, wherein

$R^1$ is $-N(R^D)R^B$, where $R^B$ is $C_{1-6}$ alkyl, $C_{3-10}$ cycloalkyl, $C_{6-10}$ aryl, 3- to 10-membered heterocyclyl or 5- to 10-membered heteroaryl, wherein the $C_{1-6}$ alkyl, $C_{3-10}$ cycloalkyl, $C_{6-10}$ aryl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl;
further preferably, $R^B$ is $C_{1-6}$ alkyl, 3- to 10-membered heterocyclyl or 5- to 10-membered heteroaryl, wherein the $C_{1-6}$ alkyl, 3- to 10-membered heterocyclyl, and 5- to 10-membered heteroaryl are all optionally substituted with 1 to 3 identical or different $R^{b1}$;
each of $R^{b1}$, if present, is independently halogen, hydroxyl, cyano, amino, nitro, oxo, formyl, $C_{1-6}$ alkyl, $-OC_{1-6}$ alkyl, $-SC_{1-6}$ alkyl or $-COC_{1-6}$ alkyl;
further preferably, $R^B$ is $C_{1-6}$ alkyl or 5- to 6-membered monocyclic heterocyclyl, wherein the $C_{1-6}$ alkyl and 5- to 6-membered monocyclic heterocyclyl are both optionally substituted with one or two identical or different $R^{b1}$; heteroatoms in the 5-to 6-membered monocyclic heterocyclyl are each independently O, N or S, and the number of heteroatoms is 1;
each of $R^{b1}$, if present, is independently halogen, oxo, formyl, $C_{1-4}$ alkyl, $-OC_{1-4}$ alkyl or $-COC_{1-4}$ alkyl;
further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;
each of $R^{b1}$, if present, is independently oxo, formyl, acetyl, methyl, ethyl, methoxy, or ethoxy;
even further preferably, $R^B$ is the following group:

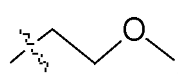, 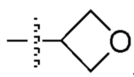,

or

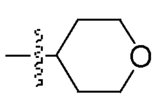;

$R^D$ is hydrogen, $C_{1-6}$ alkyl or $-OC_{1-6}$ alkyl; further preferably, $R^D$ is hydrogen or $C_{1-3}$ alkyl; even further preferably, $R^D$ is hydrogen or methyl; most preferably, $R^D$ is hydrogen.

**18.** The compound or the stereoisomer, optical isomer, pharmaceutical salt, prodrug, solvate or isotope derivative thereof

according to claim 15, wherein

$R^1$ is $R^C$, and $R^C$ is $C_{3-10}$ cycloalkyl, $C_{6-10}$ aryl, 3- to 10-membered heterocyclyl or 5- to 10-membered heteroaryl, wherein the $C_{3-10}$ cycloalkyl, $C_{6-10}$ aryl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-6}$ alkyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl or 3- to 10-membered heterocyclyl, wherein the $C_{1-6}$ alkyl, $-OC_{1-6}$ alkyl, $-SC_{1-6}$ alkyl, $-COC_{1-6}$ alkyl, $-COC_{3-6}$ cycloalkyl, $-CH_2COC_{1-6}$ alkyl, $-CH_2CON(C_{1-6}$ alkyl$)_2$, $-CH_2CONHC_{1-6}$ alkyl, $-NHC_{1-6}$ alkyl, $-N(C_{1-6}$ alkyl$)_2$, $C_{3-10}$ cycloalkyl, and 3- to 10-membered heterocyclyl are all optionally substituted with one or more substituents selected from deuterium, hydroxyl, cyano, $C_{1-3}$ alkyl, $C_{1-3}$ alkoxy or halogen;

further preferably, $R^C$ is $C_{3-10}$ cycloalkyl, 3- to 10-membered heterocyclyl or 5- to 10-membered heteroaryl, wherein the $C_{3-10}$ cycloalkyl, 3- to 10-membered heterocyclyl, and 5- to 10-membered heteroaryl are all optionally substituted with 1 to 4 identical or different $R^{c1}$;

each of $R^{c1}$, if present, is independently halogen, hydroxyl, cyano, amino, nitro, oxo, formyl, methylsulfonyl, $C_{1-4}$ alkyl, $-OC_{1-4}$ alkyl, $-SC_{1-4}$ alkyl, $-COC_{1-4}$ alkyl, $-COC_{3-6}$ cycloalkyl, $-CH_2COC_{1-4}$ alkyl, $-CH_2CON(C_{1-4}$ alkyl$)_2$, $-CH_2CONHC_{1-4}$ alkyl, $C_{3-6}$ cycloalkyl or 3- to 6-membered heterocyclyl, wherein the $C_{1-4}$ alkyl, $-OC_{1-4}$ alkyl, $-SC_{1-4}$ alkyl, $-COC_{1-4}$ alkyl, $-COC_{3-6}$ cycloalkyl, $-CH_2COC_{1-4}$ alkyl, $-CH_2CON(C_{1-4}$ alkyl$)_2$, $-CH_2CONHC_{1-4}$ alkyl, $C_{3-6}$ cycloalkyl, and 3- to 6-membered heterocyclyl are all optionally substituted with one or more substituents selected from deuterium, hydroxyl, cyano, $C_{1-3}$ alkyl, $C_{1-3}$ alkoxy or halogen;

further preferably, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$,

each of $R^{c1}$, if present, is independently halogen, hydroxyl, cyano, amino, oxo, formyl, methylsulfonyl, $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$ or 3- to 6-membered heterocyclyl, wherein the $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$, and 3- to 6-membered heterocyclyl are all optionally substituted with one or more substituents selected from deuterium, hydroxyl, cyano, methyl, methoxy or halogen;

further preferably, $R^C$ is 5- to 6-membered monocyclic heterocyclyl, 6- to 10-membered spiro heterocyclyl, 6- to 8-membered bridged heterocyclyl, 8- to 10-membered fused heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, 8- to 10-membered fused heterocyclyl, and 5-to 6-membered monocyclic heteroaryl are all optionally substituted with 1 to 4 identical or different $R^{c1}$;

each of $R^{c1}$, if present, is independently halogen, hydroxyl, cyano, amino, oxo, formyl, acetyl, propionyl, methylsulfonyl, methoxy, ethoxy, methyl, ethyl, n-propyl, isopropyl, $-CH_2CON(CH_3)_2$, -CO-cyclopropyl, -CO-cyclobutyl, 4-membered heterocyclyl, 5-membered heterocyclyl or 6-membered heterocyclyl, wherein the acetyl, propionyl, methylsulfonyl, methoxy, ethoxy, methyl, ethyl, n-propyl, isopropyl, $-CH_2CON(CH_3)_2$, -CO-cyclopropyl, -CO-cyclobutyl, 4-membered heterocyclyl, 5-membered heterocyclyl, and 6-membered heterocyclyl are all optionally substituted with one or more substituents selected from deuterium, hydroxyl, cyano, methyl, methoxy or halogen;

even further preferably, $R^C$ is 6-membered monocyclic heterocyclyl, 4-membered/6-membered spiro heterocyclyl, 4-membered/4-membered spiro heterocyclyl, 6-membered/5-membered fused heterocyclyl, 7-membered bridged heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 6-membered/4-membered fused heterocyclyl, 7-membered bridged heterocyclyl, and 6-membered monocyclic heteroaryl are all optionally substituted with 1 to 4 identical or different $R^{c1}$;

each of $R^{c1}$, if present, is independently halogen, hydroxyl, cyano, amino, oxo, formyl, acetyl, propionyl, methylsulfonyl, methoxy, ethoxy, methyl, ethyl, n-propyl, isopropyl, $-CH_2CON(CH_3)_2$, -CO-cyclopropyl, 4-membered heterocyclyl, 5-membered heterocyclyl or 6-membered heterocyclyl, wherein the acetyl, propionyl, methylsulfonyl, methoxy, ethoxy, methyl, ethyl, n-propyl, isopropyl, $-CH_2CON(CH_3)_2$, -CO-cyclopropyl, 4-membered heterocyclyl, 5-membered heterocyclyl, and 6-membered heterocyclyl are all optionally substituted with one or more substituents selected from deuterium, hydroxyl, cyano, methyl, methoxy or halogen;

further preferably, $R^C$ is

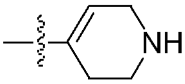, 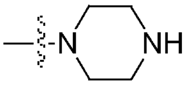, 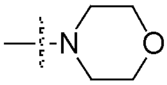,

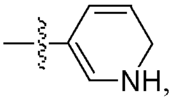 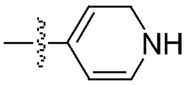, 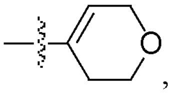, 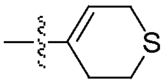, 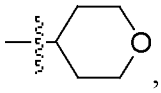, 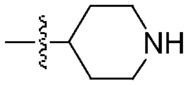,

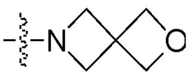, 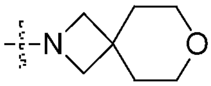, 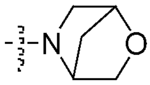, 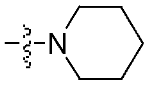, 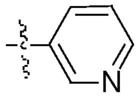, 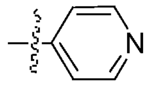,

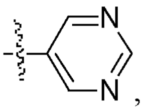, 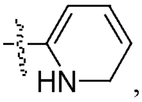, 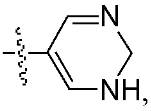 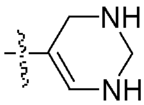 or 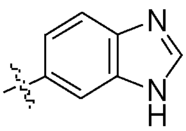,

and the $R^C$ are all optionally substituted with 1 to 4 identical or different $R^{c1}$;
each of $R^{c1}$, if present, is independently F, Cl, Br, hydroxyl, cyano, amino, oxo, methylsulfonyl, acetyl, $-COCH_2CH_3$, $-COCH_2OH$, $-COCH_2CN$, $-CH(OH)(CH_3)_2$, hydroxymethyl, hydroxyethyl, $-CH_2OCH_3$, $-CH_2CH_2OCH_3$, methoxy, methyl, $CD_3$, ethyl, isopropyl, monofluoromethyl, difluoromethyl, trifluoromethyl, monofluoroethyl, difluoroethyl, trifluoroethyl,

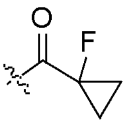, 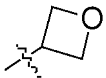,

$-CH_2CON(CH_3)_2$ or morpholinyl;
even further preferably, $R^C$ optionally substituted with $R^{c1}$ is the following group:

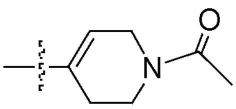, 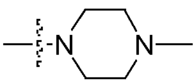, 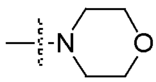, 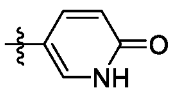, 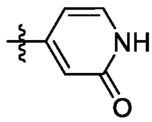,

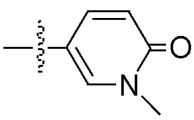, 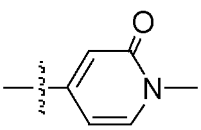, 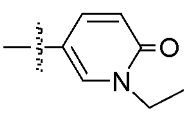, 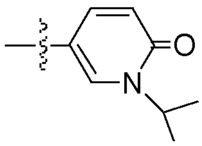, 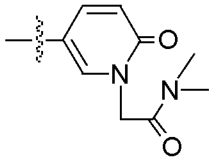,

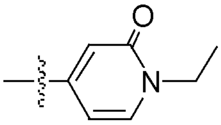, 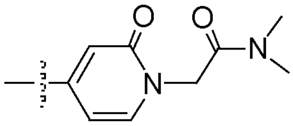, 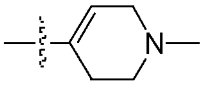, 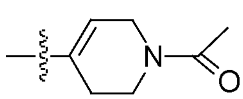,

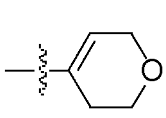, 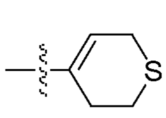, 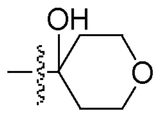, 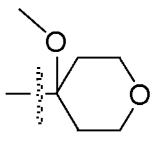, 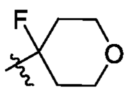, 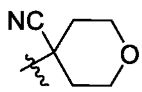,

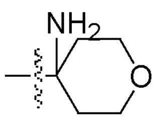, 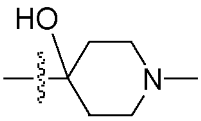, 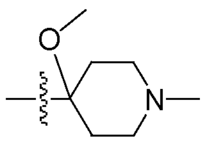, 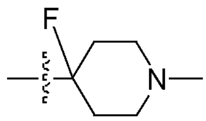, 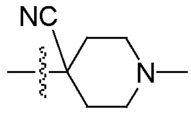,

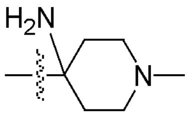, 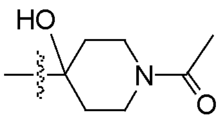, 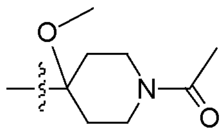, 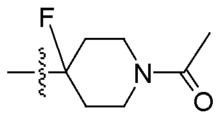,

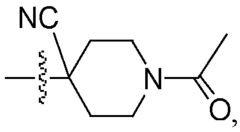, 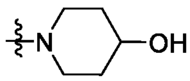, 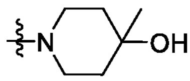, 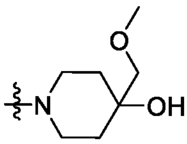, 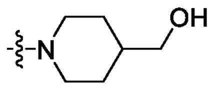,

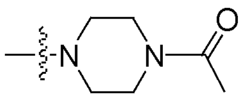, 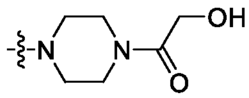, 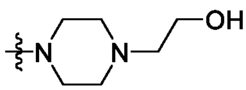, 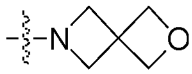,

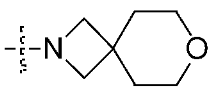, 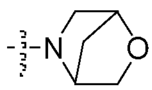, 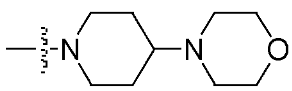, 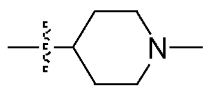,

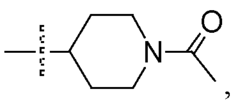, 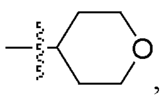, 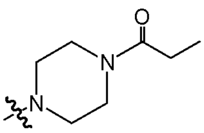, 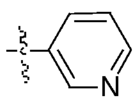, 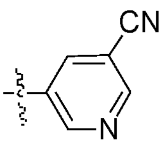, 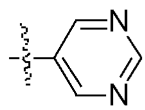,

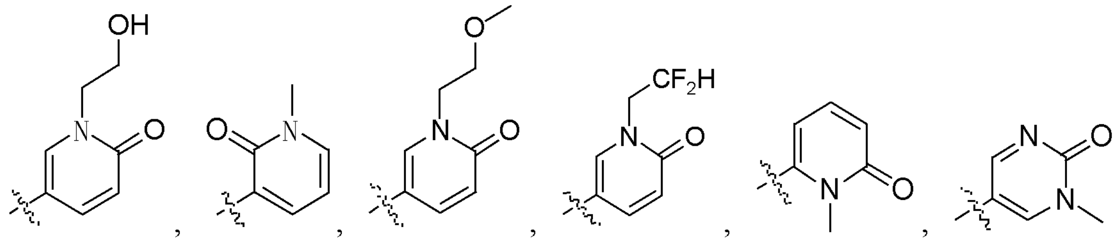, , , , , ,

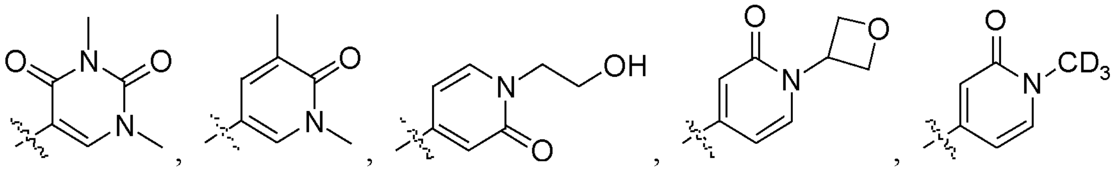, , , , ,

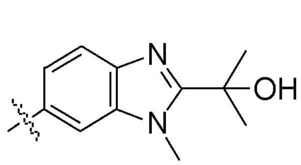, 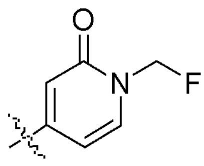, 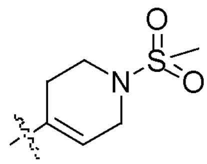, 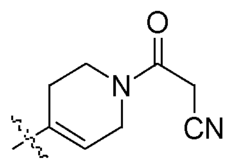,

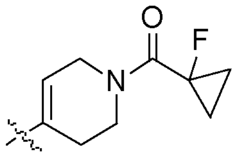 or 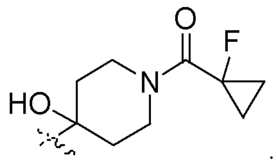.

**19.** The compound or the stereoisomer, optical isomer, pharmaceutical salt, prodrug, solvate or isotope derivative thereof according to any one of claims 15 to 18, wherein

there is one or two $R^2$, each of which at each occurrence is independently hydrogen, halogen, hydroxyl, cyano, amino, nitro, formyl, oxo, methoxy, methyl, ethyl, n-propyl or isopropyl;
further preferably, there is one or two $R^2$, each of which at each occurrence is independently hydrogen, halogen, hydroxyl, cyano, amino, nitro, methoxy or methyl;
even further preferably, there is one or two $R^2$, each of which at each occurrence is independently hydrogen or methyl;
even further preferably, $R^2$ is hydrogen.

**20.** The compound or the stereoisomer, optical isomer, pharmaceutical salt, prodrug, solvate or isotope derivative thereof according to any one of claims 15 to 19, wherein

$R^3$ is hydrogen, halogen, hydroxyl, amino, cyano, methyl, ethyl, n-propyl, isopropyl, cyclopropyl, cyclobutyl, cyclopentyl, cyclohexyl or methoxy;
further preferably, $R^3$ is hydrogen, halogen, hydroxyl, amino, cyano, methyl, ethyl, n-propyl, isopropyl or cyclopropyl;
even further preferably, $R^3$ is hydrogen, F, Cl, Br, amino, methyl, ethyl or cyclopropyl.

**21.** The compound or the stereoisomer, optical isomer, pharmaceutical salt, prodrug, solvate or isotope derivative thereof according to any one of claims 15 to 20, wherein

ring B is $C_{4-12}$ cycloalkenyl, 4- to 12-membered heterocyclyl, $C_{6-12}$ aryl, $C_{6-8}$ aryl-fused $C_{4-6}$ cycloalkyl, $C_{6-8}$ aryl-fused 4- to 6-membered heterocyclyl or 5- to 12-membered heteroaryl;
further preferably, ring B is $C_{6-10}$ aryl, 5- to 10-membered heteroaryl or $C_{6-8}$ aryl-fused 4- to 6-membered heterocyclyl;
further preferably, ring B is phenyl, pyridinyl or benzodihydrofuranyl.

**22.** The compound or the stereoisomer, optical isomer, pharmaceutical salt, prodrug, solvate or isotope derivative thereof according to any one of claims 15 to 21, wherein

each of $R^4$, if present, is independently hydrogen, cyano, halogen, amino, nitro, $C_{1-4}$ alkyl, $C_{1-4}$ haloalkyl, $C_{1-4}$ hydroxyalkyl, $C_{3-6}$ cycloalkyl, $C_{3-6}$ halocycloalkyl or 3-to 6-membered heterocyclyl, wherein the $C_{1-4}$ alkyl, $C_{1-4}$ haloalkyl, $C_{1-4}$ hydroxyalkyl, $C_{3-6}$ cycloalkyl, $C_{3-6}$ halocycloalkyl, and 3- to 6-membered heterocyclyl are all optionally substituted with one or more of the following substituents: halogen, hydroxyl, amino, -$SO_2$-$C_{1-4}$ alkyl or oxo; w is 0, 1, 2 or 3;
further preferably, each of $R^4$ is independently hydrogen, cyano, halogen, amino, nitro, $C_{1-4}$ alkyl, $C_{1-4}$ haloalkyl or $C_{1-4}$ hydroxyalkyl, wherein the $C_{1-4}$ alkyl, $C_{1-4}$ haloalkyl, and $C_{1-4}$ hydroxyalkyl are all optionally substituted with one or more of the following substituents: halogen, hydroxyl or amino; w is 1, 2 or 3;
further preferably, each of $R^4$ is independently hydrogen, cyano, halogen, amino, nitro, methyl, ethyl, n-propyl or isopropyl, wherein the methyl, ethyl, n-propyl, and isopropyl are all optionally substituted with one or more of the following substituents: halogen or hydroxyl; w is 1, 2 or 3;
further preferably, each of $R^4$ is independently hydrogen, halogen, amino, cyano, methyl, ethyl or isopropyl,

wherein the methyl, ethyl, and isopropyl are all optionally substituted with one or more of the following substituents: halogen or hydroxyl; w is 1, 2 or 3;

further preferably, each of $R^4$ is independently hydrogen, fluorine, amino, cyano, methyl, trifluoromethyl, difluoromethyl, monofluoromethyl, $-CF_2CH_2OH$, $-C(CH_3)_2OH$, $-CF_2CH_3$ or $-CH_2CHF_2$; w is 1, 2 or 3.

**23.** The compound or the stereoisomer, optical isomer, pharmaceutical salt, prodrug, solvate or isotope derivative thereof according to any one of claims 15 to 22, wherein the compound has a structure represented by formula (C):

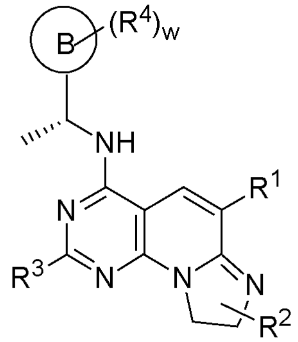

(C)

the substituents in formula (C) are as defined in formula (B).

**24.** A compound represented by formula (D), or a stereoisomer, optical isomer, pharmaceutical salt, prodrug, solvate or isotope derivative thereof,

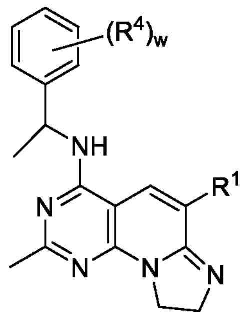

(D)

wherein each of $R^4$, if present, is independently cyano, halogen, amino, $C_{1-6}$ alkyl or $C_{1-6}$ haloalkyl, wherein the $C_{1-6}$ alkyl and $C_{1-6}$ haloalkyl are both optionally substituted with one or more hydroxyl; w is 1 or 2;

$R^1$ is $-O-R^A$, $-N(R^D)R^B$ or $R^C$;

when $R^1$ is $-O-R^A$, $R^A$ is 3- to 10-membered heterocyclyl, wherein the 3- to 10-membered heterocyclyl is optionally substituted with 1 to 3 identical or different $R^{a1}$;

each of $R^{a1}$, if present, is independently $C_{1-6}$ alkyl or $-COC_{1-6}$ alkyl;

when $R^1$ is $-N(R^D)R^B$, $R^B$ is $C_{1-6}$ alkyl or 3- to 10-membered heterocyclyl, wherein the $C_{1-6}$ alkyl and 3- to 10-membered heterocyclyl are both optionally substituted with 1 to 3 identical or different $R^{b1}$;

each of $R^{b1}$, if present, is independently $-OC_{1-6}$ alkyl;

$R^D$ is hydrogen;

when $R^1$ is $R^C$, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$,

each of $R^{c1}$, if present, is independently halogen, hydroxyl, cyano, amino, methylsulfonyl, $C_{1-6}$ alkyl, $-OC_{1-6}$ alkyl, $-COC_{1-6}$ alkyl, $-COC_{3-6}$ cycloalkyl, $-CH_2CON(C_{1-6}$ alkyl$)_2$ or 3- to 10-membered heterocyclyl, wherein the $C_{1-6}$ alkyl, $-OC_{1-6}$ alkyl, $-COC_{1-6}$ alkyl, $-COC_{3-6}$ cycloalkyl, $-CH_2CON(C_{1-6}$ alkyl$)_2$, and 3- to 10-membered heterocyclyl are all optionally substituted with one or more substituents selected from deuterium, hydroxyl, cyano, $C_{1-3}$ alkoxy or halogen;

unless otherwise stated, heteroatoms in the heteroaryl and heterocyclyl are each independently O, N or S, and the number of heteroatoms is 1, 2, 3 or 4.

**25.** The compound or the stereoisomer, optical isomer, pharmaceutical salt, prodrug, solvate or isotope derivative thereof according to claim 24, wherein

$R^1$ is -O-$R^A$, $R^A$ is 3- to 6-membered heterocyclyl, wherein the 3- to 6-membered heterocyclyl is optionally substituted with 1 to 3 identical or different $R^{a1}$;
each of $R^{a1}$, if present, is independently $C_{1-6}$ alkyl or -$COC_{1-6}$ alkyl;
further preferably, $R^A$ is 5- to 6-membered heterocyclyl, wherein the 5- to 6-membered heterocyclyl is optionally substituted with one or two identical or different $R^{a1}$;
each of $R^{a1}$, if present, is independently acetyl, methyl, ethyl, n-propyl or isopropyl;
further preferably, $R^A$ is 5- to 6-membered monocyclic heterocyclyl, wherein the 5- to 6-membered monocyclic heterocyclyl is optionally substituted with one or two identical or different $R^{a1}$; heteroatoms in the 5- to 6-membered monocyclic heterocyclyl are each independently O, N or S, and the number of heteroatoms is 1;
each of $R^{a1}$, if present, is independently acetyl, methyl or ethyl;
further preferably, $R^A$ is tetrahydrofuranyl, tetrahydrothienyl, pyrrolidinyl, piperidinyl, tetrahydropyranyl or tetrahydrothiopyranyl, wherein the tetrahydrofuranyl, tetrahydrothienyl, pyrrolidinyl, piperidinyl, tetrahydropyranyl, and tetrahydrothiopyranyl are all optionally substituted with one or two identical or different $R^{a1}$;
each of $R^{a1}$, if present, is independently acetyl, methyl or ethyl;
even further preferably, $R^A$ is the following group:

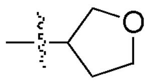 , 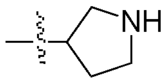 ,

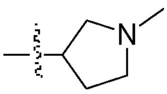 , 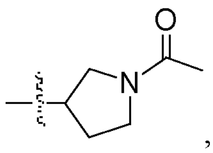 , 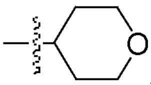 , 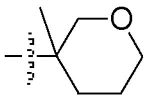 or 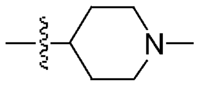 .

**26.** The compound or the stereoisomer, optical isomer, pharmaceutical salt, prodrug, solvate or isotope derivative thereof according to claim 24, wherein

$R^1$ is -N($R^D$)$R^B$, $R^B$ is $C_{1-6}$ alkyl or 3- to 6-membered monocyclic heterocyclyl, wherein the $C_{1-6}$ alkyl and 3- to 6-membered monocyclic heterocyclyl are both optionally substituted with one or two identical or different $R^{b1}$; and heteroatoms in the 3- to 6-membered monocyclic heterocyclyl are each independently O, N or S, and the number of heteroatoms is 1;
each of $R^{b1}$, if present, is independently -$OC_{1-4}$ alkyl;
further preferably, $R^B$ is methyl, ethyl, n-propyl, isopropyl, oxetanyl, tetrahydrofuranyl, tetrahydrothienyl, pyrrolidinyl, piperidinyl, tetrahydropyranyl or tetrahydrothiopyranyl, wherein the methyl, ethyl, n-propyl, isopropyl, oxetanyl, tetrahydrofuranyl, tetrahydrothienyl, pyrrolidinyl, piperidinyl, tetrahydropyranyl, and tetrahydrothiopyranyl are all optionally substituted with one or two identical or different $R^{b1}$;
each of $R^{b1}$, if present, is independently methoxy or ethoxy;
even further preferably, $R^B$ is the following group:

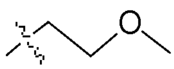 , 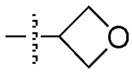

or

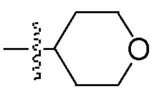 ;

$R^D$ is hydrogen, $C_{1-6}$ alkyl or -$OC_{1-6}$ alkyl; further preferably, $R^D$ is hydrogen or $C_{1-3}$ alkyl; even further preferably, $R^D$ is hydrogen or methyl; most preferably, $R^D$ is hydrogen.

**27.** The compound or the stereoisomer, optical isomer, pharmaceutical salt, prodrug, solvate or isotope derivative thereof according to claim 24, wherein

$R^1$ is $R^C$, $R^C$ is 3- to 10-membered heterocyclyl or 5- to 10-membered heteroaryl, wherein the 3- to 10-membered heterocyclyl and 5- to 10-membered heteroaryl are both optionally substituted with 1 to 4 identical or different $R^{c1}$;
each of $R^{c1}$, if present, is independently halogen, hydroxyl, cyano, amino, methylsulfonyl, $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$ or 3- to 6-membered heterocyclyl, wherein the $C_{1-4}$ alkyl, $-OC_{1-4}$ alkyl, $-COC_{1-4}$ alkyl, $-COC_{3-6}$ cycloalkyl, $-CH_2CON(C_{1-4}$ alkyl$)_2$, and 3- to 6-membered heterocyclyl are all optionally substituted with one or more substituents selected from deuterium, hydroxyl, cyano, methoxy or halogen;
further preferably, $R^C$ is 5- to 6-membered monocyclic heterocyclyl, 6- to 10-membered spiro heterocyclyl, 6- to 8-membered bridged heterocyclyl, 8- to 10-membered fused heterocyclyl or 5- to 6-membered monocyclic heteroaryl, wherein the 5- to 6-membered monocyclic heterocyclyl, 6- to 10-membered spiro heterocyclyl, 6- to 8-membered bridged heterocyclyl, 8- to 10-membered fused heterocyclyl, and 5-to 6-membered monocyclic heteroaryl are all optionally substituted with 1 to 4 identical or different $R^{c1}$;
each of $R^{c1}$, if present, is independently halogen, hydroxyl, cyano, amino, methylsulfonyl, methyl, ethyl, n-propyl, isopropyl, methoxy, ethoxy, acetyl, propionyl, -CO-cyclopropyl, -CO-cyclobutyl, $-CH_2CON(CH_3)_2$, 4-membered heterocyclyl, 5-membered heterocyclyl or 6-membered heterocyclyl, wherein the methylsulfonyl, methyl, ethyl, n-propyl, isopropyl, methoxy, ethoxy, acetyl, propionyl, -CO-cyclopropyl, -CO-cyclobutyl, $-CH_2CON(CH_3)_2$, 4-membered heterocyclyl, 5-membered heterocyclyl, and 6-membered heterocyclyl are all optionally substituted with one or more substituents selected from deuterium, hydroxyl, cyano, methoxy or halogen;
even further preferably, $R^C$ is 6-membered monocyclic heterocyclyl, 4-membered/6-membered spiro heterocyclyl, 4-membered/4-membered spiro heterocyclyl, 7-membered bridged heterocyclyl, 6-membered/5-membered fused heterocyclyl or 6-membered monocyclic heteroaryl, wherein the 6-membered monocyclic heterocyclyl, 4-membered/6-membered spiro heterocyclyl, 4-membered/4-membered spiro heterocyclyl, 7-membered bridged heterocyclyl, 6-membered/4-membered fused heterocyclyl, and 6-membered monocyclic heteroaryl are all optionally substituted with 1 to 4 identical or different $R^{c1}$;
each of $R^{c1}$, if present, is independently halogen, hydroxyl, cyano, amino, methylsulfonyl, methyl, ethyl, n-propyl, isopropyl, methoxy, ethoxy, acetyl, propionyl, -CO-cyclopropyl, $-CH_2CON(CH_3)_2$, 4-membered heterocyclyl, 5-membered heterocyclyl or 6-membered heterocyclyl, wherein the methylsulfonyl, methyl, ethyl, n-propyl, isopropyl, methoxy, ethoxy, acetyl, propionyl, -CO-cyclopropyl, 4-membered heterocyclyl, 5-membered heterocyclyl, and 6-membered heterocyclyl are all optionally substituted with one or more substituents selected from deuterium, hydroxyl, cyano, methoxy or halogen;
further preferably, $R^C$ is

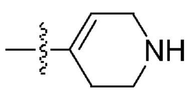, 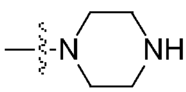, 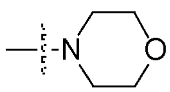,

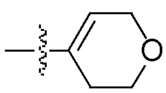, 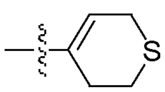, 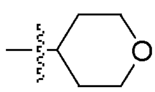, 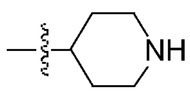, 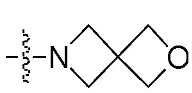,

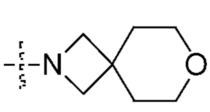, 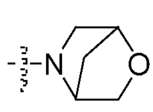, 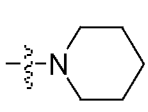, 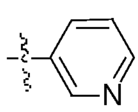, 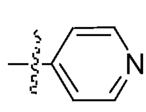, 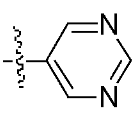 or

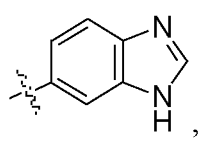,

and the $R^C$ are all optionally substituted with 1 to 4 identical or different $R^{c1}$;
each of $R^{c1}$, if present, is independently F, Cl, Br, hydroxyl, cyano, amino, methylsulfonyl, methyl, ethyl, isopropyl,

$CD_3$, hydroxymethyl, hydroxyethyl (for example, 2-hydroxyethyl), $-CH(OH)(CH_3)_2$, $-CH_2OCH_3$, $-CH_2CH_2OCH_3$, monofluoromethyl, difluoromethyl, trifluoromethyl, monofluoroethyl (for example, 2-fluoroethyl), difluoroethyl (for example, 2,2-difluoroethyl), trifluoroethyl (for example, 2,2,2-trifluoroethyl), methoxy, acetyl, $-COCH_2CH_3$, $-COCH_2OH$, $-COCH_2CN$,

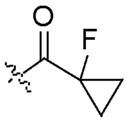,

$-CH_2CON(CH_3)_2$, oxetanyl (for example,

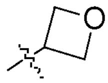

) or morpholinyl (for example, morpholin-4-yl);
even further preferably, $R^C$ optionally substituted with $R^{c1}$ is the following group:

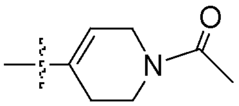, 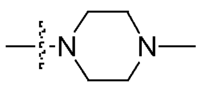, 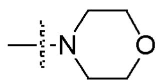, 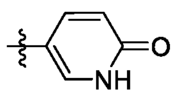, 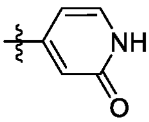,

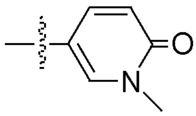, 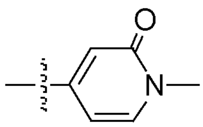, 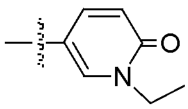, 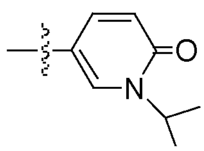, 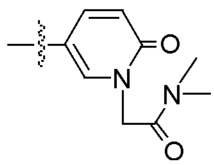,

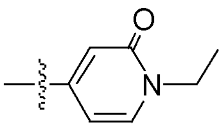, 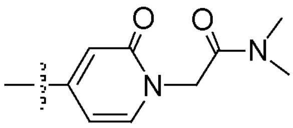, 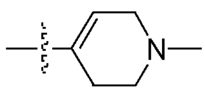, 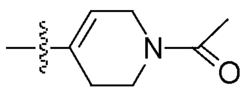,

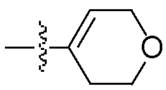, 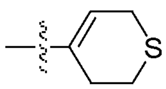, 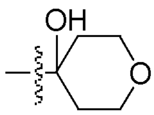, 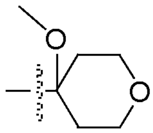, 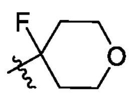, 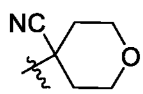,

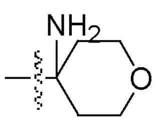, 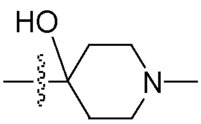, 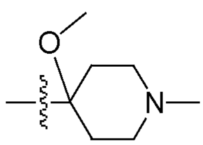, 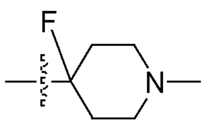, 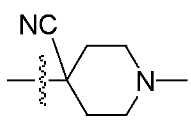,

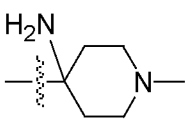, 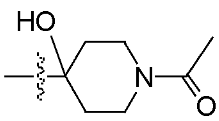, 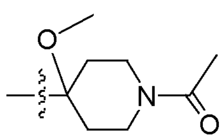, 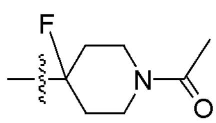,

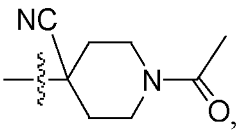, 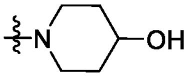, 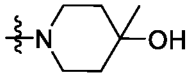, 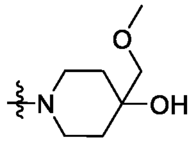, 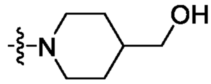,

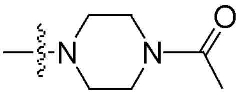, 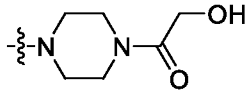, 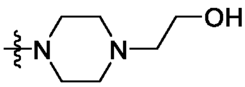, 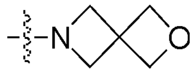,

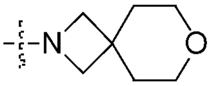, 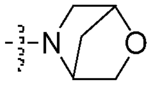, 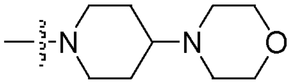, 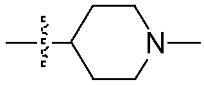,

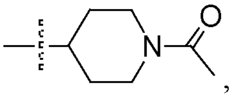, 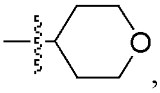, 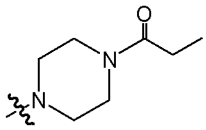, 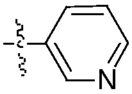, 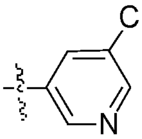, 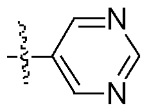,

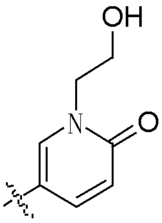, 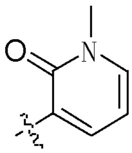, 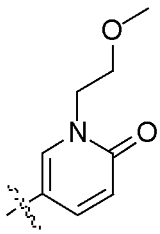, 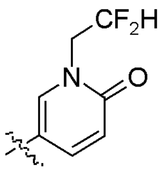, 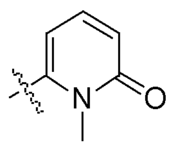, 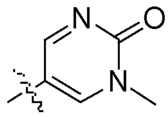,

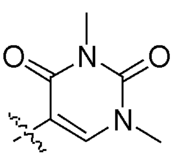, 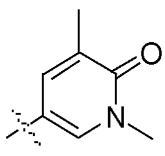, 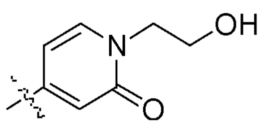, 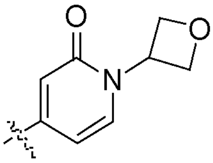, 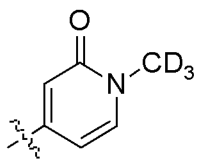,

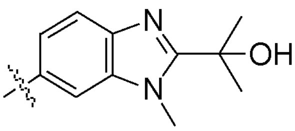, 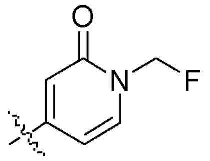, 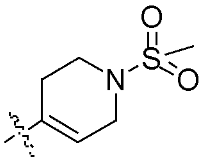, 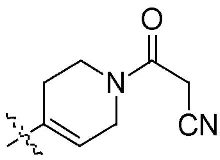,

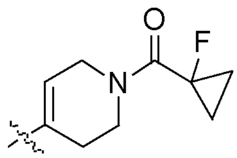 or 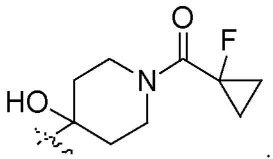.

**28.** The compound or the stereoisomer, optical isomer, pharmaceutical salt, prodrug, solvate or isotope derivative thereof according to any one of claims 24 to 27, wherein

each of $R^4$, if present, is independently cyano, halogen, amino, $C_{1-4}$ alkyl or $C_{1-4}$ haloalkyl, wherein the $C_{1-4}$ alkyl and $C_{1-4}$ haloalkyl are both optionally substituted with one or more hydroxyl; w is 1 or 2;
further preferably, each of $R^4$ is independently cyano, fluorine, amino, methyl, trifluoromethyl, difluoromethyl,

monofluoromethyl, $-CF_2CH_2OH$, $-CF_2C(CH_3)_2OH$, $-CF_2CH_3$ or $-CH_2CHF_2$; w is 1 or 2.

**29.** The compound or the stereoisomer, optical isomer, pharmaceutical salt, prodrug, solvate or isotope derivative thereof according to any one of claims 24 to 28, wherein the compound has a structure represented by formula (E):

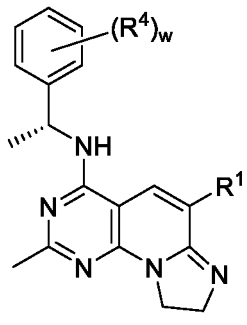

(E)

the substituents in formula (E) are as defined in formula (D).

**30.** A compound, or a stereoisomer, optical isomer, pharmaceutical salt, prodrug, solvate or isotope derivative thereof, wherein the compound is one of the following compounds:

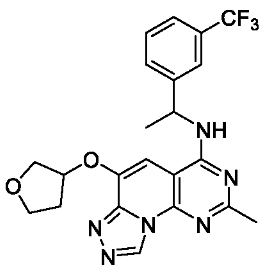
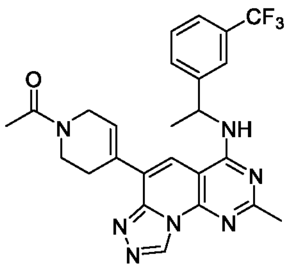
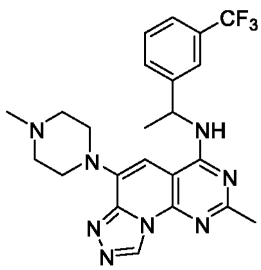
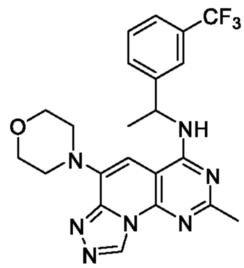

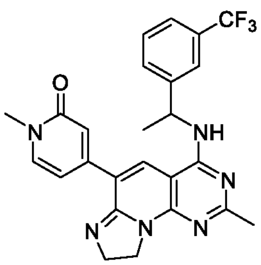
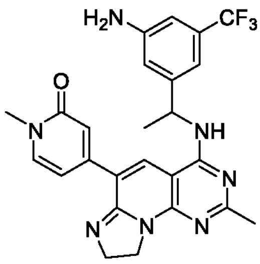
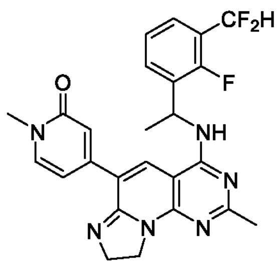
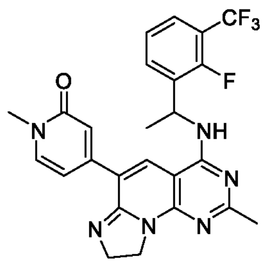

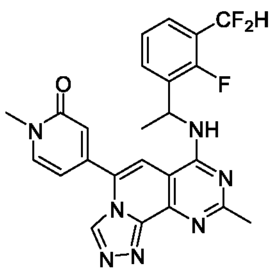
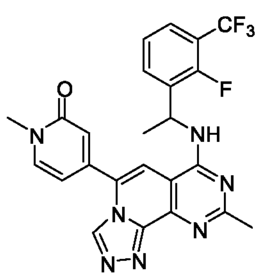
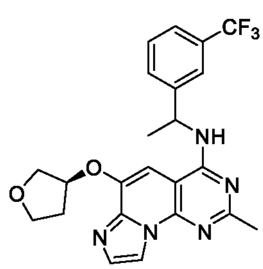
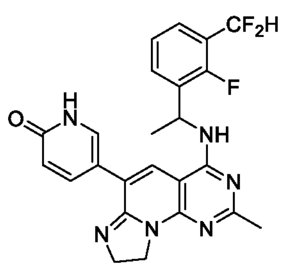

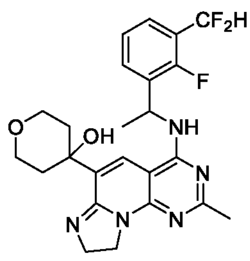 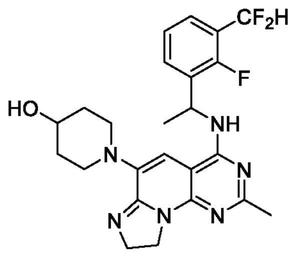 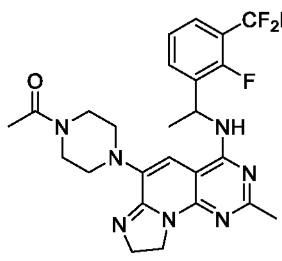 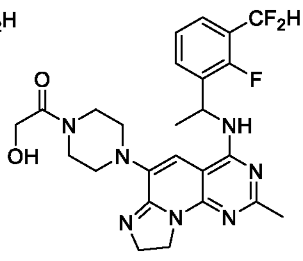

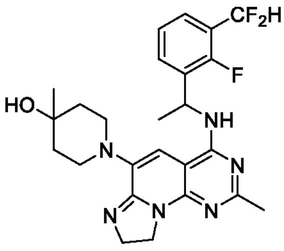 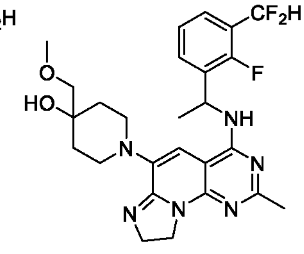 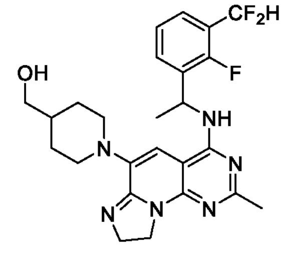 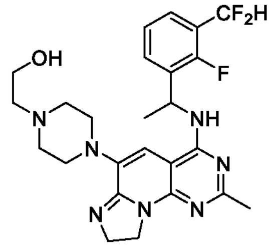

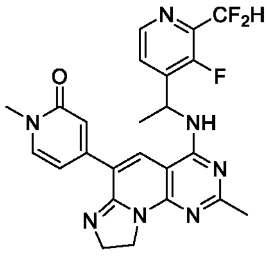 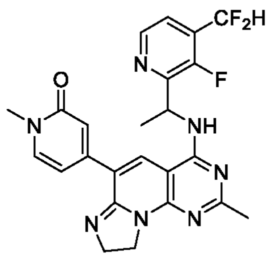 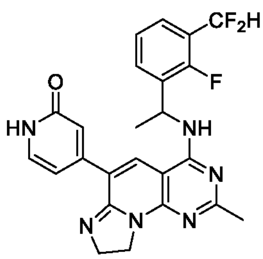 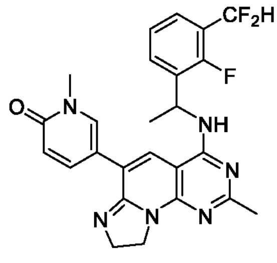

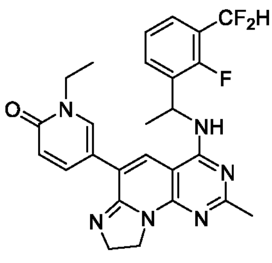 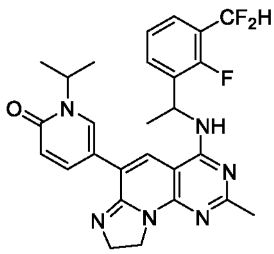 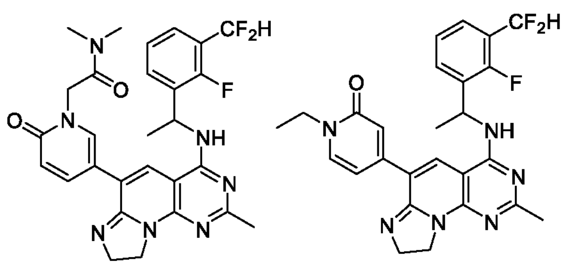

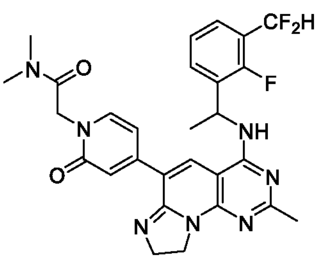 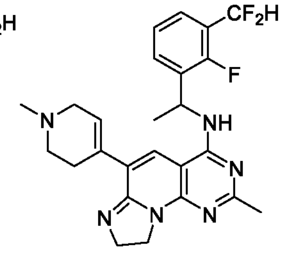 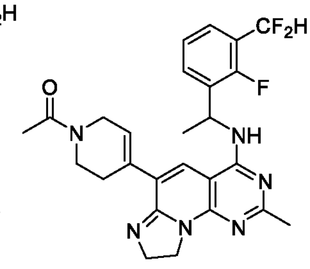 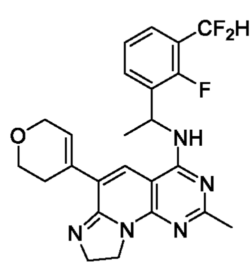

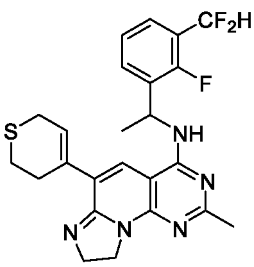
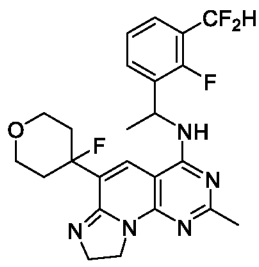
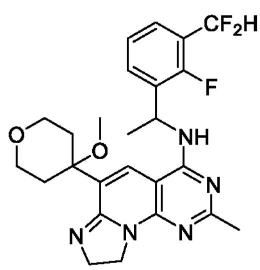
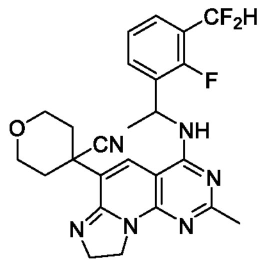

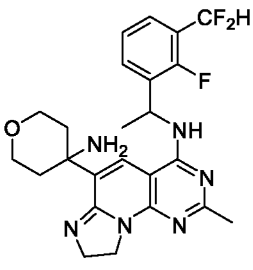
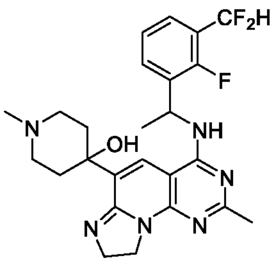
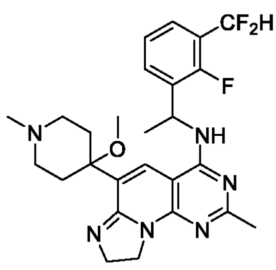
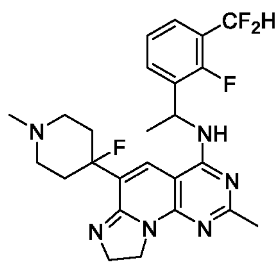

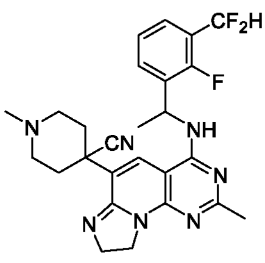
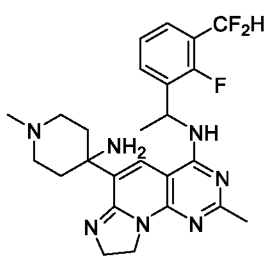
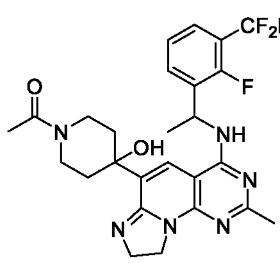
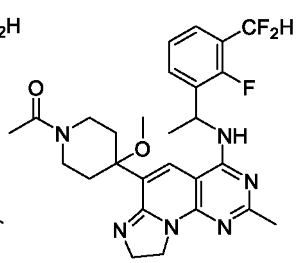

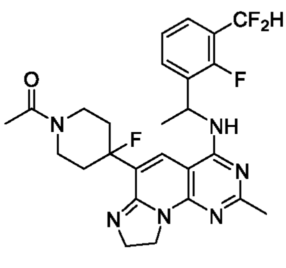
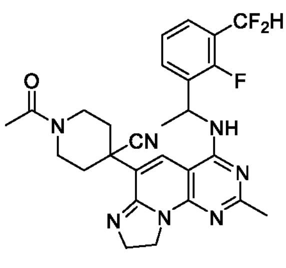
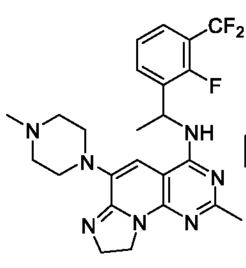
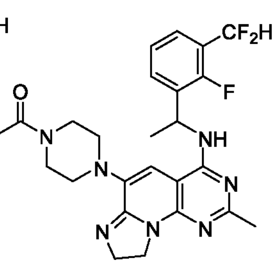

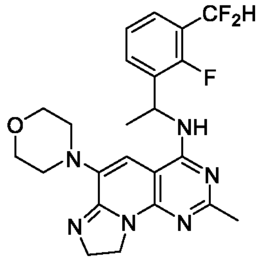
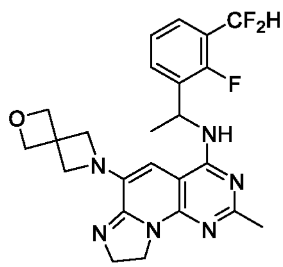
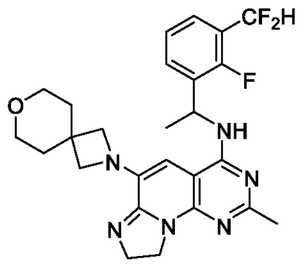
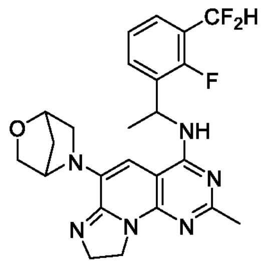

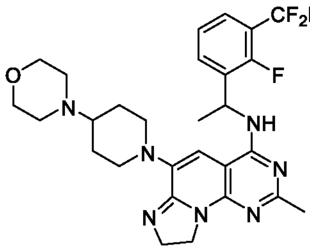 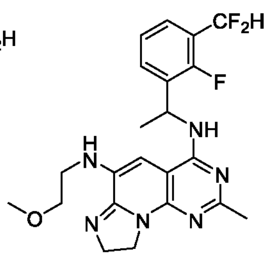 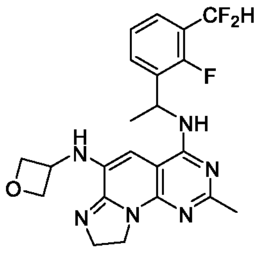 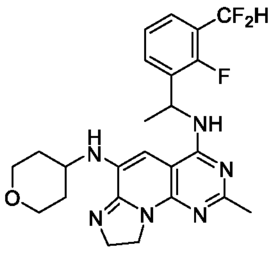

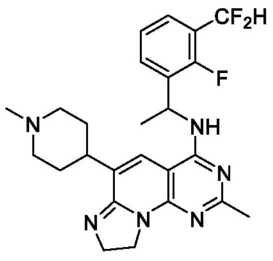 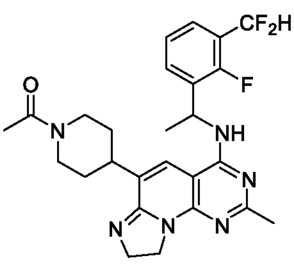 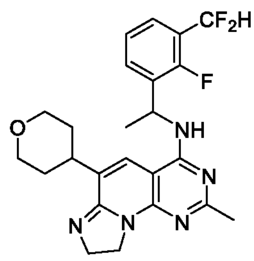 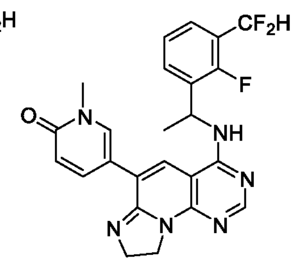

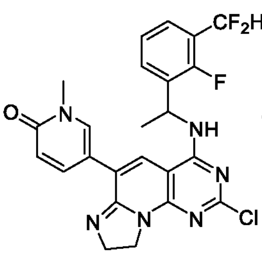 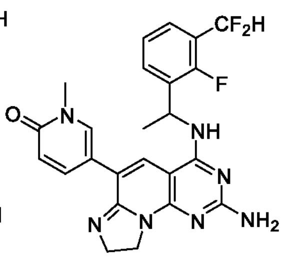 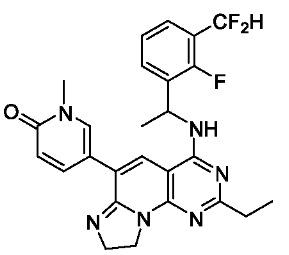 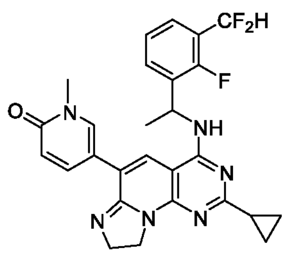

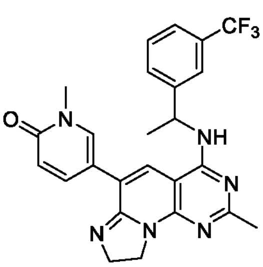 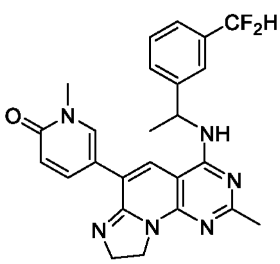 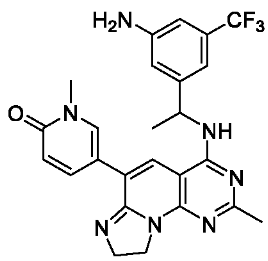 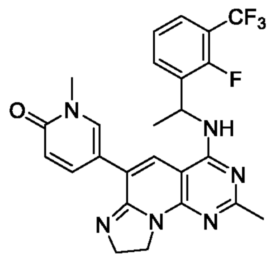

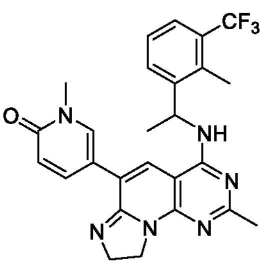 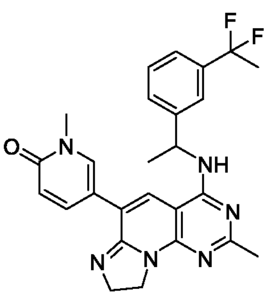 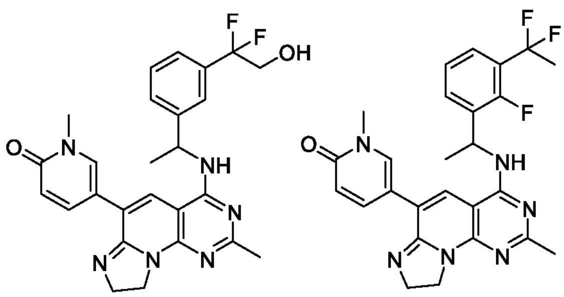

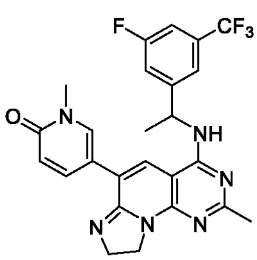
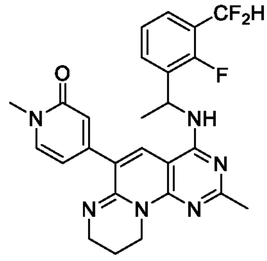
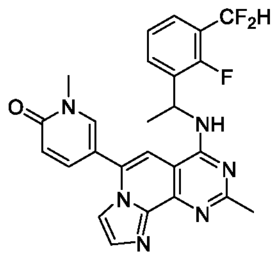
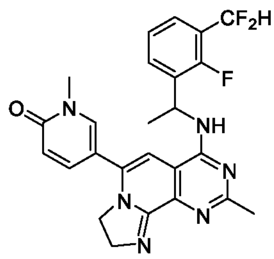
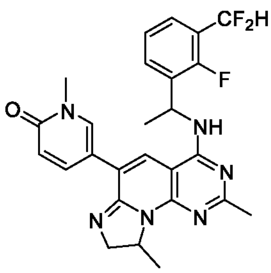
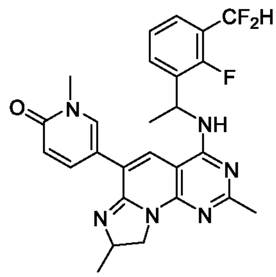
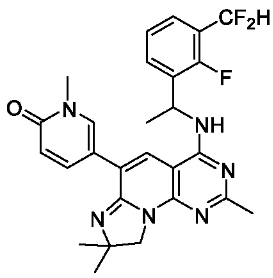
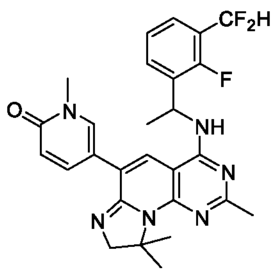
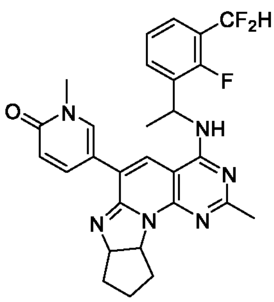
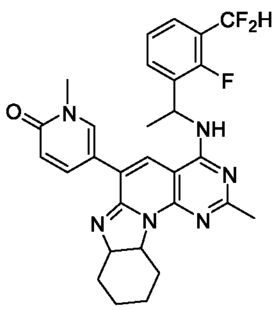
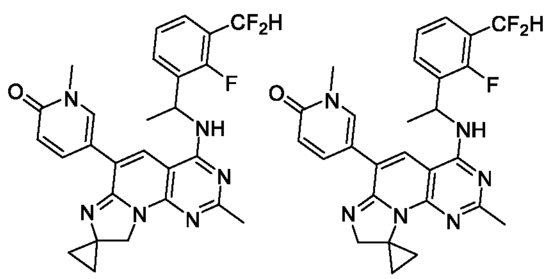
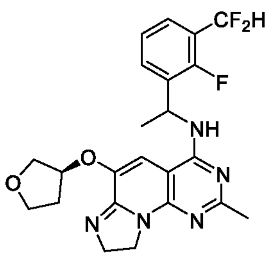
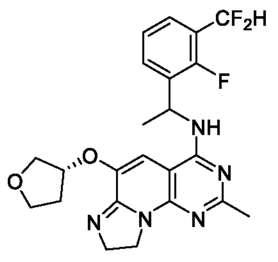
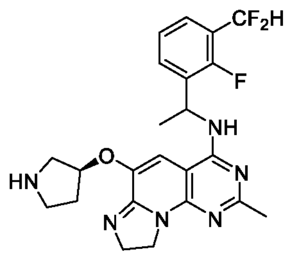
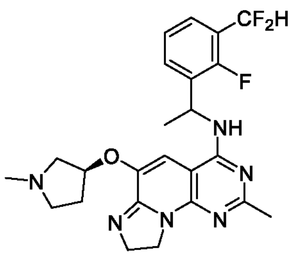
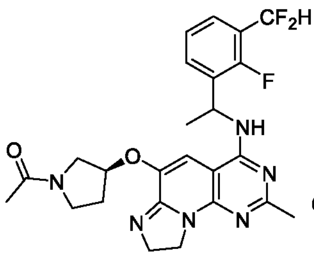
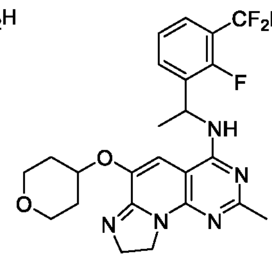
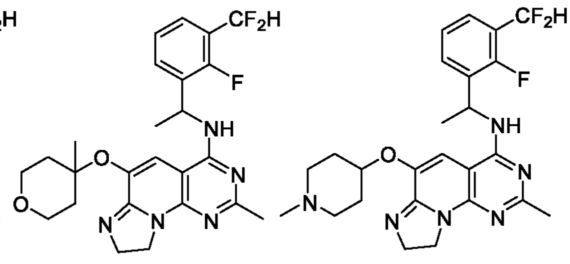

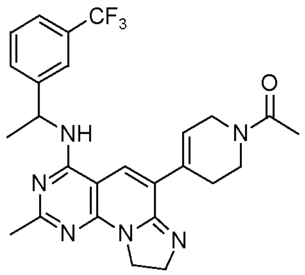
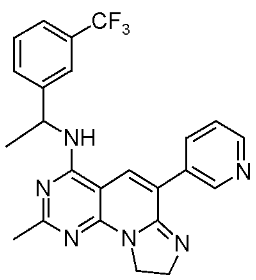
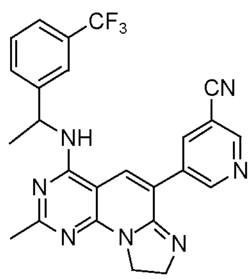
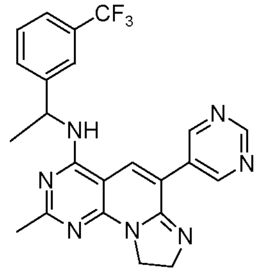
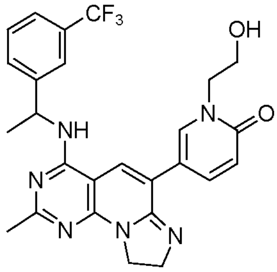
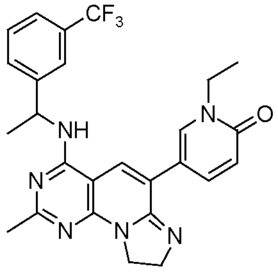
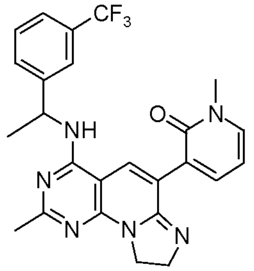
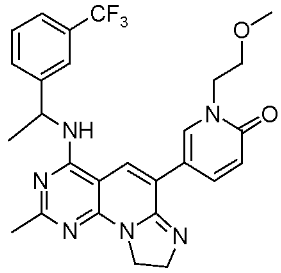
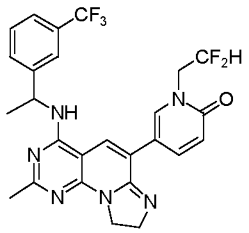
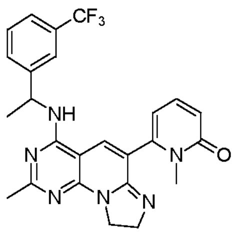
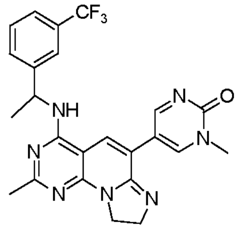
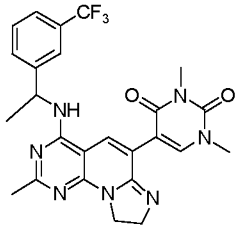
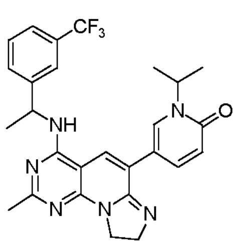
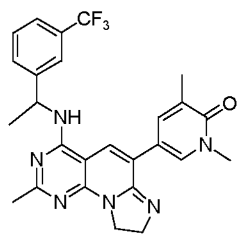
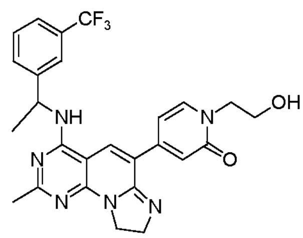
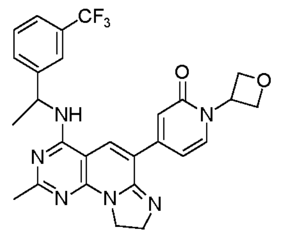
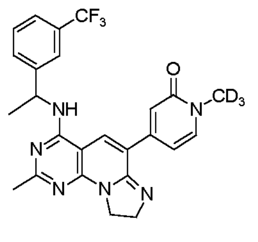
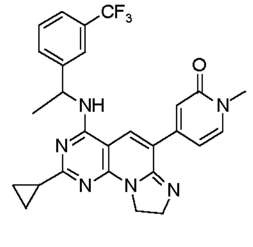
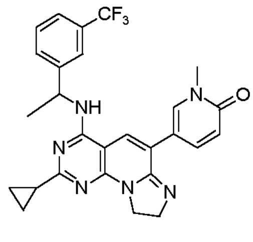
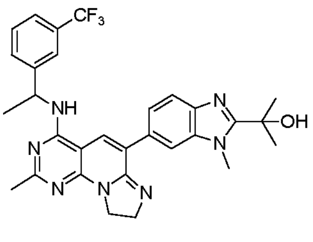
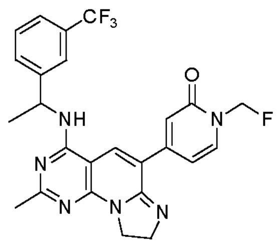
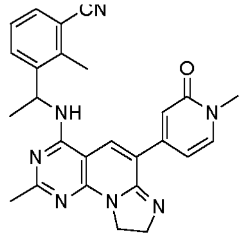
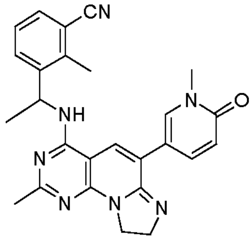
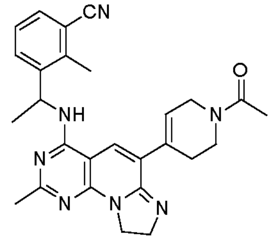

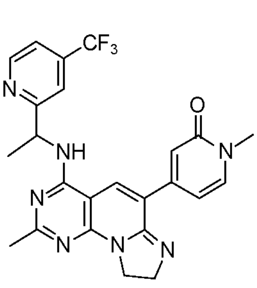
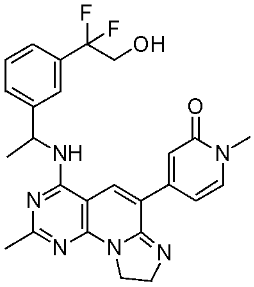
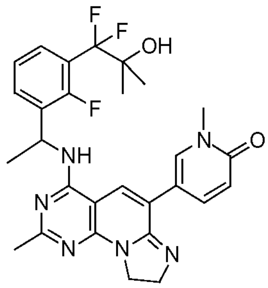
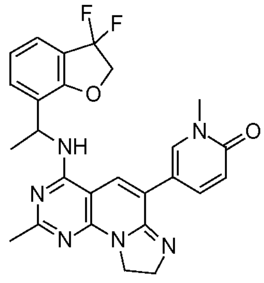
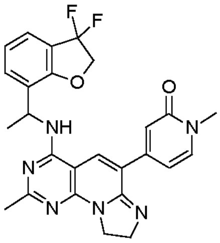
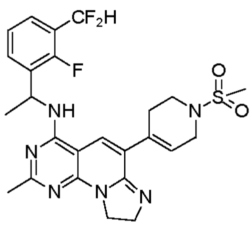
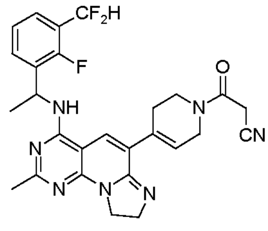
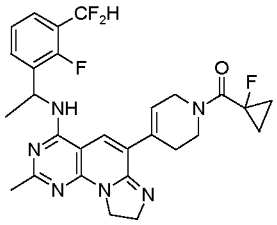
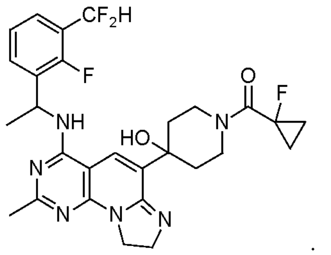

31. A compound, or a stereoisomer, optical isomer, pharmaceutical salt, prodrug, solvate or isotope derivative thereof, wherein the compound is one of the following compounds:

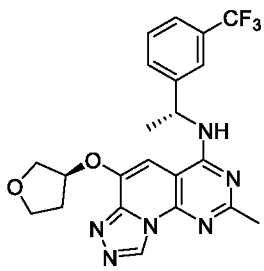
1

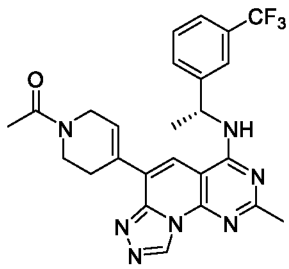
2

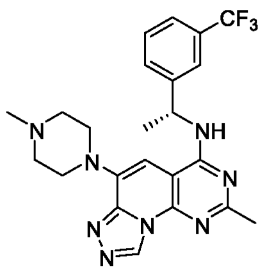
3

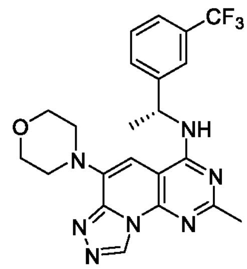
4

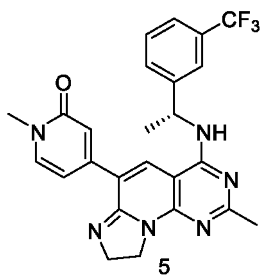
5

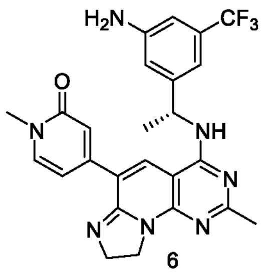
6

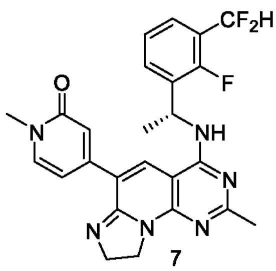
7

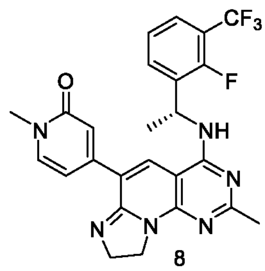
8

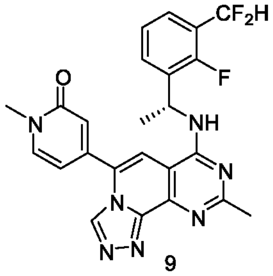
9

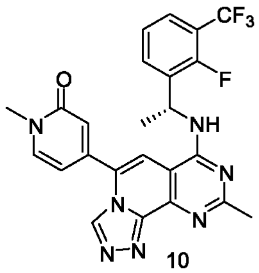
10

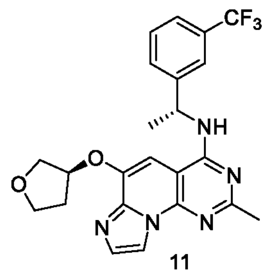
11

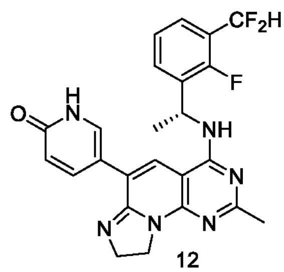
12

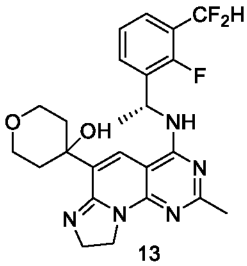
13

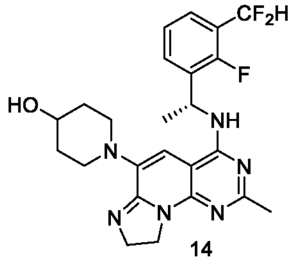
14

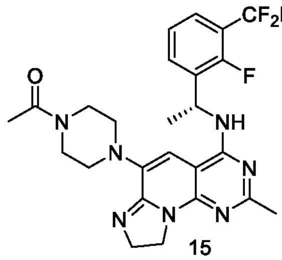
15

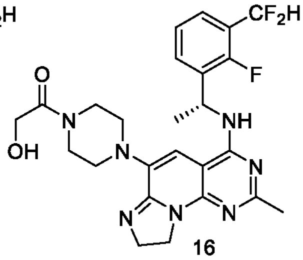
16

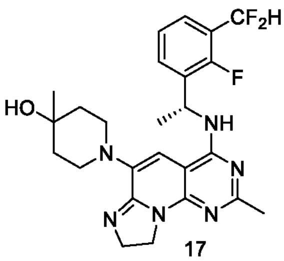
17

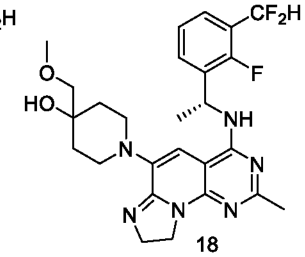
18

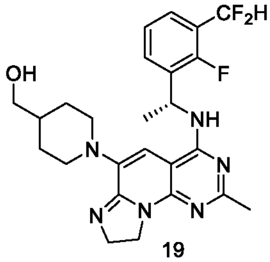
19

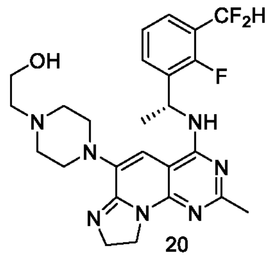
20

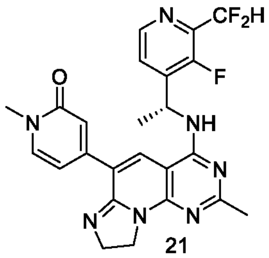
21

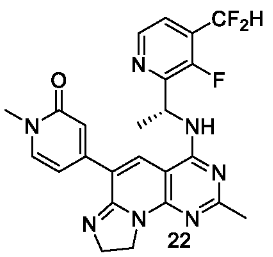
22

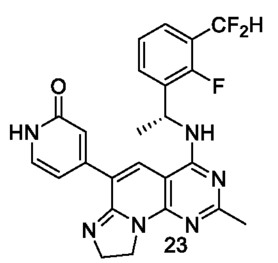
23

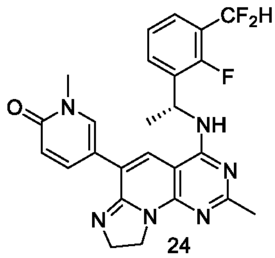
24

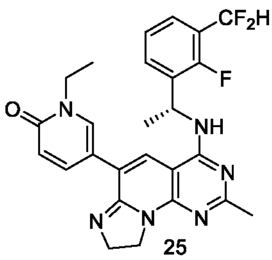
25

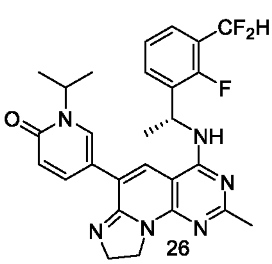
26

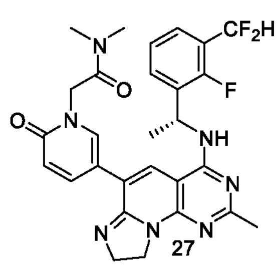
27

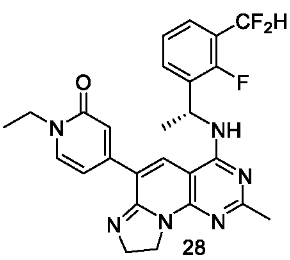
28

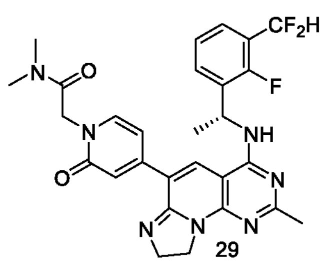
29

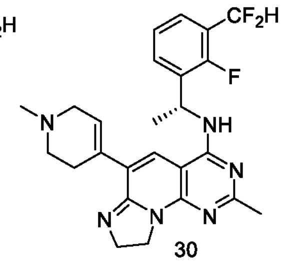
30

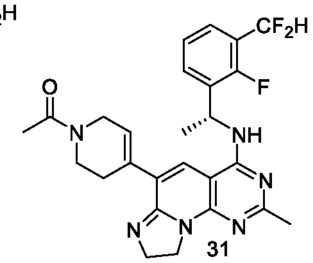
31

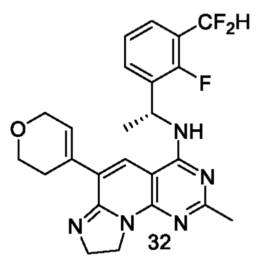
32

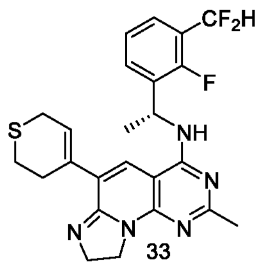
33

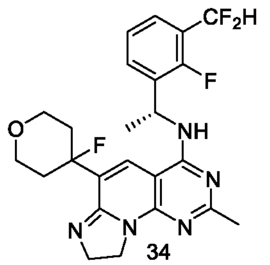
34

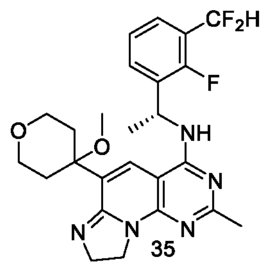
35

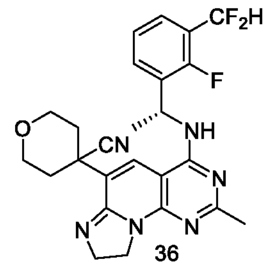
36

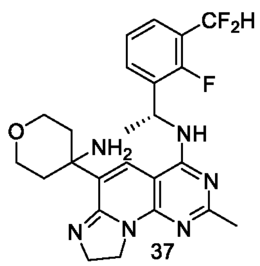
37

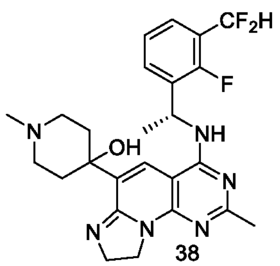
38

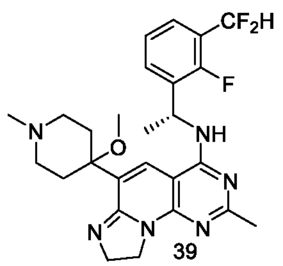
39

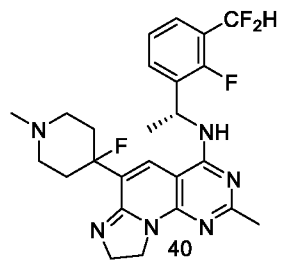
40

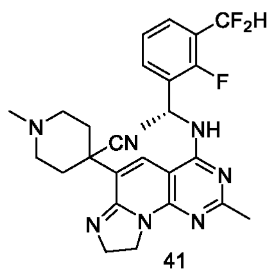
41

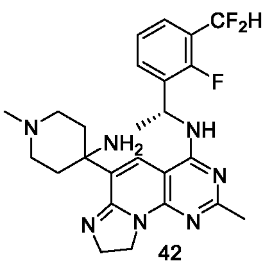
42

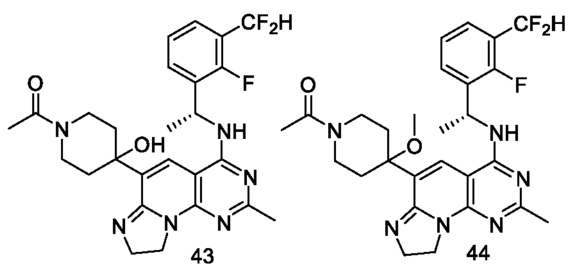
43

44

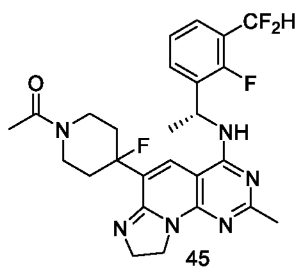
45

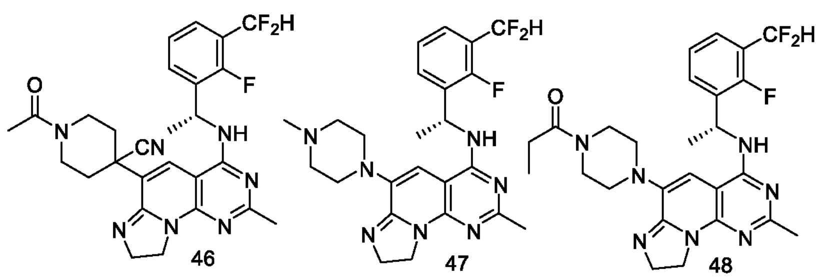
46

47

48

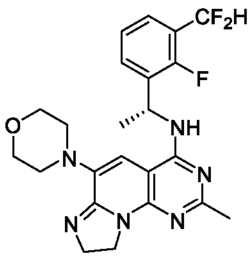

49

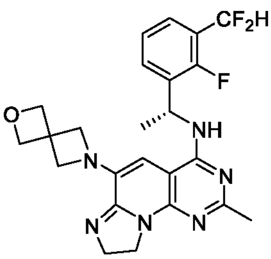

50

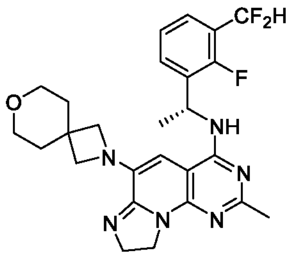

51

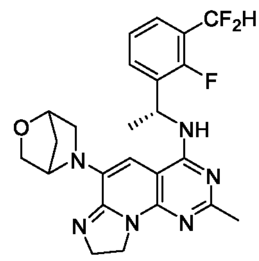

52

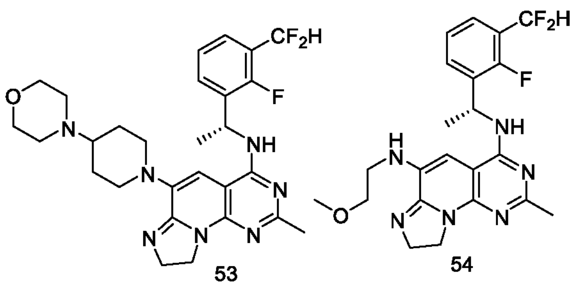

53

54

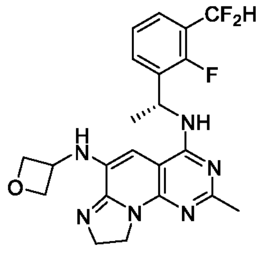

55

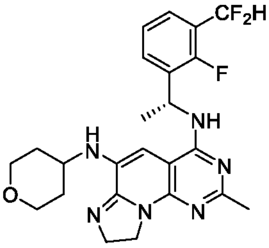

56

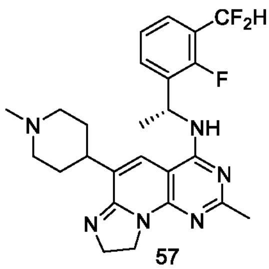

57

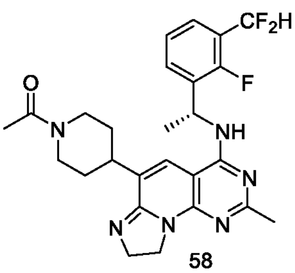

58

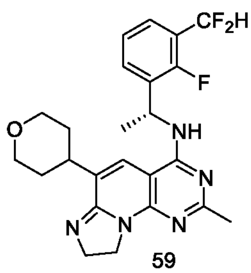

59

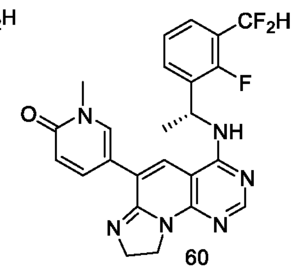

60

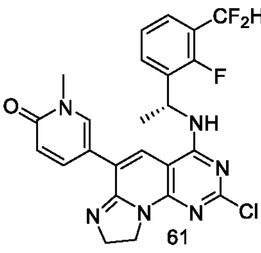

61

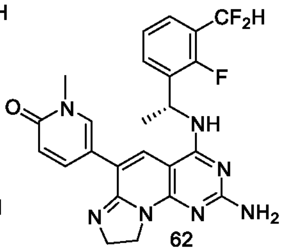

62

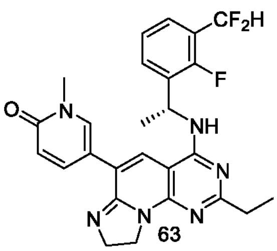

63

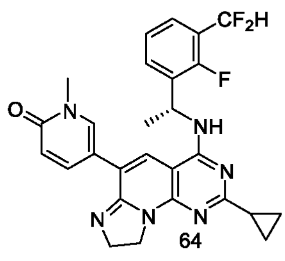

64

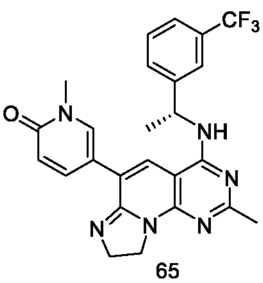

65

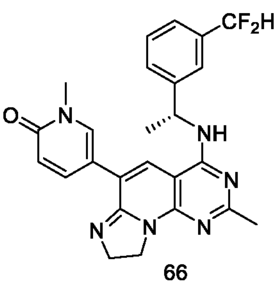

66

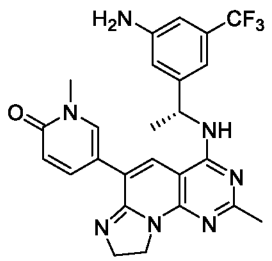

67

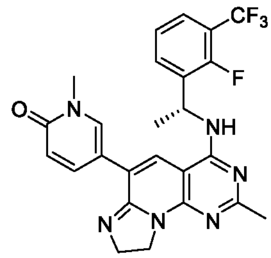

68

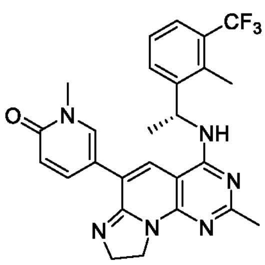
69
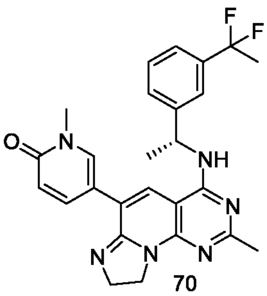
70
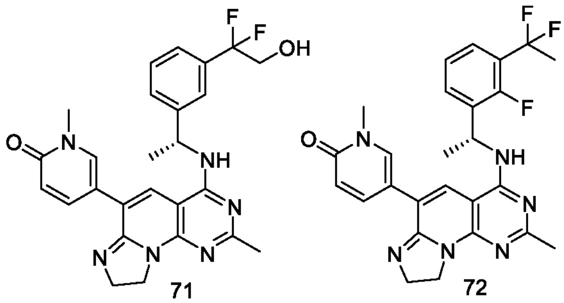
71 72

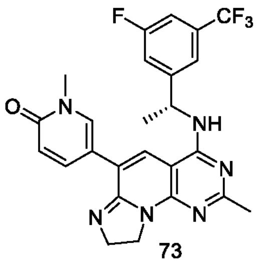
73
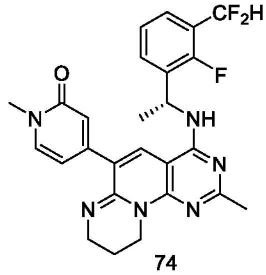
74
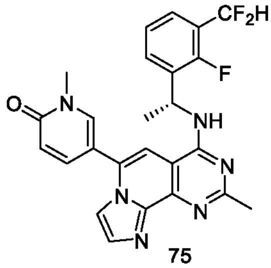
75
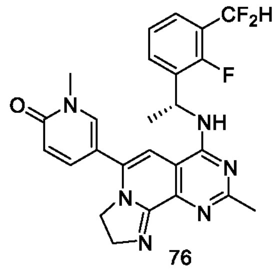
76

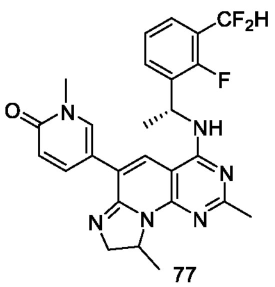
77
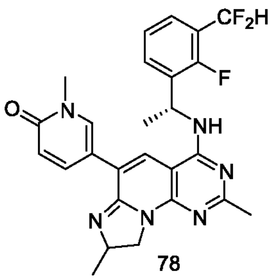
78
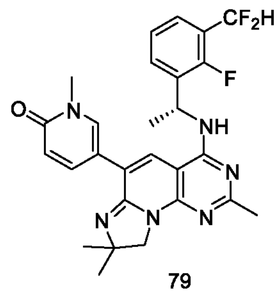
79
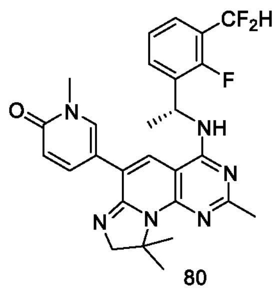
80

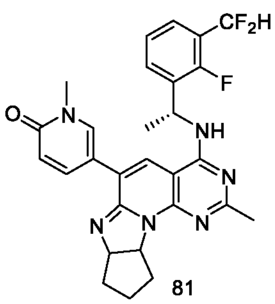
81
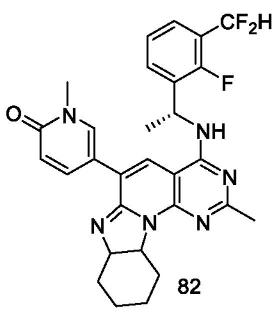
82
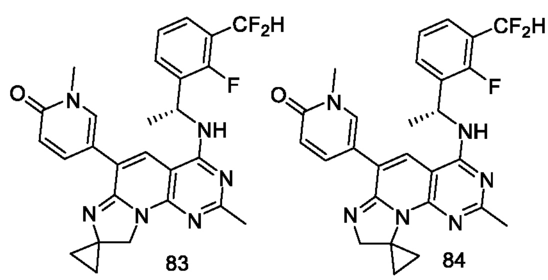
83 84

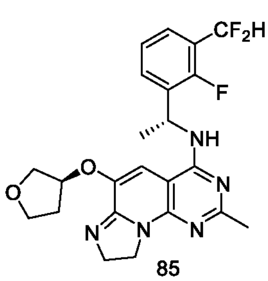
85
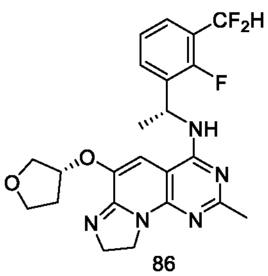
86
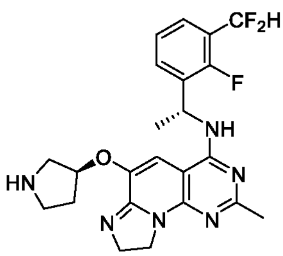
87
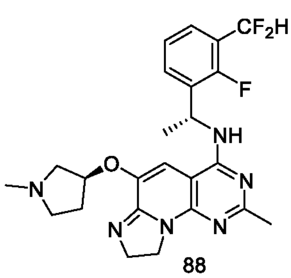
88

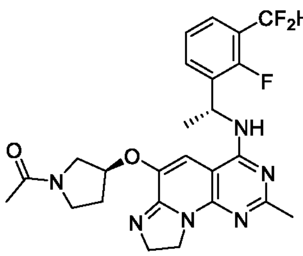
89
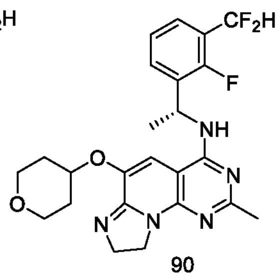
90
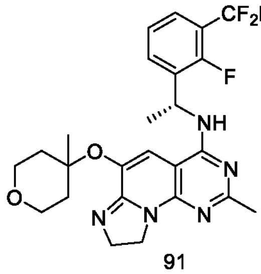
91
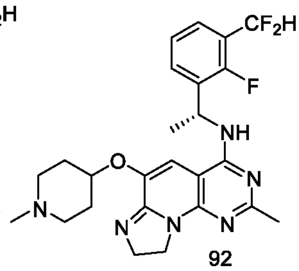
92

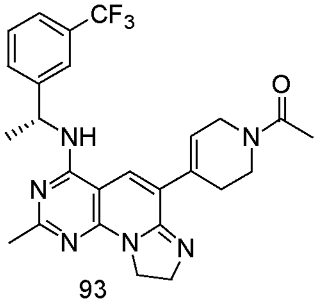
93
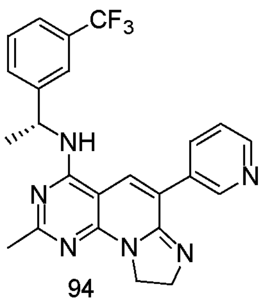
94
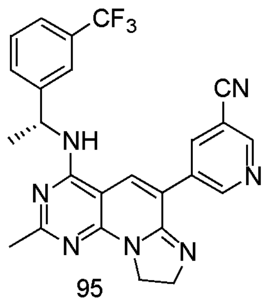
95
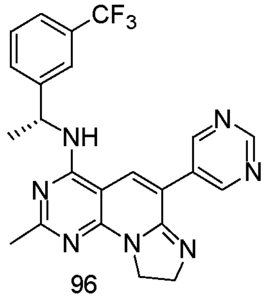
96

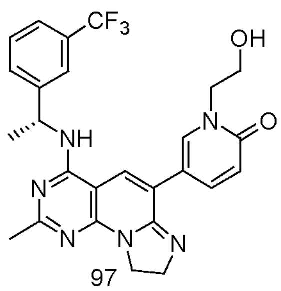
97
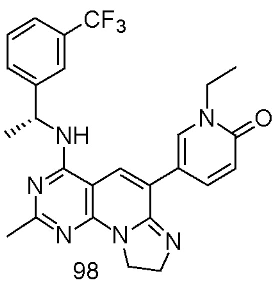
98
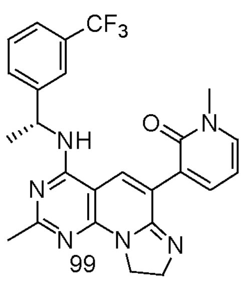
99
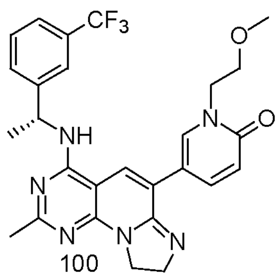
100

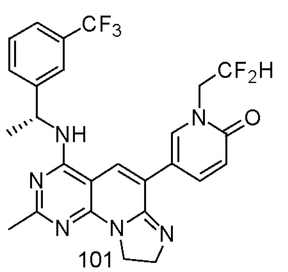
101
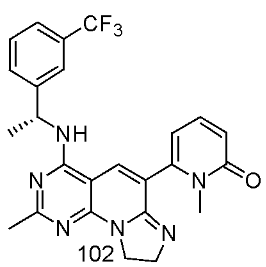
102
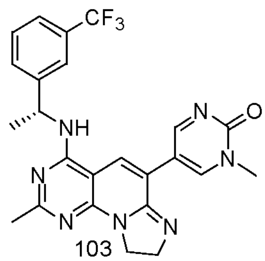
103
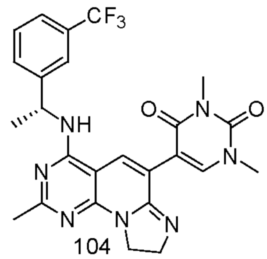
104

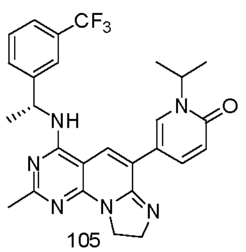
105
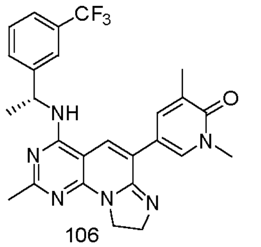
106
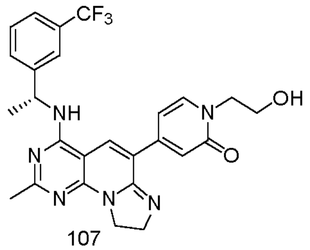
107
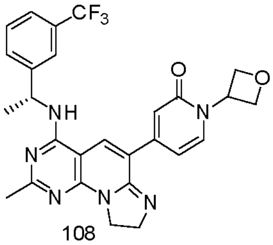
108

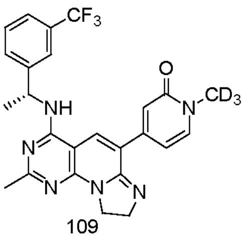
109

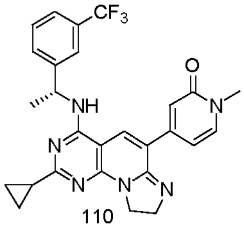
110

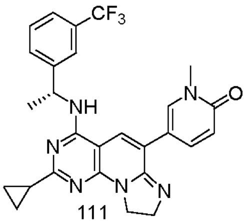
111

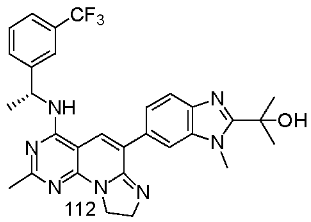
112

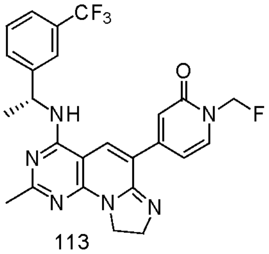
113

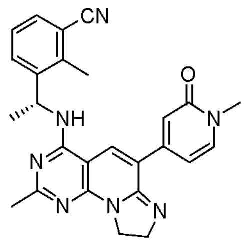
114

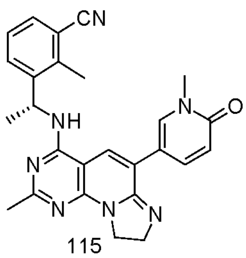
115

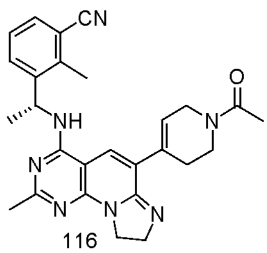
116

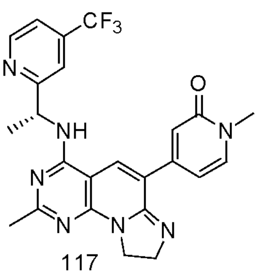
117

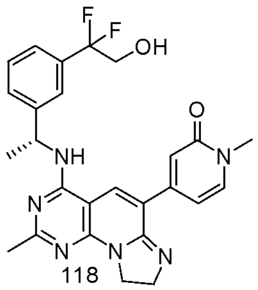
118

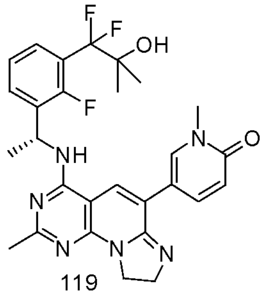
119

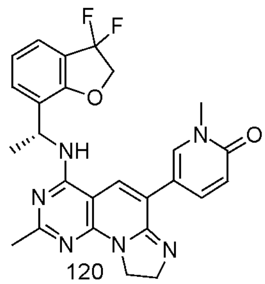
120

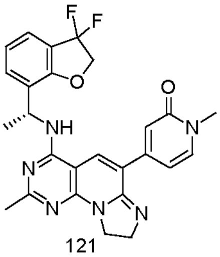
121

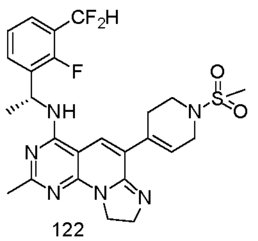
122

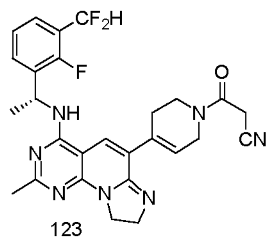
123

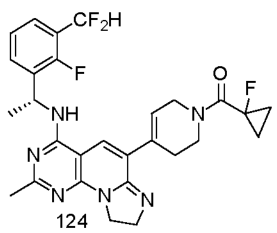
124

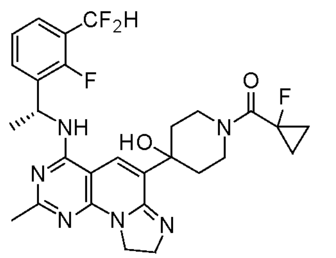
125

**32.** A pharmaceutical composition, comprising the compound or the stereoisomer, optical isomer, pharmaceutical salt, prodrug, solvate or isotope derivative thereof according to any one of claims 1 to 31;
preferably, further comprising a pharmaceutically acceptable excipient.

**33.** Use of the compound or the stereoisomer, optical isomer, pharmaceutical salt, prodrug, solvate or isotope derivative thereof according to any one of claims 1 to 31, or the pharmaceutical composition according to claim 32 in the manufacture of a medicament for preventing and/or treating a disease mediated by SOS1;

preferably, the disease mediated by SOS1 being cancer or a tumor;
further preferably, the disease mediated by SOS1 being lung cancer, preferably non-small cell lung cancer.

**34.** An intermediate compound represented by formula (V), or a stereoisomer, optical isomer, pharmaceutical salt, prodrug, solvate or isotope derivative thereof,

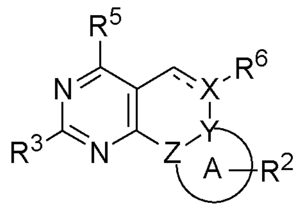
(V)

wherein $R^2$, $R^3$, ring A, X, Y, and Z are as defined in formula (A), (I) or (II);
$R^5$ is halogen, hydroxyl, -O-methylsulfonyl, -O-p-toluenesulfonyl or -O-trifluoromethylsulfonyl, preferably chlorine or hydroxyl;
$R^6$ is halogen, preferably bromine or iodine.

**35.** An intermediate compound represented by formula (VI), or a stereoisomer, optical isomer, pharmaceutical salt, prodrug, solvate or isotope derivative thereof,

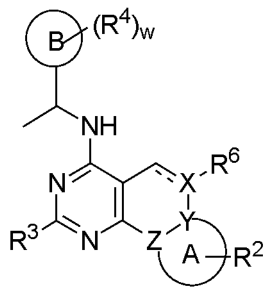
(VI)

wherein $R^2$, $R^3$, $R^4$, w, ring A, X, Y, and Z are as defined in formula (A) or (I);
$R^6$ is halogen, preferably bromine or iodine.

**36.** An intermediate compound represented by formula (VII), or a stereoisomer, optical isomer, pharmaceutical salt, prodrug, solvate or isotope derivative thereof,

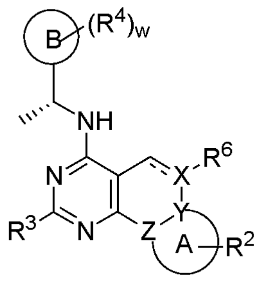
(VII)

wherein $R^2$, $R^3$, $R^4$, w, ring A, X, Y, and Z are as defined in formula (II);
$R^6$ is halogen, preferably bromine or iodine.

**37.** An intermediate compound represented by formula (VIII), or a stereoisomer, optical isomer, pharmaceutical salt, prodrug, solvate or isotope derivative thereof,

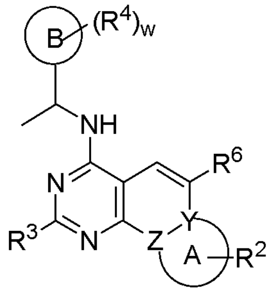

(VIII)

wherein $R^2$, $R^3$, $R^4$, w, ring A, Y, and Z are as defined in formula (III);
$R^6$ is halogen, preferably bromine or iodine.

**38.** An intermediate compound represented by formula (IX), or a stereoisomer, optical isomer, pharmaceutical salt, prodrug, solvate or isotope derivative thereof,

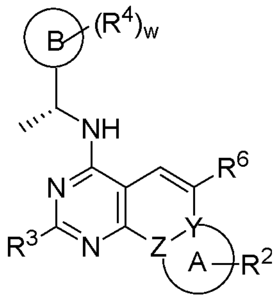

(IX)

wherein $R^2$, $R^3$, $R^4$, w, ring A, Y, and Z are as defined in formula (IV);
$R^6$ is halogen, preferably bromine or iodine.

**INTERNATIONAL SEARCH REPORT**

International application No.
**PCT/CN2022/139447**

**A. CLASSIFICATION OF SUBJECT MATTER**

C07D471/14(2006.01)i;C07D519/00(2006.01)i;A61K31/519(2006.01)i;A61P35/00(2006.01)i

According to International Patent Classification (IPC) or to both national classification and IPC

**B. FIELDS SEARCHED**

Minimum documentation searched (classification system followed by classification symbols)

C07D，A61K，A61P

Documentation searched other than minimum documentation to the extent that such documents are included in the fields searched

Electronic data base consulted during the international search (name of data base and, where practicable, search terms used)

CNPAT, CNKI, DWPI, ISI Web of Knowledge, STN-Registry, STN-Caplus: 石药集团, SOS1, 抑制, 肿瘤, 癌症, 嘧啶, 并环, inhibitor+, cancer, pyrimido+, heterocyclic, 结构检索 structural search.

**C. DOCUMENTS CONSIDERED TO BE RELEVANT**

| Category* | Citation of document, with indication, where appropriate, of the relevant passages | Relevant to claim No. |
|---|---|---|
| PX | WO 2022161480 A1 (SUZHOU ZELGEN BIOPHARMACEUTICALS CO., LTD. et al.) 04 August 2022 (2022-08-04)<br>abstract, claims 1-13, and description, page 18 | 1-38 |
| PX | CN 114835719 A (SUZHOU ZELGEN BIOPHARMACEUTICALS CO., LTD.et al.) 02 August 2022 (2022-08-02)<br>abstract, claims 1-10, and description, page 17 | 1-38 |
| PX | CN 114685487 A (SHANGHAI RINGENE BIOPHARMA CO., LTD.) 01 July 2022 (2022-07-01)<br>abstract, claims 1-10, and description, pages 50-55 | 1-38 |
| A | WO 2021105960 A1 (LUPIN LTD.) 03 June 2021 (2021-06-03)<br>abstract, claims 1-33, and description, pages 125-128 | 1-38 |
| A | WO 2021130731 A1 (LUPIN LTD.) 01 July 2021 (2021-07-01)<br>abstract, claims 1-32, and description, pages 47-57, and 200-201 | 1-38 |
| A | CN 111372932 A (BOEHRINGER INGELHEIM INTERNATIONAL GMBH) 03 July 2020 (2020-07-03)<br>abstract, claims 1-27, and description, page 122 | 1-38 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 March 2023** | **08 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2022/139447**

| Patent document cited in search report | | | Publication date (day/month/year) | Patent family member(s) | | | Publication date (day/month/year) |
|---|---|---|---|---|---|---|---|
| WO | 2022161480 | A1 | 04 August 2022 | None | | | |
| CN | 114835719 | A | 02 August 2022 | None | | | |
| CN | 114685487 | A | 01 July 2022 | WO | 2022135590 | A1 | 30 June 2022 |
| WO | 2021105960 | A1 | 03 June 2021 | PE | 20221336 | A1 | 13 September 2022 |
| | | | | BR | 112022010383 | A2 | 23 August 2022 |
| | | | | JP | 2023504113 | A | 01 February 2023 |
| | | | | CR | 20220312 | A | 05 August 2022 |
| | | | | KR | 20220110241 | A | 05 August 2022 |
| | | | | CA | 3154914 | A1 | 03 June 2021 |
| | | | | EP | 4065575 | A1 | 05 October 2022 |
| | | | | AU | 2020393205 | A1 | 30 June 2022 |
| | | | | ZA | 202206253 | B | 25 January 2023 |
| | | | | ECSP | 22050936 | A | 29 July 2022 |
| | | | | CO | 2022008997 | A2 | 19 July 2022 |
| | | | | IL | 292721 | A | 01 July 2022 |
| | | | | US | 2023013778 | A1 | 19 January 2023 |
| WO | 2021130731 | A1 | 01 July 2021 | AU | 2020412429 | A1 | 18 August 2022 |
| | | | | ECSP | 22058816 | A | 31 August 2022 |
| | | | | EP | 4081521 | A1 | 02 November 2022 |
| | | | | CA | 3165864 | A1 | 01 July 2021 |
| | | | | BR | 112022012641 | A2 | 06 September 2022 |
| | | | | IL | 294198 | A | 01 August 2022 |
| | | | | CO | 2022010460 | A2 | 09 August 2022 |
| | | | | CR | 20220363 | A | 23 September 2022 |
| | | | | KR | 20220132543 | A | 30 September 2022 |
| | | | | PE | 20221283 | A1 | 05 September 2022 |
| CN | 111372932 | A | 03 July 2020 | AU | 2018390927 | A1 | 28 May 2020 |
| | | | | AU | 2018390927 | B2 | 12 January 2023 |
| | | | | IL | 275379 | A | 30 July 2020 |
| | | | | US | 2021009588 | A1 | 14 January 2021 |
| | | | | WO | 2019122129 | A1 | 27 June 2019 |
| | | | | US | 2019194192 | A1 | 27 June 2019 |
| | | | | US | 10829487 | B2 | 10 November 2020 |
| | | | | CR | 20210307 | A | 27 July 2021 |
| | | | | PE | 20210163 | A1 | 26 January 2021 |
| | | | | EA | 202091491 | A1 | 13 November 2020 |
| | | | | CO | 2020007218 | A2 | 19 June 2020 |
| | | | | TW | 201938557 | A | 01 October 2019 |
| | | | | KR | 20200111163 | A | 28 September 2020 |
| | | | | CL | 2021000907 | A1 | 29 October 2021 |
| | | | | CL | 2020001501 | A1 | 13 November 2020 |
| | | | | CA | 3085835 | A1 | 27 June 2019 |
| | | | | JP | 2021506864 | A | 22 February 2021 |
| | | | | JP | 7189956 | B2 | 14 December 2022 |
| | | | | ECSP | 20040257 | A | 31 August 2020 |
| | | | | MX | 2020006438 | A | 17 September 2020 |
| | | | | CR | 20200312 | A | 11 September 2020 |
| | | | | SG | 11202005881 | YA | 29 July 2020 |
| | | | | MA | 51290 | A | 31 March 2021 |

Form PCT/ISA/210 (patent family annex) (July 2022)

**INTERNATIONAL SEARCH REPORT**
**Information on patent family members**

International application No.
**PCT/CN2022/139447**

| Patent document cited in search report | Publication date (day/month/year) | Patent family member(s) | | | Publication date (day/month/year) |
|---|---|---|---|---|---|
| | | JOP | 20200154 | A1 | 18 June 2020 |
| | | EP | 3728254 | A1 | 28 October 2020 |
| | | EP | 3728254 | B1 | 15 February 2023 |
| | | BR | 112020010123 | A2 | 10 November 2020 |
| | | AR | 114164 | A1 | 29 July 2020 |
| | | UA | 126173 | C2 | 25 August 2022 |
| | | SA | 520412278 | B1 | 17 August 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202111561282
- CN 202210634934
- WO 2021105960 A1
- CN 110167928 A